# EUROPEAN PATENT APPLICATION

(11) **EP 4 477 266 A2**
(43) Date of publication of application: **18.12.2024**
(21) Application number: 24190849.0
(22) Date of filing: 26.05.2022
(51) Int. Cl.: A61P 25/28

(54) **METHODS AND MATERIALS FOR TREATING PROTEINOPATHIES**

(30) Priority: 27.05.2021 US 202163193927 P; 15.10.2021 US 202163256318 P
(62) Divisional of application: 22812112.5
(71) Applicant: Mayo Foundation for Medical Education and Research, Rochester, MN 55905 (US)
(72) Inventor: KHALIL, Bilal, Jacksonville, Florida, 32224 (US); ROSSOLL, Wilfried O., Jacksonville Beach, Florida, 32250-6462 (US)
(74) Representative: Fish & Richardson P.C.

(57) **Abstract**

This document provides methods and materials for treating a mammal (e.g., a human) having, or at risk of developing, a proteinopathy. For example, one or more importin polypeptides (and/or nucleic acids designed to express an importin polypeptide) can be administered to a mammal (e.g., a human) having, or at risk of developing, a proteinopathy to treat the mammal.

## Description

### CROSS-REFERENCE TO RELATED APPLICATIONS

This application claims the benefit of U.S. Patent Application Serial No. 63/193,927, filed on May 27, 2021, and U.S. Patent Application Serial No. 63/256,318, filed on October 15, 2021. The disclosures of the prior applications are considered part of (and are incorporated by reference in) the disclosure of this application.

### SEQUENCE LISTING

This document includes a Sequence Listing that has been submitted electronically as an ASCII text file named 07039-2043WO1_SL_ST25.txt. The ASCII text file, created on May 16, 2022, is 362 kilobytes in size. The material in the ASCII text file is hereby incorporated by reference in its entirety.

### TECHNICAL FIELD

This document relates to methods and materials for treating a mammal (e.g., a human) having, or at risk of developing, a proteinopathy. For example, one or more importin polypeptides (and/or nucleic acids designed to express an importin polypeptide) can be administered to a mammal (e.g., a human) having, or at risk of developing, a proteinopathy to treat the mammal.

### BACKGROUND INFORMATION

Common age-related proteinopathies such as Alzheimer's disease and the frontotemporal dementia (FTD) and amyotrophic lateral sclerosis (ALS) disease spectrum are characterized by defects in protein homeostasis and the propagation and spreading of pathological protein aggregates (Jucker et al., Nature, 501(7465):45-51 (2013)). Currently there is a lack of understanding what causes these lesions, and how they can be reversed by effective therapeutics (Ciechanover et al., Exp. Mol. Med., 47:e147 2015; Klaips et al., J. Cell. Biol., 217(1):51-63 (2018)).

### SUMMARY

This document provides methods and materials for treating a mammal (e.g., a human) having, or at risk of developing, a proteinopathy (e.g., a proteinopathy associated with aggregation of TAR DNA-binding protein 43 (TDP-43) polypeptides). Proteinopathies associated with the aggregation of TDP-43 polypeptides can also be referred to as TDP-43 proteinopathies. For example, one or more importin polypeptides and/or fragments thereof (and/or nucleic acids designed to express an importin polypeptide and/or a fragment thereof) can be administered to a mammal (e.g., a human) having, or at risk of developing, a proteinopathy (e.g., a TDP-43 proteinopathy) to treat the mammal.

As demonstrated herein, importin polypeptides and fragments of importin polypeptides can reduce neurodegeneration in TDP-43 proteinopathies. For example, importin polypeptides and fragments of importin polypeptides can prevent TDP-43 from undergoing pathological phase transition, can reverse formation of insoluble aggregates, can restore TDP-43 nuclear localization, and can restore TDP-43 function in the nucleus. In some cases, importin polypeptides and fragments of importin polypeptides can slow, delay, or prevent progression of neurodegeneration in the central nervous system (CNS; e.g., the brain) of a mammal. In some cases, importin polypeptides and fragments of importin polypeptides can slow, delay, or prevent the development of neurodegeneration in the CNS of a mammal. Having the ability to reduce neurodegeneration in TDP-43 proteinopathies as described herein (e.g., by administering one or more importin polypeptides and/or fragments thereof and/or nucleic acids designed to express an importin polypeptide and/or a fragment thereof) provides a unique and unrealized opportunity to treat mammal having, or at risk of developing, a TDP-43 proteinopathy.

In general, one aspect of this document features methods for treating a mammal having a TDP-43 proteinopathy. The methods can include, or consist essentially of, administering an importin-3 (IPO3) polypeptide or a fragment of the IPO3 polypeptide to a mammal having a TDP-43 proteinopathy. The IPO3 polypeptide or the fragment can be effective to reduce a symptom of the TDP-43 proteinopathy. The symptom can be depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, behavior variant frontotemporal dementia (bvFTD), or primary progressive aphasia (PPA). The method can include administering the IPO3 polypeptide to the mammal. The method can include administering the fragment of the IPO3 polypeptide to the mammal. The fragment of the IPO3 polypeptide can consist of the amino acid sequence set forth in any one of SEQ ID NOs:26-55. The mammal can be a human. The TDP-43 proteinopathy can be Alzheimer's disease, FTD, ALS, traumatic brain injury (TBI), chronic traumatic encephalopathy (CTE), limbic-predominant age-related TDP-43 encephalopathy (LATE), dementia with Lewy bodies (DLB), Parkinson's disease, Huntington's disease, argyrophilic grain disease (AGD), hippocampal sclerosis (HS), Guam ALS, Guam parkinsonism-dementia complex (G-PDC), Perry disease, facial onset sensory and motor neuronopathy (FOSMN), inclusion body myositis (IBM), hereditary inclusion body myopathy, oculopharyngeal muscular dystrophy (OPMD), or distal myopathies with rimmed vacuoles. The administering can include intracerebral injection. The administering can include intrathecal injection.

In another aspect, this document features methods for reducing aggregation of TDP-43 polypeptides in a CNS of a mammal having a TDP-43 proteinopathy. The methods can include, or consist essentially of, administering an IPO3 polypeptide or a fragment of the IPO3 polypeptide to a mammal having a TDP-43 proteinopathy. The method can include administering the IPO3 polypeptide to the mammal. The method can include administering the fragment of the IPO3 polypeptide to the mammal. The fragment of the IPO3 polypeptide can consist of the amino acid sequence set forth in any one of SEQ ID NOs:26-55. The mammal can be a human. The TDP-43 proteinopathy can be Alzheimer's disease, FTD, ALS, traumatic brain injury (TBI), chronic traumatic encephalopathy (CTE), limbic-predominant age-related TDP-43 encephalopathy (LATE), dementia with Lewy bodies (DLB), Parkinson's disease, Huntington's disease, argyrophilic grain disease (AGD), hippocampal sclerosis (HS), Guam ALS, Guam parkinsonism-dementia complex (G-PDC), Perry disease, facial onset sensory and motor neuronopathy (FOSMN), inclusion body myositis (IBM), hereditary inclusion body myopathy, oculopharyngeal muscular dystrophy (OPMD), or distal myopathies with rimmed vacuoles. The administering can include intracerebral injection. The administering can include intrathecal injection.

In another aspect, this document features methods for treating a mammal having a TDP-43 proteinopathy. The methods can include, or consist essentially of, administering nucleic acid encoding an IPO3 polypeptide or a fragment of the IPO3 polypeptide to a mammal having a TDP-43 proteinopathy, where the IPO3 polypeptide or the fragment is expressed by cells in a CNS of the mammal. The IPO3 polypeptide or the fragment can be effective to reduce a symptom of the TDP-43 proteinopathy. The symptom can be depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, bvFTD, or PPA. The nucleic acid can encode the IPO3 polypeptide. The nucleic acid can encode the fragment of the IPO3 polypeptide. The fragment of the IPO3 polypeptide can consist of the amino acid sequence set forth in any one of SEQ ID NOs:26-55. The said nucleic acid can be in the form of a vector. The vector can be an adeno-associated virus (AAV) vector. The vector can be an expression plasmid. The nucleic acid can be contained within a nanoparticle. The mammal can be a human. The TDP-43 proteinopathy can be Alzheimer's disease, FTD, ALS, traumatic brain injury (TBI), chronic traumatic encephalopathy (CTE), limbic-predominant age-related TDP-43 encephalopathy (LATE), dementia with Lewy bodies (DLB), Parkinson's disease, Huntington's disease, argyrophilic grain disease (AGD), hippocampal sclerosis (HS), Guam ALS, Guam parkinsonism-dementia complex (G-PDC), Perry disease, facial onset sensory and motor neuronopathy (FOSMN), inclusion body myositis (IBM), hereditary inclusion body myopathy, oculopharyngeal muscular dystrophy (OPMD), or distal myopathies with rimmed vacuoles. The administering can include intracerebral injection. The administering can include intrathecal injection.

In another aspect, this document features methods for reducing aggregation of TDP-43 polypeptides in a CNS of a mammal. The methods can include, or consist essentially of, administering nucleic acid encoding an IPO3 polypeptide or a fragment of the IPO3 polypeptide to a mammal where the IPO3 polypeptide or the fragment is expressed by cells in the CNS of the mammal. The nucleic acid can encode the IPO3 polypeptide. The nucleic acid can encode the fragment of the IPO3 polypeptide. The fragment of the IPO3 polypeptide can consist of the amino acid sequence set forth in any one of SEQ ID NOs:26-55. The said nucleic acid can be in the form of a vector. The vector can be an adeno-associated virus (AAV) vector. The vector can be an expression plasmid. The nucleic acid can be contained within a nanoparticle. The mammal can be a human. The TDP-43 proteinopathy can be Alzheimer's disease, FTD, ALS, traumatic brain injury (TBI), chronic traumatic encephalopathy (CTE), limbic-predominant age-related TDP-43 encephalopathy (LATE), dementia with Lewy bodies (DLB), Parkinson's disease, Huntington's disease, argyrophilic grain disease (AGD), hippocampal sclerosis (HS), Guam ALS, Guam parkinsonism-dementia complex (G-PDC), Perry disease, facial onset sensory and motor neuronopathy (FOSMN), inclusion body myositis (IBM), hereditary inclusion body myopathy, oculopharyngeal muscular dystrophy (OPMD), or distal myopathies with rimmed vacuoles. The administering can include intracerebral injection. The administering can include intrathecal injection.

In another aspect, this document features methods for treating a mammal having a TDP-43 proteinopathy. The methods can include, or consist essentially of, administering an importin-13 (IPO13) polypeptide or a fragment of the IPO 13 polypeptide to a mammal having a TDP-43 proteinopathy. The IPO13 polypeptide or the fragment can be effective to reduce a symptom of the TDP-43 proteinopathy. The symptom can be depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, bvFTD, or PPA. The method can include administering the IPO13 polypeptide to the mammal. The method can include administering the fragment of the IPO13 polypeptide to the mammal. The fragment of the IPO13 polypeptide can consist of the amino acid sequence set forth in any one of SEQ ID NOs:56-77. The mammal can be a human. The TDP-43 proteinopathy can be Alzheimer's disease, FTD, ALS, TBI, CTE, LATE, DLB, Parkinson's disease, Huntington's disease, AGD, HS, Guam ALS, G-PDC, Perry disease, FOSMN, IBM, hereditary inclusion body myopathy, OPMD, or distal myopathies with rimmed vacuoles. The administering can include intracerebral injection. The administering can include intrathecal injection.

In another aspect, this document features methods for reducing aggregation of TDP-43 polypeptides in a CNS of a mammal having a TDP-43 proteinopathy. The methods can include, or consist essentially of, administering an IPO13 polypeptide or a fragment of the IPO13 polypeptide to a mammal having a TDP-43 proteinopathy. The method can include administering the IPO13 polypeptide to the mammal. The method can include administering the fragment of the IPO 13 polypeptide to the mammal. The fragment of the IPO 13 polypeptide can consist of the amino acid sequence set forth in any one of SEQ ID NOs:56-77. The mammal can be a human. The TDP-43 proteinopathy can be Alzheimer's disease, FTD, ALS, TBI, CTE, LATE, DLB, Parkinson's disease, Huntington's disease, AGD, HS, Guam ALS, G-PDC, Perry disease, FOSMN, IBM, hereditary inclusion body myopathy, OPMD, or distal myopathies with rimmed vacuoles. The administering can include intracerebral injection. The administering can include intrathecal injection.

In another aspect, this document features methods for treating a mammal having a TDP-43 proteinopathy. The methods can include, or consist essentially of, administering nucleic acid encoding an IPO 13 polypeptide or a fragment of the IPO 13 polypeptide to a mammal having a TDP-43 proteinopathy, where the IPO13 polypeptide or the fragment is expressed by cells in the CNS of the mammal. The IPO 13 polypeptide or the fragment can be effective to reduce a symptom of the TDP-43 proteinopathy. The symptom can be depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, bvFTD, or PPA. The nucleic acid can encode the IPO 13 polypeptide. The nucleic acid can encode the fragment of the IPO 13 polypeptide. The fragment of the IPO 13 polypeptide can consist of the amino acid sequence set forth in any one of SEQ ID NOs:56-77. The nucleic acid can be in the form of a vector. The vector can be an AAV vector. The vector can be an expression plasmid. The nucleic acid can be contained within a nanoparticle. The mammal can be a human. The TDP-43 proteinopathy can be Alzheimer's disease, FTD, ALS, TBI, CTE, LATE, DLB, Parkinson's disease, Huntington's disease, AGD, HS, Guam ALS, G-PDC, Perry disease, FOSMN, IBM, hereditary inclusion body myopathy, OPMD, or distal myopathies with rimmed vacuoles. The administering can include intracerebral injection. The administering can include intrathecal injection.

In another aspect, this document features methods for reducing aggregation of TDP-43 polypeptides in a CNS of a mammal. The methods can include, or consist essentially of, administering nucleic acid encoding an IPO13 polypeptide or a fragment of the IPO13 polypeptide to a mammal having a TDP-43 proteinopathy, where the IPO13 polypeptide or the fragment is expressed by cells in the CNS of the mammal. The nucleic acid can encode the IPO 13 polypeptide. The nucleic acid can encode the fragment of the IPO 13 polypeptide. The fragment of the IPO 13 polypeptide can consist of the amino acid sequence set forth in any one of SEQ ID NOs:56-77. The nucleic acid can be in the form of a vector. The vector can be an AAV vector. The vector can be an expression plasmid. The nucleic acid can be contained within a nanoparticle. The mammal can be a human. The TDP-43 proteinopathy can be Alzheimer's disease, FTD, ALS, TBI, CTE, LATE, DLB, Parkinson's disease, Huntington's disease, AGD, HS, Guam ALS, G-PDC, Perry disease, FOSMN, IBM, hereditary inclusion body myopathy, OPMD, or distal myopathies with rimmed vacuoles. The administering can include intracerebral injection. The administering can include intrathecal injection.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

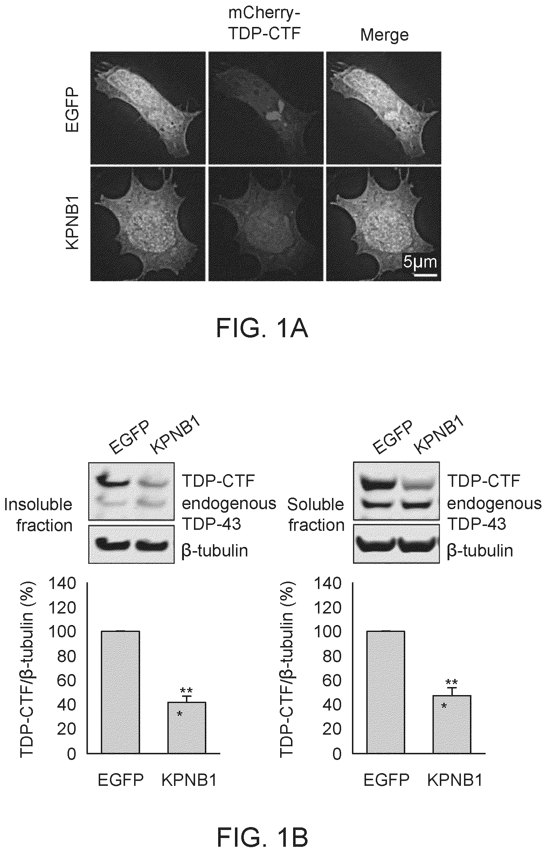

Figures 1A-1B: KPNB1 expression reduces aggregation and toxicity of a C-terminal fragment of TDP. Figure 1A) Representative images of SH-SY5Y human neuroblastoma cells expressing a mCherry-tagged C-terminal fragment of human TDP-43 from amino acid 207-414 (TDP-CTF) with either EGFP or EGFP-KPNB 1. KPNB1 expression reduces pathological TDP-CTF aggregates. Figure 1B) Western blot analyses of the soluble and insoluble fractions of SH-SY5Y cells co-expressing TDP-CTF with EGFP or EGFP-KPNB 1. KPNB1 significantly reduced both the RIPA-buffer soluble and insoluble levels of TDP-CTF, without affecting endogenous TDP-43 protein levels. N=6; ***p<0.005.

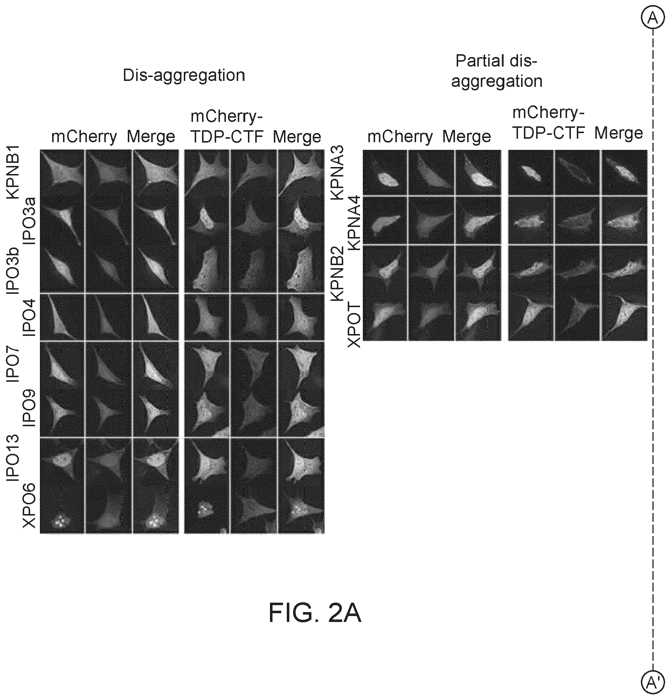

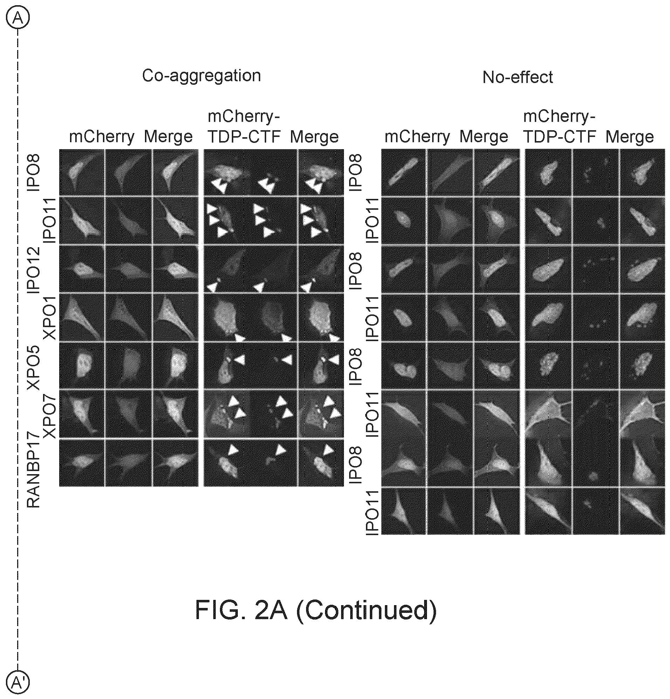

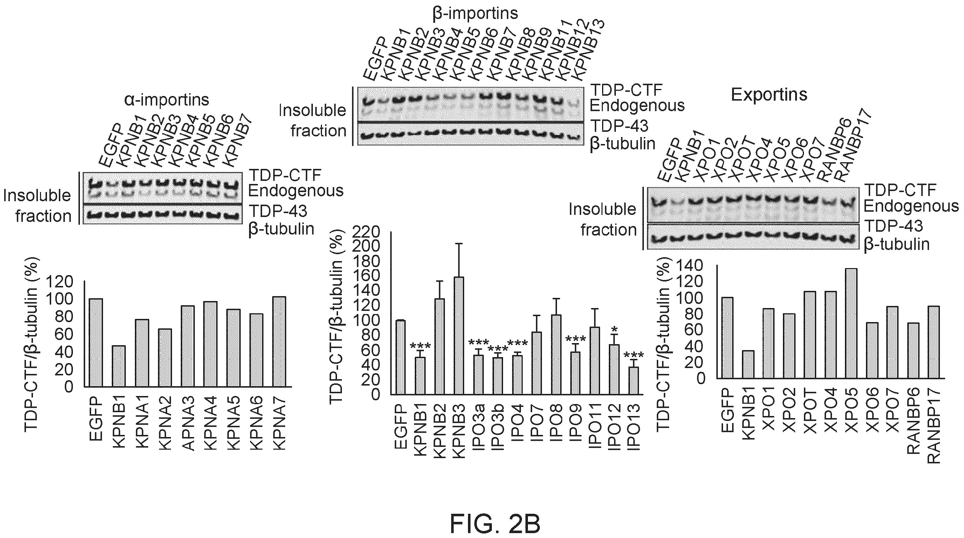

Figures 2A-2B: KPNB1 and other β-importin nuclear import receptors (NIRs) reduce TDP-CTF aggregation. Figure 2A) SH-SY5Y human neuroblastoma cells were co-transfected with mCherry or mCherry-TDP-CTF together with GFP-tagged karyopherin α and karyopherin β nuclear transport receptor proteins. Expression of KPNB 1 and other specific NIRs reduces the formation of TDP-CTF aggregates. Scale bar: 5 µm. Figure 2B) Western blot analysis and quantification of the insoluble levels of mCherry-TDP-CTF co-transfected with expression plasmids for different karyopherin α and karyopherin β transport receptors in SH-SY5Y cells shows that KPNB 1 and other β-importins significantly reduce TDP-CTF aggregation. N=4; *p<0.5, ***p<0.005.

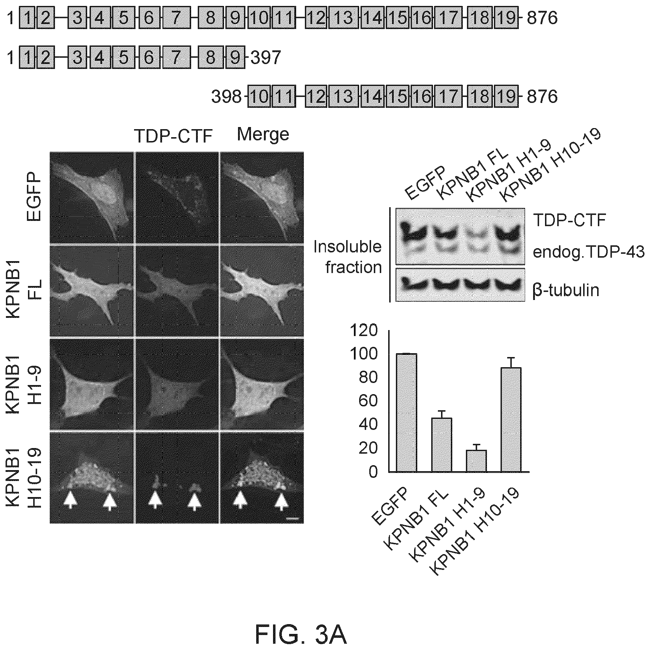

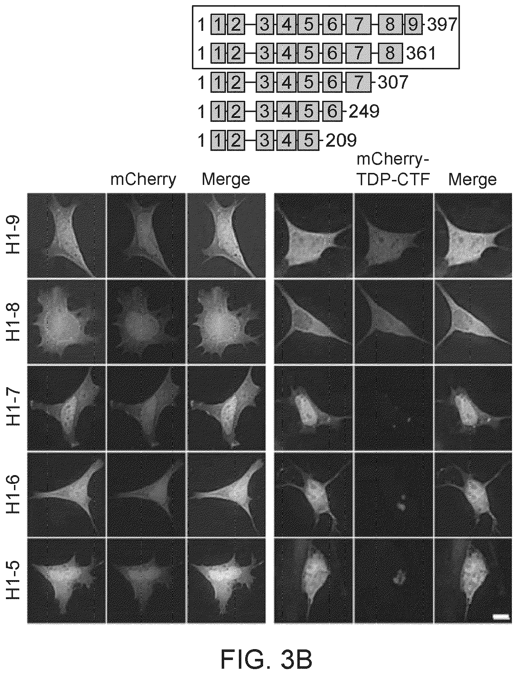

Figures 3A-3B: The N-terminal fragment of KPNB 1 is required and sufficient to reduce TDP-CTF aggregates. Figure 3A) Top: Schematic of the HEAT repeat domains in KPNB1 (H1-19). Bottom left: Representative images of SH-SY5Y cells expressing mCherry-TDP-CTF with either EGFP, EGFP-KPNB1 full-length (FL), the N-terminal fragment of KPNB 1 that includes HEAT repeat 1-9 (H1-9) or the C-terminal fragment of KPNB1 (H10-19). Both full-length KPNB1 and its N-terminal fragment strongly reduce the appearance of cytoplasmic TDP-CTF aggregates, whereas the C-terminal half of KPNB 1 co-aggregates with TDP-CTF (arrows). Bottom right: Both full-length KPNB1 and its N-terminal fragment but not its C-terminal fragment strongly reduce the amount of RIPA buffer insoluble TDP-CTF. Figure 3B) Different truncated constructs of the N-terminal half of KPNB1 were co-transfected with either mCherry or mCherry-TDP-CTF. Only constructs H1-9 and H1-8 completely suppressed TDP-CTF aggregates and led to diffuse nucleocytoplasmic distribution of mCherry-TDP-CTF. Scale bar=5µm.

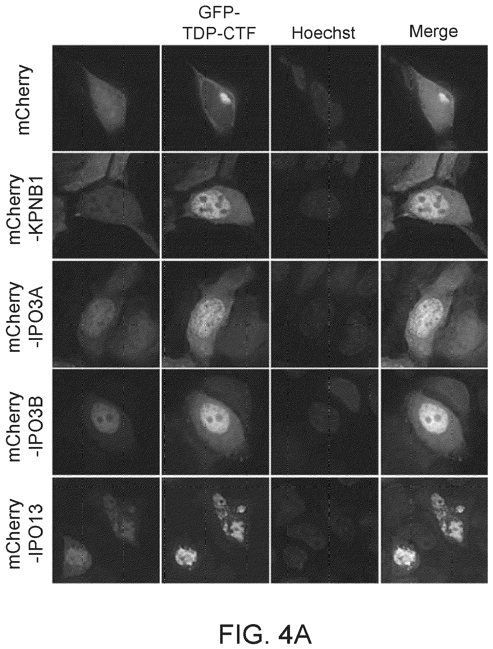

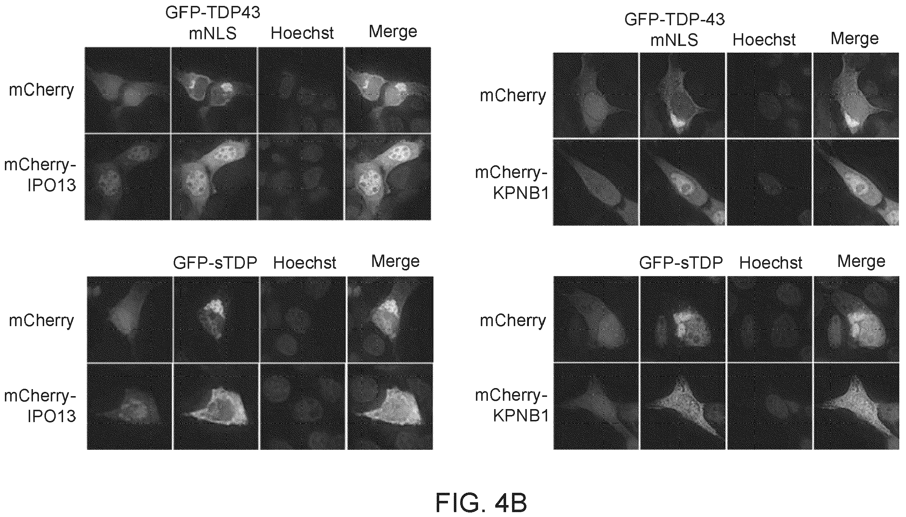

Figures 4A-4B: Selected NIRs mediate nuclear localization of TDP-43 variants even if they are lacking a functional nuclear localization sequence/signal (NLS). Representative images of SH-SY5Y human neuroblastoma cells expressing GFP-tagged TDP-CTF or full-length TDP-43 with a mutated, inactive NLS (TDP-43^{mNLS}) with mCherry-tagged NIRs. Figure 4A) KPNB1, TNPO2/IPO3 splicing isoforms IPO3A and IPO3B, and IPO13 can recruit TDP-CTF into the nucleus. Figure 4B) KPNB 1 and IPO13 can recruit full length TDP-43^{mNLS} into the nucleus. A shortened N-terminal splicing isoform of TDP-43 (sTDP-43) is partially disaggregated but remains largely cytoplasmic, probably due to the presence of a nuclear export signal.

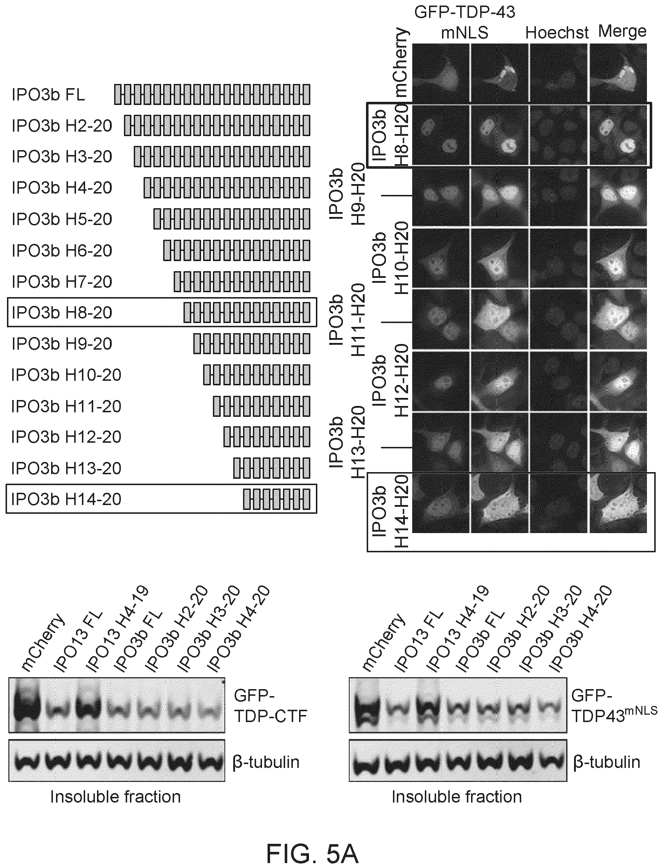

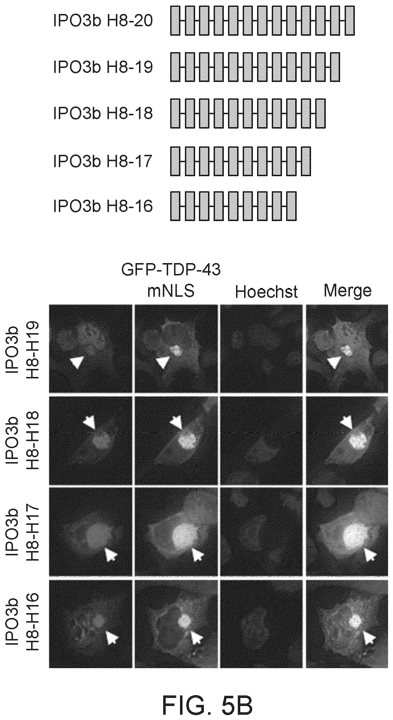

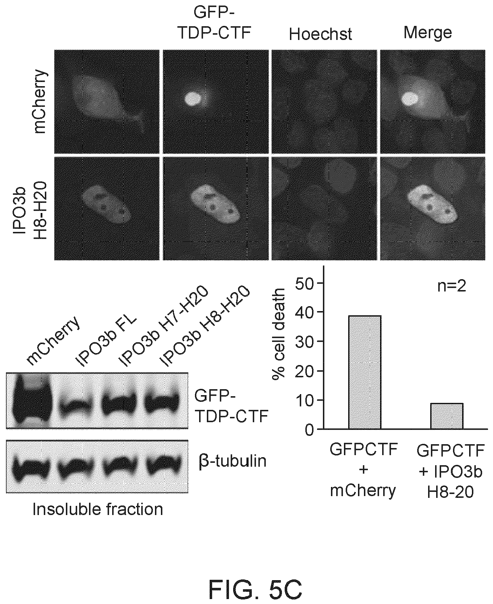

Figure 5A-5C: The C-terminal fragment of IPO3B is required and sufficient to mediate the nuclear localization of TDP-CTF and TDP-43^{mNLS}. . Figure 5A) Top: Schematic of the HEAT repeat domains in IPO3B (H1-20). Bottom: Representative images of SH-SY5Y cells expressing mCherry-TDP-CTF with either EGFP or EGFP-IPO3B constructs. Deletions of N-terminal HEAT repeats beyond H13 (H14-20) cause a drop in nuclear localization but still reduce TDP-43 aggregation. The strongest nuclear import activity was observed for IPO3b H8-H20. Bottom: Full-length IPO3 and IPO13, as well as the truncated IPO3 constructs H2-20, H3-20 and H4-20 reduce the levels of RIPA buffer insoluble GFP-TDP-CTF and GFP- TDP-43^{mNLS} protein levels in transfected HEK293 cells. Figure 5B) C-terminal deletions of IPO3B H20 (H8-19) and beyond cause strong cytoplasmic co-aggregation. These data demonstrate that H13-20 is required for the nuclear localization of TDP-43^{mNLS}. H20 is required for both nuclear transport and disaggregation. Figure 5C) IPO3B H8-H20 causes nuclear localization of TDP-CTF, reduces its insoluble aggregation, and ameliorates TDP-CTF-dependent toxicity in SH-SY5Y cells.

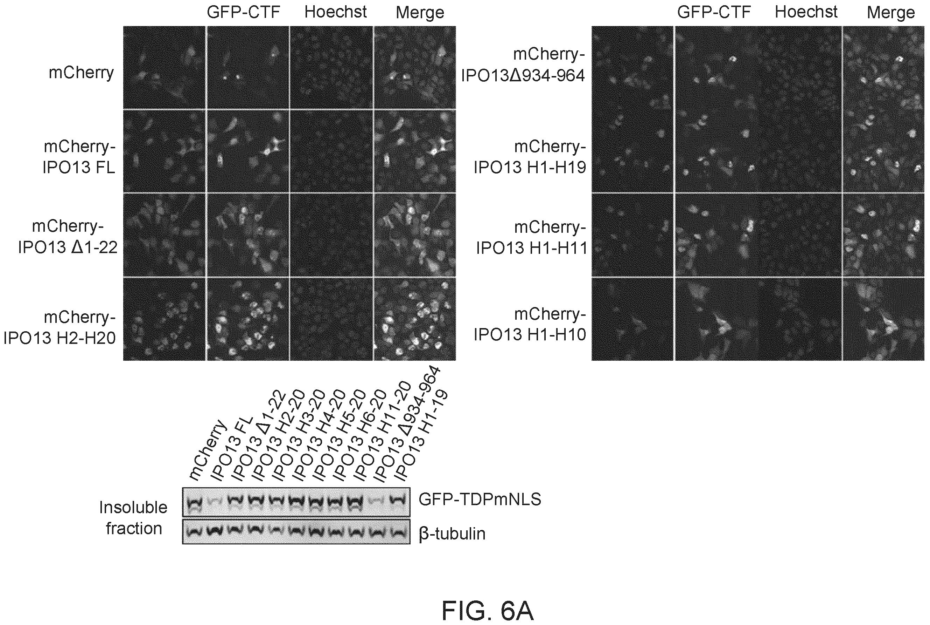

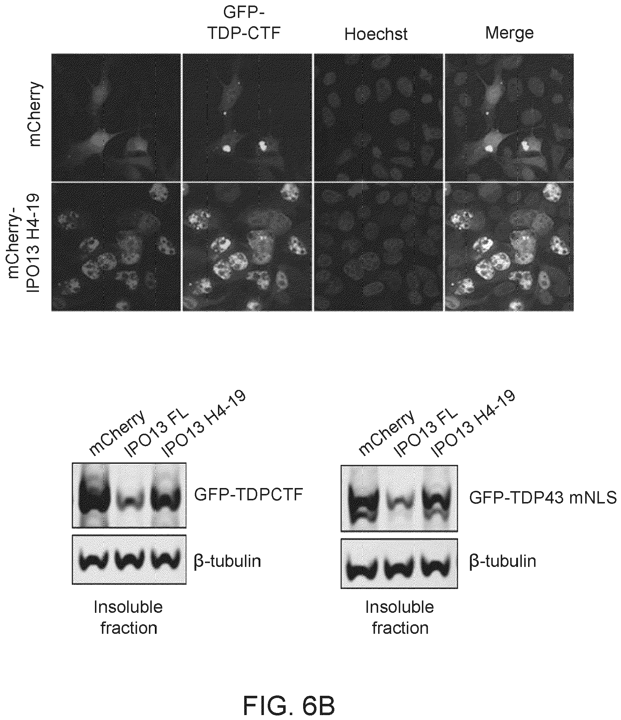

Figure 6A-6B. IPO13 fragments mediate the nuclear localization of TDP-CTF. Figure 6A) TDP-CTF becomes nuclear but appears aggregated upon co-expression of the IPO13 deletion constructs H1-19, H2-20, and H1-10, and to a lesser extent with construct H1-11. Western blot analysis of the insoluble proteome shows that only full-length and an IPO13 construct with deletion of the C-terminus after H20 (Δ934-964) strongly reduce detergent-insoluble GFP-TDP-43^{mNLS} protein levels. The other tested IPO13 constructs make TDP-CTF more nuclear yet it appears aggregated, which would explain why no effect is observed on the insoluble TDP-43 levels. Figure 6B. N-terminal deletions show that IPO13 H4-19 strongly mediates the nuclear localization of TDP-CTF but not the disaggregation of TDP-CTF and TDP-43^{mNLS}.

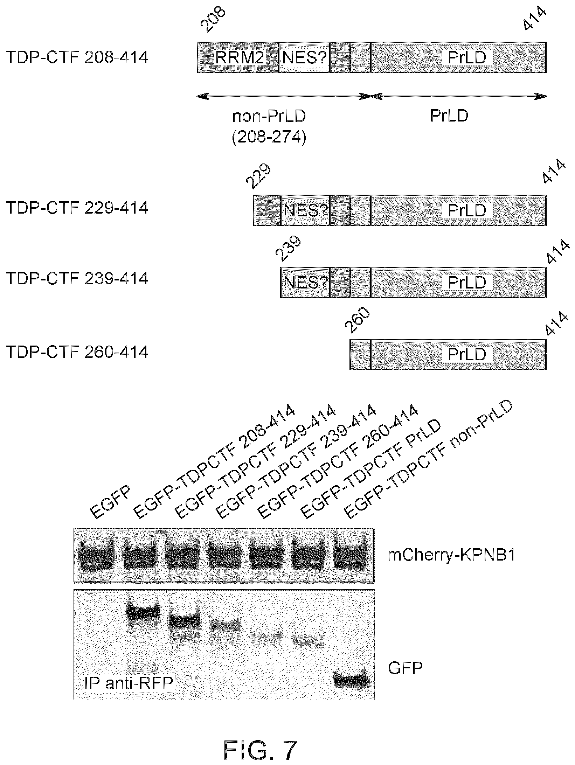

Figure 7: KPNB1 interacts with the C-terminal domain of TDP-43 in the absence of the NLS. Co-IPs of mCherry-tagged TDP-43 deletion constructs with GFP-KPNB1 show that the association does not require the intrinsically disordered aggregation-prone prion-like domain (PrLD).

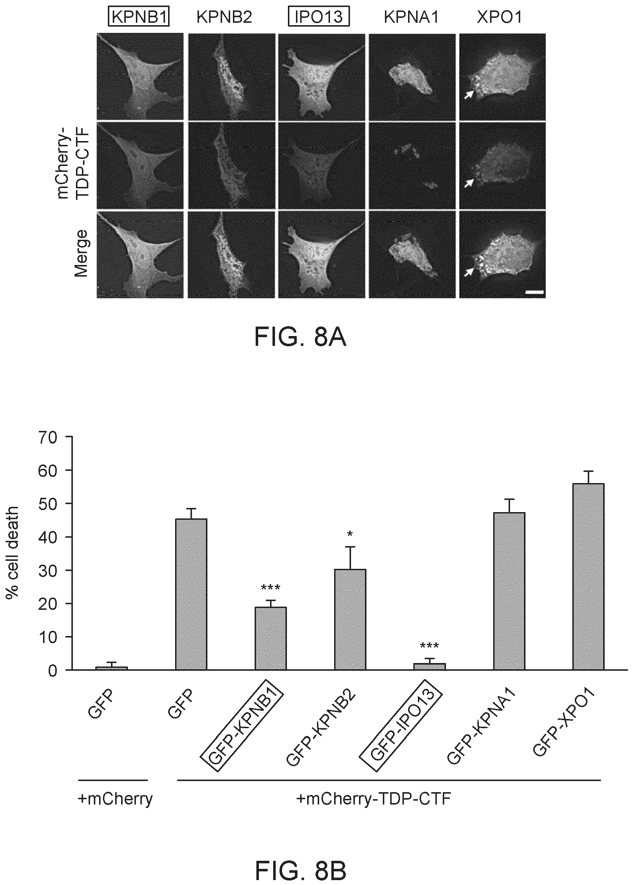

Figures 8A-8B: NIRs preventing TDP-CTF aggregation also reduce TDP-CTF induced cell death in neuronal SH-SY5Y cells. Figure 8A) Representative images of SH-SY5Y cells co-expressing EGFP-tagged NIRs with mCherry-TDP-CTF shows that KPNB 1, IPO13, and to a lesser degree KPNB2/TNPO1 reduce aggregation. Scale bar: 5 µm. Figure 8B) KPNB1 and IPO13 expression strongly reduces TDP-CTF-dependent cell death, while KPNB2/TNPO1 has a weaker effect. N=3; *p<0.5, ***p<0.005.

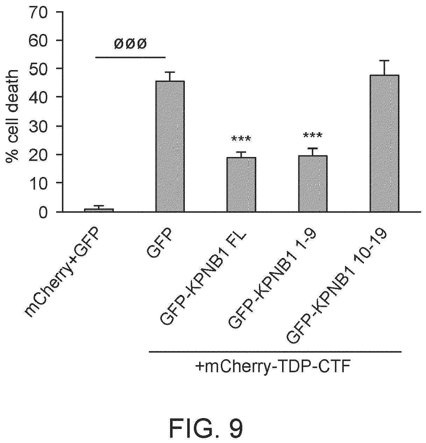

Figure 9. KPNB1 fragments that reduce TDP-CTF aggregation also prevent TDP-CTF induced cell death. Co-expression of mCherry-TDP-CTF with GFP-tagged full length KPNB1, KPNB1 (H1-9), and KPNB1 (H10-19) in neuronal SH-SY5Y cells show that only the disaggregating full length KPNB1 and its N-terminal fragment (H1-9) reduce TDP-CTF induced cell death.

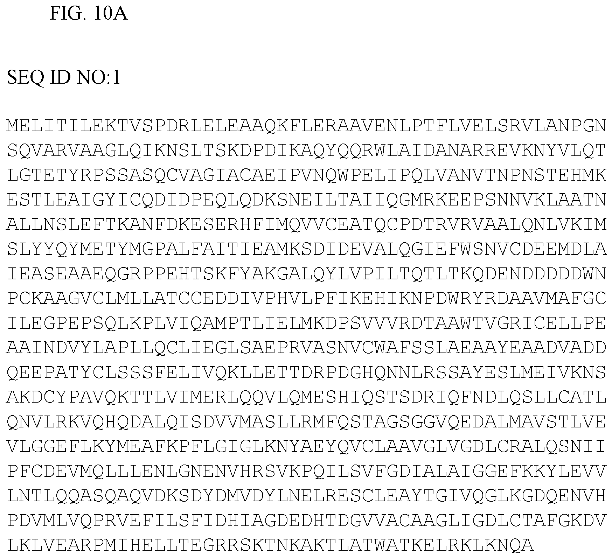

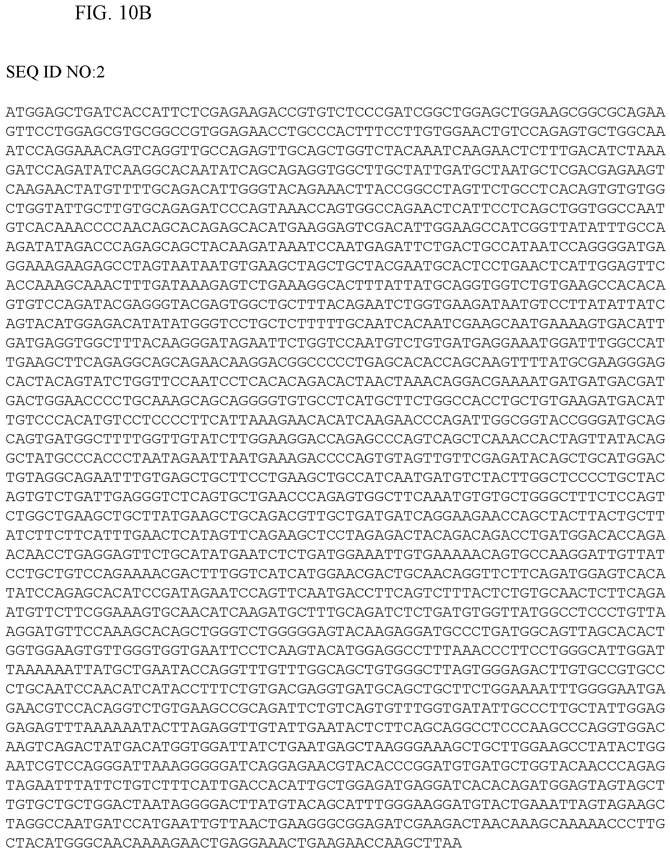

Figures 10A and 10B. Figure 10A) An amino acid sequence of an exemplary KPNB 1 polypeptide (SEQ ID NO:1). Figure 10B) An exemplary nucleic acid encoding a KPNB 1 polypeptide (SEQ ID NO:2).

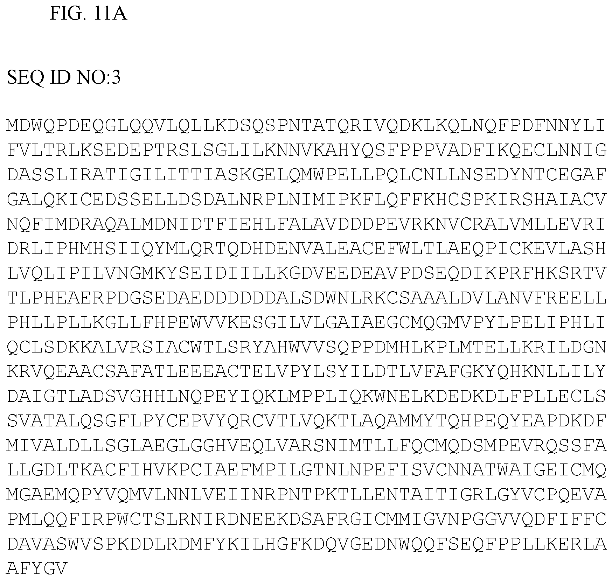

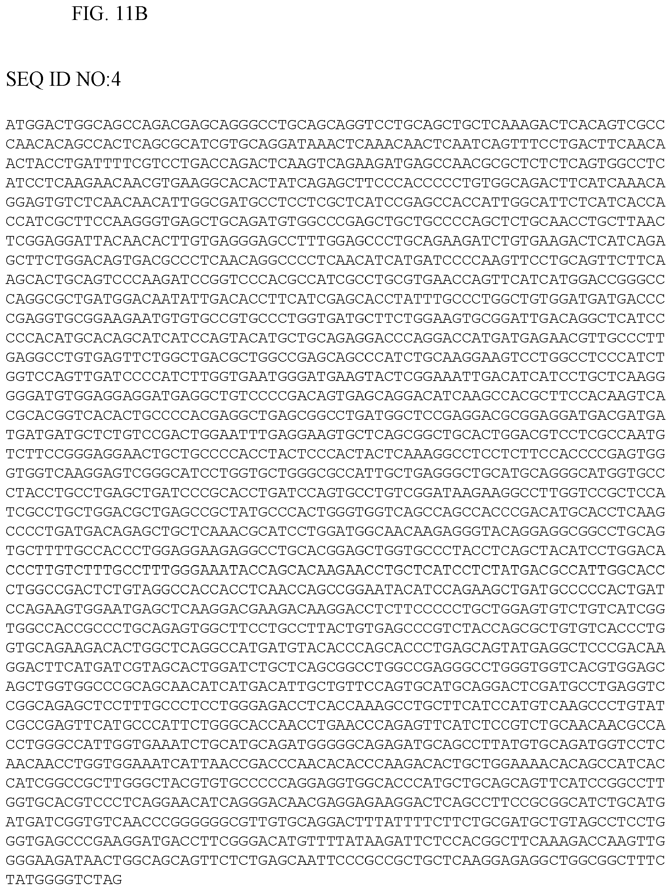

Figures 11A and 11B. Figure 11A) An amino acid sequence of an exemplary IPO3 polypeptide (SEQ ID NO:3). Figure 11B) An exemplary nucleic acid encoding a IPO3 polypeptide (SEQ ID NO:4).

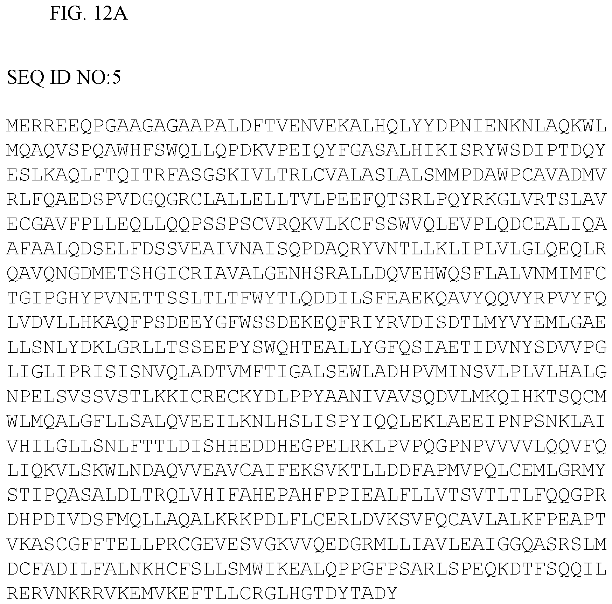

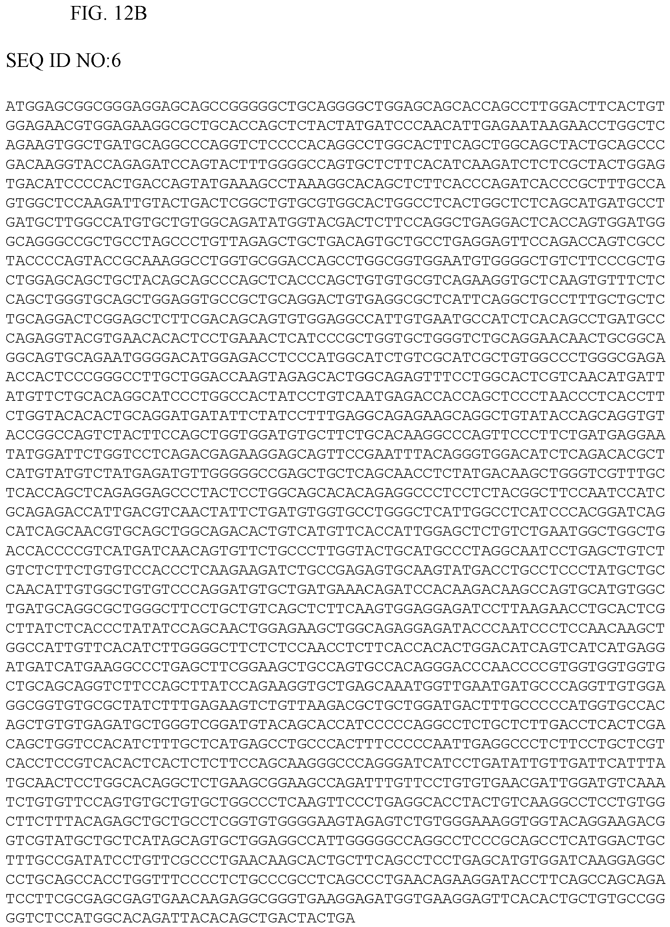

Figures 12A and 12B. Figure 12A) An amino acid sequence of an exemplary IPO13 polypeptide (SEQ ID NO:5). Figure 12B) An exemplary nucleic acid encoding a IPO13 polypeptide (SEQ ID NO:6).

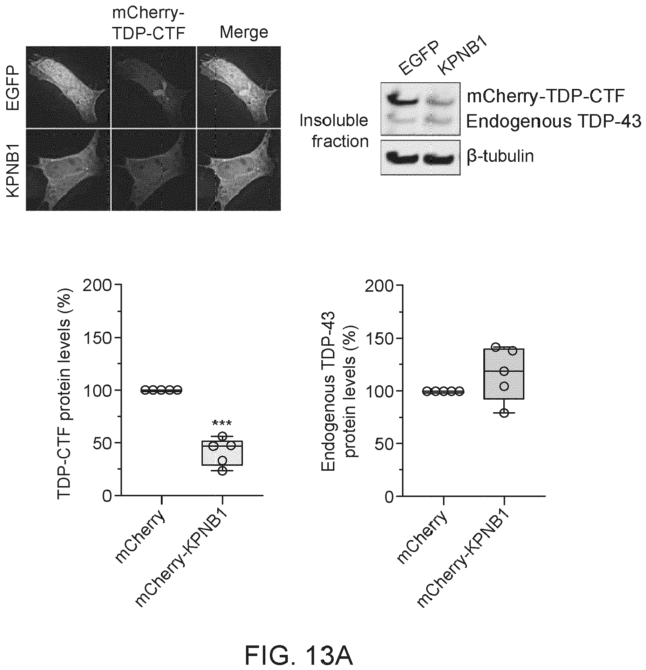

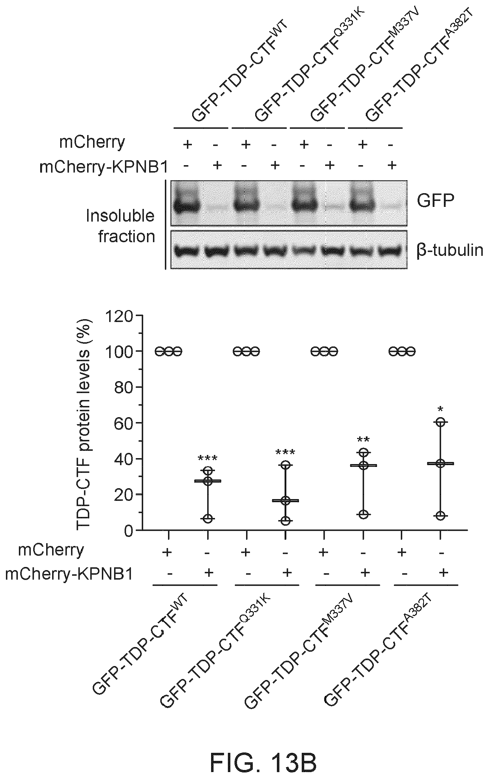

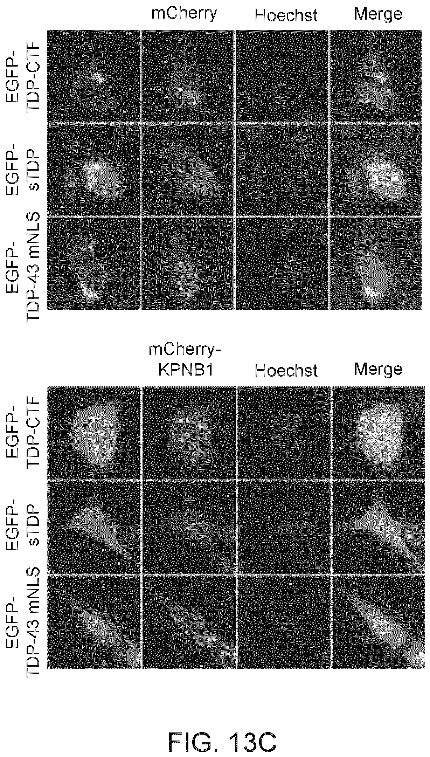

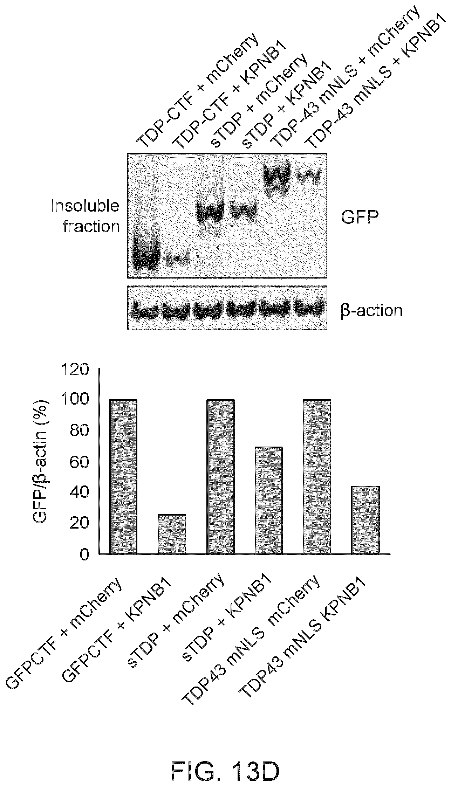

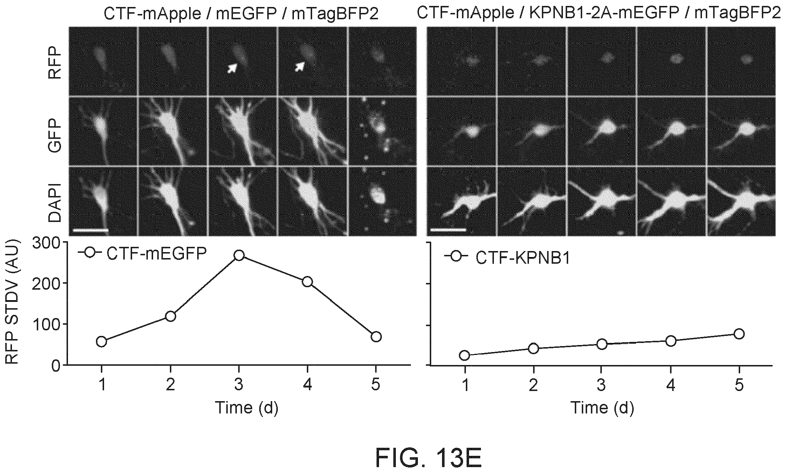

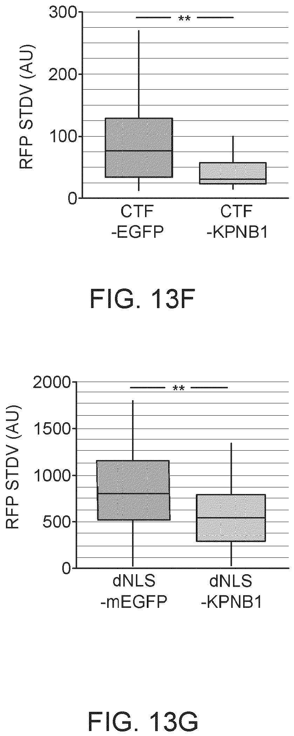

Figures 13A-13G: KPNB1 reduces the aggregation of TDP-43 in cellular models of ALS/FTD. Figure 13A) Cells co-transfected with mCherry-TDP-CTF and either EGFP or EGFP-KPNB 1 demonstrate that KPNB 1 reduces the aggregation of TDP-43 through fluorescence microscopy. Western blot analysis of the insoluble fraction further demonstrates this reduction in TDP-43 aggregation. Figure 13B) Western blot analysis of the insoluble fraction of co-transfected cells demonstrates that the disaggregation activity of KPNB1 against TDP-43 carrying known familial mutations Q331K, M337V, or A382T similar to that of wild type TDP-43. Figure 13C) Fluorescence microscopy shows KPNB1 has disaggregation activity against wild type GFP-tagged TDP-CTF, as well as the splicing isoform which lacks the C-terminal prion-like domain (sTDP) and TDP-43 with a mutated nuclear localization signal (mNLS). Figure 13D) Western blot analysis shows KPNB1 has disaggregation activity against wild type GFP-tagged TDP-CTF, TDP-43 sTDP, and TDP-43 mNLS. Figure 13E) Live-cell fluorescence microscopy analysis of primary cortical neurons co-transfected with expression constructs for TDP-CTF-mApple, mTagBFP2, and either mEGFP or KPNB 1-2A-mEGFP demonstrated that the increase of TDP-CTF-mEGFP fluorescence over time is prevented by KPNB1 expression. Figure 13F) Induced expression of KPNB 1 prevents the increase of fluorescence of a TDP-CTF construct (CTF) over time. Figure 13G) Induced expression of KPNB1 prevents the increase of fluorescence of a TDP-dNLS construct (CTF) over time.

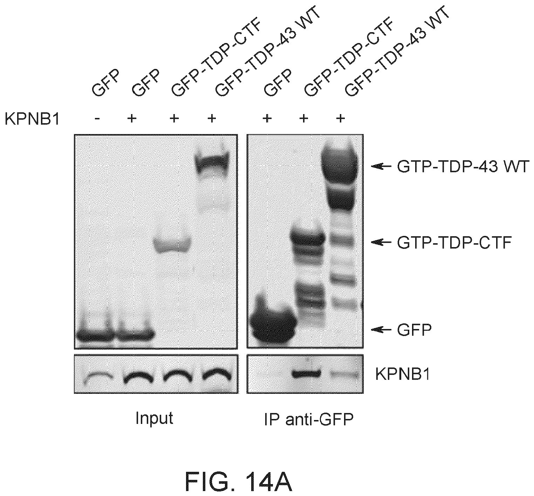

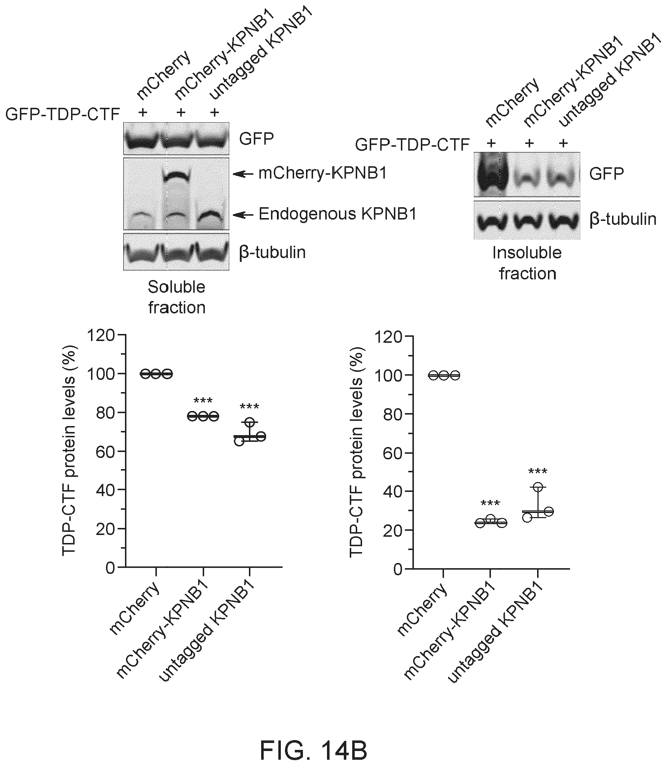

Figures 14A-14B: KPNB1 interacts with TDP-CTF and reduces its aggregation. Figure 14A) Western blot analysis of an immunoprecipitation show that both GFP-TDP-43 and GFP-TDP-CTF interact with KPNB1 despite GFP-TDP-CTF lacking the nuclear localization signal. Figure 14B) Western blot analysis of cells co-transfected with expression constructs for GFP-TDP-CTF and either mCherry-tagged KPNB1 or untagged KPNB 1 demonstrate similar reduction of RIPA insoluble TDP-CTF levels compared to untreated, regardless of the presence or absence of the mCherry tag.

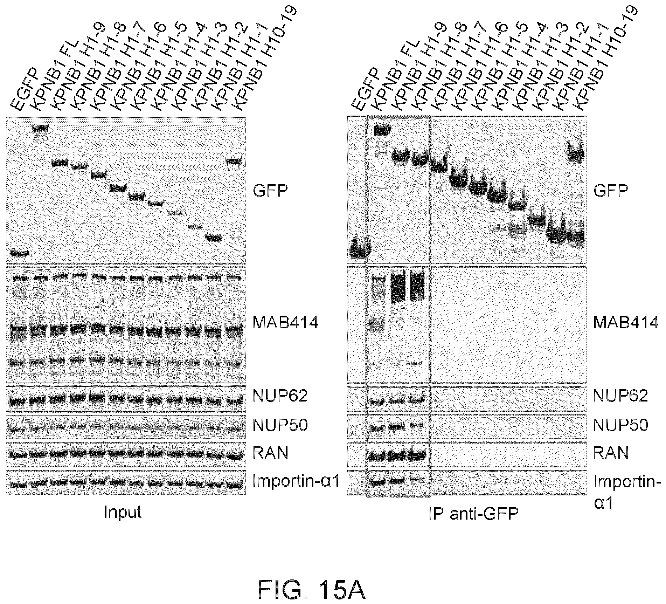

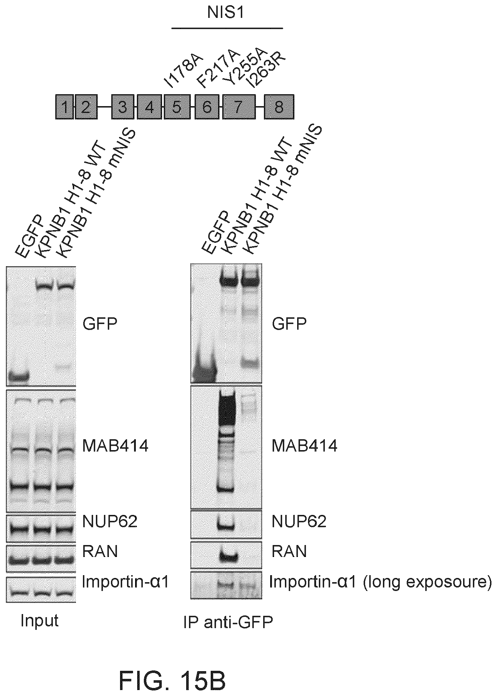

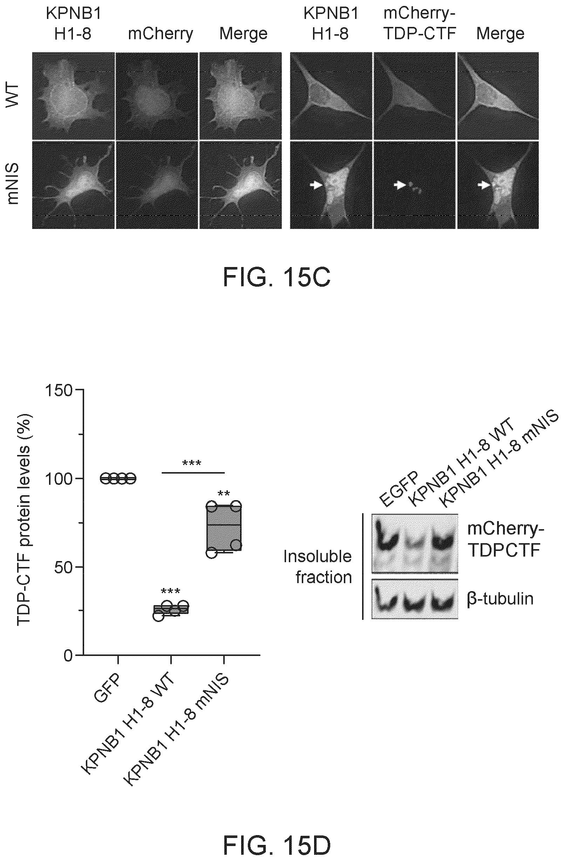

Figures 15A-15D: The N-terminal domain of KPNB1 interacts with phenylalanine and glycine rich nucleoporins. Figure 15A) Western blot analysis of co-immunoprecipitation experiments with GFP-tagged KPNB1 truncations demonstrate that KPNB 1 FL, KPNB1 H1-8, and KPNB1 H1-9, but not smaller truncations, bind to the phenylalanine and glycine rich nucleoporins (FG-Nups) NUP62, NUP50, RAN, and importin-a1. Figure 15B) Co-immunoprecipitation of the FG-Nup Nup62 by KPNB1 is abrogated by mutation in the nucleoporin-interaction sequence (I178A, F217A, Y255A, and I263R; mNIS). Figure 15C) Fluorescence microscopy of cells co-expressing TDP-43 and either KPNB1 H1-8 or KPNB1 H1-8 mNIS demonstrate reduced disaggregation activity for the mNIS variant. 15D) Western blot analysis of the insoluble fraction of cells co-expressing TDP-43 and either KPNB 1 H1-8 or KPNB 1 H1-8 mNIS demonstrate reduced disaggregation activity for the mNIS variant.

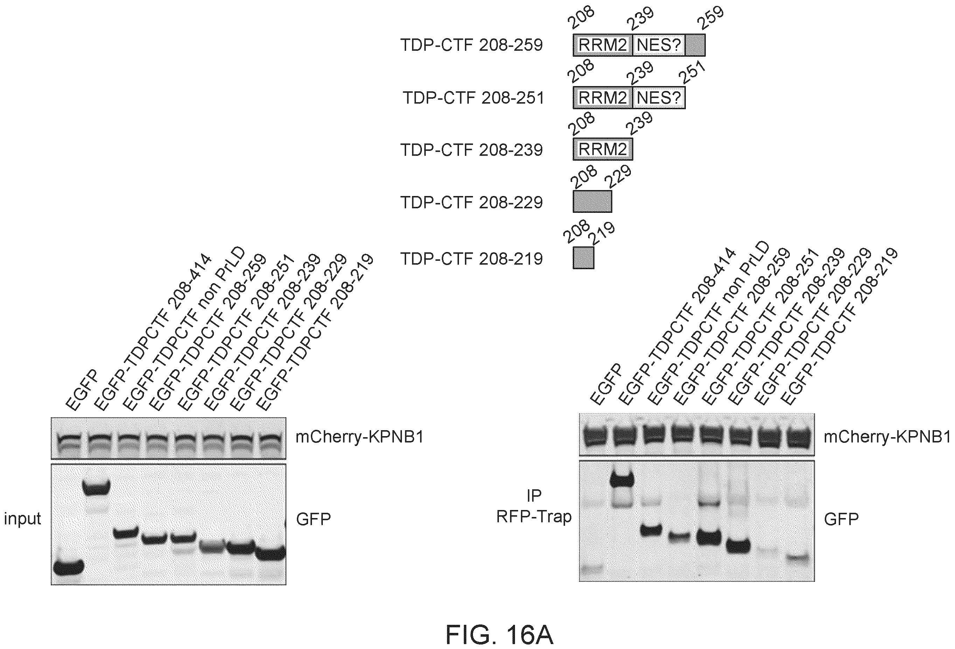

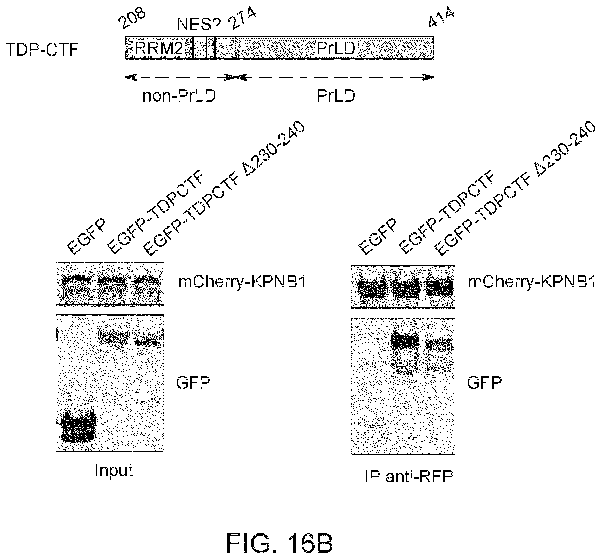

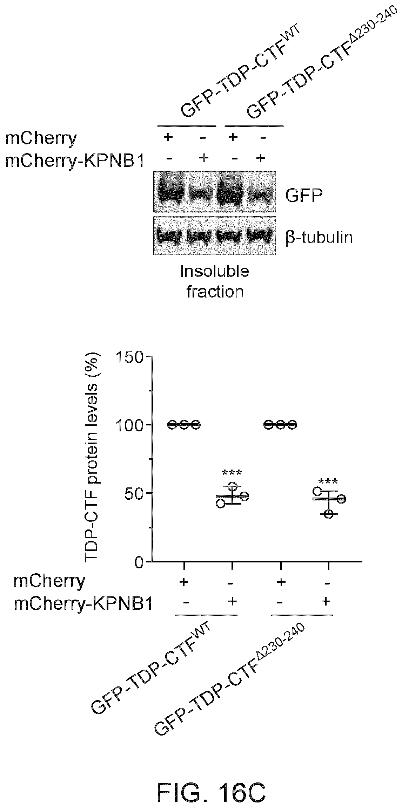

Figures 16A-16C: TDP-CTF binds to KPNB1 via its RRM2 and PrLD domains. Figure 16A) Western blot analysis of co-immunoprecipitation experiments show stronger mCherry-KPNB 1 interaction of TDP-CTF 208-239 vs. TDP-CTF 208-229. Figure 16B) Western blot analysis of co-immunoprecipitation experiments show that TDP-CTF fragment lacking aa230-240 (D230-240) shows reduced interaction with mCherry-KPNB 1 as compared with TDP-CTF. Figure 16C) Western blot analysis of the insoluble fraction of co-transfected cells show that both GFP-TDP-CTF and GFP-TDP-CTF del(aa230-240) are disaggregated by mCherry-KPNB 1 at similar levels.

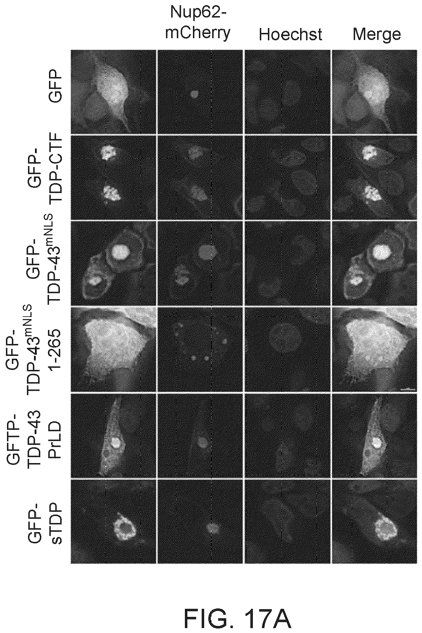

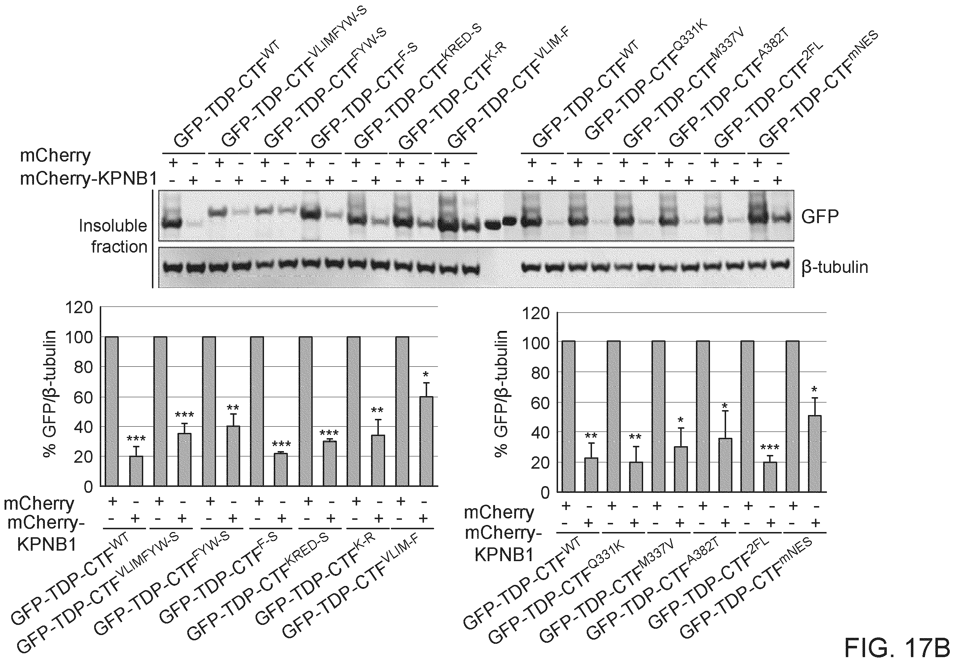

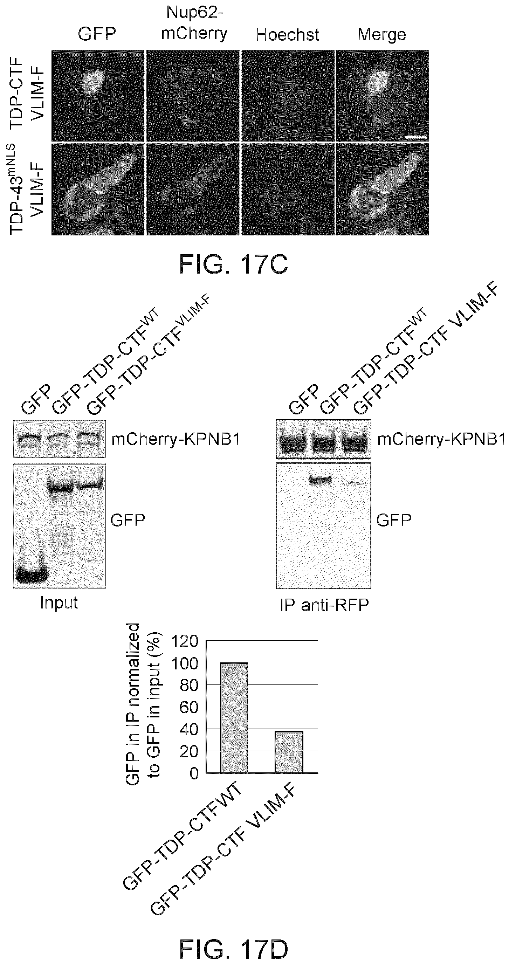

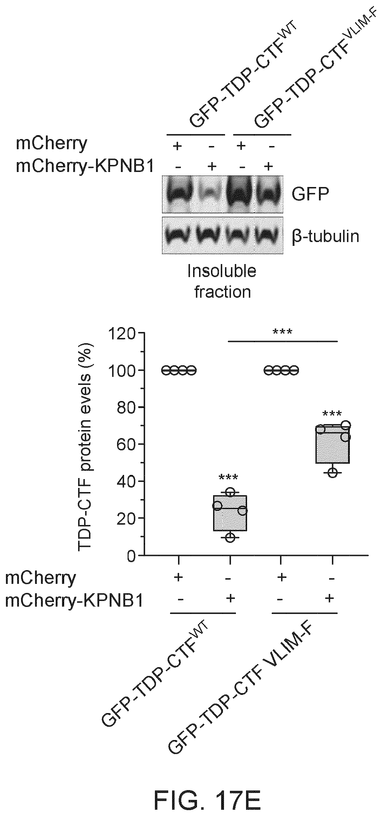

Figures 17A-17E. FG-Nups mediate the interaction and disaggregation activity of KPNB1 versus TDP-43. Figure 17A) Fluorescence microscopy of co-transfected cell shows that GFP-tagged TDP-CTF, TDP-43mutNLS, and TDP PrLD co-localize with Nup62, but N-terminal fragment TDP-43mutNLS aa1-265 and sTDP, lacking the PrLD, do not. Figure 17B) Western blot analysis of the insoluble fractions of cells expressing mutant variants of TDP-CTF where residues in the PrLD were replaced by other residues as indicated demonstrate that mutations of V, L, I, and M residues to F reduced disaggregation by KPNB1. Figure 17C) Co-immunoprecipitation experiments show that the VLIM-F variant of TDP-CTF has reduced interaction with mCherry-tagged KPNB1. Figure 17D) Immunocytochemistry of cells expressing GFP-tagged TDP-CTF and TDP-43 (mutNLS) variants where V, L, I, and M amino acid residues in the PrLD were all mutated into F (VLIM-F) show reduced co-localization with both Nup62 and KPNB1. Figure 17E) Western blot analysis of the insoluble fraction of co-transfected cells demonstrate that the VLM-F variant of TDP-CTF is less disaggregated by KPNB1 than TDP-CTF.

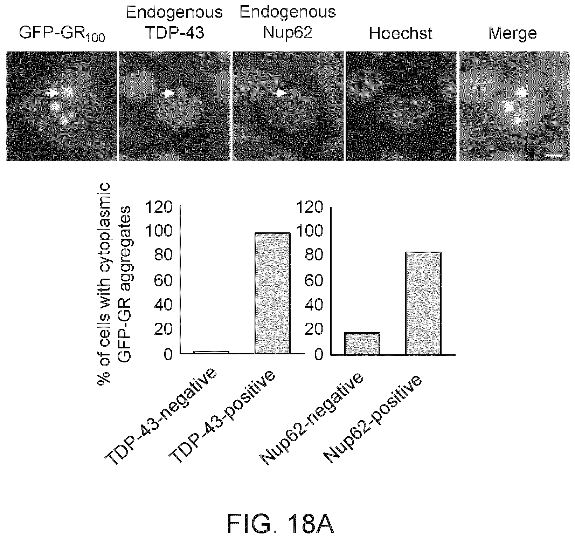

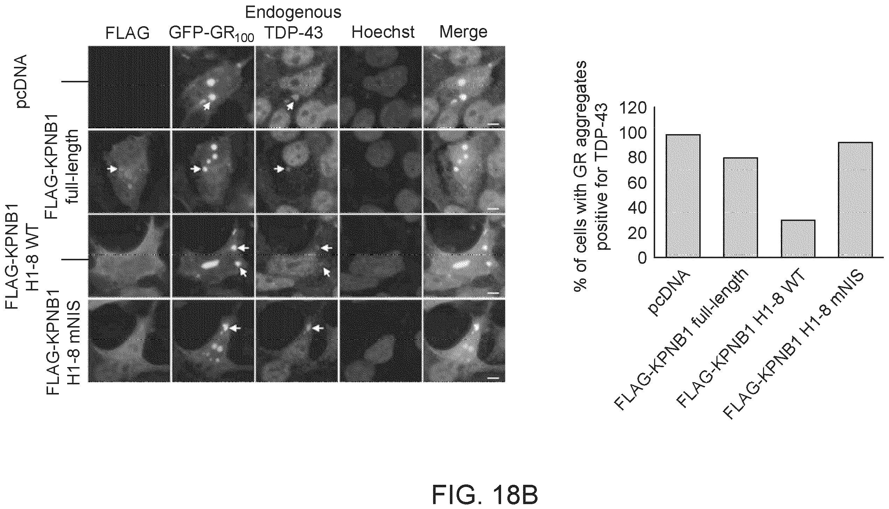

Figures 18A-18B. KPNB1 reduces the aggregation of endogenous TDP-43 in a poly-GR model of C9orf72-related ALS (C9ALS). Figure 18A) Cells transfected to express GFP-GRx100 C9orf72 to induce C9ALS demonstrate the co-aggregation of TDP-43 with GRx100 C9orf72 and Nup62. Figure 18B) Full length KPNB1 and its truncation H1-8 reduced the number of GR C9orf72 aggregates containing mislocalized TDP-43, but H1-8 with a mutated NIS was inactive.

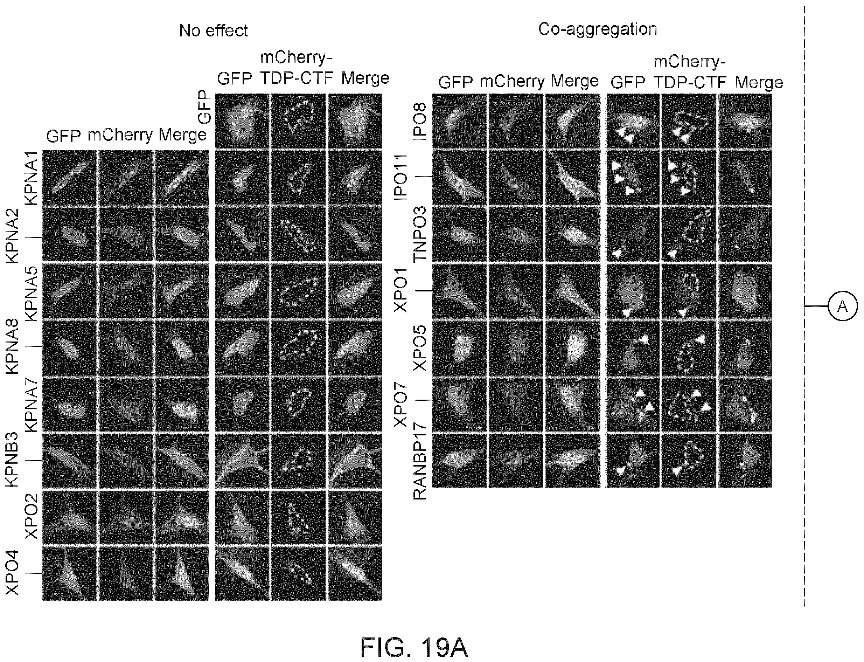

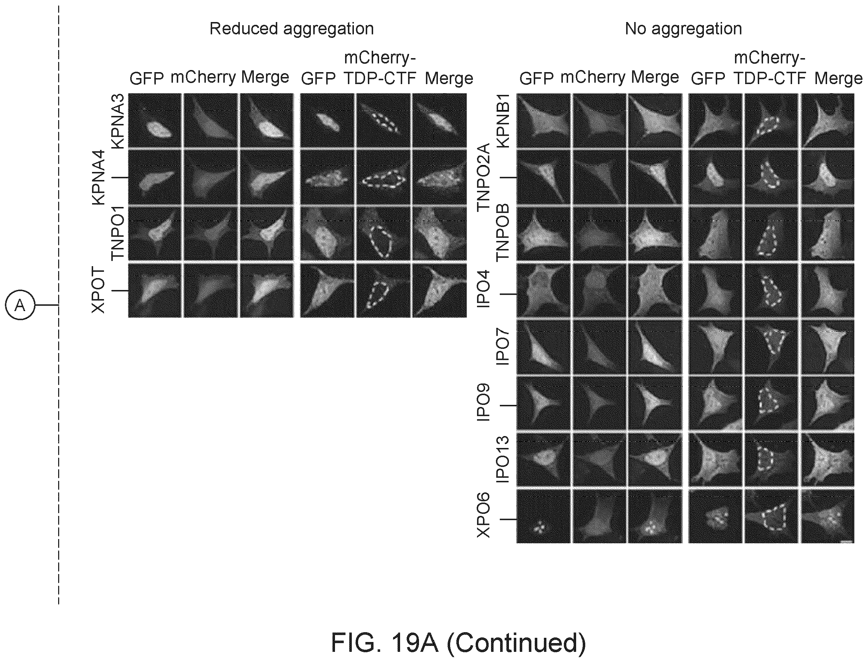

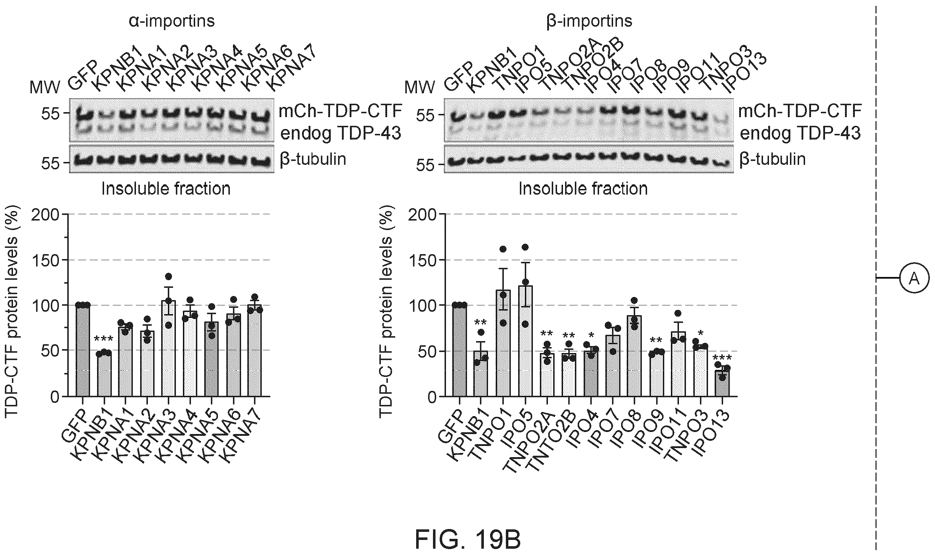

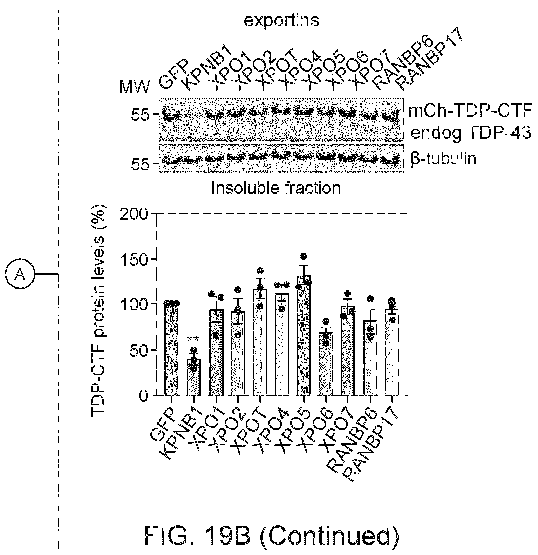

Figures 19A - 19B: KPNB1 and other β-type importins reduce the pathological aggregation of TDP-CTF. Figure 19A) Immunofluorescence of SH-SY5Y human neuroblastoma cells co-expressing mCherry or mCherry-TDP-CTF with 27 GFP-tagged transport receptors. Screen results were subdivided in four categories according to their activity: "no effect", "co-aggregation", "reduced aggregation" (=transport proteins that only reduce the size of TDP-CTF aggregates), and "no aggregation" that include most β-importins. Arrowheads indicate co-aggregation. Hoechst staining was used to outline nuclei. Scale bar: 5 µm. Figure 19B) Western blot analysis and quantification of insoluble mCherry-TDP-CTF protein levels in SH-SY5Y cells expressing GFP or one of each 27 GFP-tagged transport receptors. Multiple β-importins significantly reduced insoluble TDP-CTF levels. KPNB1 (in bold) was used as a reference. β-tubulin was used as a loading control. Statistical analysis was performed using one-way ANOVA and Bonferroni's post hoc test (*p<0.05, **p<0.01, ***p<0.001, n=3).

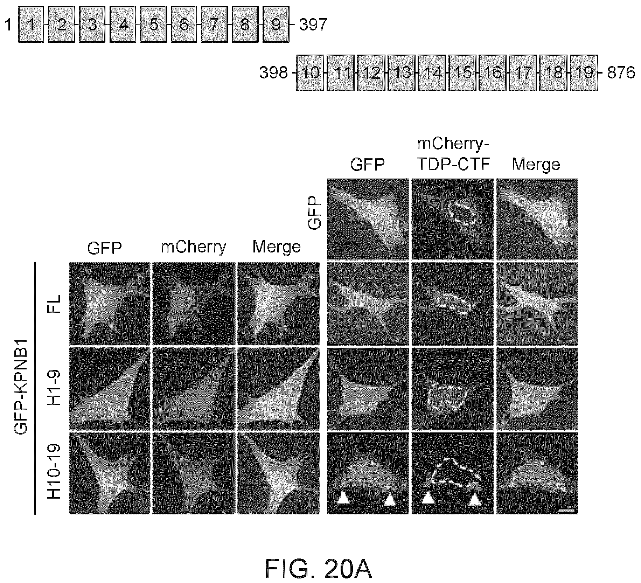

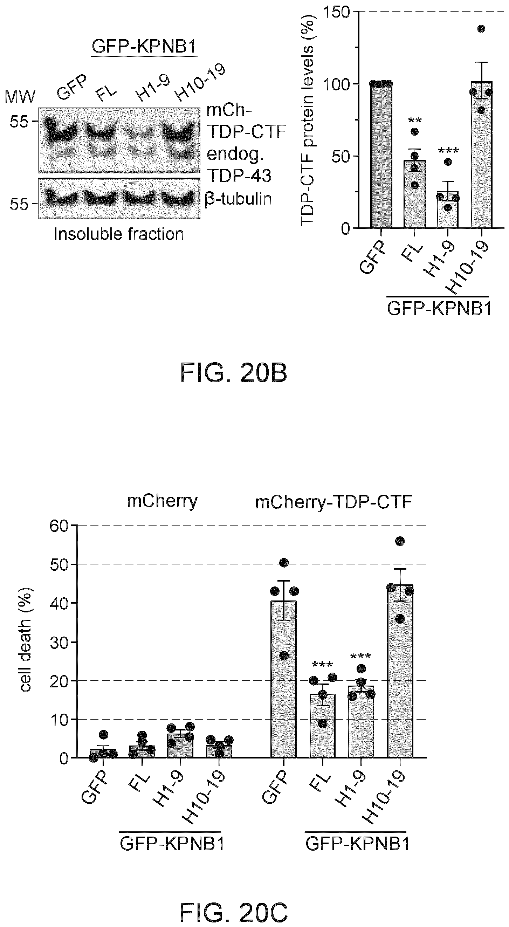

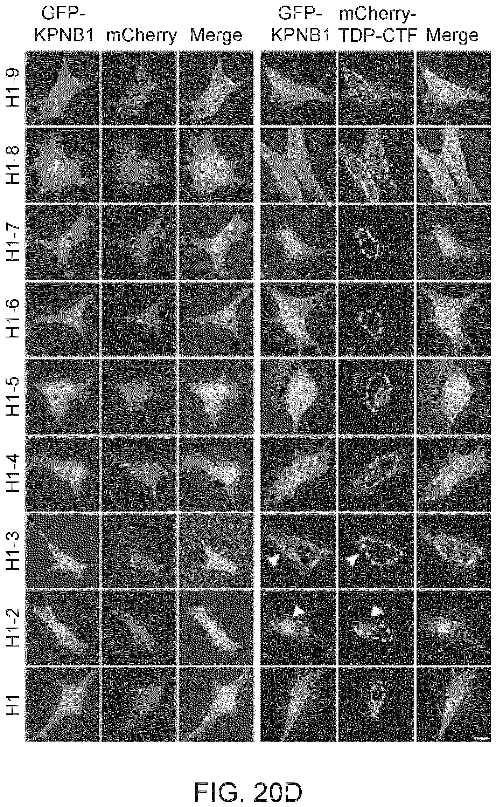

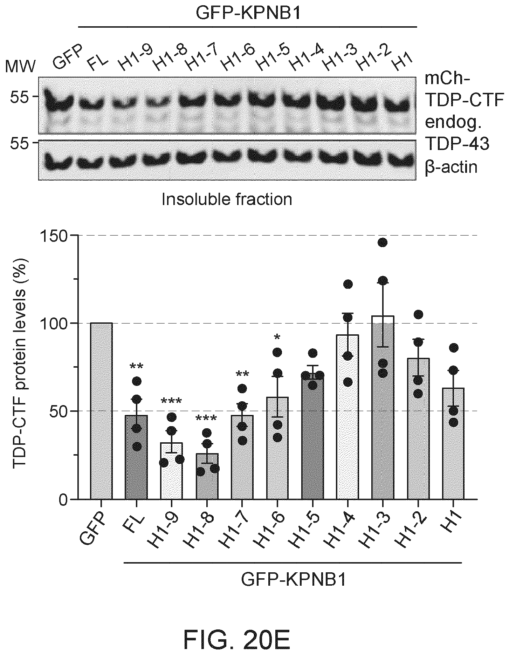

Figures 20A - 20E: The N-terminal half of KPNB 1 is required and sufficient to reduce TDP-CTF aggregation and toxicity. Figure 20A) (Top) Schematic domain structure of the N-terminal half (HEAT repeats 1-9, H1-9) and C-terminal half (HEAT repeats 10-19, H10-19) of KPNB1. (Bottom) Immunofluorescence (IF) of SH-SY5Y cells co-expressing mCherry or mCherry-TDP-CTF with GFP, GFP-KPNB1 full-length (FL), H1-9 or H10-19. Both full-length and the N-terminal half of KPNB 1 reduce TDP-CTF aggregation, whereas the C-terminal half of KPNB 1 co-aggregates with TDP-CTF in the cytoplasm. Arrowheads indicate co-aggregation. Hoechst staining was used to outline nuclei. Scale bar: 5 µm. Figure 20B) Western blot analysis and quantification of insoluble mCherry-TDP-CTF protein levels in SH-SY5Y cells expressing GFP, GFP-KPNB1 full-length (FL), H1-9 or H10-19. Both full-length and the N-terminal half of KPNB 1 significantly reduced insoluble TDP-CTF levels. β-tubulin was used as a loading control. Statistical analysis was performed using one-way ANOVA and Bonferroni's post hoc test (**p<0.01, ***p<0.001, n=4). Figure 20C) Quantification of TDP-CTF-induced cytotoxicity upon overexpression of KPNB1. mCherry or mCherry-TDP-CTF were co-expressed in SH-SY5Y cells with GFP, GFP-KPNB1 full-length (FL), H1-9 or H10-19. 48 hours after transfection, cells were labelled with the Live- or-Dye^{™} 640/662 dye and dying cells were quantified for each group. Both full-length and the N-terminal half of KPNB1 significantly ameliorated TDP-CTF-mediated toxicity. Statistical analysis was performed with two-way ANOVA and Bonferroni's post hoc test (four independent experiments; ***p<0.001. mCherry: GFP (n=400), GFP-KPNB1^{FL} (n=405), GFP-KPNB1^{H1-9} (n=426), GFP-KPNB1^{H10-19} (n=406); mCherry-TDP-CTF: GFP (n=405), GFP-KPNB1^{FL} (n=401), GFP-KPNB1^{H1-9} (n=405), GFP-KPNB1^{H10-19} (n=400)). Figure 20D) IF of SH-SY5Y cells co-expressing mCherry or mCherry-TDP-CTF with GFP-KPNB1 H1-9 or C-terminal deletion constructs. TDP-CTF appeared nucleocytoplasmic with KPNB1 H1-9 and H1-8, while it formed small aggregates in presence of KPNB1 H1-7 or H1-6. Arrowheads indicate co-aggregation. Hoechst staining was used to outline nuclei. Scale bar: 5 µm. Figure 20E) Western blot analysis and quantification of insoluble mCherry-TDP-CTF protein levels in SH-SY5Y cells expressing GFP, GFP-KPNB1 full-length (FL), H1-9 or C-terminal deletion constructs. KPNB1 H1-8 was the most active fragment in reducing insoluble TDP-CTF levels. β-actin was used as a loading control. Statistical analysis was performed using one-way ANOVA and Bonferroni's post hoc test (*p<0.05, **p<0.01, ***p<0.001, n=4).

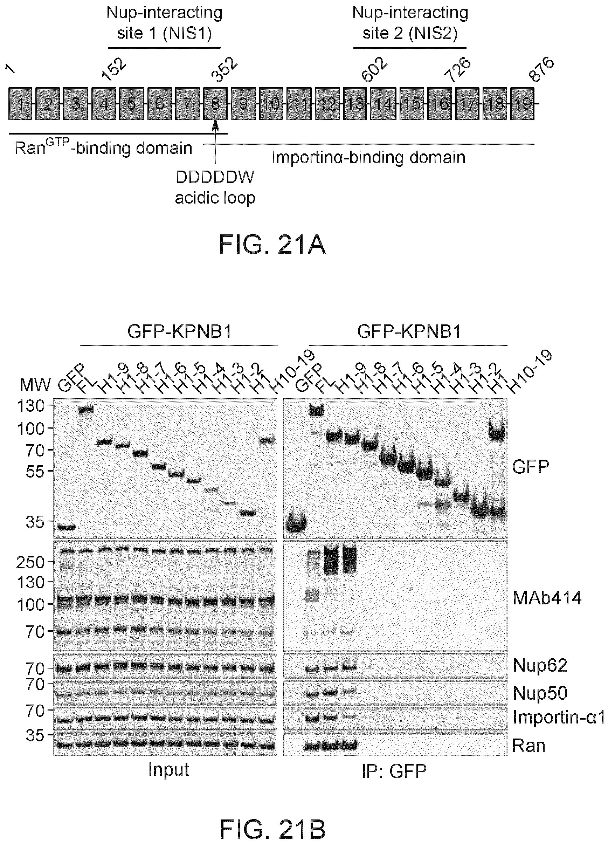

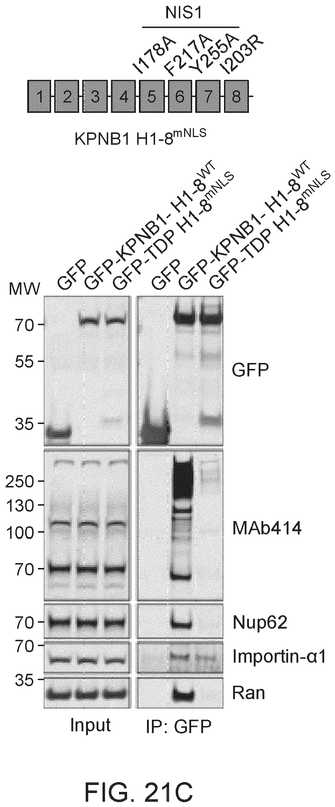

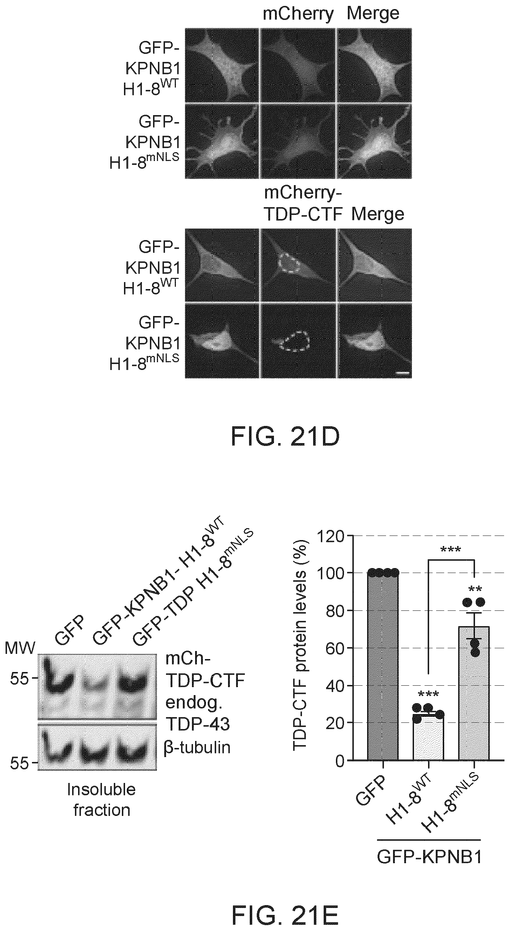

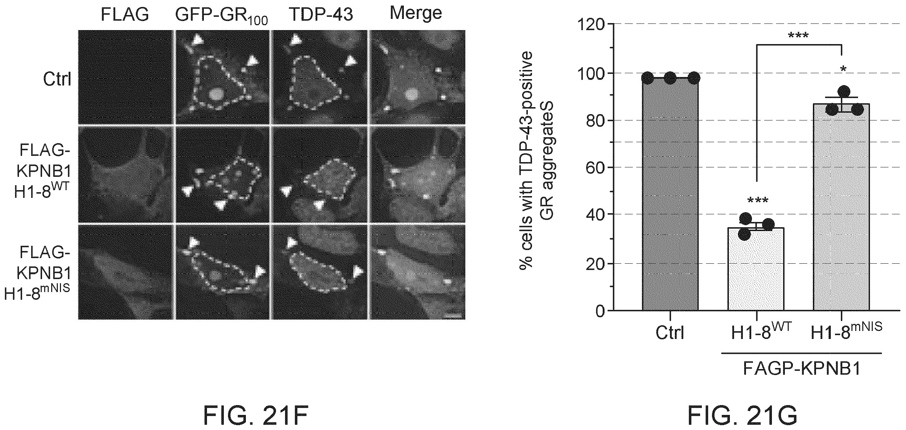

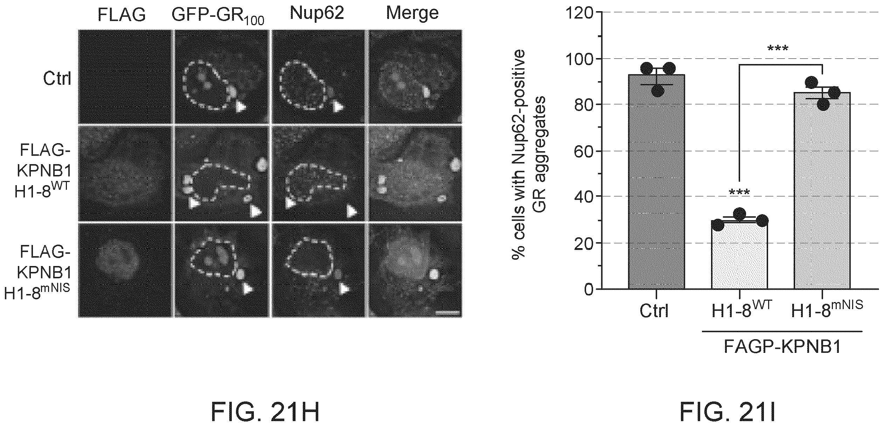

Figures 21A - 21I: KPNB 1-mediated reduction of TDP-CTF and TDP-43 aggregates depends on its FG-Nup interaction domain. Figure 21A) Schematic domain structure of KPNB1. KPNB1 is comprised of 19 HEAT repeats (H1-19). Ran^{GTP} and importin-α interact with H1-8 and H8-19, respectively. FG-Nups bind to two regions of KPNB1, H5-7 and H14-16. Figure 21B) Lysates from HEK293T cells expressing GFP, GFP-KPNB1 full-length (FL), N-terminal fragments of KPNB1 (H1-9...H1) or the C-terminal fragment of KPNB1 (H10-19) were subjected to immunoprecipitation with GFP-Trap magnetic beads. Whole cell lysates (input) and immunoprecipitates (IP) were subjected to western blot analysis using indicated antibodies. KPNB1 H1-8, the smallest fully active KPNB1 fragment in reducing TDP-43 aggregation, strongly interacts with FG-Nups and Ran, and weakly with importin-α1 in comparison with full-length KPNB1. Figure 21C) (Top) Schematic domain structure of KPNB1 H1-8^{mNIS} harboring four missense mutations (I178A, F217A, Y255A, I263R) in the nucleoporin-interacting site (NIS). (Bottom) Lysates from HEK293T cells expressing GFP, GFP-KPNB1 H1-8^{WT} or H1-8^{mNIS} were subjected to immunoprecipitation with GFP-Trap magnetic beads. Whole cell lysates (input) and immunoprecipitates (IP) were subjected to western blot analysis using indicated antibodies. KPNB1 H1-8^{mNIS} shows strongly reduced interaction with FG-Nups. Figure 21D) Immunofluorescence (IF) of SH-SY5Y cells co-expressing mCherry or mCherry-TDP-CTF with GFP-KPNB1 H1-8^{WT} or H1-8^{mNIS}. KPNB1 H1-8^{mNIS} does not reduce cytoplasmic TDP-CTF aggregates. Hoechst staining was used to outline nuclei. Scale bar: 5 µm. Figure 21E) Western blot analysis and quantification of insoluble mCherry-TDP-CTF in SH-SY5Y cells expressing GFP, GFP-KPNB1 H1-8^{WT} or H1-8^{mNIS}. KPNB1 H1-8^{WT} strongly decreased insoluble TDP-CTF levels, whereas KPNB1 H1-8^{mNIS} did not. β-tubulin was used as a loading control. Statistical analysis was performed using one-way ANOVA and Bonferroni's post hoc test (**p<0.01, ***p<0.001, n=4). Figure 21F) IF of HEK293T cells co-expressing GFP-(GR)₁₀₀ with an empty plasmid (ctrl), FLAG-tagged KPNB1 H1-8^{WT} or H1-8^{mNIS} and stained for endogenous TDP-43. Unlike KPNB1 H1-8^{mNIS}, KPNB1 H1-8^{WT} prevents the sequestration of endogenous TDP-43 in cytoplasmic GR aggregates. Arrowheads point to cytoplasmic GR aggregates. Hoechst staining was used to outline nuclei. Scale bar: 5 µm. Figure 21G) Quantification of the percentage of cells with cytoplasmic GR aggregates positive for endogenous TDP-43. KPNB1 H1-8^{WT} significantly reduced the number of TDP-43-positive GR aggregates, while KPNB1 H1-8^{mNIS} had no effect. Statistical analysis was performed using one-way ANOVA and Bonferroni's post hoc test (three independent experiments; *p<0.05, ***p<0.001. n=50-52 cells per group). Figure 21H) IF of HEK293T cells co-expressing GFP-(GR)₁₀₀ with an empty plasmid (ctrl), FLAG-tagged KPNB1 H1-8^{WT} or H1-8^{mNIS} and stained for endogenous Nup62. Unlike KPNB1 H1-8^{mNIS}, KPNB1 H1-8^{WT} prevents the sequestration of endogenous Nup62 in cytoplasmic GR aggregates. Arrowheads point to cytoplasmic GR aggregates. Hoechst staining was used to outline nuclei. Scale bar: 5 µm. Figure 21I) Quantification of the percentage of cells with cytoplasmic GR aggregates positive for endogenous Nup62. KPNB1 H1-8^{WT} significantly reduced the number of Nup62-positive GR aggregates, while KPNB1 H1-8^{mNIS} had no effect. Statistical analysis was performed using one-way ANOVA and Bonferroni's post hoc test (three independent experiments; ***p<0.001. n=50-51 cells per group).

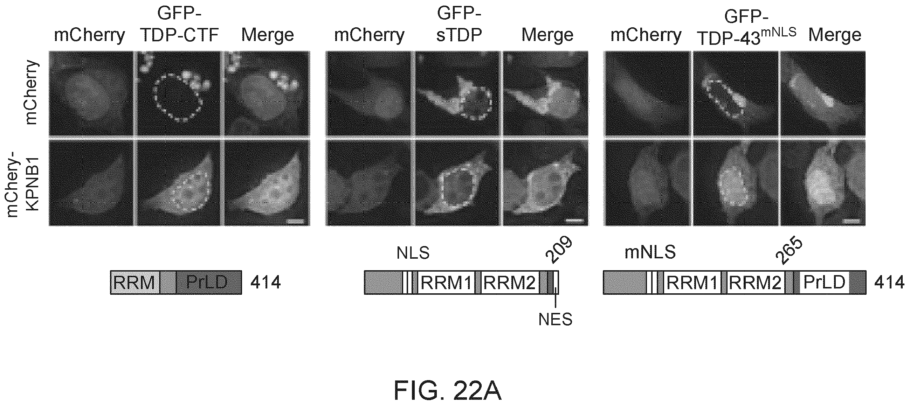

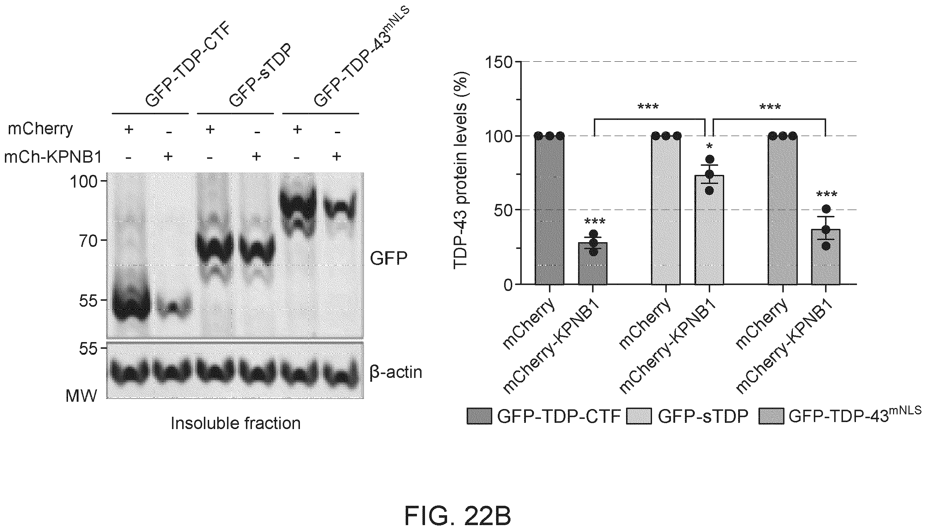

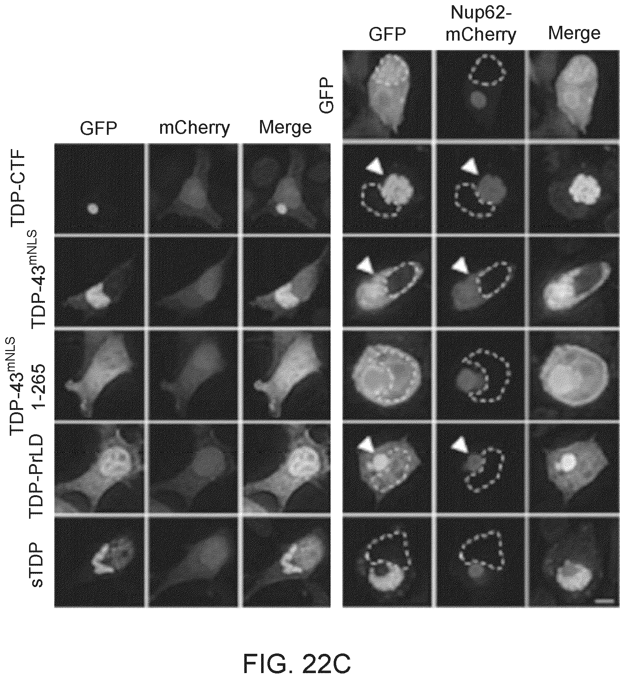

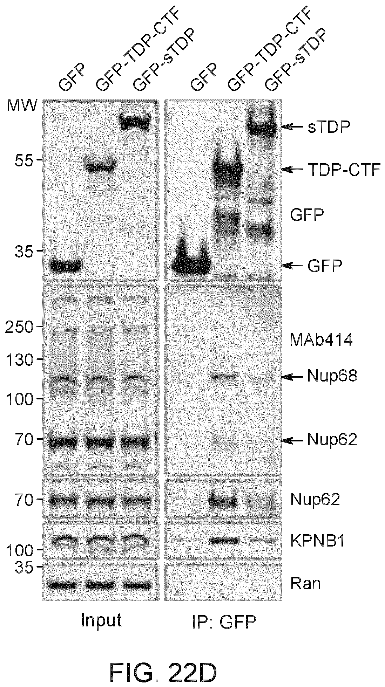

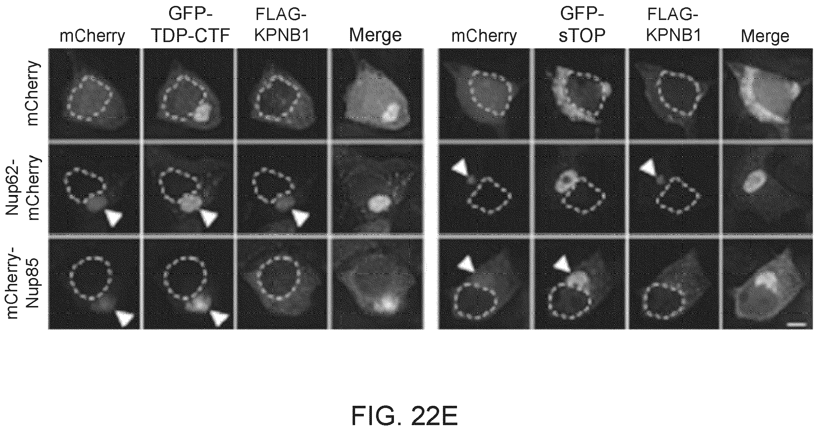

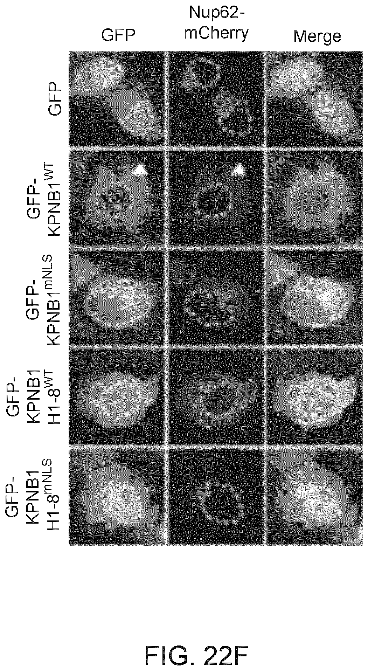

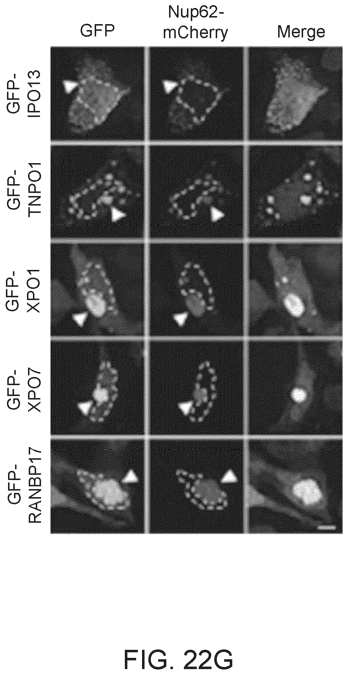

Figures 22A - 22G: Nup62 co-aggregates with TDP-43 PrLD and recruits KPNB1 to facilitate reduction of aggregation. Figure 22A) Immunofluorescence (IF) of HEK293T cells co-expressing GFP-tagged TDP-CTF, sTDP or TDP-43^{mNLS} with mCherry or mCherry - KPNB1. KPNB1 abolished TDP-CTF and TDP-43^{mNLS} aggregates, whereas sTDP aggregates were only reduced in size. Hoechst staining was used to outline nuclei. Scale bar: 5 µm. Figure 22B) Western blot analysis and quantification of insoluble GFP-tagged TDP-CTF, sTDP and TDP-43^{mNLS} in HEK293T cells expressing mCherry or mCherry-KPNB1. KPNB1 strongly reduced insoluble TDP-CTF and TDP-43^{mNLS} protein levels, and sTDP levels to a lesser extent. β-actin was used as a loading control. Statistical analysis was performed using two-way ANOVA and Bonferroni's post hoc test (*p<0.05, ***p<0.001, n=3). Figure 22C) IF of HEK293T cells co-expressing mCherry or Nup62-mCherry with GFP or GFP-tagged TDP-CTF^{WT}, TDP-43^{mNLS}, TDP-43^{mNLS} 1-265, TDP-PrLD or sTDP. TDP-CTF^{WT} and TDP-43^{mNLS} strongly colocalize with Nup62 via their PrLD, whereas sTDP lacking this domain does not. Arrowheads indicate co-aggregation. Hoechst staining was used to outline nuclei. Scale bar: 5 µm. Figure 22D) Lysates from HEK293T cells expressing GFP, GFP-TDP-CTF or GFP-sTDP were subjected to immunoprecipitation with GFP-Trap magnetic beads. Whole cell lysates (input) and immunoprecipitates (IP) were subjected to western blot analysis using indicated antibodies. Unlike sTDP, TDP-CTF strongly interacts with endogenous Nup62 and KPNB1. Figure 22E) IF of HEK293T cells co-expressing mCherry/Nup62-mCherry/mCherry-Nup85, GFP-TDP-CTF/GFP-sTDP and FLAG-KPNB 1. Nup62 recruits KPNB1 to TDP-CTF but not sTDP aggregates. Nup85 which lacks FG repeats does not recruit KPNB 1 to either TDP-CTF or sTDP, although it colocalizes with both aggregates. Arrowheads indicate co-aggregation. Hoechst staining was used to outline nuclei. Scale bar: 5 µm. Figure 22F) IF of HEK293T cells co-expressing Nup62-mCherry with GFP or GFP-tagged KPNB1^{WT}, KPNB1^{mNIS}, KPNB1 H1-8^{WT} or H1-8^{mNIS}. KPNB1^{WT} associated with Nup62 aggregates and strongly reduced their size, whereas KPNB1 H1-8^{WT} rendered Nup62 completely soluble. Arrowheads indicate co-aggregation. Hoechst staining was used to outline nuclei. Scale bar: 5 µm. Figure 22G) IF of HEK293T cells co-expressing Nup62-mCherry with GFP or GFP-tagged IPO13, TNPO1, XPO1, XPO7 or RANBP17. IPO13 associated with Nup62 aggregates and strongly reduced their size. TNPO1 and exportins co-aggregated with Nup62 but only TNPO1 slightly reduced individual aggregate size. Arrowheads indicate co-aggregation. Hoechst staining was used to outline nuclei. Scale bar: 5 µm.

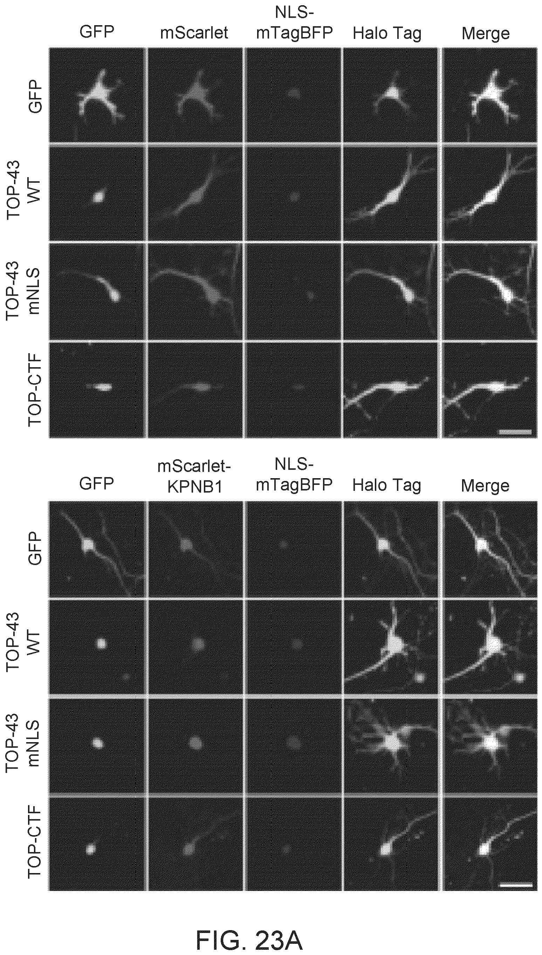

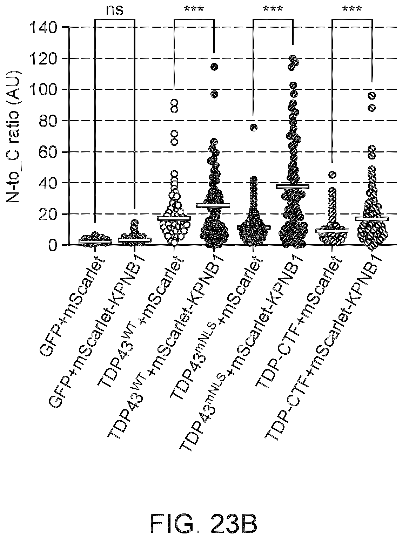

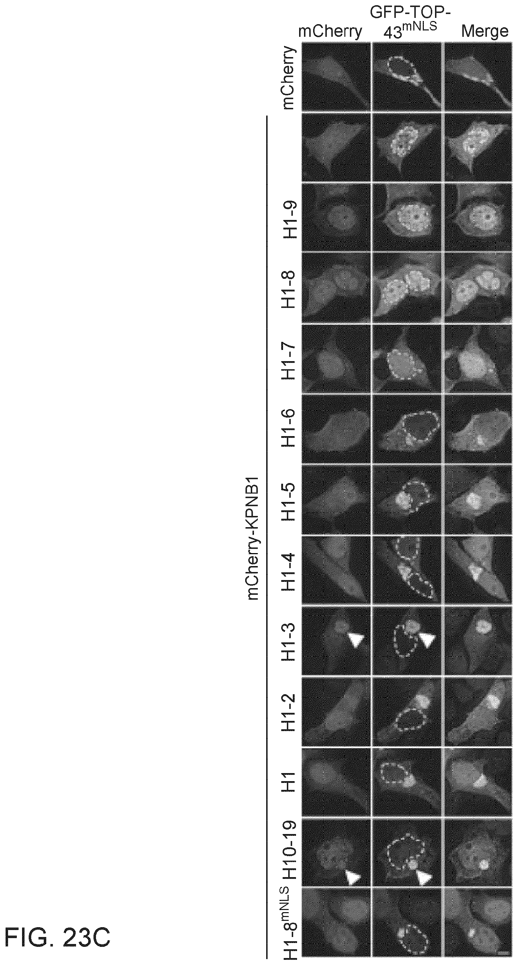

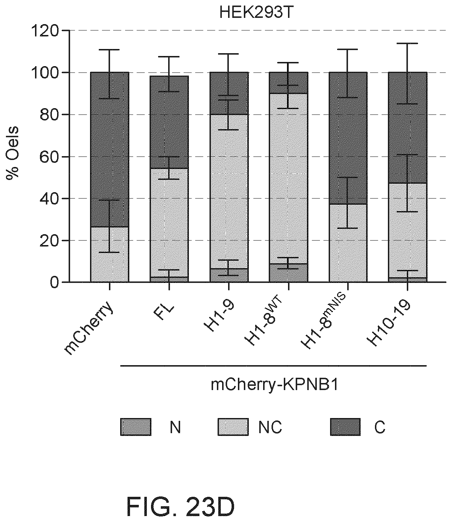

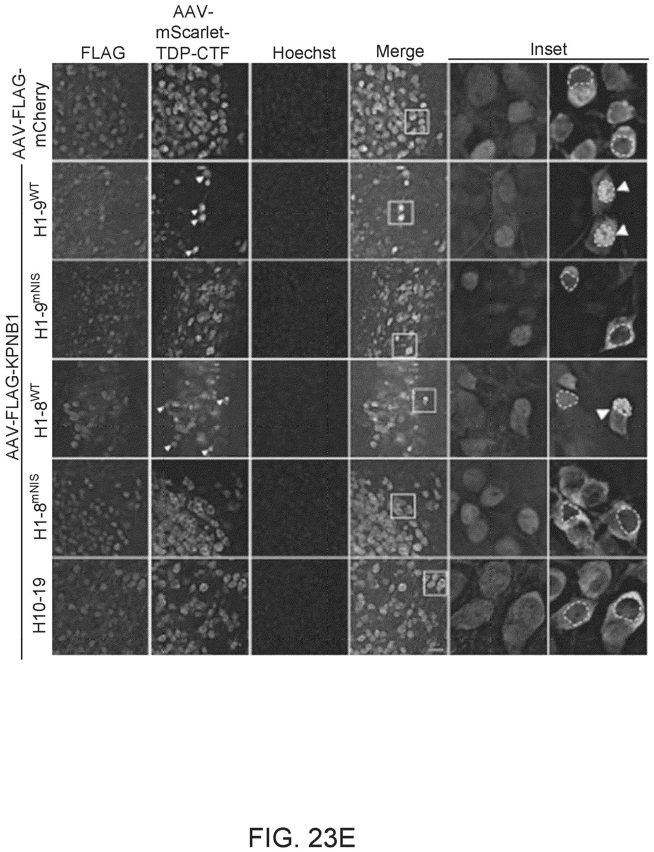

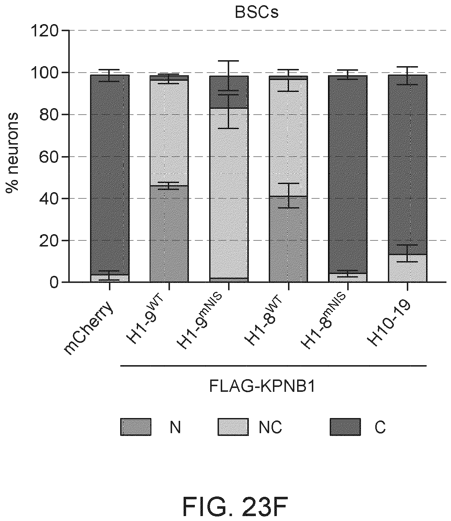

Figures 23A - 23F: KPNB1 promotes nuclear localization of TDP-43^{mNLS} in primary neurons and mouse brain tissue. Figure 23A) Immunofluorescence (IF) of primary cortical neurons co-expressing NLS-mTagBFP (nuclear marker), HaloTag (cell marker), GFP or GFP-tagged TDP-43^{WT}/TDP-43^{mNLS}/TDP-CTF and mScarlet/mScarlet-KPNB1. KPNB1 increased the nuclear localization of TDP-43^{mNLS} and TDP-CTF. Scale bar: 25 µm. Figure 23B) Quantitative analysis of the N-to-C ratio of GFP or GFP-tagged TDP-43^{WT}, TDP-43^{mNLS} or TDP-CTF in primary neurons expressing mScarlet or mScarlet-KPNB1 72 hours post-transfection. Statistical analysis was performed using one-way ANOVA and Bonferroni's post hoc test (**p<0.01, ***p<0.001, n=52-264 neurons per group). Figure 23C) IF of HEK293T cells co-expressing GFP-TDP-43^{mNLS} with mCherry or mCherry-tagged full-length KPNB1 (FL) or its fragments. KPNB1 FL, H1-9 and H1-8 constructs increase the nuclear localization of TDP-43^{mNLS}. Arrowheads point to co-aggregation. Hoechst staining was used to outline nuclei. Scale bar: 5 µm. Figure 23D) Quantification of the percentage of HEK293T cells exhibiting nuclear (N), nucleocytoplasmic (NC) or cytoplasmic (C) GFP-TDP-43^{mNLS} distribution upon expression of different mCherry-KPNB1 constructs. Statistical analysis was performed using two-way ANOVA and Bonferroni's post hoc test (three independent experiments; n=66-81 cells per group; statistics are summarized in Table 8). Figure 23E) IF of mouse brain slice cultures (DIV12) co-expressing AAV-GFP-TDP-43^{mNLS} with AAV-FLAG-mCherry or AAV-FLAG-KPNB1 H1-9^{WT}, H1-9^{mNIS}, H1-8^{WT}, H1-8^{mNIS} or H10-19. KPNB1 H1-9^{WT} and H1-8^{WT} increase the nuclear localization of TDP-43^{mNLS}, whereas NIS mutations abolished this effect. Hoechst staining was used to outline nuclei. Scale bar: 50 µm. Figure 23F) Quantification of the percentage of neurons exhibiting nuclear (N), nucleocytoplasmic (NC) or cytoplasmic (C) GFP-TDP-43^{mNLS} distribution upon expression of different AAV-KPNB1 constructs. Statistical analysis was performed using two-way ANOVA and Bonferroni's post hoc test (three independent experiments; n=150-154 neurons per group; statistics are summarized in Table 8).

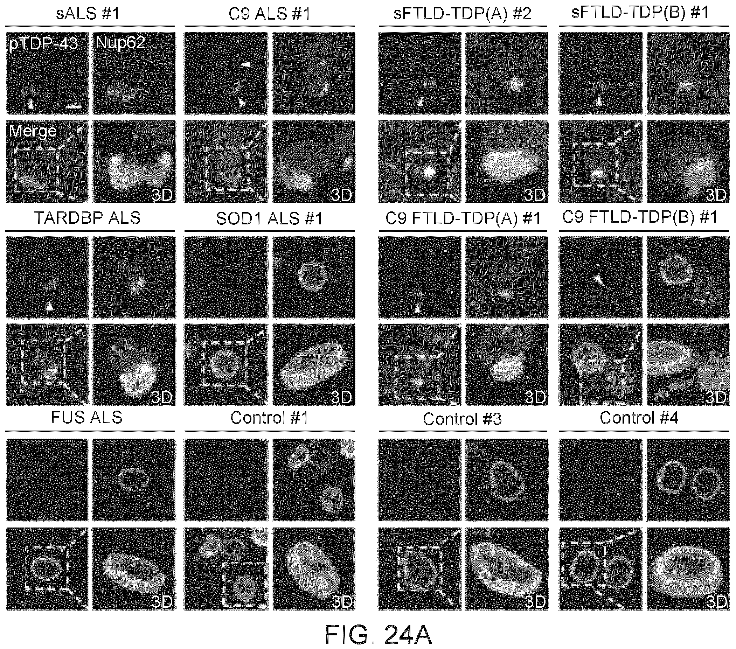

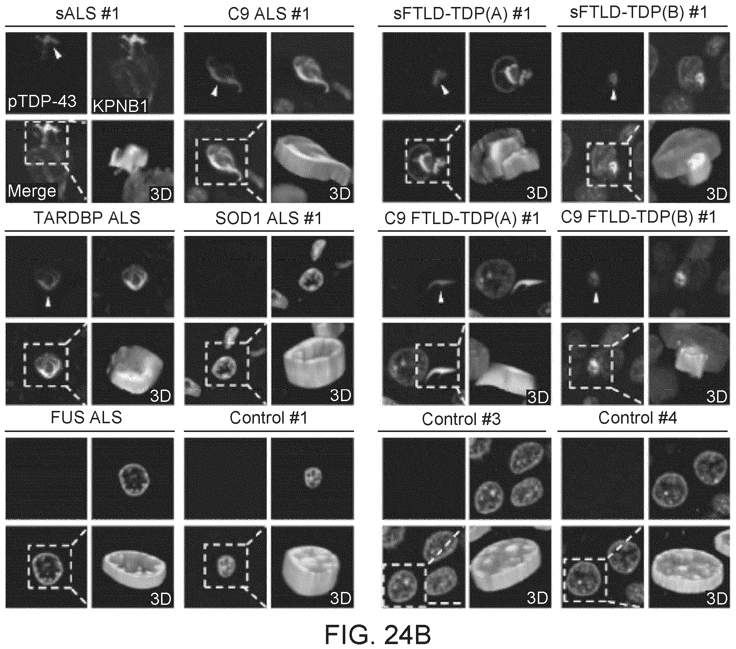

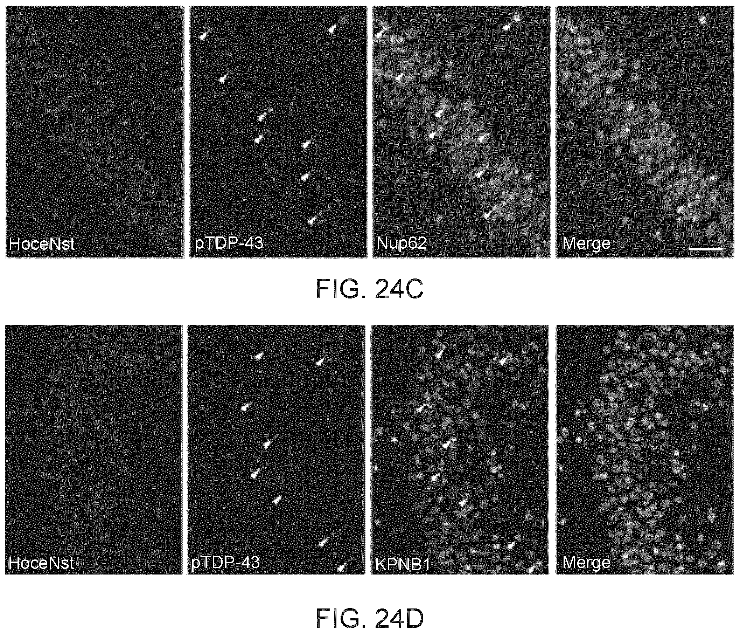

Figures 24A - 24D Nup62 and KPNB1 colocalize with pathological pTDP-43 aggregates in human post-mortem brain tissue. Co-immunofluorescence (IF) staining was conducted in fixed spinal cord or posterior hippocampus of neuropathologically diagnosed ALS and FTLD cases, respectively, and including unaffected control tissue. Figures 24A and 24B) High resolution co-IF staining of pTDP-43 and Nup62 or KPNB1 with Hoechst. Each image is shown as a panel of projected optical sections from each z-series for red and green channels and merged with Hoechst DNA staining. Volume rendered z-series of all channels are shown as the 3D inset. Scale bar: 5 µm. Figures 24C and 24D) Hippocampal tile scan images of Nup62 or KPNB 1 co-IF with pTDP-43 aggregates in case sFTLD-B#1. Scale bar: 50 µm.

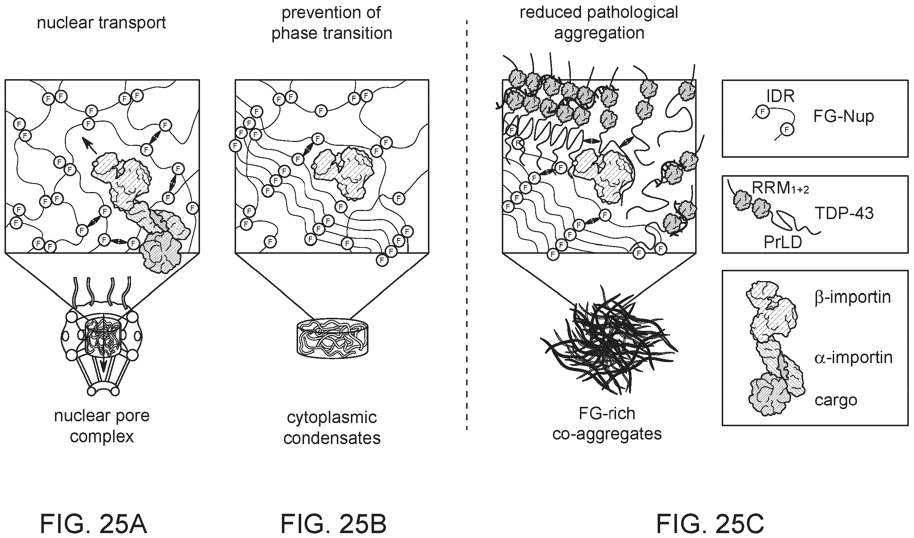

Figures 25A - 25C: A model for NIRs as molecular chaperones for FG-Nup-containing assemblies. Figure 25A) NIRs facilitate nuclear transport by disengaging the hydrophobic interactions between the FG-repeats that form the hydrogel barrier within the nuclear pore complexes. Figure 25B) NIRs prevent detrimental phase transition of FG-Nups and other aggregation-prone proteins that form cytoplasmic foci under cellular crowding conditions. Figure 25C) NIRs are recruited into pathological FG-Nups and TDP-43 co-aggregates in an NLS-independent manner, counter their aberrant phase transition, and relocate TDP-43 into the nucleus.

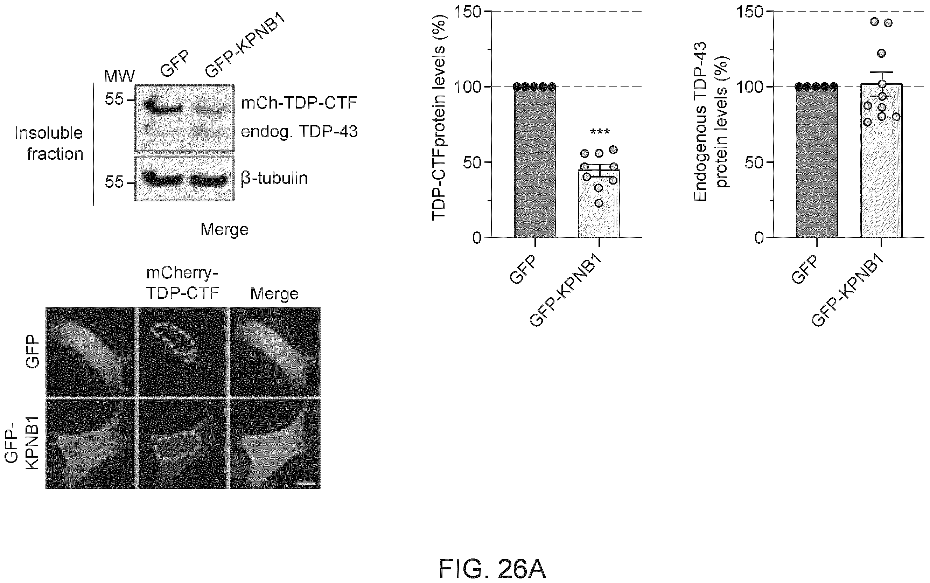

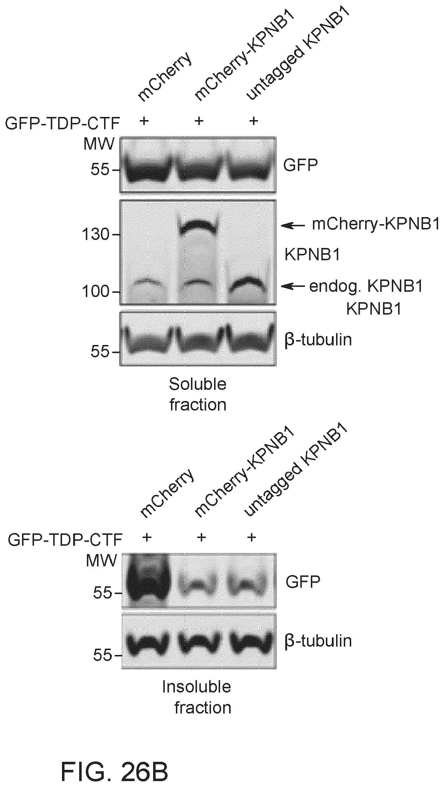

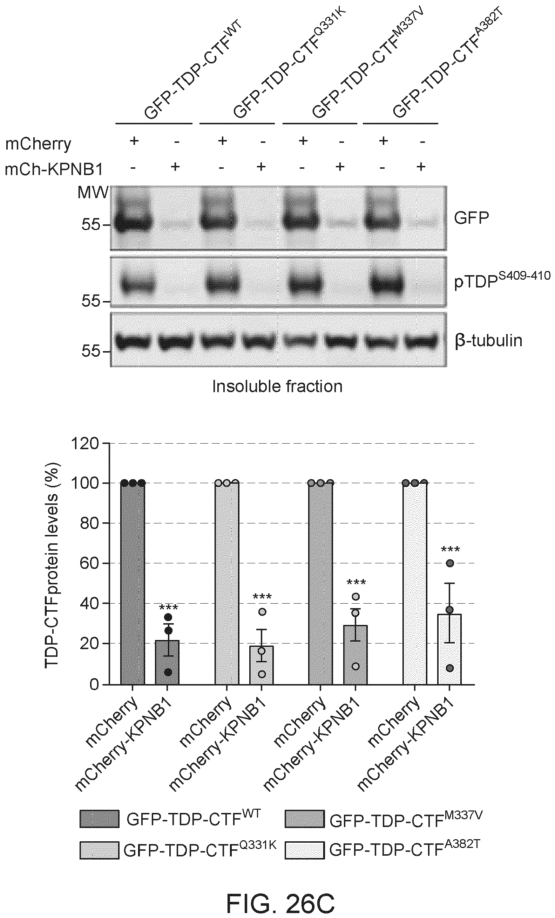

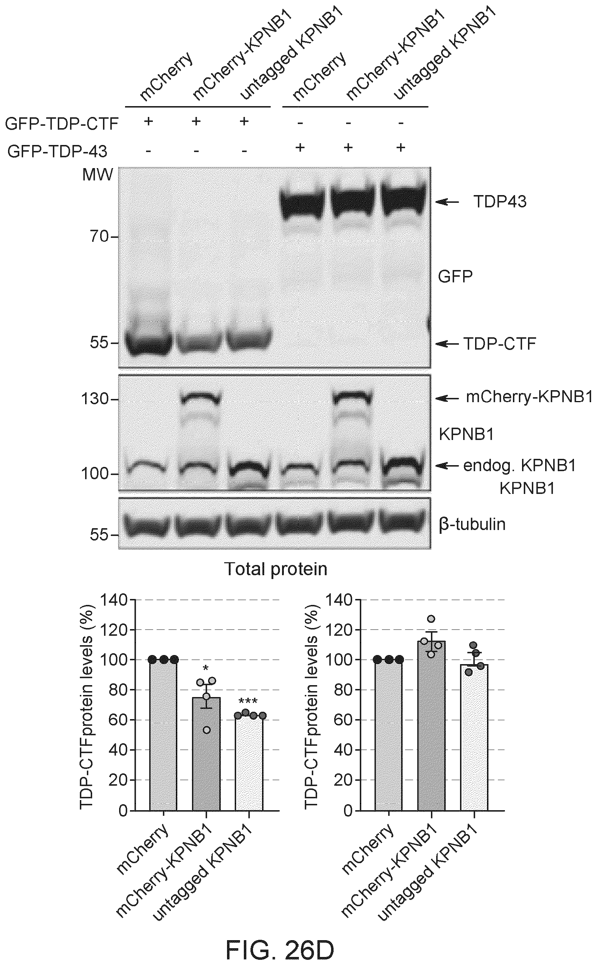

Figures 26A - 26E: KPNB1 specifically reduces insoluble protein levels of TDP-CTF. Figure 26A) (Top) Western blot analysis and quantification of insoluble mCherry-TDP-CTF and endogenous TDP-43 protein levels in SH-SY5Y cells expressing GFP or GFP-KPNB1. KPNB1 significantly reduced insoluble TDP-CTF levels, while endogenous TDP-43 levels were unaffected. β-tubulin was used as a loading control. Statistical analysis was performed using Student's *t*-test (***p<0.001, n=10). (Bottom) Immunofluorescence of SH-SY5Y cells co-expressing mCherry-TDP-CTF with GFP or GFP-KPNB1. mCherry-TDP-CTF forms cytoplasmic aggregates but shows diffuse localization in the presence of GFP-KPNB1. Hoechst staining was used to outline nuclei. Scale bar: 5 µm. Figure 26B) Western blot analysis of soluble and insoluble GFP-TDP-CTF co-expressed with mCherry, mCherry-KPNB1 or untagged KPNB1 in HEK293T cells. KPNB1 only reduces insoluble TDP-CTF levels. β-tubulin was used as a loading control. Figure 26C) Western blot analysis and quantification of insoluble GFP-tagged TDP-CTF^{WT}, TDP-CTF^{Q331K}, TDP-CTF^{M337V} and TDP-CTF^{A382T} in HEK293T cells expressing mCherry or mCherry-KPNB1. KPNB1 similarly reduced insoluble wild-type and ALS-derived mutant TDP-CTF protein levels. β-tubulin was used as a loading control. Statistical analysis was performed using Student's *t-*test (***p<0.001, n=3). Figure 26D) Western blot analysis of total protein levels of GFP-tagged TDP-CTF or TDP-43^{WT} in HEK293T cells expressing mCherry, mCherry-KPNB1 or untagged KPNB 1. Cells were lysed in 7M urea buffer to collect total protein. KPNB 1 reduces total protein levels of TDP-CTF, but not TDP-43^{WT}. β-tubulin was used as a loading control. Statistical analysis was performed using one-way ANOVA and Bonferroni's post hoc test (*p<0.05, ***p<0.001, n=4). e, *TDP-CTF* and *TDP-43* transcript levels were quantified in HEK293T cells co-expressing GFP-tagged TDP-CTF or TDP-43 with mCherry, mCherry-KPNB 1, or untagged KPNB1. *GAPDH* was used as a reference to normalize the transcript levels. Statistical analysis was performed using one-way ANOVA and Bonferroni's post hoc test (n=3).

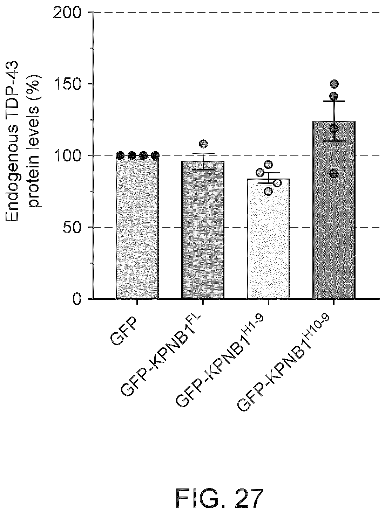

Figure 27: Expression of full-length KPNB1 and its N-terminal half do not reduce endogenous TDP-43 levels. Quantification of endogenous insoluble TDP-43 protein levels in SH-SY5Y cells co-expressing mCherry-TDP-CTF with GFP, GFP-KPNB1 full-length (FL), H1-9 or H10-19. β-tubulin was used as a loading control. Statistical analysis was performed using one-way ANOVA and Bonferroni's post hoc test (*p<0.05, n=4).

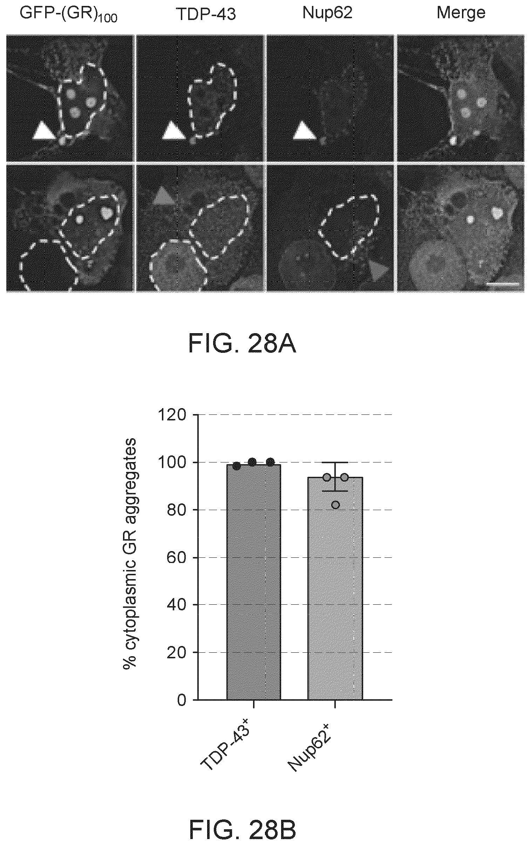

Figures 28A - 28B: Cytoplasmic GFP-(GR)₁₀₀ aggregates sequester endogenous TDP-43 and Nup62. Figure 28A) Immunofluorescence of HEK293T cells expressing GFP-(GR)₁₀₀ and stained for endogenous TDP-43 and Nup62. Only cytoplasmic GFP-(GR)₁₀₀ aggregates are immunopositive for TDP-43 and Nup62. Nuclear GFP-(GR)₁₀₀ aggregates render TDP-43 staining diffuse (purple arrowhead), while Nup62 accumulates as puncta in the cytoplasm (red arrowhead). White arrowheads point to cytoplasmic GR aggregates. Hoechst staining was used to outline nuclei. Scale bar: 5 µm. Figure 28B) Quantification of the percentage of cytoplasmic GFP-(GR)₁₀₀ aggregates positive for TDP-43 or Nup62 in HEK293T cells. Almost all cells are positive for both proteins. Statistical analysis was performed using Student's *t*-test (three independent experiments; n=239-249 cells per group).

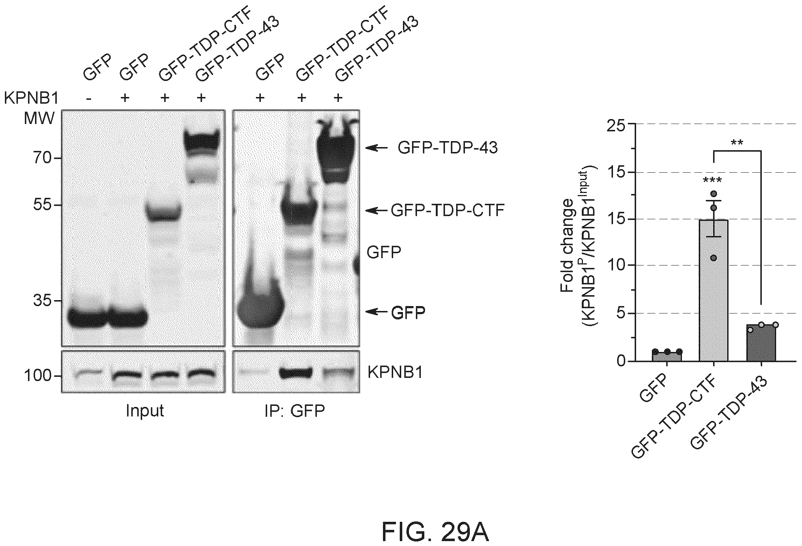

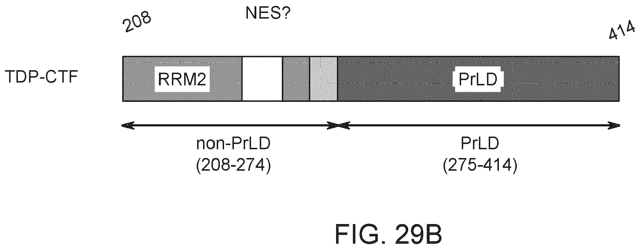

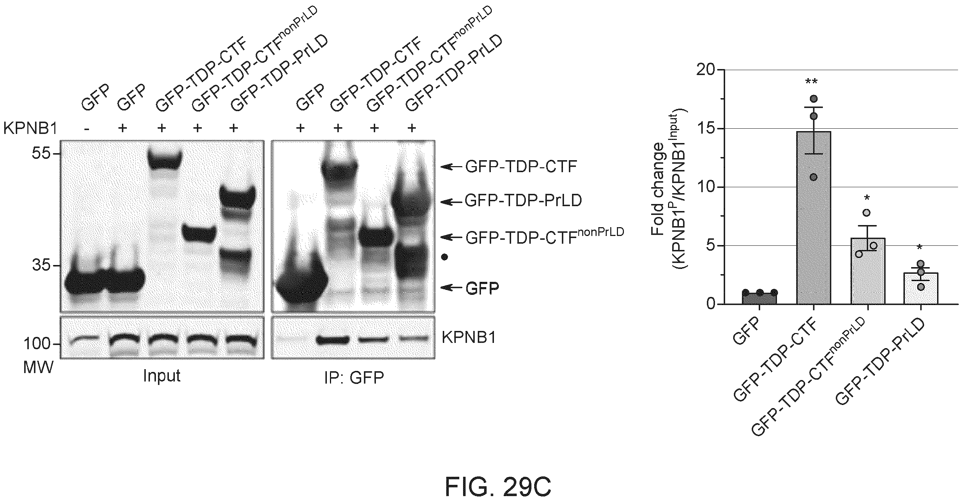

Figures 29A - 29C: Untagged KPNB1 interacts with both the RRM2 and PrLD of TDP-CTF. Figure 29A) (Left) Lysates from HEK293T cells expressing GFP alone, or untagged KPNB1 with GFP, GFP-TDP-CTF or GFP-TDP43^{WT} were subjected to immunoprecipitation with GFP-Trap magnetic beads. Whole cell lysates (input) and immunoprecipitates (IP) were subjected to western blot analysis using indicated antibodies. (Right) Quantification of relative KPNB1 levels in IP normalized by KPNB1 levels in input. Statistical analysis was performed using one-way ANOVA and Bonferroni's post hoc test (**p<0.01, ***p<0.001, n=3). Figure 29B) Schematic domain structure of TDP-CTF full-length (208-414). TDP-CTF contains part of the RRM2 (non-PrLD, 208-274) and the C-terminal PrLD (275-414). Figure 29C) (Left) Lysates from HEK293T cells expressing GFP alone, or untagged KPNB1 with GFP, GFP-TDP-CTF, GFP-TDP-CTF^{non-PrLD} or GFP-TDP-PrLD were subjected to immunoprecipitation with GFP-Trap magnetic beads. Whole cell lysates (input) and immunoprecipitates (IP) were subjected to western blot analysis using indicated antibodies. (Right) Quantification of relative KPNB1 levels in IP normalized by KPNB1 levels in input. Statistical analysis was performed using one-way ANOVA and Bonferroni's post hoc test (***p<0.001, n=3).

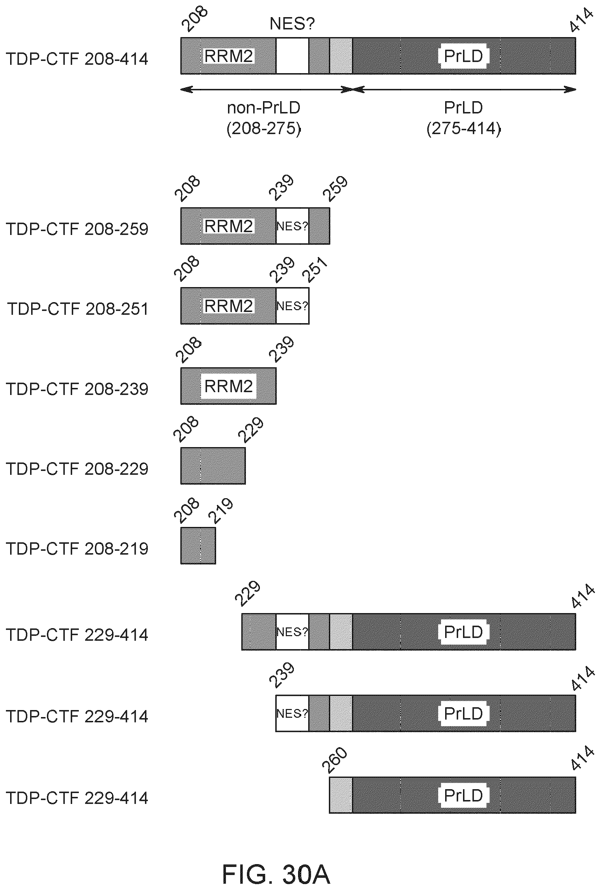

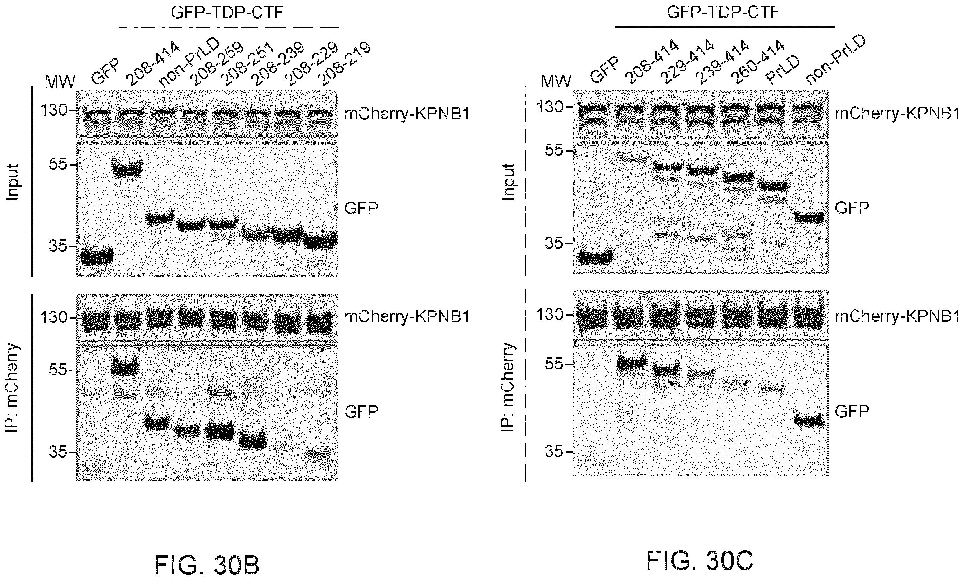

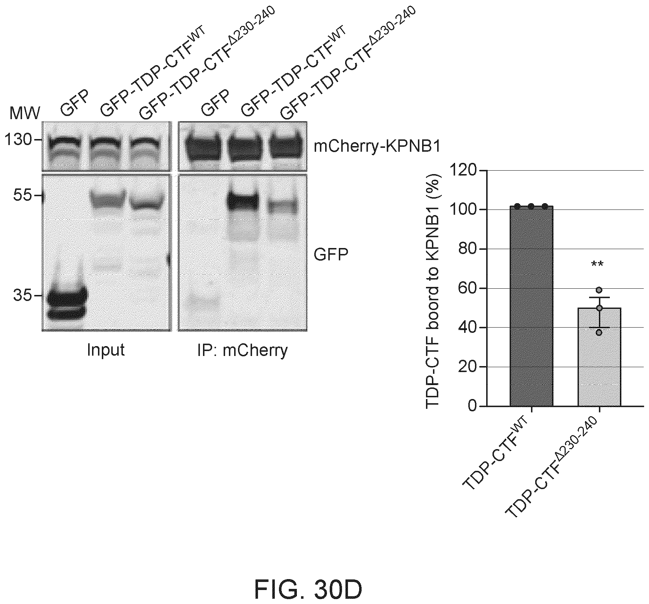

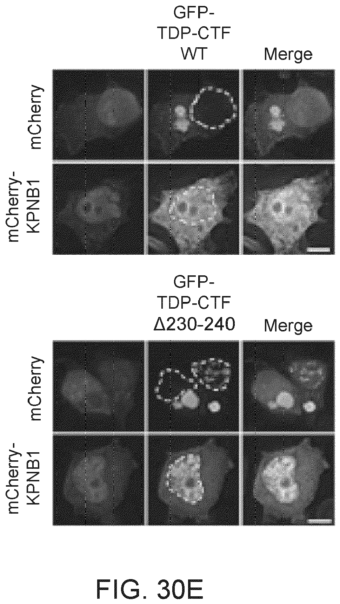

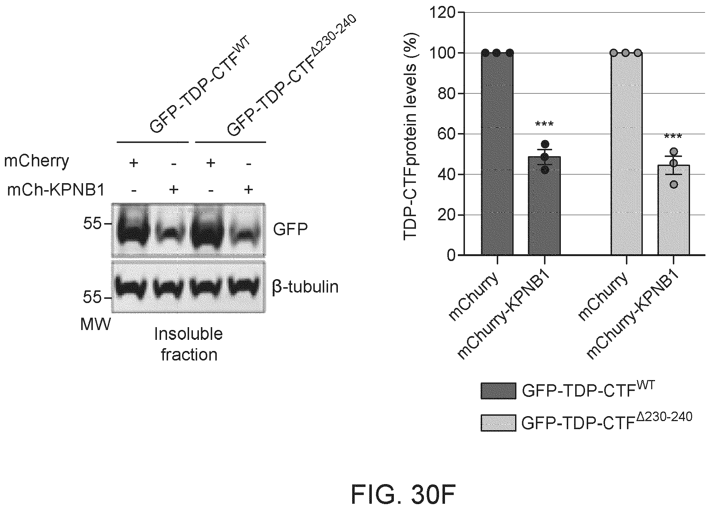

Figures 30A - 30F: KPNB1 reduces TDP-CTF aggregation independent of its interaction with the RRM2 region. Figure 30A) Schematic domain structures of full-length TDP-CTF (208-414), and its C-terminal and N-terminal deletion constructs used for co-IP experiments. Figure 30B) Lysates from HEK293T cells co-expressing mCherry-KPNB1 with GFP, GFP-TDP-CTF or GFP-TDP-CTF C-terminal deletion constructs were subjected to immunoprecipitation with RFP-Trap magnetic beads. Whole cell lysates (input) and immunoprecipitates (IP) were subjected to western blot analysis using indicated antibodies. Deletion of amino-acids 230-240 in TDP-CTF reduced its binding to KPNB1. Figure 30C) Lysates from HEK293T cells co-expressing mCherry-KPNB1 with GFP, GFP-TDP-CTF or GFP-TDP-CTF N-terminal deletion constructs were subjected to immunoprecipitation with RFP-Trap magnetic beads. Whole cell lysates (input) and immunoprecipitates (IP) were subjected to western blot analysis using indicated antibodies. Deletion of amino-acids 230-240 in TDP-CTF reduces its binding to KPNB1. TDP-PrLD weakly interacts with KPNB1. Figure 30D) (Left) Lysates from HEK293T cells co-expressing mCherry-KPNB1 with GFP, GFP-TDP-CTF^{WT} or GFP-TDP-CTF^{Δ230-240} were subjected to immunoprecipitation with RFP-Trap magnetic beads. Whole cell lysates (input) and immunoprecipitates (IP) were subjected to western blot analysis using indicated antibodies. KPNB1 interacts less with TDP-CTF^{Δ230-240}. (Right) Quantification of relative GFP levels in IP normalized by GFP levels in input confirm that GFP-TDP-CTF^{Δ230-240} interacts less with mCherry-KPNB 1. Figure 30E) Immunofluorescence of HEK293T cells co-expressing GFP-tagged TDP-CTF^{WT} or TDP-CTF^{Δ230-240} with mCherry or mCherry-KPNB 1. TDP-CTF^{Δ230-240} forms small nuclear and big cytoplasmic aggregates. KPNB1 reduces both TDP-CTF^{WT} and TDP-CTF^{Δ230-240} aggregates, and makes TDP-CTF^{Δ230-240} more nuclear. Hoechst staining was used to outline nuclei. Scale bar: 5 µm. Figure 30F) Western blot analysis and quantification of insoluble GFP-tagged TDP-CTF^{WT} or TDP-CTF^{Δ230-240} in HEK293T cells expressing mCherry or mCherry-KPNB 1. KPNB1 equally reduced insoluble TDP-CTF^{WT} and TDP-CTF^{Δ230-240} protein levels. β-tubulin was used as a loading control. Statistical analysis was performed using two-way ANOVA and Bonferroni's post hoc test (***p<0.001, n=3).

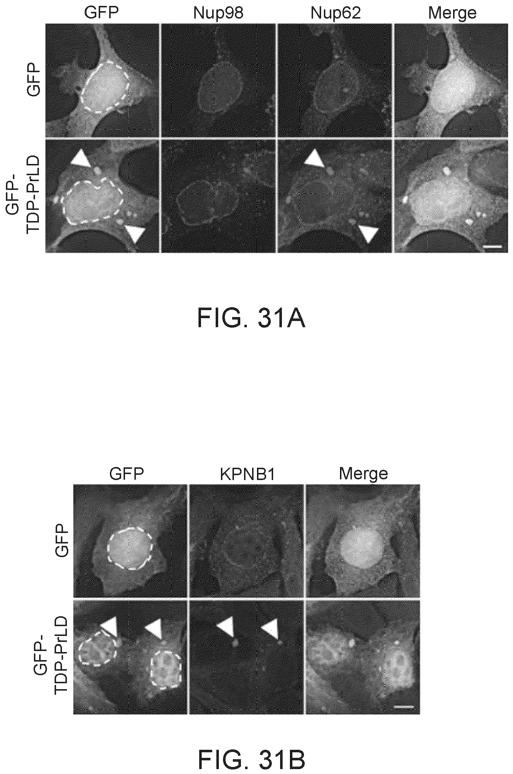

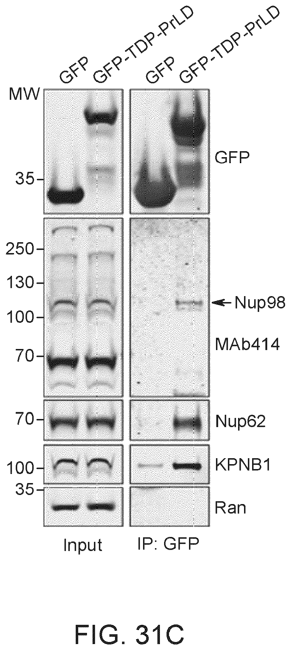

Figures 31A - 31C: The PrLD of TDP-43 interacts with FG-Nups and KPNB1. Figure 31A) Immunofluorescence (IF) of HEK293T cells expressing GFP or GFP-TDP-PrLD and stained for endogenous Nup98 and Nup62. TDP-PrLD forms small cytoplasmic foci positive for Nup62. Figure 31B) IF of HEK293T cells expressing GFP or GFP-TDP-PrLD and stained for endogenous KPNB1. KPNB1 colocalizes with TDP-PrLD foci in the cytoplasm. Hoechst staining was used to outline nuclei. Scale bar: 5 µm. Figure 31C) Lysates from HEK293T cells expressing GFP or GFP-TDP-PrLD were subjected to immunoprecipitation with GFP-Trap magnetic beads. Whole cell lysates (input) and immunoprecipitates (IP) were subjected to western blot analysis using indicated antibodies. The PrLD of TDP-43 interacts with Nup62, Nup98 and KPNB1, but not with Ran.

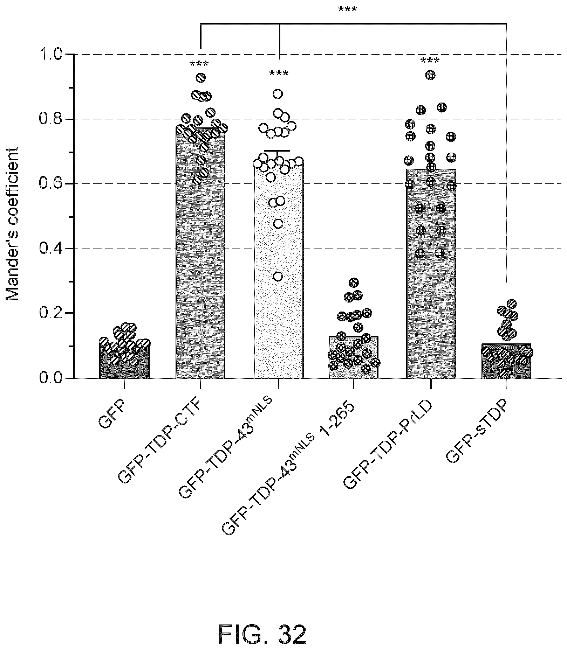

Figure 32: Colocalization of Nup62 with TDP-43 constructs depends on the PrLD. Colocalization between Nup62-mCherry and GFP or GFP-tagged TDP-43 constructs was measured using the Mander's overlap coefficient. Values close to 0 and 1 indicate a weak and strong colocalization, respectively. Statistical analysis was performed using one-way ANOVA and Bonferroni's post hoc test (***p<0.001. n=21-23 cells per group).

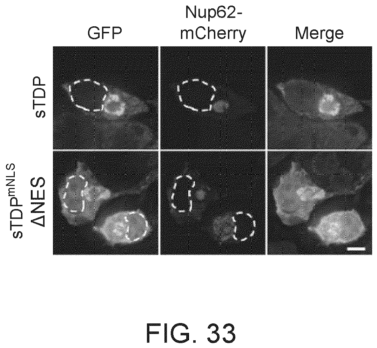

Figure 33: The NES of sTDP does not prevent it from binding to Nup62 aggregates. Immunofluorescence of HEK293T cells co-expressing Nup62-mCherry with either sTDP or sTDP^{mNLS} ΔNES. Nup62 aggregates do not colocalize with sTDP even after mutating its NLS and NES. Hoechst staining was used to outline nuclei. Scale bar: 5 µm.

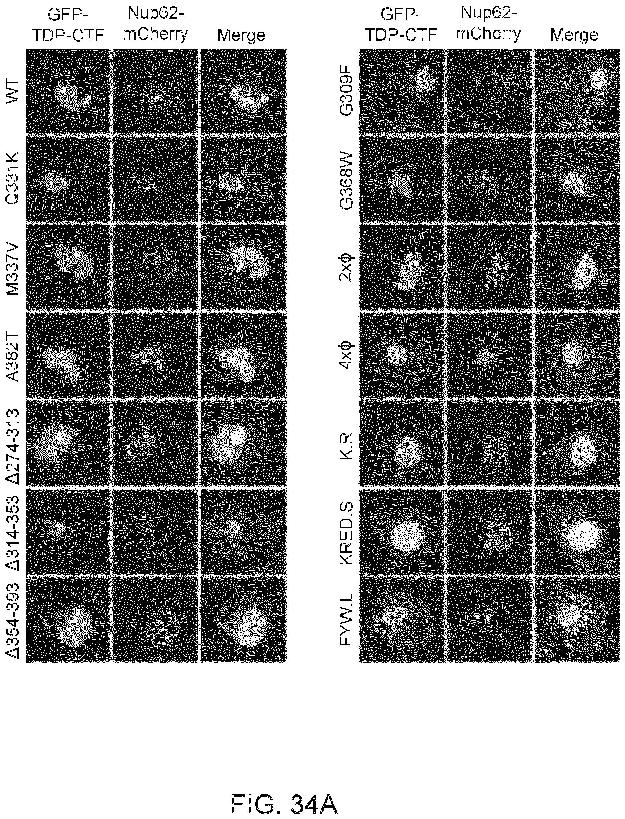

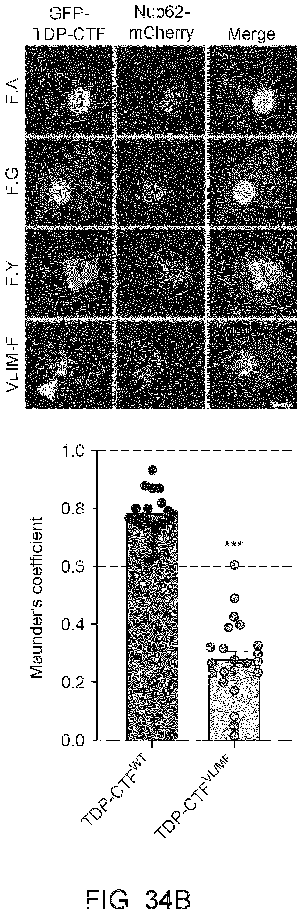

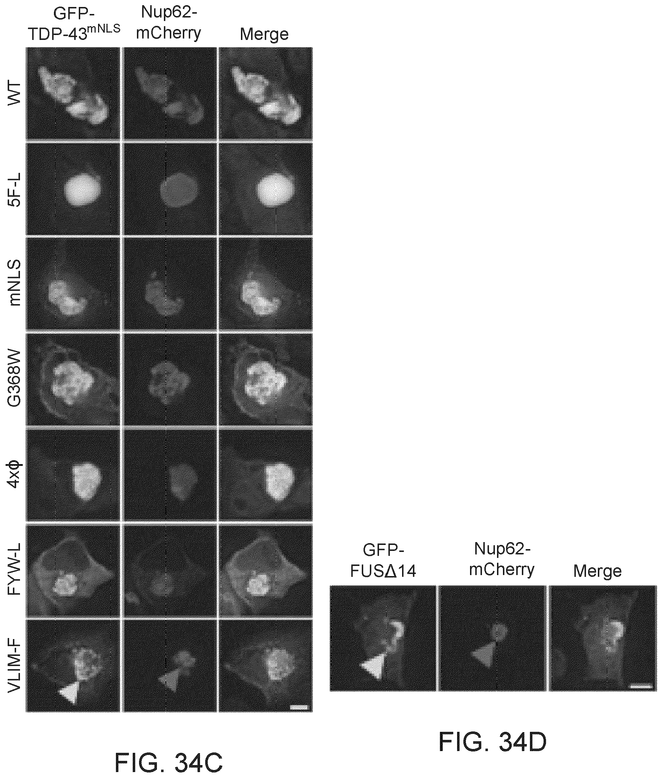

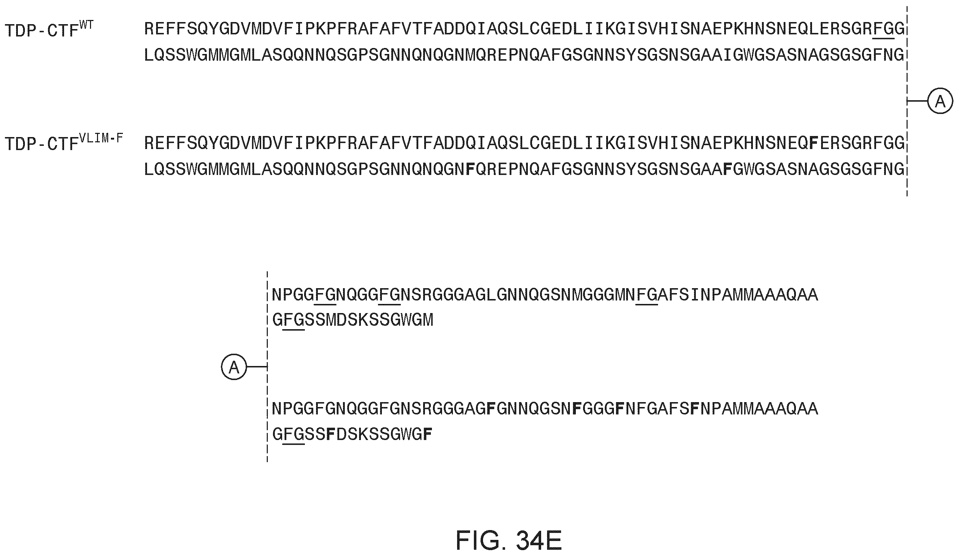

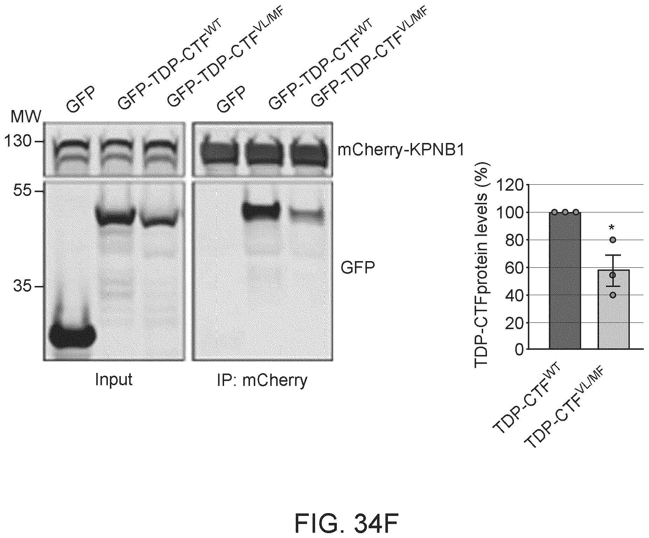

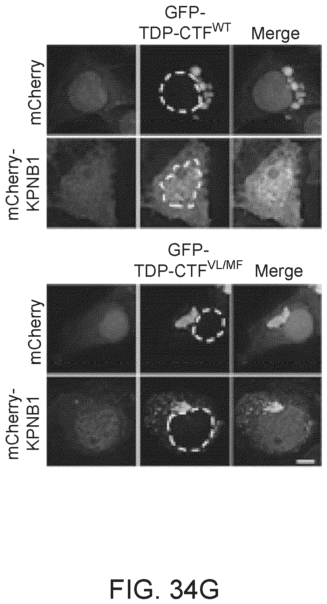

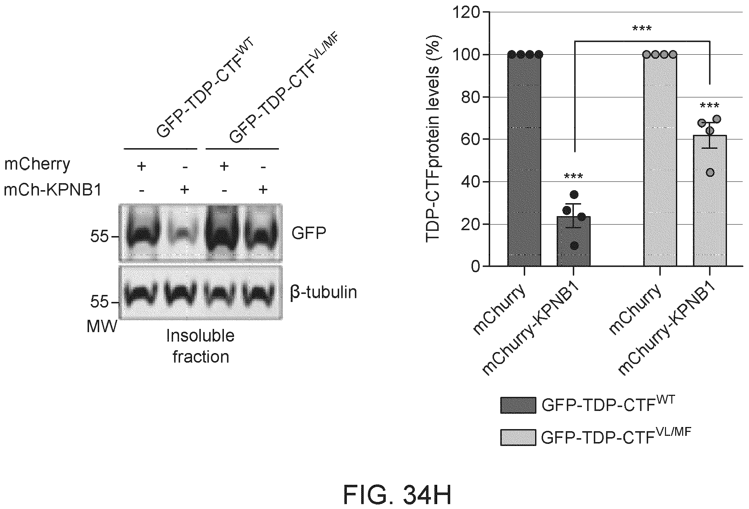

Figures 34A - 34H: TDP-CTF mutations that reduce its association with Nup62 abrogate its interaction and reduced aggregation by KPNB1. Figure 34A) Immunofluorescence (IF) of HEK293T cells co-expressing Nup62-mCherry with either GFP-tagged TDP-CTF^{WT} or TDP-CTF constructs harboring different mutations in the PrLD. All TDP-CTF mutant variants colocalize with Nup62 aggregates, except for TDP-CTF^{VLIM-F} (green arrowhead) which accumulates in close proximity to Nup62 (red arrowhead). Scale bar: 5 µm. Figure 34B) Colocalization between Nup62-mCherry and GFP-tagged TDP-CTF^{WT} or TDP-CTF^{VLIM-F} was measured using the Mander's overlap coefficient. Values close to 0 and 1 indicate a weak and strong colocalization, respectively. Statistical analysis was performed using Student's t-test (***p<0.001, n=21-22 cells per group). Figure 34C) IF of HEK293T cells co-expressing Nup62-mCherry with either GFP-tagged wild-type or mutant TDP-43^{mNLS} constructs. RNA-binding deficient TDP-43^{mNLS} (TDP-43^{mNLS} 5F-L) can still colocalize with Nup62. Only TDP-43^{mNLS} VLIM-F aggregates (green arrowhead) do not contain Nup62 (red arrowhead). Figure 34D) IF of HEK293T cells co-expressing Nup62-mCherry with GFP-FUSΔ14. Cytoplasmic FUS aggregates (green arrowhead) do not colocalize with Nup62 (red arrowhead). Scale bar: 5 µm. Figure 34E) Protein sequences of TDP-CTF^{WT} (SEQ ID NO:78) and TDP-CTF^{VLIM-F} (SEQ ID NO:79) showing FG repeats (underlined) and VLIM-F mutations (in red) in the PrLD. Figure 34F) (Left) Lysates from HEK293T cells co-expressing mCherry-KPNB1 with GFP, GFP-tagged TDP-CTF^{WT} or TDP-CTF^{VLIM-F} were subjected to immunoprecipitation with RFP-Trap magnetic beads. Whole cell lysates (input) and immunoprecipitates (IP) were subjected to western blot analysis using indicated antibodies. Introducing VLIM-F mutations in TDP-CTF significantly reduces its binding to KPNB 1. (Right) Relative GFP levels in IP normalized by GFP levels in input confirm that GFP-TDP-CTF^{VLIM-F} interacts less with mCherry-KPNB 1. Statistical analysis was performed using Student's t-test (*p<0.05, n=3). Figure 34G) IF of HEK293T cells co-expressing GFP-tagged TDP-CTF^{WT} or TDP-CTF^{VLIM-F} with mCherry or mCherry-KPNB 1. KPNB1 only reduces the size TDP-CTF^{VLIM-F} aggregates. Hoechst staining was used to outline nuclei. Scale bar: 5 µm. Figure 34H) Western blot analysis and quantification of insoluble GFP-tagged TDP-CTF^{WT} or TDP-CTF^{VLIM-F} in HEK293T cells expressing mCherry or mCherry-KPNB 1. KPNB1 strongly decreased protein levels of insoluble TDP-CTF^{WT} but not TDP-CTF^{VLIM-F}. β-tubulin was used as a loading control. Statistical analysis was performed using two-way ANOVA and Bonferroni's post hoc test (***p<0.001, n=4).

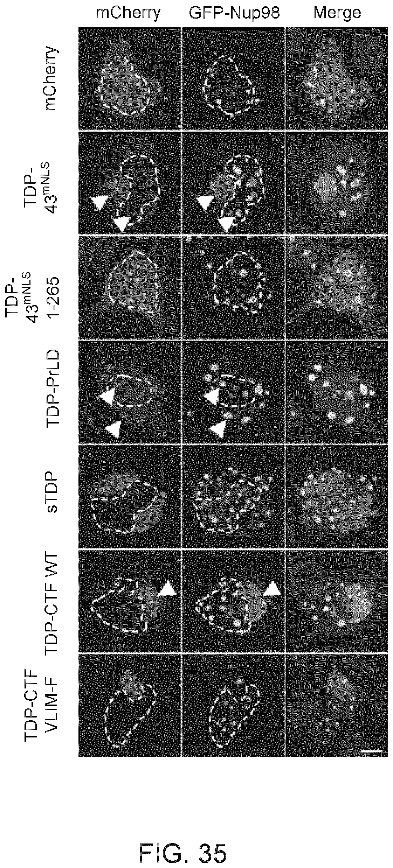

Figure 35: Nup98 strongly associates with TDP-CTF^{WT}, but not TDP-CTF^{VLIM-F} and sTDP. Immunofluorescence of HEK293T cells co-expressing GFP-Nup98 with mCherry or mCherry-tagged TDP-43^{mNLS}, TDP-43^{mNLS} 1-265, TDP-PrLD, sTDP, TDP-CTF^{WT} or TDP-CTF^{VLIM-F}. Arrowheads point to co-aggregation. Hoechst staining was used to outline nuclei. Scale bar: 5 µm.

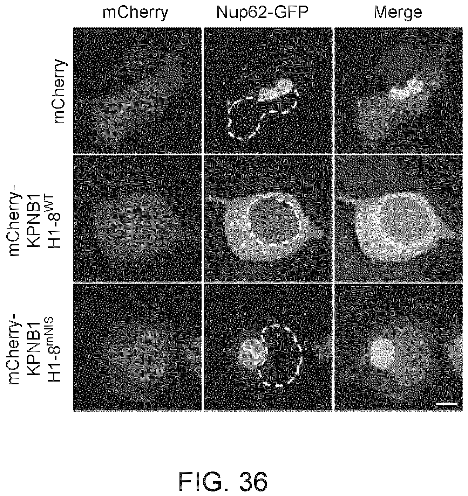

Figure 36: KPNB1 H1-8 reduces cytoplasmic Nup62 aggregates. Immunofluorescence of HEK293T cells co-expressing Nup62-GFP with mCherry, mCherry-tagged KPNB1 H1-8^{WT} or H1-8^{mNIS}. KPNB1 H1-8^{WT} suppresses Nup62 aggregates, whereas NIS mutations abolished this activity. Hoechst staining was used to outline nuclei. Scale bar: 5 µm.

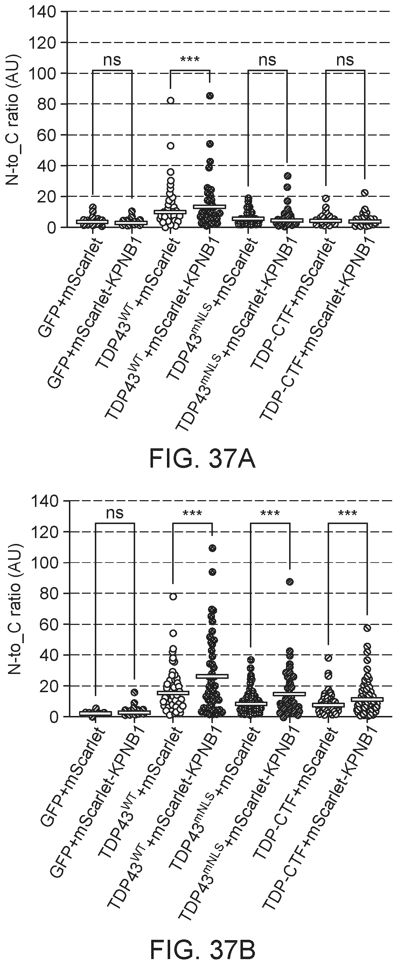

Figures 37A and 37B: KPNB1 increases the N-to-C ratio of different TDP-43 constructs in rodent primary neurons. Quantitative analysis of the N-to-C ratio of GFP or GFP-tagged TDP-43^{WT}, TDP-43^{mNLS} or TDP-CTF in primary neurons expressing mScarlet or mScarlet-KPNB 1 24 hours (Figure 37A) or 48 hours (Figure 37B) post-transfection. KPNB1 only increases nuclear levels of TDP-43^{WT} at this stage. Statistical analysis was performed using one-way ANOVA and Bonferroni's post hoc test (***p<0.001, n=52-264 neurons per group).

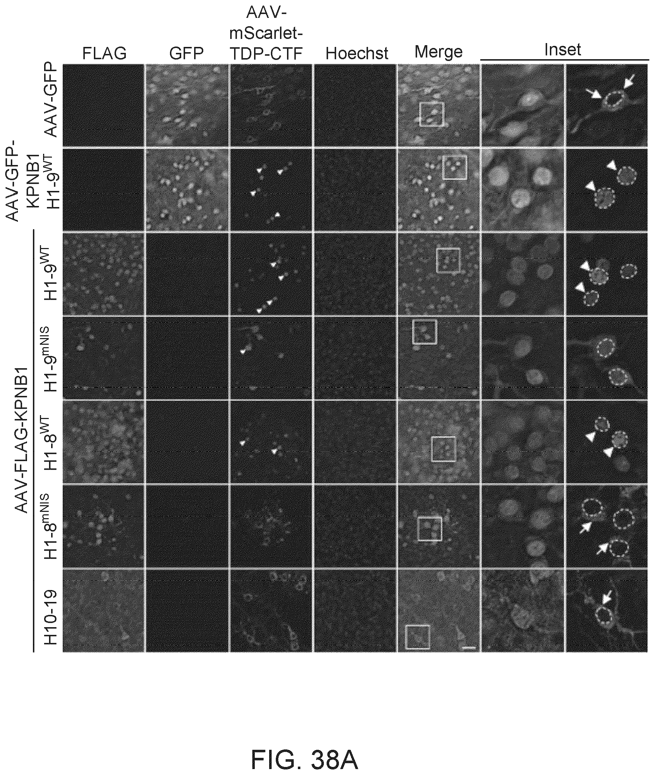

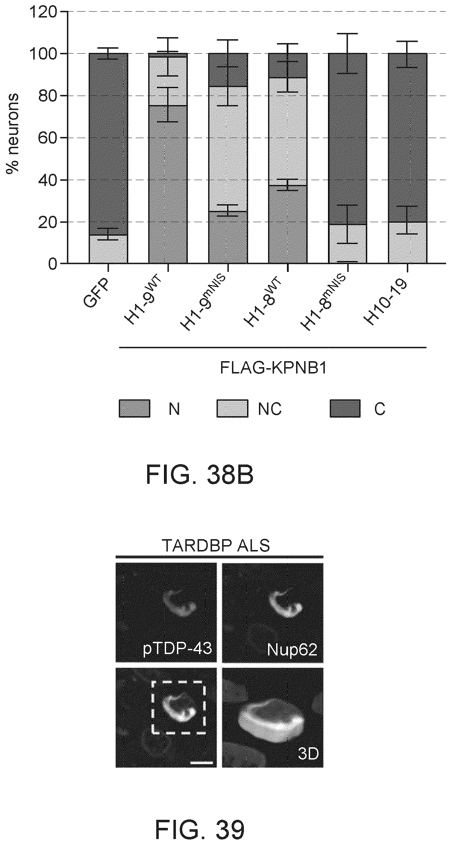

Figures 38A - 38B: KPNB1 reduces cytoplasmic aggregation of TDP-CTF and promotes nuclear localization in BSCs. Figure 38A) Immunofluorescence of mouse BSCs (DIV12) co-expressing AAV-mScarlet-TDP-CTF with AAV-GFP, AAV-GFP-KPNB1 H1-9^{WT}, AAV-FLAG-KPNB1 H1-9^{WT}, H1-9^{mNIS}, H1-8^{WT}, H1-8^{mNIS} or H10-19. KPNB1 H1-9^{WT} and H1-8^{WT} reduce TDP-CTF cytoplasmic aggregates (arrows) and render it very nuclear (arrowheads), whereas NIS mutations strongly reduce this function. Hoechst staining was used to outline nuclei. Scale bar: 50 µm. Figure 38B) Quantification of the percentage of neurons exhibiting nuclear (N), nucleocytoplasmic (NC) or cytoplasmic (C) mScarlet-TDP-CTF distribution upon expression of different AAV-KPNB1 constructs. Statistical analysis was performed using two-way ANOVA and Bonferroni's post hoc test (three independent experiments; n=150-178 neurons per group; statistics are summarized in Table 8).
Figure 39: Nup62 colocalizes with pTDP-43 aggregates in the spinal cord of TARDBP ALS patients. High resolution co-immunofluorescence staining of pTDP-43 (magenta) and Nup62 (green) with Hoechst (blue) was conducted in fixed spinal cord of a neuropathologically diagnosed TARDBP ALS case. pTDP-43 was stained with a 647/Cy5 secondary antibody and Nup62 with a 488/FITC antibody to ensure there is no bleed through between channels. The image is shown as a panel of projected optical sections from each z-series for magenta and green channels and merged with Hoechst DNA staining. Volume rendered z-series of all channels are shown as the 3D inset. Scale bar: 5 µm.

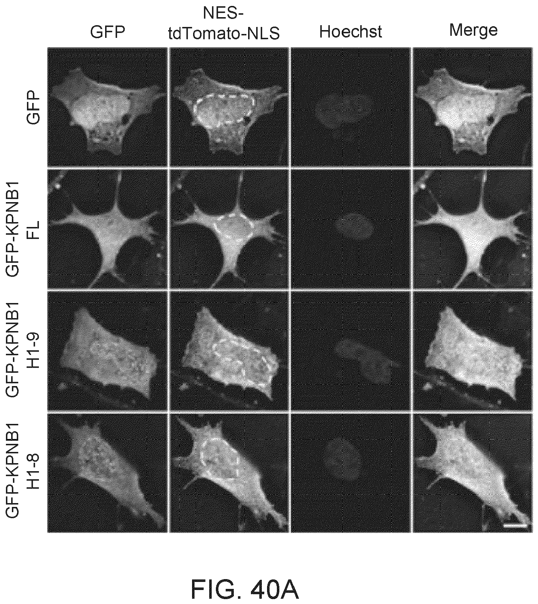

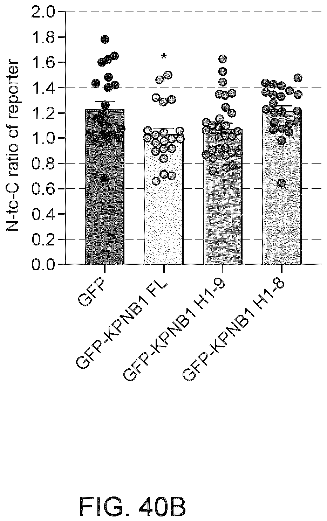

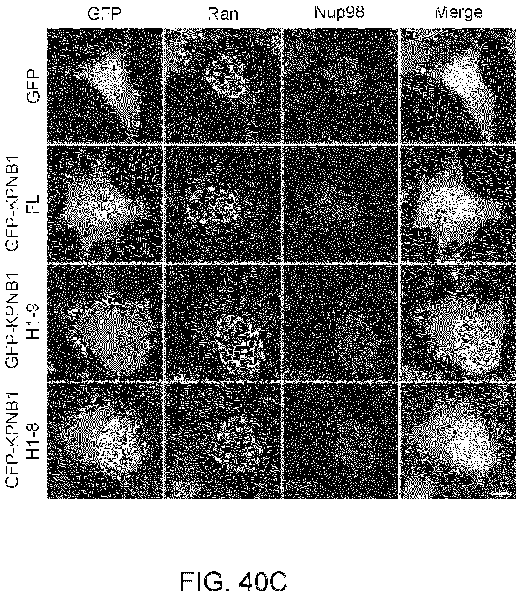

Figures 40A - 40C: KPNB1 expression does not dysregulate nucleocytoplasmic transport. Figure 40A) Immunofluorescence (IF) of SH-SY5Y cells co-expressing the transport reporter NES-tdTomato-NLS with GFP or GFP-tagged KPNB1 full-length (FL), H1-9 or H1-8. Figure 40B) Quantification of the N-to-C ratio of the NES-tdTomato-NLS reporter. Statistical analysis was performed using one-way ANOVA and Bonferroni's post hoc test (*p<0.05, n=21-29 cells per group). Figure 40C) IF of SH-SY5Y cells expressing GFP or GFP-tagged full-length KPNB1 (FL), H1-9 or H1-8 and stained for endogenous Ran and Nup98. Both proteins remain mostly nuclear in presence of KPNB1. Hoechst staining was used to outline nuclei. Scale bar: 5 µm.

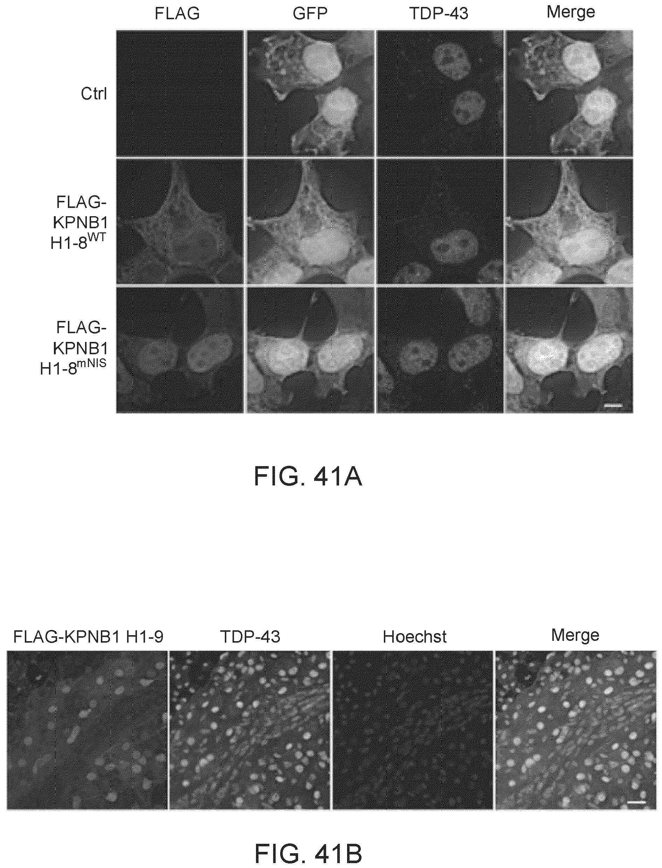

Figures 41A - 41B: Expression of the N-terminal half of KPNB1 does not affect the nuclear localization of endogenous TDP-43 in cells and BSCs. Figure 41A) Immunofluorescence (IF) of HEK293T cells co-expressing GFP with an empty plasmid (ctrl), FLAG-tagged KPNB1 H1-8^{WT} or H1-8^{mNIS} and stained for endogenous TDP-43. TDP-43 remains nuclear in presence of KPNB1 H1-8. Scale bar: 5 µm. Figure 41B) IF of mouse BSCs (DIV15) expressing AAV-FLAG-KPNB1 H1-9 and stained for endogenous TDP-43. TDP-43 remains nuclear in presence of KPNB1 H1-9. Scale bar: 25 µm.

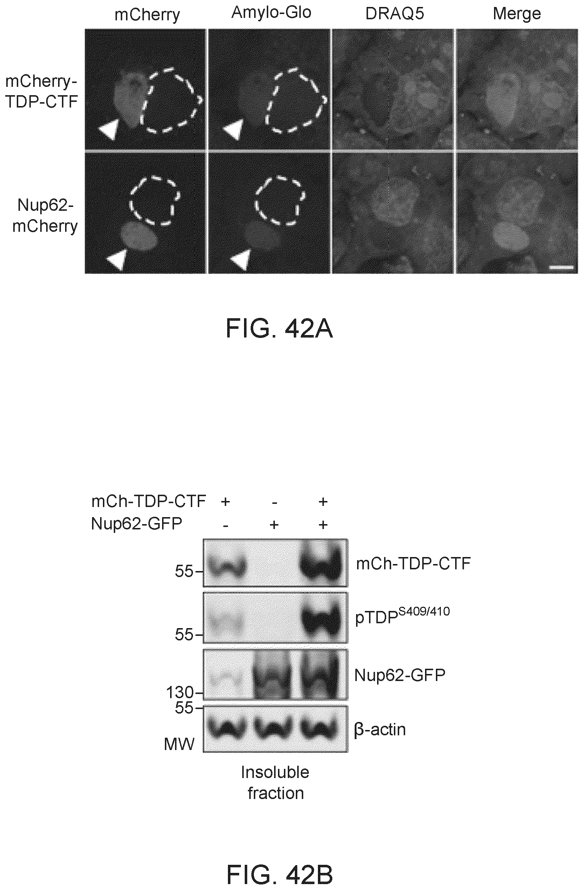

Figures 42A - 42B: Nup62 and TDP-CTF aggregates are positive for amyloid staining and their co-expression promotes TDP-CTF aggregation. Figure 42A) Immunofluorescence of HEK293T cells expressing mCherry-TDP-CTF or Nup62-mCherry which form Amylo-Glo-positive aggregates. Hoechst staining was used to outline nuclei. Scale bar: 5 µm. Figure 42B) Western blot analysis of insoluble mCherry-TDP-CTF in HEK293T cells expressing GFP or Nup62-GFP. Nup62 strongly increases insoluble levels of TDP- CTF.

### DETAILED DESCRIPTION

This document provides methods and materials for treating a mammal (e.g., a human) having, or at risk of developing, a proteinopathy (e.g., a TDP-43 proteinopathy). For example, one or more importin polypeptides and/or fragments thereof (and/or nucleic acids designed to express an importin polypeptide and/or a fragment thereof) can be administered to a mammal (e.g., a human) having, or at risk of developing, a proteinopathy (e.g., a TDP-43 proteinopathy) to treat the mammal.

In some cases, the methods and materials described herein can be used to treat a proteinopathy (e.g., a TDP-43 proteinopathy). For example, one or more importin polypeptides (and/or one or more nucleic acids designed to express an importin polypeptide) can be administered to a mammal (e.g., a human) in need thereof (e.g., a human having, or at risk of developing, a proteinopathy such as a TDP-43 proteinopathy) to slow, delay, or prevent progression of a proteinopathy (e.g., a TDP-43 proteinopathy). In some cases, one or more importin polypeptides (and/or one or more nucleic acids designed to express an importin polypeptide) can be administered to a mammal (e.g., a human) in need thereof (e.g., a human having, or at risk of developing, a proteinopathy such as a TDP-43 proteinopathy) to slow, delay, or prevent the development of a proteinopathy (e.g., a TDP-43 proteinopathy).

In some cases, the methods and materials described herein can be used to reduce or eliminate one or more symptoms of a proteinopathy (e.g., a TDP-43 proteinopathy). For example, one or more importin polypeptides (and/or one or more nucleic acids designed to express an importin polypeptide) can be administered to a mammal (e.g., a human) in need thereof (e.g., a human having, or at risk of developing, a proteinopathy such as a TDP-43 proteinopathy) to reduce or eliminate one or more symptoms of a proteinopathy (e.g., a TDP-43 proteinopathy). Examples of symptoms of a proteinopathy (e.g., a TDP-43 proteinopathy) include, without limitation, depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, and symptoms of motor neuron disease (e.g., muscle weakness, muscle atrophy, fasciculations, spasticity, dysarthria, and dysphagia), behavior variant frontotemporal dementia (bvFTD), and primary progressive aphasia (PPA). In some cases, the materials and methods described herein can be used to reduce the severity of one or more symptoms of a proteinopathy (e.g., a TDP-43 proteinopathy) in a mammal (e.g., a human) by, for example, 10, 20, 30, 40, 50, 60, 70, 80, 90, 95, or more percent.

In some cases, the methods and materials described herein can be used to reduce or eliminate neurodegeneration associated with a proteinopathy (e.g., a TDP-43 proteinopathy). For example, one or more importin polypeptides (and/or one or more nucleic acids designed to express an importin polypeptide) can be administered to a mammal (e.g., a human) in need thereof (e.g., a human having, or at risk of developing, a proteinopathy such as a TDP-43 proteinopathy) to slow, delay, or prevent progression of neurodegeneration associated with a proteinopathy (e.g., a TDP-43 proteinopathy). In some cases, one or more importin polypeptides (and/or one or more nucleic acids designed to express an importin polypeptide) can be administered to a mammal (e.g., a human) in need thereof (e.g., a human having, or at risk of developing, a proteinopathy such as a TDP-43 proteinopathy) to slow, delay, or prevent the development of neurodegeneration associated with a proteinopathy (e.g., a TDP-43 proteinopathy).

In some cases, the methods and materials described herein can be used to reduce or eliminate aggregation of TDP-43 polypeptides. For example, the materials and methods described herein can be used to reduce the number of TDP-43 polypeptide aggregates present within the CNS (e.g., the brain) of a mammal having a proteinopathy (e.g., a TDP-43 proteinopathy) by, for example, 10, 20, 30, 40, 50, 60, 70, 80, 90, 95, or more percent. For example, the materials and methods described herein can be used to reduce the size (e.g., volume) of one or more TDP-43 polypeptide aggregates present within a mammal having a proteinopathy (e.g., a TDP-43 proteinopathy) by, for example, 10, 20, 30, 40, 50, 60, 70, 80, 90, 95, or more percent.

In some cases, the methods and materials described herein can be used to reduce or eliminate detergent-insoluble TDP-43 polypeptides. For example, the materials and methods described herein can be used to reduce the number of detergent-insoluble TDP-43 polypeptides present within the CNS (e.g., the brain) of a mammal having a proteinopathy (e.g., a TDP-43 proteinopathy) by, for example, 10, 20, 30, 40, 50, 60, 70, 80, 90, 95, or more percent. For example, the materials and methods described herein can be used to reduce the fraction or percent of detergent-insoluble TDP-43 polypeptides within one or more TDP-43 polypeptide aggregates present within a mammal having a proteinopathy (e.g., a TDP-43 proteinopathy) by, for example, 10, 20, 30, 40, 50, 60, 70, 80, 90, 95, or more percent.

In some cases, the methods and materials described herein can be used to reduce or eliminate cell death associated with a proteinopathy (e.g., a TDP-43 proteinopathy). For example, the materials and methods described herein can be used to reduce the number of apoptotic cells present within the CNS (e.g., the brain) of a mammal having a proteinopathy (e.g., a TDP-43 proteinopathy) by, for example, 10, 20, 30, 40, 50, 60, 70, 80, 90, 95, or more percent.

In some cases, the methods and materials described herein can be used to restore nuclear localization of TDP-43 polypeptides. For example, the materials and methods described herein can be used to increase the number of TDP-43 polypeptides that can localize the nucleus of cells present within the CNS (e.g., the brain) of a mammal having a proteinopathy (e.g., a TDP-43 proteinopathy) by, for example, 10, 20, 30, 40, 50, 60, 70, 80, 90, 95, or more percent.

In some cases, the methods and materials described herein can be used to restore function of TDP-43 polypeptides. For example, the materials and methods described herein can be used to increase the level of TDP-43 polypeptide function (e.g., to increase expression, splicing, and poly-adenylation of transcripts from Stathmin-2 and/or UNC13A) within the nucleus of cells present within the CNS (e.g., the brain) of a mammal having a proteinopathy (e.g., a TDP-43 proteinopathy) by, for example, 10, 20, 30, 40, 50, 60, 70, 80, 90, 95, or more percent.

Any appropriate mammal having, or at risk of developing, a proteinopathy (e.g., a TDP-43 proteinopathy) can be treated as described herein (e.g., by administering one or more importin polypeptides and/or fragments thereof and/or nucleic acids designed to express an importin polypeptide and/or a fragment thereof). Examples of mammals that can be treated as described herein include, without limitation, humans, non-human primates such as monkeys, dogs, cats, horses, cows, pigs, sheep, mice, rats, rabbits, hamsters, guinea pigs, and ferrets.

When treating a mammal (e.g., a human) having, or at risk of developing, a proteinopathy (e.g., a TDP-43 proteinopathy) as described herein (e.g., by administering one or more importin polypeptides and/or fragments thereof and/or nucleic acids designed to express an importin polypeptide and/or a fragment thereof), the proteinopathy can be any type of proteinopathy. In some cases, a proteinopathy be a TDP-43 proteinopathy (e.g., can include aggregation of TDP-43 polypeptides). In some cases, a proteinopathy can be a neurodegenerative disease. Examples of types of proteinopathies that can be treated as described herein include, without limitation, Alzheimer's disease, FTD, ALS, traumatic brain injury (TBI), chronic traumatic encephalopathy (CTE), limbic-predominant age-related TDP-43 encephalopathy (LATE), dementia with Lewy bodies (DLB), Parkinson's disease, Huntington's disease, argyrophilic grain disease (AGD), hippocampal sclerosis (HS), Guam ALS, Guam parkinsonism-dementia complex (G-PDC), Perry disease, facial onset sensory and motor neuronopathy (FOSMN), and rimmed vacuole myopathies (e.g., inclusion body myositis (IBM), hereditary inclusion body myopathy, oculopharyngeal muscular dystrophy (OPMD), and distal myopathies with rimmed vacuoles). When a proteinopathy to be treated as described herein is ALS, the ALS can be a familial ALS. A familial ALS can be associated with one or more mutations (e.g., insertions, deletions, indels, substitutions, and/or expansions) in any appropriate nucleic acid (e.g., any appropriate gene). Examples of familial ALS include, without limitation, C9ALS (associated with one or more mutations in a *C9orf72* gene), ALS1 (associated with one or more mutations in a *SOD1* gene), ALS 4 (associated with one or more mutations in a *SETX* gene), ALS6 (associated with one or more mutations in a *FUS* gene), ALS8 (associated with one or more mutations in a *VABP* gene), ALS9 (associated with one or more mutations in an *ANG* gene), ALS 10 (associated with one or more mutations in a *TARDBP* gene), ALS 11 (associated with one or more mutations in a *FIG4* gene), ALS12 (associated with one or more mutations in a *OPTN* gene), ALS13 (associated with one or more mutations in an *ATXN2* gene), ALS14 (associated with one or more mutations in a *VCP* gene), ALS 15 (associated with one or more mutations in a *UBQLN2* gene), ALS 17 (associated with one or more mutations in a *CHMP2B* gene), ALS 18 (associated with one or more mutations in a *PFN1* gene), ALS19 (associated with one or more mutations in a *ERBB4* gene), ALS20 (associated with one or more mutations in a *HNRNPA1*/*A2B1* gene), ALS21 (associated with one or more mutations in a *MATR3* gene), ALS22 (associated with one or more mutations in a *TUBB4A* gene), ALS23 (associated with one or more mutations in a *ANXA11* gene), ALS24 (associated with one or more mutations in a *NEK1* gene), and ALS25 (associated with one or more mutations in a *KIF5A* gene).

In some cases, a mammal (e.g., a human) can be identified as having, or as being at risk of developing, a proteinopathy (e.g., a TDP-43 proteinopathy). For example, genetic testing, imaging techniques (e.g., CNS scanning techniques such as magnetic resonance imaging (MRI), computed tomography (CT) scanning, fluorodeoxyglucose positron emission tomography (FDG-PET) scanning, and SPECT (single proton emission CT) scanning), and/or biomarker detection using enzyme-linked immunosorbent assay (ELISA), immunohistochemistry (IHC), cerebrospinal fluid real-time quaking-induced conversion (RT-QuIC), single molecule array (Simoa), western blot analysis, and/or mass spectrometry can be used to diagnose a mammal as having, or as being at risk of developing, a proteinopathy (e.g., a TDP-43 proteinopathy).

Once identified as having, or as being at risk of developing, a proteinopathy (e.g., a TDP-43 proteinopathy), the mammal (*e.g*., the human) can be administered, or instructed to self-administer, one or more importin polypeptides and/or fragments thereof (and/or nucleic acids designed to express an importin polypeptide and/or a fragment thereof) as described herein.

Any appropriate importin polypeptide or fragment thereof (and/or nucleic acid designed to express an importin polypeptide or a fragment thereof) can be administered to a mammal (e.g., a human) having, or at risk of developing, a proteinopathy (e.g., a TDP-43 proteinopathy) as described herein. In some cases, an importin polypeptide can be a beta-karyopherin type importin polypeptide. Examples of importin polypeptides include, without limitation, KPNB1 polypeptides, IPO3 polypeptides (e.g., IPO3a polypeptides and IPO3b polypeptides), importin-4 (IPO4) polypeptides, importin-9 (IPO9) polypeptides, importin-12 (IPO12) polypeptides, and IPO13 polypeptides.

When an importin polypeptide is a KPNB1 polypeptide, any appropriate KPNB 1 polypeptide (and/or nucleic acid designed to express a KPNB1 polypeptide) can be administered to a mammal (e.g., a human) having, or at risk of developing, a proteinopathy (e.g., a TDP-43 proteinopathy) as described herein. Examples of KPNB 1 polypeptides and nucleic acids encoding KPNB1 polypeptides include, without limitation, those set forth in the National Center for Biotechnology Information (NCBI) databases at, for example, accession no. NP_002256 (version NP_002256.2), accession no. NP_001263382 (version NP_001263382.1), and accession no. NP_032405 (version NP_032405.3).

In some cases, a KPNB 1 polypeptide can have an amino acid sequence set forth in SEQ ID NO: 1 (see, e.g., Figure 10A). In some cases, a nucleic acid encoding a KPNB 1 polypeptide can have an nucleotide sequence set forth in SEQ ID NO:2 (see, e.g., Figure 10B).

When a fragment of an importin polypeptide is a KPNB 1 polypeptide fragment, any appropriate KPNB1 polypeptide fragment (and/or nucleic acid designed to express a KPNB 1 polypeptide fragment) can be administered to a mammal (e.g., a human) having, or at risk of developing, a proteinopathy (e.g., a TDP-43 proteinopathy) as described herein. In some cases, a KPNB 1 polypeptide fragment can be derived from the amino acid sequence set forth in SEQ ID NO: 1.

A KPNB 1 polypeptide fragment can be any appropriate length (e.g., can include any number of amino acids) provided that it maintains at least some function of a naturally-occurring KPNB1 polypeptide (e.g., the ability to reduce at least some TDP-43 aggregation and/or restore at least some nuclear localization of TDP-43 polypeptides). For example, a KPNB1 polypeptide fragment can be from about 30 amino acids in length to about 900 amino acids in length (e.g., from about 30 amino acids to about 850 amino acids, from about 30 amino acids to about 800 amino acids, from about 30 amino acids to about 750 amino acids, from about 30 amino acids to about 700 amino acids, from about 30 amino acids to about 650 amino acids, from about 30 amino acids to about 600 amino acids, from about 30 amino acids to about 550 amino acids, from about 30 amino acids to about 500 amino acids, from about 30 amino acids to about 450 amino acids, from about 30 amino acids to about 400 amino acids, from about 30 amino acids to about 350 amino acids, from about 30 amino acids to about 300 amino acids, from about 30 amino acids to about 250 amino acids, from about 30 amino acids to about 200 amino acids, from about 30 amino acids to about 150 amino acids, from about 30 amino acids to about 100 amino acids, from about 50 amino acids to about 900 amino acids, from about 100 amino acids to about 900 amino acids, from about 150 amino acids to about 900 amino acids, from about 200 amino acids to about 900 amino acids, from about 250 amino acids to about 900 amino acids, from about 300 amino acids to about 900 amino acids, from about 350 amino acids to about 900 amino acids, from about 400 amino acids to about 900 amino acids, from about 450 amino acids to about 900 amino acids, from about 500 amino acids to about 900 amino acids, from about 550 amino acids to about 900 amino acids, from about 600 amino acids to about 900 amino acids, from about 650 amino acids to about 900 amino acids, from about 700 amino acids to about 900 amino acids, from about 750 amino acids to about 900 amino acids, from about 800 amino acids to about 900 amino acids, from about 50 amino acids to about 800 amino acids, from about 100 amino acids to about 700 amino acids, from about 200 amino acids to about 600 amino acids, from about 300 amino acids to about 500 amino acids, from about 100 amino acids to about 300 amino acids, from about 200 amino acids to about 400 amino acids, from about 400 amino acids to about 600 amino acids, from about 500 amino acids to about 700 amino acids, or from about 600 amino acids to about 800 amino acids in length) provided that it maintains at least some function of a naturally-occurring KPNB1 polypeptide (e.g., the ability to reduce at least some TDP-43 aggregation).

In some cases, a KPNB 1 polypeptide fragment can comprise, consist essentially of, or consist of an amino acid sequence set forth in Table 1.

**Table 1. Exemplary KPNB1 polypeptide fragments.**

| Truncation | Polypeptide Sequence | SEQ ID NO: |
|---|---|---|
| H1-9 | MELITILEKTVSPDRLELEAAQKFLERAAVENLPTFLVELSRVLA NPGNSQVARVAAGLQIKNSLTSKDPDIKAQYQQRWLAIDANARRE VKNYVLQTLGTETYRPSSASQCVAGIACAEIPVNQWPELIPQLVA NVTNPNSTEHMKESTLEAIGYICQDIDPEQLQDKSNEILTAIIQG MRKEEPSNNVKLAATNALLNSLEFTKANFDKESERHFIMQVVCEA TQCPDTRVRVAALQNLVKIMSLYYQYMETYMGPALFAITIEAMKS DIDEVALQGIEFWSNVCDEEMDLAIEASEAAEQGRPPEHTSKFYA KGALQYLVPILTQTLTKQDENDDDDDWNPCKAAGVCLMLLATCCE DDIVPHVLPFIKEHIKNPDWRYRDAAVMAFGCILEGP | 7 |
| H1-8 | MELITILEKTVSPDRLELEAAQKFLERAAVENLPTFLVELSRVLA NPGNSQVARVAAGLQIKNSLTSKDPDIKAQYQQRWLAIDANARRE VKNYVLQTLGTETYRPSSASQCVAGIACAEIPVNQWPELIPQLVA NVTNPNSTEHMKESTLEAIGYICQDIDPEQLQDKSNEILTAIIQG MRKEEPSNNVKLAATNALLNSLEFTKANFDKESERHFIMQVVCEA TQCPDTRVRVAALQNLVKIMSLYYQYMETYMGPALFAITIEAMKS DIDEVALQGIEFWSNVCDEEMDLAIEASEAAEQGRPPEHTSKFYA KGALQYLVPILTQTLTKQDENDDDDDWNPCKAAGVCLMLLATCCE D | 8 |
| H1-7 | MELITILEKTVSPDRLELEAAQKFLERAAVENLPTFLVELSRVLA NPGNSQVARVAAGLQIKNSLTSKDPDIKAQYQQRWLAIDANARRE VKNYVLQTLGTETYRPSSASQCVAGIACAEIPVNQWPELIPQLVA NVTNPNSTEHMKESTLEAIGYICQDIDPEQLQDKSNEILTAIIQG MRKEEPSNNVKLAATNALLNSLEFTKANFDKESERHFIMQVVCEA TQCPDTRVRVAALQNLVKIMSLYYQYMETYMGPALFAITIEAMKS DIDEVALQGIEFWSNVCDEEMDLAIEASEAAEQGRPP | 9 |
| H1-6 | MELITILEKTVSPDRLELEAAQKFLERAAVENLPTFLVELSRVLA NPGNSQVARVAAGLQIKNSLTSKDPDIKAQYQQRWLAIDANARRE VKNYVLQTLGTETYRPSSASQCVAGIACAEIPVNQWPELIPQLVA NVTNPNSTEHMKESTLEAIGYICQDIDPEQLQDKSNEILTAIIQG MRKEEPSNNVKLAATNALLNSLEFTKANFDKESERHFIMQVVCEA TQCPDTRVRVAALQNLVKIMSLYY | 10 |
| H1-5 | MELITILEKTVSPDRLELEAAQKFLERAAVENLPTFLVELSRVLA NPGNSQVARVAAGLQIKNSLTSKDPDIKAQYQQRWLAIDANARRE VKNYVLQTLGTETYRPSSASQCVAGIACAEIPVNQWPELIPQLVA NVTNPNSTEHMKESTLEAIGYICQDIDPEQLQDKSNEILTAIIQG MRKEEPSNNVKLAATNALLNSLEFTKANF | 11 |
| H1-4 | MELITILEKTVSPDRLELEAAQKFLERAAVENLPTFLVELSRVLA NPGNSQVARVAAGLQIKNSLTSKDPDIKAQYQQRWLAIDANARRE VKNYVLQTLGTETYRPSSASQCVAGIACAEIPVNQWPELIPQLVA NVTNPNSTEHMKESTLEAIGYICQDIDPEQLQ | 12 |
| H1-3 | MELITILEKTVSPDRLELEAAQKFLERAAVENLPTFLVELSRVLA NPGNSQVARVAAGLQIKNSLTSKDPDIKAQYQQRWLAIDANARRE VKNYVLQTLGTETYRPSSASQCVAGIACAEIPVNQWP | 13 |
| H1-2 | MELITILEKTVSPDRLELEAAQKFLERAAVENLPTFLVELSRVLA NPGNSQVARVAAGLQIKNSLTSKDPD | 14 |
| H1 | MELITILEKTVSPDRLELEAAQKFLERAAVEN | 15 |
| H10-19 | MEPSQLKPLVIQAMPTLIELMKDPSVVVRDTAAWTVGRICELLPE AAINDVYLAPLLQCLIEGLSAEPRVASNVCWAFSSLAEAAYEAAD VADDQEEPATYCLSSSFELIVQKLLETTDRPDGHQNNLRSSAYES LMEIVKNSAKDCYPAVQKTTLVIMERLQQVLQMESHIQSTSDRIQ FNDLQSLLCATLQNVLRKVQHQDALQISDVVMASLLRMFQSTAGS GGVQEDALMAVSTLVEVLGGEFLKYMEAFKPFLGIGLKNYAEYQV CLAAVGLVGDLCRALQSNIIPFCDEVMQLLLENLGNENVHRSVKP QILSVFGDIALAIGGEFKKYLEVVLNTLQQASQAQVDKSDYDMVD YLNELRESCLEAYTGIVQGLKGDQENVHPDVMLVQPRVEFILSFI DHIAGDEDHTDGVVACAAGLIGDLCTAFGKDVLKLVEARPMIHEL LTEGRRSKTNKAKTLATWATKELRKLKNQA | 16 |

In some cases, a KPNB1 polypeptide fragment provided herein can include the amino acid sequence set forth in any one of SEQ ID NOs:7-16 with zero, one, two three, or more (e.g., five, eight, 12, 15, 18, 20, 25, 30, 35, 40, or more) amino acid substitutions (e.g., one or more K→R substitutions) within the articulated sequence of the sequence identifier (e.g., any one of SEQ ID NOs:7-16), with zero, one, two, three, four, or five amino acid residues preceding the articulated sequence of the sequence identifier (e.g., any one of SEQ ID NOs:7-16), and/or with zero, one, two, three, four, or five amino acid residues following the articulated sequence of the sequence identifier (e.g., any one of SEQ ID NOs:7-16), provided that the KPNB1 polypeptide fragment retains at least some activity exhibited by a naturally-occurring KPNB1 polypeptide (e.g., the ability to reduce at least some TDP-43 aggregation and/or restore at least some nuclear localization of TDP-43 polypeptides). Examples of such KPNB1 polypeptide fragments can be those set forth in Table 2.

**Table 2. Exemplary KPNB1 polypeptide fragments.**

| Polypeptide Sequence | SEQ ID NO: |
|---|---|
| MELITILERTVSPDRLELEAAQRFLERAAVENLPTFLVELSRVLANPGNSQVARVA AGLQIRNSLTSRDPDIRAQYQQRWLAIDANARREVRNYVLQTLGTETYRPSSASQC VAGIACAEIPVNQWPELIPQLVANVTNPNSTEHMRESTLEAIGYICQDIDPEQLQD RSNEILTAIIQGMRREEPSNNVRLAATNALLNSLEFTRANFDRESERHFIMQVVCE ATQCPDTRVRVAALQNLVRIMSLYYQYMETYMGPALFAITIEAMRSDIDEVALQGI EFWSNVCDEEMDLAIEASEAAEQGRPPEHTSRFYARGALQYLVPILTQTLTRQDEN DDDDDWNPCRAAGVCLMLLATCCEDDIVPHVLPFIREHIRNPDWRYRDAAVMAFGC ILEGP | 17 |
| MELITILERTVSPDRLELEAAQRFLERAAVENLPTFLVELSRVLANPGNSQVARVA AGLQIRNSLTSRDPDIRAQYQQRWLAIDANARREVRNYVLQTLGTETYRPSSASQC VAGIACAEIPVNQWPELIPQLVANVTNPNSTEHMRESTLEAIGYICQDIDPEQLQD RSNEILTAIIQGMRREEPSNNVRLAATNALLNSLEFTRANFDRESERHFIMQVVCE ATQCPDTRVRVAALQNLVRIMSLYYQYMETYMGPALFAITIEAMRSDIDEVALQGI EFWSNVCDEEMDLAIEASEAAEQGRPPEHTSRFYARGALQYLVPILTQTLTRQDEN DDDDDWNPCRAAGVCLMLLATCCED | 18 |
| MELITILERTVSPDRLELEAAQRFLERAAVENLPTFLVELSRVLANPGNSQVARVA AGLQIRNSLTSRDPDIRAQYQQRWLAIDANARREVRNYVLQTLGTETYRPSSASQC VAGIACAEIPVNQWPELIPQLVANVTNPNSTEHMRESTLEAIGYICQDIDPEQLQD RSNEILTAIIQGMRREEPSNNVRLAATNALLNSLEFTRANFDRESERHFIMQVVCE ATQCPDTRVRVAALQNLVRIMSLYYQYMETYMGPALFAITIEAMRSDIDEVALQGI EFWSNVCDEEMDLAIEASEAAEQGRPP | 19 |
| MELITILERTVSPDRLELEAAQRFLERAAVENLPTFLVELSRVLANPGNSQVARVA AGLQIRNSLTSRDPDIRAQYQQRWLAIDANARREVRNYVLQTLGTETYRPSSASQC VAGIACAEIPVNQWPELIPQLVANVTNPNSTEHMRESTLEAIGYICQDIDPEQLQD | 20 |
| RSNEILTAIIQGMRREEPSNNVRLAATNALLNSLEFTRANFDRESERHFIMQVVCE ATQCPDTRVRVAALQNLVRIMSLYY | |
| MELITILERTVSPDRLELEAAQRFLERAAVENLPTFLVELSRVLANPGNSQVARVA AGLQIRNSLTSRDPDIRAQYQQRWLAIDANARREVRNYVLQTLGTETYRPSSASQC VAGIACAEIPVNQWPELIPQLVANVTNPNSTEHMRESTLEAIGYICQDIDPEQLQD RSNEILTAIIQGMRREEPSNNVRLAATNALLNSLEFTRANF | 21 |
| MELITILERTVSPDRLELEAAQRFLERAAVENLPTFLVELSRVLANPGNSQVARVA AGLQIRNSLTSRDPDIRAQYQQRWLAIDANARREVRNYVLQTLGTETYRPSSASQC VAGIACAEIPVNQWPELIPQLVANVTNPNSTEHMRESTLEAIGYICQDIDPEQLQ | 22 |
| MELITILERTVSPDRLELEAAQRFLERAAVENLPTFLVELSRVLANPGNSQVARVA AGLQIRNSLTSRDPDIRAQYQQRWLAIDANARREVRNYVLQTLGTETYRPSSASQC VAGIACAEIPVNQWP | 23 |
| MELITILERTVSPDRLELEAAQRFLERAAVENLPTFLVELSRVLANPGNSQVARVA AGLQIRNSLTSRDPD | 24 |
| MELITILERTVSPDRLELEAAQRFLERAAVEN | 25 |

When an importin polypeptide is an IPO3 polypeptide (also referred to as a transportin-2 (TNPO2) polypeptide), any appropriate IPO3 polypeptide (and/or nucleic acid designed to express an IPO3 polypeptide) can be administered to a mammal (e.g., a human) having, or at risk of developing, a proteinopathy (e.g., a TDP-43 proteinopathy) as described herein. Examples of IPO3 polypeptides and nucleic acids encoding IPO3 polypeptides include, without limitation, those set forth in the NCBI databases at, for example, accession no. NP_001369170 (version NP_001369170 .1), accession no. NP_038461 (version NP_038461.2), and accession no. NP_001369172 (version NP_001369172.1).

In some cases, an IPO3 polypeptide can have an amino acid sequence set forth in SEQ ID NO:3 (see, e.g., Figure 11A). In some cases, a nucleic acid encoding an IPO3 polypeptide can have an nucleotide sequence set forth in SEQ ID NO:4 (see, e.g., Figure 11B).

When a fragment of an importin polypeptide is an IPO3 polypeptide fragment, any appropriate IPO3 polypeptide fragment (and/or nucleic acid designed to express a IPO3 polypeptide fragment) can be administered to a mammal (e.g., a human) having, or at risk of developing, a proteinopathy (e.g., a TDP-43 proteinopathy) as described herein. In some cases, an IPO3 polypeptide fragment can be derived from the amino acid sequence set forth in SEQ ID NO:3.

An IPO3 polypeptide fragment can be any appropriate length (e.g., can include any number of amino acids) provided that it maintains at least some function of a naturally-occurring IPO3 polypeptide (e.g., the ability to reduce at least some TDP-43 aggregation and/or restore at least some nuclear localization of TDP-43 polypeptides). For example, an IPO3 polypeptide fragment can be from about 100 amino acids in length to about 850 amino acids in length (e.g., from about 100 amino acids to about 800 amino acids, from about 100 amino acids to about 750 amino acids, from about 100 amino acids to about 700 amino acids, from about 100 amino acids to about 650 amino acids, from about 100 amino acids to about 600 amino acids, from about 100 amino acids to about 550 amino acids, from about 100 amino acids to about 500 amino acids, from about 100 amino acids to about 450 amino acids, from about 100 amino acids to about 400 amino acids, from about 100 amino acids to about 350 amino acids, from about 100 amino acids to about 300 amino acids, from about 100 amino acids to about 250 amino acids, from about 100 amino acids to about 200 amino acids, from about 150 amino acids to about 850 amino acids, from about 200 amino acids to about 850 amino acids, from about 250 amino acids to about 850 amino acids, from about 300 amino acids to about 850 amino acids, from about 350 amino acids to about 850 amino acids, from about 400 amino acids to about 850 amino acids, from about 450 amino acids to about 850 amino acids, from about 500 amino acids to about 850 amino acids, from about 550 amino acids to about 850 amino acids, from about 600 amino acids to about 850 amino acids, from about 650 amino acids to about 850 amino acids, from about 700 amino acids to about 850 amino acids, from about 750 amino acids to about 850 amino acids, from about 200 amino acids to about 800 amino acids, from about 300 amino acids to about 700 amino acids, from about 400 amino acids to about 600 amino acids, from about 200 amino acids to about 400 amino acids, from about 300 amino acids to about 500 amino acids, from about 500 amino acids to about 700 amino acids, or from about 600 amino acids to about 800 amino acids in length) provided that it maintains at least some function of a naturally-occurring IPO3 polypeptide (e.g., the ability to reduce at least some TDP-43 aggregation).

In some cases, an IPO3 polypeptide fragment can comprise, consist essentially of, or consist of an amino acid sequence set forth in Table 3.

**Table 3. Exemplary IPO3 polypeptide fragments.**

| Truncation | Polypeptide Sequence | SEQ ID NO: |
|---|---|---|
| H2-20 | MNQFPDFNNYLIFVLTRLKSEDEPTRSLSGLILKNNVKAHYQSFP PPVADFIKQECLNNIGDASSLIRATIGILITTIASKGELQMWPEL LPQLCNLLNSEDYNTCEGAFGALQKICEDSSELLDSDALNRPLNI MIPKFLQFFKHCSPKIRSHAIACVNQFIMDRAQALMDNIDTFIEH LFALAVDDDPEVRKNVCRALVMLLEVRIDRLIPHMHSIIQYMLQR TQDHDENVALEACEFWLTLAEQPICKEVLASHLVQLIPILVNGMK YSEIDIILLKGDVEEDEAVPDSEQDIKPRFHKSRTVTLPHEAERP DGSEDAEDDDDDDALSDWNLRKCSAAALDVLANVFREELLPHLLP LLKGLLFHPEWVVKESGILVLGAIAEGCMQGMVPYLPELIPHLIQ CLSDKKALVRSIACWTLSRYAHWVVSQPPDMHLKPLMTELLKRIL DGNKRVQEAACSAFATLEEEACTELVPYLSYILDTLVFAFGKYQH KNLLILYDAIGTLADSVGHHLNQPEYIQKLMPPLIQKWNELKDED KDLFPLLECLSSVATALQSGFLPYCEPVYQRCVTLVQKTLAQAMM YTQHPEQYEAPDKDFMIVALDLLSGLAEGLGGHVEQLVARSNIMT LLFQCMQDSMPEVRQSSFALLGDLTKACFIHVKPCIAEFMPILGT NLNPEFISVCNNATWAIGEICMQMGAEMQPYVQMVLNNLVEIINR PNTPKTLLENTAITIGRLGYVCPQEVAPMLQQFIRPWCTSLRNIR DNEEKDSAFRGICMMIGVNPGGVVQDFIFFCDAVASWVSPKDDLR DMFYKILHGFKDQVGEDNWQQFSEQFPPLLKERLAAFYGV | 26 |
| H3-20 | MPVADFIKQECLNNIGDASSLIRATIGILITTIASKGELQMWPEL LPQLCNLLNSEDYNTCEGAFGALQKICEDSSELLDSDALNRPLNI MIPKFLQFFKHCSPKIRSHAIACVNQFIMDRAQALMDNIDTFIEH LFALAVDDDPEVRKNVCRALVMLLEVRIDRLIPHMHSIIQYMLQR TQDHDENVALEACEFWLTLAEQPICKEVLASHLVQLIPILVNGMK YSEIDIILLKGDVEEDEAVPDSEQDIKPRFHKSRTVTLPHEAERP DGSEDAEDDDDDDALSDWNLRKCSAAALDVLANVFREELLPHLLP LLKGLLFHPEWVVKESGILVLGAIAEGCMQGMVPYLPELIPHLIQ CLSDKKALVRSIACWTLSRYAHWVVSQPPDMHLKPLMTELLKRIL DGNKRVQEAACSAFATLEEEACTELVPYLSYILDTLVFAFGKYQH KNLLILYDAIGTLADSVGHHLNQPEYIQKLMPPLIQKWNELKDED KDLFPLLECLSSVATALQSGFLPYCEPVYQRCVTLVQKTLAQAMM YTQHPEQYEAPDKDFMIVALDLLSGLAEGLGGHVEQLVARSNIMT LLFQCMQDSMPEVRQSSFALLGDLTKACFIHVKPCIAEFMPILGT NLNPEFISVCNNATWAIGEICMQMGAEMQPYVQMVLNNLVEIINR PNTPKTLLENTAITIGRLGYVCPQEVAPMLQQFIRPWCTSLRNIR DNEEKDSAFRGICMMIGVNPGGVVQDFIFFCDAVASWVSPKDDLR DMFYKILHGFKDQVGEDNWQQFSEQFPPLLKERLAAFYGV | 27 |
| H4-20 | MWPELLPQLCNLLNSEDYNTCEGAFGALQKICEDSSELLDSDALN RPLNIMIPKFLQFFKHCSPKIRSHAIACVNQFIMDRAQALMDNID TFIEHLFALAVDDDPEVRKNVCRALVMLLEVRIDRLIPHMHSIIQ YMLQRTQDHDENVALEACEFWLTLAEQPICKEVLASHLVQLIPIL VNGMKYSEIDIILLKGDVEEDEAVPDSEQDIKPRFHKSRTVTLPH EAERPDGSEDAEDDDDDDALSDWNLRKCSAAALDVLANVFREELL PHLLPLLKGLLFHPEWVVKESGILVLGAIAEGCMQGMVPYLPELI PHLIQCLSDKKALVRSIACWTLSRYAHWVVSQPPDMHLKPLMTEL LKRILDGNKRVQEAACSAFATLEEEACTELVPYLSYILDTLVFAF GKYQHKNLLILYDAIGTLADSVGHHLNQPEYIQKLMPPLIQKWNE LKDEDKDLFPLLECLSSVATALQSGFLPYCEPVYQRCVTLVQKTL AQAMMYTQHPEQYEAPDKDFMIVALDLLSGLAEGLGGHVEQLVAR SNIMTLLFQCMQDSMPEVRQSSFALLGDLTKACFIHVKPCIAEFM PILGTNLNPEFISVCNNATWAIGEICMQMGAEMQPYVQMVLNNLV EIINRPNTPKTLLENTAITIGRLGYVCPQEVAPMLQQFIRPWCTS LRNIRDNEEKDSAFRGICMMIGVNPGGVVQDFIFFCDAVASWVSP KDDLRDMFYKILHGFKDQVGEDNWQQFSEQFPPLLKERLAAFYGV | 28 |
| H5-20 | MRPLNIMIPKFLQFFKHCSPKIRSHAIACVNQFIMDRAQALMDNI DTFIEHLFALAVDDDPEVRKNVCRALVMLLEVRIDRLIPHMHSII QYMLQRTQDHDENVALEACEFWLTLAEQPICKEVLASHLVQLIPI LVNGMKYSEIDIILLKGDVEEDEAVPDSEQDIKPRFHKSRTVTLP HEAERPDGSEDAEDDDDDDALSDWNLRKCSAAALDVLANVFREEL LPHLLPLLKGLLFHPEWVVKESGILVLGAIAEGCMQGMVPYLPEL IPHLIQCLSDKKALVRSIACWTLSRYAHWVVSQPPDMHLKPLMTE LLKRILDGNKRVQEAACSAFATLEEEACTELVPYLSYILDTLVFA FGKYQHKNLLILYDAIGTLADSVGHHLNQPEYIQKLMPPLIQKWN ELKDEDKDLFPLLECLSSVATALQSGFLPYCEPVYQRCVTLVQKT LAQAMMYTQHPEQYEAPDKDFMIVALDLLSGLAEGLGGHVEQLVA RSNIMTLLFQCMQDSMPEVRQSSFALLGDLTKACFIHVKPCIAEF MPILGTNLNPEFISVCNNATWAIGEICMQMGAEMQPYVQMVLNNL VEIINRPNTPKTLLENTAITIGRLGYVCPQEVAPMLQQFIRPWCT SLRNIRDNEEKDSAFRGICMMIGVNPGGVVQDFIFFCDAVASWVS PKDDLRDMFYKILHGFKDQVGEDNWQQFSEQFPPLLKERLAAFYG V | 29 |
| H6-20 | MDNIDTFIEHLFALAVDDDPEVRKNVCRALVMLLEVRIDRLIPHM HSIIQYMLQRTQDHDENVALEACEFWLTLAEQPICKEVLASHLVQ LIPILVNGMKYSEIDIILLKGDVEEDEAVPDSEQDIKPRFHKSRT VTLPHEAERPDGSEDAEDDDDDDALSDWNLRKCSAAALDVLANVF REELLPHLLPLLKGLLFHPEWVVKESGILVLGAIAEGCMQGMVPY LPELIPHLIQCLSDKKALVRSIACWTLSRYAHWVVSQPPDMHLKP LMTELLKRILDGNKRVQEAACSAFATLEEEACTELVPYLSYILDT LVFAFGKYQHKNLLILYDAIGTLADSVGHHLNQPEYIQKLMPPLI QKWNELKDEDKDLFPLLECLSSVATALQSGFLPYCEPVYQRCVTL VQKTLAQAMMYTQHPEQYEAPDKDFMIVALDLLSGLAEGLGGHVE QLVARSNIMTLLFQCMQDSMPEVRQSSFALLGDLTKACFIHVKPC IAEFMPILGTNLNPEFISVCNNATWAIGEICMQMGAEMQPYVQMV LNNLVEIINRPNTPKTLLENTAITIGRLGYVCPQEVAPMLQQFIR PWCTSLRNIRDNEEKDSAFRGICMMIGVNPGGVVQDFIFFCDAVA SWVSPKDDLRDMFYKILHGFKDQVGEDNWQQFSEQFPPLLKERLA AFYGV | 30 |
| H7-20 | MLIPHMHSIIQYMLQRTQDHDENVALEACEFWLTLAEQPICKEVL ASHLVQLIPILVNGMKYSEIDIILLKGDVEEDEAVPDSEQDIKPR FHKSRTVTLPHEAERPDGSEDAEDDDDDDALSDWNLRKCSAAALD VLANVFREELLPHLLPLLKGLLFHPEWVVKESGILVLGAIAEGCM QGMVPYLPELIPHLIQCLSDKKALVRSIACWTLSRYAHWVVSQPP DMHLKPLMTELLKRILDGNKRVQEAACSAFATLEEEACTELVPYL SYILDTLVFAFGKYQHKNLLILYDAIGTLADSVGHHLNQPEYIQK LMPPLIQKWNELKDEDKDLFPLLECLSSVATALQSGFLPYCEPVY QRCVTLVQKTLAQAMMYTQHPEQYEAPDKDFMIVALDLLSGLAEG LGGHVEQLVARSNIMTLLFQCMQDSMPEVRQSSFALLGDLTKACF IHVKPCIAEFMPILGTNLNPEFISVCNNATWAIGEICMQMGAEMQ PYVQMVLNNLVEIINRPNTPKTLLENTAITIGRLGYVCPQEVAPM LQQFIRPWCTSLRNIRDNEEKDSAFRGICMMIGVNPGGVVQDFIF FCDAVASWVSPKDDLRDMFYKILHGFKDQVGEDNWQQFSEQFPPL LKERLAAFYGV | 31 |
| H8-20 | MCKEVLASHLVQLIPILVNGMKYSEIDIILLKGDVEEDEAVPDSE QDIKPRFHKSRTVTLPHEAERPDGSEDAEDDDDDDALSDWNLRKC SAAALDVLANVFREELLPHLLPLLKGLLFHPEWVVKESGILVLGA IAEGCMQGMVPYLPELIPHLIQCLSDKKALVRSIACWTLSRYAHW VVSQPPDMHLKPLMTELLKRILDGNKRVQEAACSAFATLEEEACT ELVPYLSYILDTLVFAFGKYQHKNLLILYDAIGTLADSVGHHLNQ PEYIQKLMPPLIQKWNELKDEDKDLFPLLECLSSVATALQSGFLP YCEPVYQRCVTLVQKTLAQAMMYTQHPEQYEAPDKDFMIVALDLL SGLAEGLGGHVEQLVARSNIMTLLFQCMQDSMPEVRQSSFALLGD LTKACFIHVKPCIAEFMPILGTNLNPEFISVCNNATWAIGEICMQ MGAEMQPYVQMVLNNLVEIINRPNTPKTLLENTAITIGRLGYVCP QEVAPMLQQFIRPWCTSLRNIRDNEEKDSAFRGICMMIGVNPGGV VQDFIFFCDAVASWVSPKDDLRDMFYKILHGFKDQVGEDNWQQFS EQFPPLLKERLAAFYGV | 32 |
| H9-20 | MLPHLLPLLKGLLFHPEWVVKESGILVLGAIAEGCMQGMVPYLPE LIPHLIQCLSDKKALVRSIACWTLSRYAHWVVSQPPDMHLKPLMT ELLKRILDGNKRVQEAACSAFATLEEEACTELVPYLSYILDTLVF AFGKYQHKNLLILYDAIGTLADSVGHHLNQPEYIQKLMPPLIQKW NELKDEDKDLFPLLECLSSVATALQSGFLPYCEPVYQRCVTLVQK TLAQAMMYTQHPEQYEAPDKDFMIVALDLLSGLAEGLGGHVEQLV ARSNIMTLLFQCMQDSMPEVRQSSFALLGDLTKACFIHVKPCIAE FMPILGTNLNPEFISVCNNATWAIGEICMQMGAEMQPYVQMVLNN LVEIINRPNTPKTLLENTAITIGRLGYVCPQEVAPMLQQFIRPWC TSLRNIRDNEEKDSAFRGICMMIGVNPGGVVQDFIFFCDAVASWV SPKDDLRDMFYKILHGFKDQVGEDNWQQFSEQFPPLLKERLAAFY GV | 33 |
| H10-20 | MVPYLPELIPHLIQCLSDKKALVRSIACWTLSRYAHWVVSQPPDM HLKPLMTELLKRILDGNKRVQEAACSAFATLEEEACTELVPYLSY ILDTLVFAFGKYQHKNLLILYDAIGTLADSVGHHLNQPEYIQKLM PPLIQKWNELKDEDKDLFPLLECLSSVATALQSGFLPYCEPVYQR CVTLVQKTLAQAMMYTQHPEQYEAPDKDFMIVALDLLSGLAEGLG GHVEQLVARSNIMTLLFQCMQDSMPEVRQSSFALLGDLTKACFIH VKPCIAEFMPILGTNLNPEFISVCNNATWAIGEICMQMGAEMQPY VQMVLNNLVEIINRPNTPKTLLENTAITIGRLGYVCPQEVAPMLQ QFIRPWCTSLRNIRDNEEKDSAFRGICMMIGVNPGGVVQDFIFFC DAVASWVSPKDDLRDMFYKILHGFKDQVGEDNWQQFSEQFPPLLK ERLAAFYGV | 34 |
| H11-20 | MQPPDMHLKPLMTELLKRILDGNKRVQEAACSAFATLEEEACTEL VPYLSYILDTLVFAFGKYQHKNLLILYDAIGTLADSVGHHLNQPE YIQKLMPPLIQKWNELKDEDKDLFPLLECLSSVATALQSGFLPYC EPVYQRCVTLVQKTLAQAMMYTQHPEQYEAPDKDFMIVALDLLSG LAEGLGGHVEQLVARSNIMTLLFQCMQDSMPEVRQSSFALLGDLT KACFIHVKPCIAEFMPILGTNLNPEFISVCNNATWAIGEICMQMG AEMQPYVQMVLNNLVEIINRPNTPKTLLENTAITIGRLGYVCPQE VAPMLQQFIRPWCTSLRNIRDNEEKDSAFRGICMMIGVNPGGVVQ DFIFFCDAVASWVSPKDDLRDMFYKILHGFKDQVGEDNWQQFSEQ FPPLLKERLAAFYGV | 35 |
| H12-20 | MVPYLSYILDTLVFAFGKYQHKNLLILYDAIGTLADSVGHHLNQP EYIQKLMPPLIQKWNELKDEDKDLFPLLECLSSVATALQSGFLPY CEPVYQRCVTLVQKTLAQAMMYTQHPEQYEAPDKDFMIVALDLLS GLAEGLGGHVEQLVARSNIMTLLFQCMQDSMPEVRQSSFALLGDL TKACFIHVKPCIAEFMPILGTNLNPEFISVCNNATWAIGEICMQM GAEMQPYVQMVLNNLVEIINRPNTPKTLLENTAITIGRLGYVCPQ EVAPMLQQFIRPWCTSLRNIRDNEEKDSAFRGICMMIGVNPGGVV QDFIFFCDAVASWVSPKDDLRDMFYKILHGFKDQVGEDNWQQFSE QFPPLLKERLAAFYGV | 36 |
| H13-20 | MNQPEYIQKLMPPLIQKWNELKDEDKDLFPLLECLSSVATALQSG FLPYCEPVYQRCVTLVQKTLAQAMMYTQHPEQYEAPDKDFMIVAL DLLSGLAEGLGGHVEQLVARSNIMTLLFQCMQDSMPEVRQSSFAL LGDLTKACFIHVKPCIAEFMPILGTNLNPEFISVCNNATWAIGEI CMQMGAEMQPYVQMVLNNLVEIINRPNTPKTLLENTAITIGRLGY VCPQEVAPMLQQFIRPWCTSLRNIRDNEEKDSAFRGICMMIGVNP GGVVQDFIFFCDAVASWVSPKDDLRDMFYKILHGFKDQVGEDNWQ QFSEQFPPLLKERLAAFYGV | 37 |
| H14-20 | MPYCEPVYQRCVTLVQKTLAQAMMYTQHPEQYEAPDKDFMIVALD LLSGLAEGLGGHVEQLVARSNIMTLLFQCMQDSMPEVRQSSFALL GDLTKACFIHVKPCIAEFMPILGTNLNPEFISVCNNATWAIGEIC MQMGAEMQPYVQMVLNNLVEIINRPNTPKTLLENTAITIGRLGYV CPQEVAPMLQQFIRPWCTSLRNIRDNEEKDSAFRGICMMIGVNPG GVVQDFIFFCDAVASWVSPKDDLRDMFYKILHGFKDQVGEDNWQQ FSEQFPPLLKERLAAFYGV | 38 |
| H8-19 | MCKEVLASHLVQLIPILVNGMKYSEIDIILLKGDVEEDEAVPDSE QDIKPRFHKSRTVTLPHEAERPDGSEDAEDDDDDDALSDWNLRKC SAAALDVLANVFREELLPHLLPLLKGLLFHPEWVVKESGILVLGA IAEGCMQGMVPYLPELIPHLIQCLSDKKALVRSIACWTLSRYAHW VVSQPPDMHLKPLMTELLKRILDGNKRVQEAACSAFATLEEEACT ELVPYLSYILDTLVFAFGKYQHKNLLILYDAIGTLADSVGHHLNQ PEYIQKLMPPLIQKWNELKDEDKDLFPLLECLSSVATALQSGFLP YCEPVYQRCVTLVQKTLAQAMMYTQHPEQYEAPDKDFMIVALDLL SGLAEGLGGHVEQLVARSNIMTLLFQCMQDSMPEVRQSSFALLGD LTKACFIHVKPCIAEFMPILGTNLNPEFISVCNNATWAIGEICMQ MGAEMQPYVQMVLNNLVEIINRPNTPKTLLENTAITIGRLGYVCP QEVAPMLQQFIRPWCTSLRNIRDNEEKDSAFRGICMMIGVNPGGV VQDFIFFCDAVASWVSPKDDLRDMFYKILHGFKDQVG | 39 |
| H8-18 | MCKEVLASHLVQLIPILVNGMKYSEIDIILLKGDVEEDEAVPDSE QDIKPRFHKSRTVTLPHEAERPDGSEDAEDDDDDDALSDWNLRKC SAAALDVLANVFREELLPHLLPLLKGLLFHPEWVVKESGILVLGA IAEGCMQGMVPYLPELIPHLIQCLSDKKALVRSIACWTLSRYAHW VVSQPPDMHLKPLMTELLKRILDGNKRVQEAACSAFATLEEEACT ELVPYLSYILDTLVFAFGKYQHKNLLILYDAIGTLADSVGHHLNQ PEYIQKLMPPLIQKWNELKDEDKDLFPLLECLSSVATALQSGFLP YCEPVYQRCVTLVQKTLAQAMMYTQHPEQYEAPDKDFMIVALDLL SGLAEGLGGHVEQLVARSNIMTLLFQCMQDSMPEVRQSSFALLGD LTKACFIHVKPCIAEFMPILGTNLNPEFISVCNNATWAIGEICMQ MGAEMQPYVQMVLNNLVEIINRPNTPKTLLENTAITIGRLGYVCP QEVAPMLQQFIRPWCTSLRNIRDNEEKDSAFRGICMMIGVNP | 40 |
| H8-17 | MCKEVLASHLVQLIPILVNGMKYSEIDIILLKGDVEEDEAVPDSE QDIKPRFHKSRTVTLPHEAERPDGSEDAEDDDDDDALSDWNLRKC SAAALDVLANVFREELLPHLLPLLKGLLFHPEWVVKESGILVLGA IAEGCMQGMVPYLPELIPHLIQCLSDKKALVRSIACWTLSRYAHW VVSQPPDMHLKPLMTELLKRILDGNKRVQEAACSAFATLEEEACT ELVPYLSYILDTLVFAFGKYQHKNLLILYDAIGTLADSVGHHLNQ PEYIQKLMPPLIQKWNELKDEDKDLFPLLECLSSVATALQSGFLP YCEPVYQRCVTLVQKTLAQAMMYTQHPEQYEAPDKDFMIVALDLL SGLAEGLGGHVEQLVARSNIMTLLFQCMQDSMPEVRQSSFALLGD LTKACFIHVKPCIAEFMPILGTNLNPEFISVCNNATWAIGEICMQ MGAEMQPYVQMVLNNLVEIINRPNTPKTLLENTAITIGRLGYVC | 41 |
| H8-16 | MCKEVLASHLVQLIPILVNGMKYSEIDIILLKGDVEEDEAVPDSE QDIKPRFHKSRTVTLPHEAERPDGSEDAEDDDDDDALSDWNLRKC SAAALDVLANVFREELLPHLLPLLKGLLFHPEWVVKESGILVLGA IAEGCMQGMVPYLPELIPHLIQCLSDKKALVRSIACWTLSRYAHW VVSQPPDMHLKPLMTELLKRILDGNKRVQEAACSAFATLEEEACT ELVPYLSYILDTLVFAFGKYQHKNLLILYDAIGTLADSVGHHLNQ PEYIQKLMPPLIQKWNELKDEDKDLFPLLECLSSVATALQSGFLP YCEPVYQRCVTLVQKTLAQAMMYTQHPEQYEAPDKDFMIVALDLL SGLAEGLGGHVEQLVARSNIMTLLFQCMQDSMPEVRQSSFALLGD LTKACFIHVKPCIAEFMPILGTNLNPEFISVCNNATWAIGEICMQ MGAEMQP | 42 |

In some cases, an IPO3 polypeptide fragment provided herein can include the amino acid sequence set forth in any one of SEQ ID NOs:26-42 with zero, one, two three, or more (e.g., five, eight, 12, 15, 18, 20, 25, 30, 35, 40, or more) amino acid substitutions (e.g., one or more K→R substitutions) within the articulated sequence of the sequence identifier (e.g., any one of SEQ ID NOs:26-42), with zero, one, two, three, four, or five amino acid residues preceding the articulated sequence of the sequence identifier (e.g., any one of SEQ ID NOs:26-42, and/or with zero, one, two, three, four, or five amino acid residues following the articulated sequence of the sequence identifier (e.g., any one of SEQ ID NOs:26-42), provided that the IPO3 polypeptide fragment provided herein retains at least some activity exhibited by a naturally-occurring IPO3 polypeptide (e.g., the ability to reduce at least some TDP-43 aggregation and/or restore at least some nuclear localization of TDP-43 polypeptides). Examples of such IPO3 polypeptide fragments can be those set forth in Table 4.

**Table 4. Exemplary IPO3 polypeptide fragments.**

| Polypeptide Sequence | SEQ ID NO: |
|---|---|
| MNQFPDFNNYLIFVLTRLRSEDEPTRSLSGLILRNNVRAHYQSFPPPVADFIRQEC LNNIGDASSLIRATIGILITTIASRGELQMWPELLPQLCNLLNSEDYNTCEGAFGA LQRICEDSSELLDSDALNRPLNIMIPRFLQFFRHCSPRIRSHAIACVNQFIMDRAQ ALMDNIDTFIEHLFALAVDDDPEVRRNVCRALVMLLEVRIDRLIPHMHSIIQYMLQ RTQDHDENVALEACEFWLTLAEQPICREVLASHLVQLIPILVNGMRYSEIDIILLR GDVEEDEAVPDSEQDIRPRFHRSRTVTLPHEAERPDGSEDAEDDDDDDALSDWNLR RCSAAALDVLANVFREELLPHLLPLLRGLLFHPEWVVRESGILVLGAIAEGCMQGM VPYLPELIPHLIQCLSDRRALVRSIACWTLSRYAHWVVSQPPDMHLRPLMTELLRR ILDGNRRVQEAACSAFATLEEEACTELVPYLSYILDTLVFAFGRYQHRNLLILYDA IGTLADSVGHHLNQPEYIQRLMPPLIQRWNELRDEDRDLFPLLECLSSVATALQSG FLPYCEPVYQRCVTLVQRTLAQAMMYTQHPEQYEAPDRDFMIVALDLLSGLAEGLG GHVEQLVARSNIMTLLFQCMQDSMPEVRQSSFALLGDLTRACFIHVRPCIAEFMPI LGTNLNPEFISVCNNATWAIGEICMQMGAEMQPYVQMVLNNLVEIINRPNTPRTLL ENTAITIGRLGYVCPQEVAPMLQQFIRPWCTSLRNIRDNEERDSAFRGICMMIGVN PGGVVQDFIFFCDAVASWVSPRDDLRDMFYRILHGFRDQVGEDNWQQFSEQFPPLL RERLAAFYGV | 43 |
| MPVADFIRQECLNNIGDASSLIRATIGILITTIASRGELQMWPELLPQLCNLLNSE DYNTCEGAFGALQRICEDSSELLDSDALNRPLNIMIPRFLQFFRHCSPRIRSHAIA CVNQFIMDRAQALMDNIDTFIEHLFALAVDDDPEVRRNVCRALVMLLEVRIDRLIP HMHSIIQYMLQRTQDHDENVALEACEFWLTLAEQPICREVLASHLVQLIPILVNGM RYSEIDIILLRGDVEEDEAVPDSEQDIRPRFHRSRTVTLPHEAERPDGSEDAEDDD DDDALSDWNLRRCSAAALDVLANVFREELLPHLLPLLRGLLFHPEWVVRESGILVL GAIAEGCMQGMVPYLPELIPHLIQCLSDRRALVRSIACWTLSRYAHWVVSQPPDMH LRPLMTELLRRILDGNRRVQEAACSAFATLEEEACTELVPYLSYILDTLVFAFGRY QHRNLLILYDAIGTLADSVGHHLNQPEYIQRLMPPLIQRWNELRDEDRDLFPLLEC LSSVATALQSGFLPYCEPVYQRCVTLVQRTLAQAMMYTQHPEQYEAPDRDFMIVAL DLLSGLAEGLGGHVEQLVARSNIMTLLFQCMQDSMPEVRQSSFALLGDLTRACFIH VRPCIAEFMPILGTNLNPEFISVCNNATWAIGEICMQMGAEMQPYVQMVLNNLVEI INRPNTPRTLLENTAITIGRLGYVCPQEVAPMLQQFIRPWCTSLRNIRDNEERDSA FRGICMMIGVNPGGVVQDFIFFCDAVASWVSPRDDLRDMFYRILHGFRDQVGEDNW QQFSEQFPPLLRERLAAFYGV | 44 |
| MWPELLPQLCNLLNSEDYNTCEGAFGALQRICEDSSELLDSDALNRPLNIMIPRFL QFFRHCSPRIRSHAIACVNQFIMDRAQALMDNIDTFIEHLFALAVDDDPEVRRNVC RALVMLLEVRIDRLIPHMHSIIQYMLQRTQDHDENVALEACEFWLTLAEQPICREV LASHLVQLIPILVNGMRYSEIDIILLRGDVEEDEAVPDSEQDIRPRFHRSRTVTLP HEAERPDGSEDAEDDDDDDALSDWNLRRCSAAALDVLANVFREELLPHLLPLLRGL LFHPEWVVRESGILVLGAIAEGCMQGMVPYLPELIPHLIQCLSDRRALVRSIACWT LSRYAHWVVSQPPDMHLRPLMTELLRRILDGNRRVQEAACSAFATLEEEACTELVP YLSYILDTLVFAFGRYQHRNLLILYDAIGTLADSVGHHLNQPEYIQRLMPPLIQRW NELRDEDRDLFPLLECLSSVATALQSGFLPYCEPVYQRCVTLVQRTLAQAMMYTQH PEQYEAPDRDFMIVALDLLSGLAEGLGGHVEQLVARSNIMTLLFQCMQDSMPEVRQ SSFALLGDLTRACFIHVRPCIAEFMPILGTNLNPEFISVCNNATWAIGEICMQMGA EMQPYVQMVLNNLVEIINRPNTPRTLLENTAITIGRLGYVCPQEVAPMLQQFIRPW CTSLRNIRDNEERDSAFRGICMMIGVNPGGVVQDFIFFCDAVASWVSPRDDLRDMF YRILHGFRDQVGEDNWQQFSEQFPPLLRERLAAFYGV | 45 |
| MRPLNIMIPRFLQFFRHCSPRIRSHAIACVNQFIMDRAQALMDNIDTFIEHLFALA VDDDPEVRRNVCRALVMLLEVRIDRLIPHMHSIIQYMLQRTQDHDENVALEACEFW LTLAEQPICREVLASHLVQLIPILVNGMRYSEIDIILLRGDVEEDEAVPDSEQDIR PRFHRSRTVTLPHEAERPDGSEDAEDDDDDDALSDWNLRRCSAAALDVLANVFREE LLPHLLPLLRGLLFHPEWVVRESGILVLGAIAEGCMQGMVPYLPELIPHLIQCLSD RRALVRSIACWTLSRYAHWVVSQPPDMHLRPLMTELLRRILDGNRRVQEAACSAFA TLEEEACTELVPYLSYILDTLVFAFGRYQHRNLLILYDAIGTLADSVGHHLNQPEY IQRLMPPLIQRWNELRDEDRDLFPLLECLSSVATALQSGFLPYCEPVYQRCVTLVQ RTLAQAMMYTQHPEQYEAPDRDFMIVALDLLSGLAEGLGGHVEQLVARSNIMTLLF QCMQDSMPEVRQSSFALLGDLTRACFIHVRPCIAEFMPILGTNLNPEFISVCNNAT WAIGEICMQMGAEMQPYVQMVLNNLVEIINRPNTPRTLLENTAITIGRLGYVCPQE VAPMLQQFIRPWCTSLRNIRDNEERDSAFRGICMMIGVNPGGVVQDFIFFCDAVAS WVSPRDDLRDMFYRILHGFRDQVGEDNWQQFSEQFPPLLRERLAAFYGV | 46 |
| MDNIDTFIEHLFALAVDDDPEVRRNVCRALVMLLEVRIDRLIPHMHSIIQYMLQRT QDHDENVALEACEFWLTLAEQPICREVLASHLVQLIPILVNGMRYSEIDIILLRGD VEEDEAVPDSEQDIRPRFHRSRTVTLPHEAERPDGSEDAEDDDDDDALSDWNLRRC SAAALDVLANVFREELLPHLLPLLRGLLFHPEWVVRESGILVLGAIAEGCMQGMVP YLPELIPHLIQCLSDRRALVRSIACWTLSRYAHWVVSQPPDMHLRPLMTELLRRIL DGNRRVQEAACSAFATLEEEACTELVPYLSYILDTLVFAFGRYQHRNLLILYDAIG TLADSVGHHLNQPEYIQRLMPPLIQRWNELRDEDRDLFPLLECLSSVATALQSGFL PYCEPVYQRCVTLVQRTLAQAMMYTQHPEQYEAPDRDFMIVALDLLSGLAEGLGGH VEQLVARSNIMTLLFQCMQDSMPEVRQSSFALLGDLTRACFIHVRPCIAEFMPILG TNLNPEFISVCNNATWAIGEICMQMGAEMQPYVQMVLNNLVEIINRPNTPRTLLEN TAITIGRLGYVCPQEVAPMLQQFIRPWCTSLRNIRDNEERDSAFRGICMMIGVNPG GVVQDFIFFCDAVASWVSPRDDLRDMFYRILHGFRDQVGEDNWQQFSEQFPPLLRE RLAAFYGV | 47 |
| MLIPHMHSIIQYMLQRTQDHDENVALEACEFWLTLAEQPICREVLASHLVQLIPIL VNGMRYSEIDIILLRGDVEEDEAVPDSEQDIRPRFHRSRTVTLPHEAERPDGSEDA EDDDDDDALSDWNLRRCSAAALDVLANVFREELLPHLLPLLRGLLFHPEWVVRESG ILVLGAIAEGCMQGMVPYLPELIPHLIQCLSDRRALVRSIACWTLSRYAHWVVSQP PDMHLRPLMTELLRRILDGNRRVQEAACSAFATLEEEACTELVPYLSYILDTLVFA FGRYQHRNLLILYDAIGTLADSVGHHLNQPEYIQRLMPPLIQRWNELRDEDRDLFP LLECLSSVATALQSGFLPYCEPVYQRCVTLVQRTLAQAMMYTQHPEQYEAPDRDFM IVALDLLSGLAEGLGGHVEQLVARSNIMTLLFQCMQDSMPEVRQSSFALLGDLTRA CFIHVRPCIAEFMPILGTNLNPEFISVCNNATWAIGEICMQMGAEMQPYVQMVLNN LVEIINRPNTPRTLLENTAITIGRLGYVCPQEVAPMLQQFIRPWCTSLRNIRDNEE RDSAFRGICMMIGVNPGGVVQDFIFFCDAVASWVSPRDDLRDMFYRILHGFRDQVG EDNWQQFSEQFPPLLRERLAAFYGV | 48 |
| MCREVLASHLVQLIPILVNGMRYSEIDIILLRGDVEEDEAVPDSEQDIRPRFHRSR TVTLPHEAERPDGSEDAEDDDDDDALSDWNLRRCSAAALDVLANVFREELLPHLLP LLRGLLFHPEWVVRESGILVLGAIAEGCMQGMVPYLPELIPHLIQCLSDRRALVRS IACWTLSRYAHWVVSQPPDMHLRPLMTELLRRILDGNRRVQEAACSAFATLEEEAC TELVPYLSYILDTLVFAFGRYQHRNLLILYDAIGTLADSVGHHLNQPEYIQRLMPP LIQRWNELRDEDRDLFPLLECLSSVATALQSGFLPYCEPVYQRCVTLVQRTLAQAM MYTQHPEQYEAPDRDFMIVALDLLSGLAEGLGGHVEQLVARSNIMTLLFQCMQDSM PEVRQSSFALLGDLTRACFIHVRPCIAEFMPILGTNLNPEFISVCNNATWAIGEIC MQMGAEMQPYVQMVLNNLVEIINRPNTPRTLLENTAITIGRLGYVCPQEVAPMLQQ FIRPWCTSLRNIRDNEERDSAFRGICMMIGVNPGGVVQDFIFFCDAVASWVSPRDD LRDMFYRILHGFRDQVGEDNWQQFSEQFPPLLRERLAAFYGV | 49 |
| MLPHLLPLLRGLLFHPEWVVRESGILVLGAIAEGCMQGMVPYLPELIPHLIQCLSD RRALVRSIACWTLSRYAHWVVSQPPDMHLRPLMTELLRRILDGNRRVQEAACSAFA TLEEEACTELVPYLSYILDTLVFAFGRYQHRNLLILYDAIGTLADSVGHHLNQPEY IQRLMPPLIQRWNELRDEDRDLFPLLECLSSVATALQSGFLPYCEPVYQRCVTLVQ RTLAQAMMYTQHPEQYEAPDRDFMIVALDLLSGLAEGLGGHVEQLVARSNIMTLLF QCMQDSMPEVRQSSFALLGDLTRACFIHVRPCIAEFMPILGTNLNPEFISVCNNAT WAIGEICMQMGAEMQPYVQMVLNNLVEIINRPNTPRTLLENTAITIGRLGYVCPQE VAPMLQQFIRPWCTSLRNIRDNEERDSAFRGICMMIGVNPGGVVQDFIFFCDAVAS WVSPRDDLRDMFYRILHGFRDQVGEDNWQQFSEQFPPLLRERLAAFYGV | 50 |
| MVPYLPELIPHLIQCLSDRRALVRSIACWTLSRYAHWVVSQPPDMHLRPLMTELLR RILDGNRRVQEAACSAFATLEEEACTELVPYLSYILDTLVFAFGRYQHRNLLILYD AIGTLADSVGHHLNQPEYIQRLMPPLIQRWNELRDEDRDLFPLLECLSSVATALQS GFLPYCEPVYQRCVTLVQRTLAQAMMYTQHPEQYEAPDRDFMIVALDLLSGLAEGL GGHVEQLVARSNIMTLLFQCMQDSMPEVRQSSFALLGDLTRACFIHVRPCIAEFMP ILGTNLNPEFISVCNNATWAIGEICMQMGAEMQPYVQMVLNNLVEIINRPNTPRTL LENTAITIGRLGYVCPQEVAPMLQQFIRPWCTSLRNIRDNEERDSAFRGICMMIGV NPGGVVQDFIFFCDAVASWVSPRDDLRDMFYRILHGFRDQVGEDNWQQFSEQFPPL LRERLAAFYGV | 51 |
| MQPPDMHLRPLMTELLRRILDGNRRVQEAACSAFATLEEEACTELVPYLSYILDTL VFAFGRYQHRNLLILYDAIGTLADSVGHHLNQPEYIQRLMPPLIQRWNELRDEDRD LFPLLECLSSVATALQSGFLPYCEPVYQRCVTLVQRTLAQAMMYTQHPEQYEAPDR DFMIVALDLLSGLAEGLGGHVEQLVARSNIMTLLFQCMQDSMPEVRQSSFALLGDL TRACFIHVRPCIAEFMPILGTNLNPEFISVCNNATWAIGEICMQMGAEMQPYVQMV LNNLVEIINRPNTPRTLLENTAITIGRLGYVCPQEVAPMLQQFIRPWCTSLRNIRD NEERDSAFRGICMMIGVNPGGVVQDFIFFCDAVASWVSPRDDLRDMFYRILHGFRD QVGEDNWQQFSEQFPPLLRERLAAFYGV | 52 |
| MVPYLSYILDTLVFAFGRYQHRNLLILYDAIGTLADSVGHHLNQPEYIQRLMPPLI QRWNELRDEDRDLFPLLECLSSVATALQSGFLPYCEPVYQRCVTLVQRTLAQAMMY TQHPEQYEAPDRDFMIVALDLLSGLAEGLGGHVEQLVARSNIMTLLFQCMQDSMPE VRQSSFALLGDLTRACFIHVRPCIAEFMPILGTNLNPEFISVCNNATWAIGEICMQ MGAEMQPYVQMVLNNLVEIINRPNTPRTLLENTAITIGRLGYVCPQEVAPMLQQFI RPWCTSLRNIRDNEERDSAFRGICMMIGVNPGGVVQDFIFFCDAVASWVSPRDDLR DMFYRILHGFRDQVGEDNWQQFSEQFPPLLRERLAAFYGV | 53 |
| MNQPEYIQRLMPPLIQRWNELRDEDRDLFPLLECLSSVATALQSGFLPYCEPVYQR CVTLVQRTLAQAMMYTQHPEQYEAPDRDFMIVALDLLSGLAEGLGGHVEQLVARSN IMTLLFQCMQDSMPEVRQSSFALLGDLTRACFIHVRPCIAEFMPILGTNLNPEFIS VCNNATWAIGEICMQMGAEMQPYVQMVLNNLVEIINRPNTPRTLLENTAITIGRLG YVCPQEVAPMLQQFIRPWCTSLRNIRDNEERDSAFRGICMMIGVNPGGVVQDFIFF CDAVASWVSPRDDLRDMFYRILHGFRDQVGEDNWQQFSEQFPPLLRERLAAFYGV | 54 |
| MPYCEPVYQRCVTLVQRTLAQAMMYTQHPEQYEAPDRDFMIVALDLLSGLAEGLGG HVEQLVARSNIMTLLFQCMQDSMPEVRQSSFALLGDLTRACFIHVRPCIAEFMPIL GTNLNPEFISVCNNATWAIGEICMQMGAEMQPYVQMVLNNLVEIINRPNTPRTLLE NTAITIGRLGYVCPQEVAPMLQQFIRPWCTSLRNIRDNEERDSAFRGICMMIGVNP GGVVQDFIFFCDAVASWVSPRDDLRDMFYRILHGFRDQVGEDNWQQFSEQFPPLLR ERLAAFYGV | 55 |

When an importin polypeptide is an IPO13 polypeptide, any appropriate IPO13 polypeptide (and/or nucleic acid designed to express an IPO13 polypeptide) can be administered to a mammal (e.g., a human) having, or at risk of developing, a proteinopathy (e.g., a TDP-43 proteinopathy) as described herein. Examples of IPO13 polypeptides and nucleic acids encoding IPO13 polypeptides include, without limitation, those set forth in the NCBI databases at, for example, accession no. NP_055467 (version NP_055467.3), accession no. XP_024306837 (version XP_024306837.1), and accession no. NP_666264 (version NP_666264.1).

In some cases, an IPO13 polypeptide can have an amino acid sequence set forth in SEQ ID NO:5 (see, e.g., Figure 12A). In some cases, a nucleic acid encoding an IPO13 polypeptide can have an nucleotide sequence set forth in SEQ ID NO:6 (see, e.g., Figure 12B).

When a fragment of an importin polypeptide is an IPO13 polypeptide fragment, any appropriate IPO13 polypeptide fragment (and/or nucleic acid designed to express a KPNB1 polypeptide fragment) can be administered to a mammal (e.g., a human) having, or at risk of developing, a proteinopathy (e.g., a TDP-43 proteinopathy) as described herein. In some cases, an IPO13 polypeptide fragment can be derived from the amino acid sequence set forth in SEQ ID NO:5.

An IPO13 polypeptide fragment can be any appropriate length (e.g., can include any number of amino acids) provided that it maintains at least some function of a naturally-occurring IPO13 polypeptide (e.g., the ability to reduce at least some TDP-43 aggregation and/or restore at least some nuclear localization of TDP-43 polypeptides). For example, an IPO13 polypeptide fragment can be from about 100 amino acids in length to about 950 amino acids in length (e.g., from about 100 amino acids to about 800 amino acids, from about 100 amino acids to about 750 amino acids, from about 100 amino acids to about 700 amino acids, from about 100 amino acids to about 650 amino acids, from about 100 amino acids to about 600 amino acids, from about 100 amino acids to about 550 amino acids, from about 100 amino acids to about 500 amino acids, from about 100 amino acids to about 450 amino acids, from about 100 amino acids to about 400 amino acids, from about 100 amino acids to about 350 amino acids, from about 100 amino acids to about 300 amino acids, from about 100 amino acids to about 250 amino acids, from about 200 amino acids to about 950 amino acids, from about 250 amino acids to about 950 amino acids, from about 300 amino acids to about 950 amino acids, from about 350 amino acids to about 950 amino acids, from about 400 amino acids to about 950 amino acids, from about 450 amino acids to about 950 amino acids, from about 500 amino acids to about 950 amino acids, from about 550 amino acids to about 950 amino acids, from about 600 amino acids to about 950 amino acids, from about 650 amino acids to about 950 amino acids, from about 700 amino acids to about 950 amino acids, from about 750 amino acids to about 950 amino acids, from about 800 amino acids to about 950 amino acids, from about 200 amino acids to about 900 amino acids, from about 300 amino acids to about 800 amino acids, from about 400 amino acids to about 700 amino acids, from about 500 amino acids to about 600 amino acids, from about 200 amino acids to about 400 amino acids, from about 300 amino acids to about 500 amino acids, from about 400 amino acids to about 600 amino acids, from about 500 amino acids to about 700 amino acids, from about 600 amino acids to about 800 amino acids, or from about 700 amino acids to about 900 amino acids in length) provided that it maintains at least some function of a naturally-occurring IPO13 polypeptide (e.g., the ability to reduce at least some TDP-43 aggregation).

In some cases, an IPO13 polypeptide fragment can comprise, consist essentially of, or consist of an amino acid sequence set forth in Table 5.

**Table 5. Exemplary IPO13 polypeptide fragments.**

| Truncation | Polypeptide Sequence | SEQ ID NO: |
|---|---|---|
| Δ1-22aa | MVENVEKALHQLYYDPNIENKNLAQKWLMQAQVSPQAWHFSWQLL QPDKVPEIQYFGASALHIKISRYWSDIPTDQYESLKAQLFTQITR FASGSKIVLTRLCVALASLALSMMPDAWPCAVADMVRLFQAEDSP VDGQGRCLALLELLTVLPEEFQTSRLPQYRKGLVRTSLAVECGAV FPLLEQLLQQPSSPSCVRQKVLKCFSSWVQLEVPLQDCEALIQAA FAALQDSELFDSSVEAIVNAISQPDAQRYVNTLLKLIPLVLGLQE QLRQAVQNGDMETSHGICRIAVALGENHSRALLDQVEHWQSFLAL VNMIMFCTGIPGHYPVNETTSSLTLTFWYTLQDDILSFEAEKQAV YQQVYRPVYFQLVDVLLHKAQFPSDEEYGFWSSDEKEQFRIYRVD ISDTLMYVYEMLGAELLSNLYDKLGRLLTSSEEPYSWQHTEALLY GFQSIAETIDVNYSDVVPGLIGLIPRISISNVQLADTVMFTIGAL SEWLADHPVMINSVLPLVLHALGNPELSVSSVSTLKKICRECKYD LPPYAANIVAVSQDVLMKQIHKTSQCMWLMQALGFLLSALQVEEI LKNLHSLISPYIQQLEKLAEEIPNPSNKLAIVHILGLLSNLFTTL DISHHEDDHEGPELRKLPVPQGPNPVVVVLQQVFQLIQKVLSKWL NDAQVVEAVCAIFEKSVKTLLDDFAPMVPQLCEMLGRMYSTIPQA SALDLTRQLVHIFAHEPAHFPPIEALFLLVTSVTLTLFQQGPRDH PDIVDSFMQLLAQALKRKPDLFLCERLDVKSVFQCAVLALKFPEA PTVKASCGFFTELLPRCGEVESVGKVVQEDGRMLLIAVLEAIGGQ ASRSLMDCFADILFALNKHCFSLLSMWIKEALQPPGFPSARLSPE QKDTFSQQILRERVNKRRVKEMVKEFTLLCRGLHGTDYTADY | 56 |
| H2-20 | MQAWHFSWQLLQPDKVPEIQYFGASALHIKISRYWSDIPTDQYES LKAQLFTQITRFASGSKIVLTRLCVALASLALSMMPDAWPCAVAD MVRLFQAEDSPVDGQGRCLALLELLTVLPEEFQTSRLPQYRKGLV RTSLAVECGAVFPLLEQLLQQPSSPSCVRQKVLKCFSSWVQLEVP LQDCEALIQAAFAALQDSELFDSSVEAIVNAISQPDAQRYVNTLL KLIPLVLGLQEQLRQAVQNGDMETSHGICRIAVALGENHSRALLD QVEHWQSFLALVNMIMFCTGIPGHYPVNETTSSLTLTFWYTLQDD ILSFEAEKQAVYQQVYRPVYFQLVDVLLHKAQFPSDEEYGFWSSD EKEQFRIYRVDISDTLMYVYEMLGAELLSNLYDKLGRLLTSSEEP YSWQHTEALLYGFQSIAETIDVNYSDVVPGLIGLIPRISISNVQL ADTVMFTIGALSEWLADHPVMINSVLPLVLHALGNPELSVSSVST LKKICRECKYDLPPYAANIVAVSQDVLMKQIHKTSQCMWLMQALG FLLSALQVEEILKNLHSLISPYIQQLEKLAEEIPNPSNKLAIVHI LGLLSNLFTTLDISHHEDDHEGPELRKLPVPQGPNPVVVVLQQVF QLIQKVLSKWLNDAQVVEAVCAIFEKSVKTLLDDFAPMVPQLCEM LGRMYSTIPQASALDLTRQLVHIFAHEPAHFPPIEALFLLVTSVT LTLFQQGPRDHPDIVDSFMQLLAQALKRKPDLFLCERLDVKSVFQ CAVLALKFPEAPTVKASCGFFTELLPRCGEVESVGKVVQEDGRML LIAVLEAIGGQASRSLMDCFADILFALNKHCFSLLSMWIKEALQP PGFPSARLSPEQKDTFSQQILRERVNKRRVKEMVKEFTLLCRGLH GTDYTADY | 57 |
| H3-20 | MIPTDQYESLKAQLFTQITRFASGSKIVLTRLCVALASLALSMMP DAWPCAVADMVRLFQAEDSPVDGQGRCLALLELLTVLPEEFQTSR LPQYRKGLVRTSLAVECGAVFPLLEQLLQQPSSPSCVRQKVLKCF SSWVQLEVPLQDCEALIQAAFAALQDSELFDSSVEAIVNAISQPD AQRYVNTLLKLIPLVLGLQEQLRQAVQNGDMETSHGICRIAVALG ENHSRALLDQVEHWQSFLALVNMIMFCTGIPGHYPVNETTSSLTL TFWYTLQDDILSFEAEKQAVYQQVYRPVYFQLVDVLLHKAQFPSD EEYGFWSSDEKEQFRIYRVDISDTLMYVYEMLGAELLSNLYDKLG RLLTSSEEPYSWQHTEALLYGFQSIAETIDVNYSDVVPGLIGLIP RISISNVQLADTVMFTIGALSEWLADHPVMINSVLPLVLHALGNP ELSVSSVSTLKKICRECKYDLPPYAANIVAVSQDVLMKQIHKTSQ CMWLMQALGFLLSALQVEEILKNLHSLISPYIQQLEKLAEEIPNP SNKLAIVHILGLLSNLFTTLDISHHEDDHEGPELRKLPVPQGPNP VVVVLQQVFQLIQKVLSKWLNDAQVVEAVCAIFEKSVKTLLDDFA PMVPQLCEMLGRMYSTIPQASALDLTRQLVHIFAHEPAHFPPIEA LFLLVTSVTLTLFQQGPRDHPDIVDSFMQLLAQALKRKPDLFLCE RLDVKSVFQCAVLALKFPEAPTVKASCGFFTELLPRCGEVESVGK VVQEDGRMLLIAVLEAIGGQASRSLMDCFADILFALNKHCFSLLS MWIKEALQPPGFPSARLSPEQKDTFSQQILRERVNKRRVKEMVKE FTLLCRGLHGTDYTADY | 58 |
| H4-20 | MWPCAVADMVRLFQAEDSPVDGQGRCLALLELLTVLPEEFQTSRL PQYRKGLVRTSLAVECGAVFPLLEQLLQQPSSPSCVRQKVLKCFS SWVQLEVPLQDCEALIQAAFAALQDSELFDSSVEAIVNAISQPDA QRYVNTLLKLIPLVLGLQEQLRQAVQNGDMETSHGICRIAVALGE NHSRALLDQVEHWQSFLALVNMIMFCTGIPGHYPVNETTSSLTLT FWYTLQDDILSFEAEKQAVYQQVYRPVYFQLVDVLLHKAQFPSDE EYGFWSSDEKEQFRIYRVDISDTLMYVYEMLGAELLSNLYDKLGR LLTSSEEPYSWQHTEALLYGFQSIAETIDVNYSDVVPGLIGLIPR ISISNVQLADTVMFTIGALSEWLADHPVMINSVLPLVLHALGNPE LSVSSVSTLKKICRECKYDLPPYAANIVAVSQDVLMKQIHKTSQC MWLMQALGFLLSALQVEEILKNLHSLISPYIQQLEKLAEEIPNPS NKLAIVHILGLLSNLFTTLDISHHEDDHEGPELRKLPVPQGPNPV VVVLQQVFQLIQKVLSKWLNDAQVVEAVCAIFEKSVKTLLDDFAP MVPQLCEMLGRMYSTIPQASALDLTRQLVHIFAHEPAHFPPIEAL FLLVTSVTLTLFQQGPRDHPDIVDSFMQLLAQALKRKPDLFLCER LDVKSVFQCAVLALKFPEAPTVKASCGFFTELLPRCGEVESVGKV VQEDGRMLLIAVLEAIGGQASRSLMDCFADILFALNKHCFSLLSM WIKEALQPPGFPSARLSPEQKDTFSQQILRERVNKRRVKEMVKEF TLLCRGLHGTDYTADY | 59 |
| H5-20 | MLPQYRKGLVRTSLAVECGAVFPLLEQLLQQPSSPSCVRQKVLKC FSSWVQLEVPLQDCEALIQAAFAALQDSELFDSSVEAIVNAISQP DAQRYVNTLLKLIPLVLGLQEQLRQAVQNGDMETSHGICRIAVAL GENHSRALLDQVEHWQSFLALVNMIMFCTGIPGHYPVNETTSSLT LTFWYTLQDDILSFEAEKQAVYQQVYRPVYFQLVDVLLHKAQFPS DEEYGFWSSDEKEQFRIYRVDISDTLMYVYEMLGAELLSNLYDKL GRLLTSSEEPYSWQHTEALLYGFQSIAETIDVNYSDVVPGLIGLI PRISISNVQLADTVMFTIGALSEWLADHPVMINSVLPLVLHALGN PELSVSSVSTLKKICRECKYDLPPYAANIVAVSQDVLMKQIHKTS QCMWLMQALGFLLSALQVEEILKNLHSLISPYIQQLEKLAEEIPN PSNKLAIVHILGLLSNLFTTLDISHHEDDHEGPELRKLPVPQGPN PVVVVLQQVFQLIQKVLSKWLNDAQVVEAVCAIFEKSVKTLLDDF APMVPQLCEMLGRMYSTIPQASALDLTRQLVHIFAHEPAHFPPIE ALFLLVTSVTLTLFQQGPRDHPDIVDSFMQLLAQALKRKPDLFLC ERLDVKSVFQCAVLALKFPEAPTVKASCGFFTELLPRCGEVESVG KVVQEDGRMLLIAVLEAIGGQASRSLMDCFADILFALNKHCFSLL SMWIKEALQPPGFPSARLSPEQKDTFSQQILRERVNKRRVKEMVK EFTLLCRGLHGTDYTADY | 60 |
| H6-20 | MEVPLQDCEALIQAAFAALQDSELFDSSVEAIVNAISQPDAQRYV NTLLKLIPLVLGLQEQLRQAVQNGDMETSHGICRIAVALGENHSR ALLDQVEHWQSFLALVNMIMFCTGIPGHYPVNETTSSLTLTFWYT LQDDILSFEAEKQAVYQQVYRPVYFQLVDVLLHKAQFPSDEEYGF WSSDEKEQFRIYRVDISDTLMYVYEMLGAELLSNLYDKLGRLLTS SEEPYSWQHTEALLYGFQSIAETIDVNYSDVVPGLIGLIPRISIS NVQLADTVMFTIGALSEWLADHPVMINSVLPLVLHALGNPELSVS SVSTLKKICRECKYDLPPYAANIVAVSQDVLMKQIHKTSQCMWLM QALGFLLSALQVEEILKNLHSLISPYIQQLEKLAEEIPNPSNKLA IVHILGLLSNLFTTLDISHHEDDHEGPELRKLPVPQGPNPVVVVL QQVFQLIQKVLSKWLNDAQVVEAVCAIFEKSVKTLLDDFAPMVPQ LCEMLGRMYSTIPQASALDLTRQLVHIFAHEPAHFPPIEALFLLV TSVTLTLFQQGPRDHPDIVDSFMQLLAQALKRKPDLFLCERLDVK SVFQCAVLALKFPEAPTVKASCGFFTELLPRCGEVESVGKVVQED GRMLLIAVLEAIGGQASRSLMDCFADILFALNKHCFSLLSMWIKE ALQPPGFPSARLSPEQKDTFSQQILRERVNKRRVKEMVKEFTLLC RGLHGTDYTADY | 61 |
| H11-20 | MAETIDVNYSDVVPGLIGLIPRISISNVQLADTVMFTIGALSEWL ADHPVMINSVLPLVLHALGNPELSVSSVSTLKKICRECKYDLPPY AANIVAVSQDVLMKQIHKTSQCMWLMQALGFLLSALQVEEILKNL HSLISPYIQQLEKLAEEIPNPSNKLAIVHILGLLSNLFTTLDISH HEDDHEGPELRKLPVPQGPNPVVVVLQQVFQLIQKVLSKWLNDAQ VVEAVCAIFEKSVKTLLDDFAPMVPQLCEMLGRMYSTIPQASALD LTRQLVHIFAHEPAHFPPIEALFLLVTSVTLTLFQQGPRDHPDIV DSFMQLLAQALKRKPDLFLCERLDVKSVFQCAVLALKFPEAPTVK ASCGFFTELLPRCGEVESVGKVVQEDGRMLLIAVLEAIGGQASRS LMDCFADILFALNKHCFSLLSMWIKEALQPPGFPSARLSPEQKDT FSQQILRERVNKRRVKEMVKEFTLLCRGLHGTDYTADY | 62 |
| Δ934-964aa | MERREEQPGAAGAGAAPALDFTVENVEKALHQLYYDPNIENKNLA QKWLMQAQVSPQAWHFSWQLLQPDKVPEIQYFGASALHIKISRYW SDIPTDQYESLKAQLFTQITRFASGSKIVLTRLCVALASLALSMM PDAWPCAVADMVRLFQAEDSPVDGQGRCLALLELLTVLPEEFQTS RLPQYRKGLVRTSLAVECGAVFPLLEQLLQQPSSPSCVRQKVLKC FSSWVQLEVPLQDCEALIQAAFAALQDSELFDSSVEAIVNAISQP DAQRYVNTLLKLIPLVLGLQEQLRQAVQNGDMETSHGICRIAVAL GENHSRALLDQVEHWQSFLALVNMIMFCTGIPGHYPVNETTSSLT LTFWYTLQDDILSFEAEKQAVYQQVYRPVYFQLVDVLLHKAQFPS DEEYGFWSSDEKEQFRIYRVDISDTLMYVYEMLGAELLSNLYDKL GRLLTSSEEPYSWQHTEALLYGFQSIAETIDVNYSDVVPGLIGLI PRISISNVQLADTVMFTIGALSEWLADHPVMINSVLPLVLHALGN PELSVSSVSTLKKICRECKYDLPPYAANIVAVSQDVLMKQIHKTS QCMWLMQALGFLLSALQVEEILKNLHSLISPYIQQLEKLAEEIPN PSNKLAIVHILGLLSNLFTTLDISHHEDDHEGPELRKLPVPQGPN PVVVVLQQVFQLIQKVLSKWLNDAQVVEAVCAIFEKSVKTLLDDF APMVPQLCEMLGRMYSTIPQASALDLTRQLVHIFAHEPAHFPPIE ALFLLVTSVTLTLFQQGPRDHPDIVDSFMQLLAQALKRKPDLFLC ERLDVKSVFQCAVLALKFPEAPTVKASCGFFTELLPRCGEVESVG KVVQEDGRMLLIAVLEAIGGQASRSLMDCFADILFALNKHCFSLL SMWIKEALQPPGFPSARLSPEQKDTFSQQILRE | 63 |
| H1-19 | MERREEQPGAAGAGAAPALDFTVENVEKALHQLYYDPNIENKNLA QKWLMQAQVSPQAWHFSWQLLQPDKVPEIQYFGASALHIKISRYW SDIPTDQYESLKAQLFTQITRFASGSKIVLTRLCVALASLALSMM PDAWPCAVADMVRLFQAEDSPVDGQGRCLALLELLTVLPEEFQTS RLPQYRKGLVRTSLAVECGAVFPLLEQLLQQPSSPSCVRQKVLKC FSSWVQLEVPLQDCEALIQAAFAALQDSELFDSSVEAIVNAISQP DAQRYVNTLLKLIPLVLGLQEQLRQAVQNGDMETSHGICRIAVAL GENHSRALLDQVEHWQSFLALVNMIMFCTGIPGHYPVNETTSSLT LTFWYTLQDDILSFEAEKQAVYQQVYRPVYFQLVDVLLHKAQFPS DEEYGFWSSDEKEQFRIYRVDISDTLMYVYEMLGAELLSNLYDKL GRLLTSSEEPYSWQHTEALLYGFQSIAETIDVNYSDVVPGLIGLI PRISISNVQLADTVMFTIGALSEWLADHPVMINSVLPLVLHALGN PELSVSSVSTLKKICRECKYDLPPYAANIVAVSQDVLMKQIHKTS QCMWLMQALGFLLSALQVEEILKNLHSLISPYIQQLEKLAEEIPN PSNKLAIVHILGLLSNLFTTLDISHHEDDHEGPELRKLPVPQGPN PVVVVLQQVFQLIQKVLSKWLNDAQVVEAVCAIFEKSVKTLLDDF APMVPQLCEMLGRMYSTIPQASALDLTRQLVHIFAHEPAHFPPIE ALFLLVTSVTLTLFQQGPRDHPDIVDSFMQLLAQALKRKPDLFLC ERLDVKSVFQCAVLALKFPEAPTVKASCGFFTELLPRCGEVESVG KVVQEDGRMLLIAVLEAIGGQASRSLMDCFADILFALNKHC | 64 |
| H1-10 | MERREEQPGAAGAGAAPALDFTVENVEKALHQLYYDPNIENKNLA QKWLMQAQVSPQAWHFSWQLLQPDKVPEIQYFGASALHIKISRYW SDIPTDQYESLKAQLFTQITRFASGSKIVLTRLCVALASLALSMM PDAWPCAVADMVRLFQAEDSPVDGQGRCLALLELLTVLPEEFQTS RLPQYRKGLVRTSLAVECGAVFPLLEQLLQQPSSPSCVRQKVLKC FSSWVQLEVPLQDCEALIQAAFAALQDSELFDSSVEAIVNAISQP DAQRYVNTLLKLIPLVLGLQEQLRQAVQNGDMETSHGICRIAVAL GENHSRALLDQVEHWQSFLALVNMIMFCTGIPGHYPVNETTSSLT LTFWYTLQDDILSFEAEKQAVYQQVYRPVYFQLVDVLLHKAQFPS DEEYGFWSSDEKEQFRIYRVDISDTLMYVYEMLGAELLSNLYDKL GRLLTSSEEPYSWQHTEALLYGFQSI | 65 |
| H1-11 | MERREEQPGAAGAGAAPALDFTVENVEKALHQLYYDPNIENKNLA QKWLMQAQVSPQAWHFSWQLLQPDKVPEIQYFGASALHIKISRYW SDIPTDQYESLKAQLFTQITRFASGSKIVLTRLCVALASLALSMM PDAWPCAVADMVRLFQAEDSPVDGQGRCLALLELLTVLPEEFQTS RLPQYRKGLVRTSLAVECGAVFPLLEQLLQQPSSPSCVRQKVLKC FSSWVQLEVPLQDCEALIQAAFAALQDSELFDSSVEAIVNAISQP DAQRYVNTLLKLIPLVLGLQEQLRQAVQNGDMETSHGICRIAVAL GENHSRALLDQVEHWQSFLALVNMIMFCTGIPGHYPVNETTSSLT LTFWYTLQDDILSFEAEKQAVYQQVYRPVYFQLVDVLLHKAQFPS DEEYGFWSSDEKEQFRIYRVDISDTLMYVYEMLGAELLSNLYDKL GRLLTSSEEPYSWQHTEALLYGFQSIAETIDVNYSDVVPGLIGLI PRISISNVQLADTVMFTIGALSEWLADH | 66 |

In some cases, an IPO13 polypeptide fragment provided herein can include the amino acid sequence set forth in any one of SEQ ID NOs:56-66 with zero, one, two three, or more (e.g., five, eight, 12, 15, 18, 20, 25, 30, 35, 40, or more) amino acid substitutions (e.g., one or more K→R substitutions) within the articulated sequence of the sequence identifier (e.g., any one of SEQ ID NOs: 56-66), with zero, one, two, three, four, or five amino acid residues preceding the articulated sequence of the sequence identifier (e.g., any one of SEQ ID NOs: 56-66), and/or with zero, one, two, three, four, or five amino acid residues following the articulated sequence of the sequence identifier (e.g., any one of SEQ ID NOs: 56-66), provided that the IPO13 polypeptide fragment retains at least some activity exhibited by a naturally-occurring IPO13 polypeptide (e.g., the ability to reduce at least some TDP-43 aggregation and/or restore at least some nuclear localization of TDP-43 polypeptides). Examples of such IPO13 polypeptide fragments can be those set forth in Table 6.

**Table 6. Exemplary IPO13 polypeptide fragments.**

| Polypeptide Sequence | SEQ ID NO: |
|---|---|
| MVENVERALHQLYYDPNIENRNLAQRWLMQAQVSPQAWHFSWQLLQPDRVPEIQYF GASALHIRISRYWSDIPTDQYESLRAQLFTQITRFASGSRIVLTRLCVALASLALS MMPDAWPCAVADMVRLFQAEDSPVDGQGRCLALLELLTVLPEEFQTSRLPQYRRGL VRTSLAVECGAVFPLLEQLLQQPSSPSCVRQRVLRCFSSWVQLEVPLQDCEALIQA AFAALQDSELFDSSVEAIVNAISQPDAQRYVNTLLRLIPLVLGLQEQLRQAVQNGD METSHGICRIAVALGENHSRALLDQVEHWQSFLALVNMIMFCTGIPGHYPVNETTS SLTLTFWYTLQDDILSFEAERQAVYQQVYRPVYFQLVDVLLHRAQFPSDEEYGFWS SDEREQFRIYRVDISDTLMYVYEMLGAELLSNLYDRLGRLLTSSEEPYSWQHTEAL LYGFQSIAETIDVNYSDVVPGLIGLIPRISISNVQLADTVMFTIGALSEWLADHPV MINSVLPLVLHALGNPELSVSSVSTLRRICRECRYDLPPYAANIVAVSQDVLMRQI HRTSQCMWLMQALGFLLSALQVEEILRNLHSLISPYIQQLERLAEEIPNPSNRLAI VHILGLLSNLFTTLDISHHEDDHEGPELRRLPVPQGPNPVVVVLQQVFQLIQRVLS RWLNDAQVVEAVCAIFERSVRTLLDDFAPMVPQLCEMLGRMYSTIPQASALDLTRQ LVHIFAHEPAHFPPIEALFLLVTSVTLTLFQQGPRDHPDIVDSFMQLLAQALRRRP DLFLCERLDVRSVFQCAVLALRFPEAPTVRASCGFFTELLPRCGEVESVGRVVQED GRMLLIAVLEAIGGQASRSLMDCFADILFALNRHCFSLLSMWIREALQPPGFPSAR LSPEQRDTFSQQILRERVNRRRVREMVREFTLLCRGLHGTDYTADY | 67 |
| MQAWHFSWQLLQPDRVPEIQYFGASALHIRISRYWSDIPTDQYESLRAQLFTQITR FASGSRIVLTRLCVALASLALSMMPDAWPCAVADMVRLFQAEDSPVDGQGRCLALL ELLTVLPEEFQTSRLPQYRRGLVRTSLAVECGAVFPLLEQLLQQPSSPSCVRQRVL RCFSSWVQLEVPLQDCEALIQAAFAALQDSELFDSSVEAIVNAISQPDAQRYVNTL LRLIPLVLGLQEQLRQAVQNGDMETSHGICRIAVALGENHSRALLDQVEHWQSFLA LVNMIMFCTGIPGHYPVNETTSSLTLTFWYTLQDDILSFEAERQAVYQQVYRPVYF QLVDVLLHRAQFPSDEEYGFWSSDEREQFRIYRVDISDTLMYVYEMLGAELLSNLY DRLGRLLTSSEEPYSWQHTEALLYGFQSIAETIDVNYSDVVPGLIGLIPRISISNV QLADTVMFTIGALSEWLADHPVMINSVLPLVLHALGNPELSVSSVSTLRRICRECR YDLPPYAANIVAVSQDVLMRQIHRTSQCMWLMQALGFLLSALQVEEILRNLHSLIS PYIQQLERLAEEIPNPSNRLAIVHILGLLSNLFTTLDISHHEDDHEGPELRRLPVP QGPNPVVVVLQQVFQLIQRVLSRWLNDAQVVEAVCAIFERSVRTLLDDFAPMVPQL CEMLGRMYSTIPQASALDLTRQLVHIFAHEPAHFPPIEALFLLVTSVTLTLFQQGP RDHPDIVDSFMQLLAQALRRRPDLFLCERLDVRSVFQCAVLALRFPEAPTVRASCG FFTELLPRCGEVESVGRVVQEDGRMLLIAVLEAIGGQASRSLMDCFADILFALNRH CFSLLSMWIREALQPPGFPSARLSPEQRDTFSQQILRERVNRRRVREMVREFTLLC RGLHGTDYTADY | 68 |
| MIPTDQYESLRAQLFTQITRFASGSRIVLTRLCVALASLALSMMPDAWPCAVADMV RLFQAEDSPVDGQGRCLALLELLTVLPEEFQTSRLPQYRRGLVRTSLAVECGAVFP LLEQLLQQPSSPSCVRQRVLRCFSSWVQLEVPLQDCEALIQAAFAALQDSELFDSS VEAIVNAISQPDAQRYVNTLLRLIPLVLGLQEQLRQAVQNGDMETSHGICRIAVAL GENHSRALLDQVEHWQSFLALVNMIMFCTGIPGHYPVNETTSSLTLTFWYTLQDDI LSFEAERQAVYQQVYRPVYFQLVDVLLHRAQFPSDEEYGFWSSDEREQFRIYRVDI SDTLMYVYEMLGAELLSNLYDRLGRLLTSSEEPYSWQHTEALLYGFQSIAETIDVN YSDVVPGLIGLIPRISISNVQLADTVMFTIGALSEWLADHPVMINSVLPLVLHALG NPELSVSSVSTLRRICRECRYDLPPYAANIVAVSQDVLMRQIHRTSQCMWLMQALG FLLSALQVEEILRNLHSLISPYIQQLERLAEEIPNPSNRLAIVHILGLLSNLFTTL DISHHEDDHEGPELRRLPVPQGPNPVVVVLQQVFQLIQRVLSRWLNDAQVVEAVCA IFERSVRTLLDDFAPMVPQLCEMLGRMYSTIPQASALDLTRQLVHIFAHEPAHFPP IEALFLLVTSVTLTLFQQGPRDHPDIVDSFMQLLAQALRRRPDLFLCERLDVRSVF QCAVLALRFPEAPTVRASCGFFTELLPRCGEVESVGRVVQEDGRMLLIAVLEAIGG QASRSLMDCFADILFALNRHCFSLLSMWIREALQPPGFPSARLSPEQRDTFSQQIL RERVNRRRVREMVREFTLLCRGLHGTDYTADY | 69 |
| MWPCAVADMVRLFQAEDSPVDGQGRCLALLELLTVLPEEFQTSRLPQYRRGLVRTS LAVECGAVFPLLEQLLQQPSSPSCVRQRVLRCFSSWVQLEVPLQDCEALIQAAFAA LQDSELFDSSVEAIVNAISQPDAQRYVNTLLRLIPLVLGLQEQLRQAVQNGDMETS HGICRIAVALGENHSRALLDQVEHWQSFLALVNMIMFCTGIPGHYPVNETTSSLTL TFWYTLQDDILSFEAERQAVYQQVYRPVYFQLVDVLLHRAQFPSDEEYGFWSSDER EQFRIYRVDISDTLMYVYEMLGAELLSNLYDRLGRLLTSSEEPYSWQHTEALLYGF QSIAETIDVNYSDVVPGLIGLIPRISISNVQLADTVMFTIGALSEWLADHPVMINS VLPLVLHALGNPELSVSSVSTLRRICRECRYDLPPYAANIVAVSQDVLMRQIHRTS QCMWLMQALGFLLSALQVEEILRNLHSLISPYIQQLERLAEEIPNPSNRLAIVHIL GLLSNLFTTLDISHHEDDHEGPELRRLPVPQGPNPVVVVLQQVFQLIQRVLSRWLN DAQVVEAVCAIFERSVRTLLDDFAPMVPQLCEMLGRMYSTIPQASALDLTRQLVHI FAHEPAHFPPIEALFLLVTSVTLTLFQQGPRDHPDIVDSFMQLLAQALRRRPDLFL CERLDVRSVFQCAVLALRFPEAPTVRASCGFFTELLPRCGEVESVGRVVQEDGRML LIAVLEAIGGQASRSLMDCFADILFALNRHCFSLLSMWIREALQPPGFPSARLSPE QRDTFSQQILRERVNRRRVREMVREFTLLCRGLHGTDYTADY | 70 |
| MLPQYRRGLVRTSLAVECGAVFPLLEQLLQQPSSPSCVRQRVLRCFSSWVQLEVPL QDCEALIQAAFAALQDSELFDSSVEAIVNAISQPDAQRYVNTLLRLIPLVLGLQEQ LRQAVQNGDMETSHGICRIAVALGENHSRALLDQVEHWQSFLALVNMIMFCTGIPG HYPVNETTSSLTLTFWYTLQDDILSFEAERQAVYQQVYRPVYFQLVDVLLHRAQFP SDEEYGFWSSDEREQFRIYRVDISDTLMYVYEMLGAELLSNLYDRLGRLLTSSEEP YSWQHTEALLYGFQSIAETIDVNYSDVVPGLIGLIPRISISNVQLADTVMFTIGAL SEWLADHPVMINSVLPLVLHALGNPELSVSSVSTLRRICRECRYDLPPYAANIVAV SQDVLMRQIHRTSQCMWLMQALGFLLSALQVEEILRNLHSLISPYIQQLERLAEEI PNPSNRLAIVHILGLLSNLFTTLDISHHEDDHEGPELRRLPVPQGPNPVVVVLQQV FQLIQRVLSRWLNDAQVVEAVCAIFERSVRTLLDDFAPMVPQLCEMLGRMYSTIPQ ASALDLTRQLVHIFAHEPAHFPPIEALFLLVTSVTLTLFQQGPRDHPDIVDSFMQL LAQALRRRPDLFLCERLDVRSVFQCAVLALRFPEAPTVRASCGFFTELLPRCGEVE SVGRVVQEDGRMLLIAVLEAIGGQASRSLMDCFADILFALNRHCFSLLSMWIREAL QPPGFPSARLSPEQRDTFSQQILRERVNRRRVREMVREFTLLCRGLHGTDYTADY | 71 |
| MEVPLQDCEALIQAAFAALQDSELFDSSVEAIVNAISQPDAQRYVNTLLRLIPLVL GLQEQLRQAVQNGDMETSHGICRIAVALGENHSRALLDQVEHWQSFLALVNMIMFC TGIPGHYPVNETTSSLTLTFWYTLQDDILSFEAERQAVYQQVYRPVYFQLVDVLLH RAQFPSDEEYGFWSSDEREQFRIYRVDISDTLMYVYEMLGAELLSNLYDRLGRLLT SSEEPYSWQHTEALLYGFQSIAETIDVNYSDVVPGLIGLIPRISISNVQLADTVMF TIGALSEWLADHPVMINSVLPLVLHALGNPELSVSSVSTLRRICRECRYDLPPYAA NIVAVSQDVLMRQIHRTSQCMWLMQALGFLLSALQVEEILRNLHSLISPYIQQLER LAEEIPNPSNRLAIVHILGLLSNLFTTLDISHHEDDHEGPELRRLPVPQGPNPVVV VLQQVFQLIQRVLSRWLNDAQVVEAVCAIFERSVRTLLDDFAPMVPQLCEMLGRMY STIPQASALDLTRQLVHIFAHEPAHFPPIEALFLLVTSVTLTLFQQGPRDHPDIVD SFMQLLAQALRRRPDLFLCERLDVRSVFQCAVLALRFPEAPTVRASCGFFTELLPR CGEVESVGRVVQEDGRMLLIAVLEAIGGQASRSLMDCFADILFALNRHCFSLLSMW IREALQPPGFPSARLSPEQRDTFSQQILRERVNRRRVREMVREFTLLCRGLHGTDY TADY | 72 |
| MAETIDVNYSDVVPGLIGLIPRISISNVQLADTVMFTIGALSEWLADHPVMINSVL PLVLHALGNPELSVSSVSTLRRICRECRYDLPPYAANIVAVSQDVLMRQIHRTSQC MWLMQALGFLLSALQVEEILRNLHSLISPYIQQLERLAEEIPNPSNRLAIVHILGL LSNLFTTLDISHHEDDHEGPELRRLPVPQGPNPVVVVLQQVFQLIQRVLSRWLNDA QVVEAVCAIFERSVRTLLDDFAPMVPQLCEMLGRMYSTIPQASALDLTRQLVHIFA HEPAHFPPIEALFLLVTSVTLTLFQQGPRDHPDIVDSFMQLLAQALRRRPDLFLCE RLDVRSVFQCAVLALRFPEAPTVRASCGFFTELLPRCGEVESVGRVVQEDGRMLLI AVLEAIGGQASRSLMDCFADILFALNRHCFSLLSMWIREALQPPGFPSARLSPEQR DTFSQQILRERVNRRRVREMVREFTLLCRGLHGTDYTADY | 73 |
| MERREEQPGAAGAGAAPALDFTVENVERALHQLYYDPNIENRNLAQRWLMQAQVSP QAWHFSWQLLQPDRVPEIQYFGASALHIRISRYWSDIPTDQYESLRAQLFTQITRF ASGSRIVLTRLCVALASLALSMMPDAWPCAVADMVRLFQAEDSPVDGQGRCLALLE LLTVLPEEFQTSRLPQYRRGLVRTSLAVECGAVFPLLEQLLQQPSSPSCVRQRVLR CFSSWVQLEVPLQDCEALIQAAFAALQDSELFDSSVEAIVNAISQPDAQRYVNTLL RLIPLVLGLQEQLRQAVQNGDMETSHGICRIAVALGENHSRALLDQVEHWQSFLAL VNMIMFCTGIPGHYPVNETTSSLTLTFWYTLQDDILSFEAERQAVYQQVYRPVYFQ LVDVLLHRAQFPSDEEYGFWSSDEREQFRIYRVDISDTLMYVYEMLGAELLSNLYD RLGRLLTSSEEPYSWQHTEALLYGFQSIAETIDVNYSDVVPGLIGLIPRISISNVQ LADTVMFTIGALSEWLADHPVMINSVLPLVLHALGNPELSVSSVSTLRRICRECRY DLPPYAANIVAVSQDVLMRQIHRTSQCMWLMQALGFLLSALQVEEILRNLHSLISP YIQQLERLAEEIPNPSNRLAIVHILGLLSNLFTTLDISHHEDDHEGPELRRLPVPQ GPNPVVVVLQQVFQLIQRVLSRWLNDAQVVEAVCAIFERSVRTLLDDFAPMVPQLC EMLGRMYSTIPQASALDLTRQLVHIFAHEPAHFPPIEALFLLVTSVTLTLFQQGPR DHPDIVDSFMQLLAQALRRRPDLFLCERLDVRSVFQCAVLALRFPEAPTVRASCGF FTELLPRCGEVESVGRVVQEDGRMLLIAVLEAIGGQASRSLMDCFADILFALNRHC FSLLSMWIREALQPPGFPSARLSPEQRDTFSQQILRE | 74 |
| MERREEQPGAAGAGAAPALDFTVENVERALHQLYYDPNIENRNLAQRWLMQAQVSP QAWHFSWQLLQPDRVPEIQYFGASALHIRISRYWSDIPTDQYESLRAQLFTQITRF ASGSRIVLTRLCVALASLALSMMPDAWPCAVADMVRLFQAEDSPVDGQGRCLALLE LLTVLPEEFQTSRLPQYRRGLVRTSLAVECGAVFPLLEQLLQQPSSPSCVRQRVLR CFSSWVQLEVPLQDCEALIQAAFAALQDSELFDSSVEAIVNAISQPDAQRYVNTLL RLIPLVLGLQEQLRQAVQNGDMETSHGICRIAVALGENHSRALLDQVEHWQSFLAL VNMIMFCTGIPGHYPVNETTSSLTLTFWYTLQDDILSFEAERQAVYQQVYRPVYFQ LVDVLLHRAQFPSDEEYGFWSSDEREQFRIYRVDISDTLMYVYEMLGAELLSNLYD RLGRLLTSSEEPYSWQHTEALLYGFQSIAETIDVNYSDVVPGLIGLIPRISISNVQ LADTVMFTIGALSEWLADHPVMINSVLPLVLHALGNPELSVSSVSTLRRICRECRY DLPPYAANIVAVSQDVLMRQIHRTSQCMWLMQALGFLLSALQVEEILRNLHSLISP YIQQLERLAEEIPNPSNRLAIVHILGLLSNLFTTLDISHHEDDHEGPELRRLPVPQ GPNPVVVVLQQVFQLIQRVLSRWLNDAQVVEAVCAIFERSVRTLLDDFAPMVPQLC EMLGRMYSTIPQASALDLTRQLVHIFAHEPAHFPPIEALFLLVTSVTLTLFQQGPR DHPDIVDSFMQLLAQALRRRPDLFLCERLDVRSVFQCAVLALRFPEAPTVRASCGF FTELLPRCGEVESVGRVVQEDGRMLLIAVLEAIGGQASRSLMDCFADILFALNRHC | 75 |
| MERREEQPGAAGAGAAPALDFTVENVERALHQLYYDPNIENRNLAQRWLMQAQVSP QAWHFSWQLLQPDRVPEIQYFGASALHIRISRYWSDIPTDQYESLRAQLFTQITRF ASGSRIVLTRLCVALASLALSMMPDAWPCAVADMVRLFQAEDSPVDGQGRCLALLE LLTVLPEEFQTSRLPQYRRGLVRTSLAVECGAVFPLLEQLLQQPSSPSCVRQRVLR CFSSWVQLEVPLQDCEALIQAAFAALQDSELFDSSVEAIVNAISQPDAQRYVNTLL RLIPLVLGLQEQLRQAVQNGDMETSHGICRIAVALGENHSRALLDQVEHWQSFLAL VNMIMFCTGIPGHYPVNETTSSLTLTFWYTLQDDILSFEAERQAVYQQVYRPVYFQ LVDVLLHRAQFPSDEEYGFWSSDEREQFRIYRVDISDTLMYVYEMLGAELLSNLYD RLGRLLTSSEEPYSWQHTEALLYGFQSI | 76 |
| MERREEQPGAAGAGAAPALDFTVENVERALHQLYYDPNIENRNLAQRWLMQAQVSP QAWHFSWQLLQPDRVPEIQYFGASALHIRISRYWSDIPTDQYESLRAQLFTQITRF ASGSRIVLTRLCVALASLALSMMPDAWPCAVADMVRLFQAEDSPVDGQGRCLALLE LLTVLPEEFQTSRLPQYRRGLVRTSLAVECGAVFPLLEQLLQQPSSPSCVRQRVLR CFSSWVQLEVPLQDCEALIQAAFAALQDSELFDSSVEAIVNAISQPDAQRYVNTLL RLIPLVLGLQEQLRQAVQNGDMETSHGICRIAVALGENHSRALLDQVEHWQSFLAL VNMIMFCTGIPGHYPVNETTSSLTLTFWYTLQDDILSFEAERQAVYQQVYRPVYFQ LVDVLLHRAQFPSDEEYGFWSSDEREQFRIYRVDISDTLMYVYEMLGAELLSNLYD RLGRLLTSSEEPYSWQHTEALLYGFQSIAETIDVNYSDVVPGLIGLIPRISISNVQ LADTVMFTIGALSEWLADH | 77 |

Any appropriate method can be used to deliver one or more importin polypeptides or fragments thereof (and/or nucleic acids designed to express an importin polypeptide or a fragment thereof) to a mammal. When one or more importin polypeptides or fragments thereof (and/or nucleic acids designed to express an importin polypeptide or a fragment thereof) are administered to a mammal (e.g., a human), the one or more importin polypeptides or fragments thereof (and/or nucleic acids designed to express an importin polypeptide or a fragment thereof) can be administered to the CNS (e.g., the brain) of a mammal (e.g., a human). In some cases, one or more importin polypeptides or fragments thereof (and/or nucleic acids designed to express an importin polypeptide or a fragment thereof) can be administered to the CNS (e.g., the brain) of a mammal (e.g., a human) by direct injection into the CNS (e.g., into the brain and/or spinal cord).

Any appropriate method can be used to obtain an importin polypeptide or fragment thereof provided herein (e.g., a polypeptide that comprises, consists essentially of, or consists of the amino acid sequence set forth in any one of SEQ ID NOs:7-77). For example, an importin polypeptide or fragment thereof provided herein (e.g., a polypeptide that comprises, consists essentially of, or consists of the amino acid sequence set forth in any one of SEQ ID NOs:7-77) can be obtained by synthesizing the polypeptide of interest using appropriate polypeptide synthesizing techniques such as those described elsewhere (see, e.g., Fields et al., Curr. Protoc. Protein Sci., Chapter 18:Unit 18.1 (2002); Hartrampf et al., Science, 368(6494):980-987 (2020); and Merrifield, J. Am. Chem. Soc., 85:2149-2154 (1963)).

When one or more nucleic acids designed to express an importin polypeptide or a fragment thereof are administered to a mammal (e.g., a human), the nucleic acid can be in the form of a vector (e.g., a viral vector or a non-viral vector).

When nucleic acid encoding an importin polypeptide or a fragment thereof is administered to a mammal, the nucleic acid can be used for transient expression of an importin polypeptide or a fragment thereof or for stable expression of an importin polypeptide or a fragment thereof. In cases where a nucleic acid encoding an importin polypeptide or a fragment thereof is used for stable expression of an importin polypeptide or a fragment thereof, the nucleic acid encoding an importin polypeptide or a fragment thereof can be engineered to integrate into the genome of a cell. Nucleic acid can be engineered to integrate into the genome of a cell using any appropriate method. For example, gene editing techniques (e.g., CRISPR or TALEN gene editing) can be used to integrate nucleic acid designed to express an importin polypeptide or a fragment thereof into the genome of a cell.

When a vector used to deliver nucleic acid encoding an importin polypeptide or a fragment thereof to a mammal (e.g., a human) is a viral vector, any appropriate viral vector can be used. A viral vector can be derived from a positive-strand virus or a negative-strand virus. A viral vector can be derived from a virus with a DNA genome or a RNA genome. In some cases, a viral vector can be a chimeric viral vector. In some cases, a viral vector can infect dividing cells. In some cases, a viral vector can infect non-dividing cells. Examples virus-based vectors that can be used to deliver nucleic acid encoding an importin polypeptide to a mammal (e.g., a human) include, without limitation, virus-based vectors based on adenoviruses, AAVs, Sendai viruses, retroviruses, lentiviruses, herpes simplex viruses (HSV), vaccinia viruses, and baculoviruses.

When a vector used to deliver nucleic acid encoding an importin polypeptide or a fragment thereof to a mammal (e.g., a human) is a non-viral vector, any appropriate non-viral vector can be used. In some cases, a non-viral vector can be an expression plasmid (e.g., a cDNA expression vector).

When one or more nucleic acids designed to express an importin polypeptide or a fragment thereof are administered to a mammal (e.g., a human), the nucleic acid can be contained within a nanoparticle (e.g., a liposome).

In addition to nucleic acid encoding an importin polypeptide or a fragment thereof, a vector (e.g., a viral vector or a non-viral vector) can contain one or more regulatory elements operably linked to the nucleic acid encoding an importin polypeptide or a fragment thereof. Such regulatory elements can include promoter sequences, enhancer sequences, response elements, signal peptides, internal ribosome entry sequences, polyadenylation signals, terminators, and inducible elements that modulate expression (e.g., transcription or translation) of a nucleic acid. The choice of regulatory element(s) that can be included in a vector depends on several factors, including, without limitation, inducibility, targeting, and the level of expression desired. For example, a promoter can be included in a vector to facilitate transcription of a nucleic acid encoding an importin polypeptide. A promoter can be a naturally occurring promoter or a recombinant promoter. A promoter can be ubiquitous or inducible (e.g., in the presence of tetracycline), and can affect the expression of a nucleic acid encoding a polypeptide in a general or tissue-specific manner (e.g., prion protein (Prp) promoters, synapsin promoters, methyl-CpG-binding protein-2 (MeCP2) promoters, neuron-specific enolase (NSE) promoters, vesicular glutamate transporter promoter (vGLUT) promoters, and Hb9 promoters). Examples of promoters that can be used to drive expression of an importin polypeptide in cells include, without limitation, cytomegalovirus/chicken beta-actin (CBA) promoters, cytomegalovirus (CMV) promoters, ubiquitin C (UbC) promoters, Prp promoters, synapsin promoters, MeCP2 promoters, NSE promoters, vGLUT promoters, and Hb9 promoters. As used herein, "operably linked" refers to positioning of a regulatory element in a vector relative to a nucleic acid encoding a polypeptide in such a way as to permit or facilitate expression of the encoded polypeptide. For example, a vector can contain a promoter and nucleic acid encoding an importin polypeptide or a fragment thereof. In this case, the promoter is operably linked to a nucleic acid encoding an importin polypeptide or a fragment thereof such that it drives expression of the importin polypeptide or a fragment thereof in cells.

In some cases, nucleic acid encoding an importin polypeptide or a fragment thereof can contain nucleic acid encoding a detectable label. For example, a vector can include nucleic acid encoding an importin polypeptide or a fragment thereof and nucleic acid encoding a detectable label positioned such that the encoded polypeptide is a fusion polypeptide that includes an importin polypeptide or a fragment thereof fused to a detectable polypeptide. In some cases, a detectable label can be a peptide tag. In some cases, a detectable label can be a fluorescent molecule (e.g., fluorescent dyes and fluorescent polypeptides). Examples of detectable labels that can be used as described herein include, without limitation, an HA tag, a Myc-tag, a FLAG-tag, green fluorescent polypeptides (GFPs; e.g., enhanced GFPs), red fluorescent polypeptides (e.g. mCherry) polypeptides, Halo Tags, SNAP-tags, 6xHis-tags, GST-tags, MBP-tags, Strep-Tags, and V5-tags.

Nucleic acid encoding an importin polypeptide or a fragment thereof can be produced by techniques including, without limitation, common molecular cloning, polymerase chain reaction (PCR), chemical nucleic acid synthesis techniques, and combinations of such techniques. For example, PCR or RT-PCR can be used with oligonucleotide primers designed to amplify nucleic acid (e.g., genomic DNA or RNA) encoding an importin polypeptide.

In some cases, one or more importin polypeptides or fragments thereof (and/or nucleic acids designed to express an importin polypeptide or a fragment thereof) can be formulated into a composition (*e.g*., a pharmaceutical composition) for administration to a mammal (*e.g*., a human). For example, one or more importin polypeptides or fragments thereof (and/or nucleic acids designed to express an importin polypeptide or a fragment thereof) can be formulated into a pharmaceutically acceptable composition for administration to a mammal (e.g., a human) having, or at risk of developing, a proteinopathy (e.g., a TDP-43 proteinopathy). In some cases, one or more importin polypeptides or fragments thereof (and/or nucleic acids designed to express an importin polypeptide or a fragment thereof) can be formulated together with one or more pharmaceutically acceptable carriers (additives), excipients, and/or diluents. Examples of pharmaceutically acceptable carriers, excipients, and diluents that can be used in a composition described herein include, without limitation, sucrose, lactose, starch (*e.g.*, starch glycolate), cellulose, cellulose derivatives (*e.g.*, modified celluloses such as microcrystalline cellulose and cellulose ethers like hydroxypropyl cellulose (HPC) and cellulose ether hydroxypropyl methylcellulose (HPMC)), xylitol, sorbitol, mannitol, gelatin, polymers (*e.g*., polyvinylpyrrolidone (PVP), polyethylene glycol (PEG), crosslinked polyvinylpyrrolidone (crospovidone), carboxymethyl cellulose, polyethylene-polyoxypropylene-block polymers, and crosslinked sodium carboxymethyl cellulose (croscarmellose sodium)), titanium oxide, azo dyes, silica gel, fumed silica, talc, magnesium carbonate, vegetable stearin, magnesium stearate, aluminum stearate, stearic acid, antioxidants (*e.g*., vitamin A, vitamin E, vitamin C, retinyl palmitate, and selenium), citric acid, sodium citrate, parabens (*e.g*., methyl paraben and propyl paraben), petrolatum, dimethyl sulfoxide, mineral oil, serum proteins (*e.g*., human serum albumin), glycine, sorbic acid, potassium sorbate, water, salts or electrolytes (*e.g*., saline, protamine sulfate, disodium hydrogen phosphate, potassium hydrogen phosphate, sodium chloride, sodium acetate, and zinc salts), colloidal silica, magnesium trisilicate, polyacrylates, waxes, wool fat, SM-102, dimyristoyl glycerol, cholesterol, tromethamine, and lecithin.

A composition (*e.g*., a pharmaceutical composition) containing one or more importin polypeptides or fragments thereof (and/or nucleic acids designed to express an importin polypeptide or a fragment thereof) can be formulated into any appropriate dosage form. Examples of dosage forms include solid or liquid forms including, without limitation, gels, liquids, suspensions, solutions (*e.g*., sterile solutions), sustained-release formulations, and delayed-release formulations.

A composition (*e.g*., a pharmaceutical composition) containing one or more importin polypeptides or fragments thereof (and/or nucleic acids designed to express an importin polypeptide or a fragment thereof) can be designed for parenteral (e.g., intravenous, intracerebral, intracerebroventricular, and intrathecal) administration. Compositions suitable for parenteral administration include aqueous and non-aqueous sterile injection solutions that can contain anti-oxidants, buffers, bacteriostats, and solutes which render the formulation isotonic with the blood of the intended recipient; and aqueous and non-aqueous sterile suspensions which may include suspending agents and thickening agents. The formulations can be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules, and tablets.

A composition (*e.g*., a pharmaceutical composition) containing one or more importin polypeptides or fragments thereof (and/or nucleic acids designed to express an importin polypeptide or a fragment thereof) can be administered locally or systemically. For example, a composition containing one or more importin polypeptides or fragments thereof (and/or nucleic acids designed to express an importin polypeptide or a fragment thereof) can be administered locally by direct injection (e.g., an intracerebral injection) to the CNS (e.g., the brain) of a mammal (*e.g*., a human).

An effective amount of a composition (*e.g*., a pharmaceutical composition) containing one or more importin polypeptides or fragments thereof (and/or nucleic acids designed to express an importin polypeptide or a fragment thereof) can be any amount that can treat the mammal without producing significant toxicity to the mammal. For example, an effective amount of one or more importin polypeptides or fragments thereof can be from about 0.1 milligrams of polypeptide(s) per kilogram bodyweight of the mammal (mg/kg) per dose to about 100 mg/kg per does (e.g., from about 0.1 mg/kg to about 75 mg/kg, from about 0.1 mg/kg to about 50 mg/kg, from about 0.1 mg/kg to about 30 mg/kg, from about 0.1 mg/kg to about 20 mg/kg, from about 0.1 mg/kg to about 10 mg/kg, from about 0.1 mg/kg to about 1 mg/kg, from about 1 mg/kg to about 100 mg/kg, from about 10 mg/kg to about 100 mg/kg, from about 20 mg/kg to about 100 mg/kg, from about 30 mg/kg to about 100 mg/kg, from about 50 mg/kg to about 100 mg/kg, from about 75 mg/kg to about 100 mg/kg, from about 1 mg/kg to about 75 mg/kg, from about 10 mg/kg to about 50 mg/kg, from about 20 mg/kg to about 40 mg/kg, from about 1 mg/kg to about 10 mg/kg, from about 10 mg/kg to about 30 mg/kg, from about 30 mg/kg to about 50 mg/kg, from about 40 mg/kg to about 60 mg/kg, from about 50 mg/kg to about 70 mg/kg, from about 60 mg/kg to about 80 mg/kg, or from about 70 mg/kg to about 90 mg/kg per dose). The effective amount can remain constant or can be adjusted as a sliding scale or variable dose depending on the mammal's response to treatment. Various factors can influence the actual effective amount used for a particular application. For example, the frequency of administration, duration of treatment, use of multiple treatment agents, route of administration, and severity of the condition may require an increase or decrease in the actual effective amount administered.

The frequency of administration of a composition (*e.g*., a pharmaceutical composition) containing one or more importin polypeptides or fragments thereof (and/or nucleic acids designed to express an importin polypeptide or a fragment thereof) can be any frequency that can treat a mammal (e.g., a human) having, or at risk of developing, a proteinopathy (e.g., a TDP-43 proteinopathy) without producing significant toxicity to the mammal. For example, the frequency of administration can be from about two times a day to about once a week, from about twice a day to about twice a week, or from about once a day to about twice a week. The frequency of administration can remain constant or can be variable during the duration of treatment. A course of treatment with a composition containing one or more chimeric vesiculoviruses provided herein can include rest periods. As with the effective amount, various factors can influence the actual frequency of administration used for a particular application. For example, the effective amount, duration of treatment, use of multiple treatment agents, route of administration, and severity of the condition may require an increase or decrease in administration frequency.

An effective duration for administering a composition (*e.g*., a pharmaceutical composition) containing one or more importin polypeptides or fragments thereof (and/or nucleic acids designed to express an importin polypeptide or a fragment thereof) can be any duration that treat a mammal (e.g., a human) having, or at risk of developing, a proteinopathy (e.g., a TDP-43 proteinopathy) without producing significant toxicity to the mammal. For example, the effective duration can vary from several days to several weeks, months, or years. In some cases, the effective duration for the treatment of a mammal (e.g., a human) having, or at risk of developing, a proteinopathy (e.g., a TDP-43 proteinopathy) can range in duration from about one month to about 10 years. Multiple factors can influence the actual effective duration used for a particular treatment. For example, an effective duration can vary with the frequency of administration, effective amount, use of multiple treatment agents, route of administration, and severity of the condition being treated.

In some cases, the one or more importin polypeptides or fragments thereof (and/or nucleic acids designed to express an importin polypeptide or a fragment thereof) can be used as the sole active agent used to treat a mammal (e.g., a human) having, or at risk of developing, a proteinopathy (e.g., a TDP-43 proteinopathy).

In some cases, the methods and materials described herein can include one or more (e.g., one, two, three, four, five or more) additional therapeutic agents used to treat a mammal (e.g., a human) having, or at risk of developing, a proteinopathy (e.g., a TDP-43 proteinopathy). Examples of therapeutic agents used to treat a proteinopathy (e.g., a TDP-43 proteinopathy) that can be administered to a mammal (e.g., a human) having, or at risk of developing, a proteinopathy (e.g., a TDP-43 proteinopathy) together with one or more importin polypeptides or fragments thereof (and/or nucleic acids designed to express an importin polypeptide or a fragment thereof) include, without limitation, antidepressants, antipsychotics, riluzole (e.g., RILUTEK^{®}), edavarone (e.g., RADICAVA^{®}), molecules (e.g., antisense oligonucleotides) that can target C9orf72 repeat expansions, molecules (e.g., antibodies) that can target amyloid beta (Aβ) polypeptides ( e.g., Aβ polypeptides present in amyloid plaques), and molecules (e.g., antibodies) that can target tau polypeptides (e.g., tau polypeptides present in neurofibrillary tangles). In some cases, the one or more additional therapeutic agents can be administered together with one or more importin polypeptides or fragments thereof (and/or nucleic acids designed to express an importin polypeptide or a fragment thereof; e.g., in the same composition). In some cases, the one or more additional therapeutic agents can be administered independent of the one or more importin polypeptides or fragments thereof (and/or nucleic acids designed to express an importin polypeptide or a fragment thereof). When the one or more additional therapeutic agents are administered independent of the one or more importin polypeptides or fragments thereof (and/or nucleic acids designed to express an importin polypeptide or a fragment thereof), the one or more importin polypeptides or fragments thereof (and/or nucleic acids designed to express an importin polypeptide or a fragment thereof) can be administered first, and the one or more additional therapeutic agents administered second, or vice versa.

In some cases, the methods and materials described herein can include subjecting a mammal having, or at risk of developing, a proteinopathy (e.g., a TDP-43 proteinopathy) to one or more (*e.g.*, one, two, three, four, five or more) additional treatments (*e.g.*, therapeutic interventions) that are effective to treat a proteinopathy (e.g., a TDP-43 proteinopathy). Examples of therapies that can be used to treat a proteinopathy include, without limitation, physical therapy, occupational therapy, speech therapy, and any combinations thereof. In some cases, the one or more additional treatments that are effective to treat a proteinopathy (e.g., a TDP-43 proteinopathy) can be performed at the same time as the administration of the one or more importin polypeptides or fragments thereof (and/or nucleic acids designed to express an importin polypeptide or a fragment thereof). In some cases, the one or more additional treatments that are effective to treat a proteinopathy (e.g., a TDP-43 proteinopathy) can be performed before and/or after the administration of the one or more importin polypeptides or fragments thereof (and/or nucleic acids designed to express an importin polypeptide or a fragment thereof).

The invention will be further described in the following examples, which do not limit the scope of the invention described in the claims.

### EXAMPLES

### Example 1: Nuclear import receptors (NIRs) reduce TDP-CTF aggregates

This Example describes the discovery that NIR polypeptides can reduce TDP-43 aggregation.

### METHODS

### Cell culture and transfection

Human HEK293 cells were cultured in DMEM media (Life Technologies) containing 10% FBS and transfected with PolyMag Neo (Oz Biosciences) according to the manufacturer's protocol. SH-SY5Y neuroblastoma cells were cultured in DMEM/F12 media (Life Technologies) containing 10% FBS and transfected with NeuroMag Neo (Oz Biosciences) according to the manufacturer's protocol. HEK293 and SH-S5Y cells were obtained from ATCC (CRL-1573 and CRL-2266, respectively).

### Immunofluorescence and image acquisition

Cells were plated on coverslips in 12-well plates and transfected for 24 hours, and then were fixed with 4% paraformaldehyde in PBS for 15 minutes at room temperature. For high-resolution imaging, z-series image stacks were acquired using an epifluorescence microscope (Nikon Eclipse Ti2) equipped with a Zyla camera (Zyla sCMOS, Andor). Within each experiment, all groups were imaged with the same acquisition settings. Image stacks were deconvolved using a 3D blind constrained iterative algorithm (Nikon NIS Elements).

### Cell lysis, subcellular fractionation, and immunoblotting

Cells were seeded in 12-well plates and transfected for 48 hours. Cells were lysed in RIPA Lysis and Extraction buffer (Fisher) supplemented with a protease inhibitor cocktail (Roche), and centrifuged at 15000 rpm for 20 minutes at 4°C. The supernatant was collected as the detergent-soluble fraction and the insoluble pellet was recovered in urea buffer (7M urea, 2 M thiourea, 4% CHAPS, 50 mM Tris pH 8, Roche complete protease inhibitor cocktail). The samples were left at room temperature for 20 minutes then briefly sonicated and centrifuged at 15000 rpm for 20 minutes. The supernatant was collected as the detergent-insoluble fraction.

For immunoblotting, the samples were boiled in Laemmli sample buffer for 5 minutes at 98°C and separated in BoltTM 4 to 12% Bis-Tris gels (Fisher). Proteins were blotted onto nitrocellulose membranes using an iBlotTM 2 Gel Transfer device (Fisher). Membranes were blocked with Odyssey blocking buffer (LiCor) for 1 hour followed by incubation with primary antibodies (anti-TDP-43 (1:1000, 12892-1-AP, Proteintech) and anti-β-tubulin (1:1000, DHSB)) overnight at 4°C and incubation with secondary antibodies (1:10000, LiCor) in PBS blocking buffer with 0.05% Tween20 for 1 hour at room temperature. Blots were imaged using an Odyssey scanner (LiCor).

### Immunoprecipitation

HEK293 cells were seeded in 6-well plates and transfected for 48 hours, were then lysed in IP Lysis buffer (Fisher) supplemented with a protease inhibitor cocktail (Roche), and were centrifuged at 15000r pm for 20 minutes at 4°C. A small aliquot of the recovered supernatant was set aside (input). Pull-down was performed using GFP-and RFP-TRAP magnetic beads (Chromotek) according to the manufacturer's protocol. Briefly, beads were washed twice in wash buffer (10 mM Tris/Cl pH 7.5, 150 mM NaCl, 0.5 mM EDTA). The lysate was then incubated with the equilibrated beads overnight at 4°C with end-over-end rotation. The next day, beads were magnetically separated from the supernatant (flow-through) and washed 3 times with wash buffer. Bound proteins were released from the beads in an elution buffer (200 mM glycine pH 2.5). Samples were boiled in Laemmli buffer for 10 minutes at 95°C and used for immunoblotting.

### Cell death assays

Cell death of SH-SY5Y cells was assessed by the uptake of the membrane-impermeant Live-or-Dye^{™} Fixable Dead Cell Dye 640/662 (Live-or-Dye^{™} Fixable Viability Staining Kit, 32007, Biotium). The dye is able to penetrate into dead cells that have compromised membrane integrity and label amines on intracellular proteins. Cells were co-transfected with mCherry/mCherry-TDP-CTF and EGFP/EGFP-tagged transportin constructs using FuGene6 (Promega). 48 hours after transfection, cells were incubated with the dye for 30 minutes at 37°C protected from light, then fixed in 4% paraformaldehyde and imaged using an epifluorescence microscope (Nikon Eclipse Ti2). Cell death was assessed by counting the number of dead cells that incorporated the dye.

### Results

### The import receptor KPNB1 reduces TDP-CTF aggregates.

It was discovered that the expression of importin β1 / karyopherin β1 (KPNB 1) reduced the aggregation of insoluble TDP-CTF protein in the cytoplasm and caused a strong reduction of TDP-CTF protein levels, while not affecting endogenous TDP-43 levels (Figure 1).

### Specific nuclear import receptors (NIRs) reduces TDP-CTF aggregates

To find out whether this effect was specific for KPNB 1, all 7 alpha-type and 20 beta-type karyopherins were obtained or cloned as expression constructs and were tested for their ability to reduce TDP-CTF aggregates in SH-SY5Y cells. KPNB 1/IPO1, IPO3, 4, 9, and 13 had the strongest effect on reducing TDP-CTF aggregation (Figure 2), whereas the exportins and 7 α-karyopherins had no major effects.

### The N-terminal fragment of KPNB1 reduces TDP-CTF aggregates

To determine the minimal domain of KPNB 1 that is required and sufficient for the activity toward TDP-43, extensive series of truncated KPNB1 constructs were generated based on serial deletions of the alpha-helical HEAT repeats (H1-19). These constructs were used to locate the maximally active domain in the N-terminal half of KPNB1 H1-8 (Figure 3). Shorter fragments become instable or progressively lose their activity towards TDP-CTF.

### Specific NIRs restore nuclear localization of TDP-43 variants lacking a functional NLS

Certain NIRs also restored nuclear localization of TDP-43 constructs that lack a functional NLS (nuclear localization signal), including the C-terminal fragment TDP-CTF (TDP-43²⁰⁸⁻⁴¹⁴) and full length TDP-43 with a mutated NLS (TDP-43^{ΔNLS}). The NIRs KPNB1, IPO3A and 3B, and to a lesser extent also IPO13 caused nuclear localization of both normally cytoplasmic TDP-43 constructs (Figure 4). The localization of the truncated TDP-43 (short TDP; sTDP) splicing isoform with an active NES (nuclear export sequence) at its C-terminus was less affected. To determine which domain of IPO3 is required and sufficient for its activity toward TDP-43 constructs, serial truncation mutants were generated and tested. The results show that the IPO3b H8-20 fragment has the strongest activity to restore nuclear localization of TDP-43^{mNLS} (Figure 5A and 5C). Further N-terminal truncations cause TDP-43^{mNLS} to become progressively more cytoplasmic. C-terminal deletions cause IPO3b fragments to co-aggregate with TDP-43^{mNLS} in the cytoplasm. The active IPO3b H8-20 fragment also reduces insoluble TDP-CTF protein levels and TDP-CTF-induced cell death.

N- and C-terminally truncated fragments of IPO13 restore TDP-CTF nuclear localization.

In the case of IPO13, the full-length protein has a strong activity to reduce TDP-CTF (Figure 2B) and TDP-43^{ΔNLS} aggregates (Figure 6A), and in some cells it mediates the nuclear localization of TDP-CTF (Figure 4A). This activities are different from truncated constructs, where the N-terminal fragments H1-11 and the C-terminal H4-19 fragments are sufficient to mediate strong nuclear localization of TDP-CTF but do not have a strong effect on the solubility of TDP-43^{ΔNLS} (Figure 6A and 6B). This demonstrates that the strongest activity mediating nuclear localization of TDP-CTF resides between H4 and H11, while the strongest activity to reduce aggregation of TDP-43^{ΔNLS} requires all 20 HEAT repeats and resides between amino acid residue 1-934 (Figure 6A and 6B).

### TDP-43 associates with KPNB1 via its RRM2 domain

A series of TDP-43 constructs was tested to determine which domain is required and sufficient to mediate association with KPNB1 (Figure 7). The strongest association in pull-down experiments from lysates was found with the sequence from aa229-259 within the RRM2 domain. Weaker association was mediated via its C-terminal prion like domain. This demonstrates that the NLS is not required for association of KPNB1 with TDP-43 fragments.

### Specific NIRs reduce TDP-43 mediated toxicity.

To determine whether restoring solubility of pathological TDP-43 is beneficial for the survival of cells, cell death assays of SH-SY5Y cells expressing toxic TDP-43 constructs and selected NIRs were conducted. Experiments with TDP-CTF show that co-expressed KPNB 1 and even more so IPO13 reduced cell death, whereas the non-disaggregating NIRs KPNA1 and XPO1 do not show such activity (Figure 8), demonstrating thatNIRs that reduce TDP-43 aggregation also reduce TDP-43 toxicity *in vitro.*

Comparing the activity of the KPNB1 constructs towards TDP-CTF induced cell death show that only full-length KPNB1 and its N-terminal fragment but not the C-terminal fragment reduced cell death (Figure 9), matching their activity towards reducing TDP-CTF aggregates (Figure 3A).

Together these results demonstrate that NIR polypeptides can be used to treat proteinopathies associated with TDP-43 polypeptide aggregation. For example, NIR polypeptides can be used to prevent TDP-43 polypeptides from undergoing pathological phase transition, to reverse formation of insoluble aggregates, to restore TDP-43 nuclear localization, and/or to restore TDP-43 function in the nucleus.

### Example 2: KPNB1 reduces TDP-43 aggregation in multiple cellular models containing disease associated mutations.

### METHODS

### Cell culture and transfection

Human HEK293T cells were cultured in DMEM media (Life Technologies) containing 10% FBS and transfected with PolyMag Neo (Oz Biosciences) according to the manufacturer's protocol. SH-SY5Y neuroblastoma cells were cultured in DMEM/F12 media (Life Technologies) containing 10% FBS and transfected with NeuroMag Neo (Oz Biosciences) according to the manufacturer's protocol. HEK293T and SH-SY5Y cells were obtained from ATCC (CRL-1573 and CRL-2266, respectively).

### Immunofluorescence and image acquisition

Cells were plated on coverslips in 12-well plates and transfected for 24 hours, and then were fixed with 4% paraformaldehyde in PBS for 15 minutes at room temperature. For high-resolution imaging, z-series image stacks were acquired using an epifluorescence microscope (Nikon Eclipse Ti2) equipped with a Zyla camera (Zyla sCMOS, Andor). Within each experiment, all groups were imaged with the same acquisition settings. Image stacks were deconvolved using a 3D blind constrained iterative algorithm (Nikon NIS Elements).

### Cell lysis, subcellular fractionation, and immunoblotting

Cells were seeded in 12-well plates and transfected for 48 hours. Cells were lysed in RIPA Lysis and Extraction buffer (Fisher) supplemented with a protease inhibitor cocktail (Roche), and centrifuged at 15000 rpm for 20 minutes at 4°C. The supernatant was collected as the detergent-soluble fraction and the insoluble pellet was recovered in urea buffer (7M urea, 2M thiourea, 4% CHAPS, 50 mM Tris pH 8, Roche complete protease inhibitor cocktail). The samples were left at room temperature for 20 minutes then briefly sonicated and centrifuged at 15000 rpm for 20 minutes. The supernatant was collected as the detergent-insoluble fraction. For immunoblotting, the samples were boiled in Laemmli sample buffer for 5 minutes at 98°C and separated in BoltTM 4 to 12% Bis-Tris gels (Fisher). Proteins were blotted onto nitrocellulose membranes using an iBlotTM 2 Gel Transfer device (Fisher). Membranes were blocked with Odyssey blocking buffer (LiCor) for 1 hour followed by incubation with primary antibodies (anti-TDP-43 (1:1000, 12892-1-AP, Proteintech), anti-GFP (1:2000, Aves Labs) anti-β-tubulin (1:1000, DHSB)) overnight at 4°C and incubation with secondary antibodies (1:10000, LiCor) in PBS blocking buffer with 0.05% Tween 20 for 1 hour at room temperature. Blots were imaged using an Odyssey scanner (LiCor).

### RESULTS

Full length KPNB1 successfully blocks the aggregation of TDP-43-CTF in co-transfected cells (Figure 13A). TDP-43 containing various disease associated mutations (Q331K, M337V, or A382T) are similarly affected in a transfected cell model (Figure 13B). The aggregation of TDP-43-CTF engineered to either lack the C-terminal prion-like domain (sTDP) or containing a mutated nuclear localization signal (mNLS) are is also reduced by the expression of KPNB1 (Figure 13C & 13D). The inhibitory activity of KPNB1 is also maintained in more physiologically relevant primary cortical neurons (Figure 13E-13G). KPNB1 demonstrates robust activity against a range of disease-relevant forms of TDP-43 in multiple cellular environments.

### Example 3: Modifications of KPNB1 maintain its functional activity.

### METHODS

### Cell culture and transfection

Human HEK293T cells were cultured in DMEM media (Life Technologies) containing 10% FBS and transfected with PolyMag Neo (Oz Biosciences) according to the manufacturer's protocol. SH-SY5Y neuroblastoma cells were cultured in DMEM/F12 media (Life Technologies) containing 10% FBS and transfected with NeuroMag Neo (Oz Biosciences) according to the manufacturer's protocol. HEK293T and SH-SY5Y cells were obtained from ATCC (CRL-1573 and CRL-2266, respectively).

### Cell lysis, subcellular fractionation, and immunoblotting

Cells were seeded in 12-well plates and transfected for 48 hours. Cells were lysed in RIPA Lysis and Extraction buffer (Fisher) supplemented with a protease inhibitor cocktail (Roche), and centrifuged at 15000 rpm for 20 minutes at 4°C. The supernatant was collected as the detergent-soluble fraction and the insoluble pellet was recovered in urea buffer (7M urea, 2M thiourea, 4% CHAPS, 50 mM Tris pH 8, Roche complete protease inhibitor cocktail). The samples were left at room temperature for 20 minutes then briefly sonicated and centrifuged at 15000 rpm for 20 minutes. The supernatant was collected as the detergent-insoluble fraction. For immunoblotting, the samples were boiled in Laemmli sample buffer for 5 minutes at 98°C and separated in BoltTM 4 to 12% Bis-Tris gels (Fisher). Proteins were blotted onto nitrocellulose membranes using an iBlotTM 2 Gel Transfer device (Fisher). Membranes were blocked with Odyssey blocking buffer (LiCor) for 1 hour followed by incubation with primary antibodies (anti-TDP-43 (1:1000, 12892-1-AP, Proteintech), anti-GFP (1:2000, Aves Labs) anti-β-tubulin (1:1000, DHSB)) overnight at 4°C and incubation with secondary antibodies (1:10000, LiCor) in PBS blocking buffer with 0.05% Tween 20 for 1 hour at room temperature. Blots were imaged using an Odyssey scanner (LiCor).

### Immunoprecipitation

HEK293T cells were seeded in 6-well plates and transfected for 48 hours. Cells were then lysed in IP Lysis buffer (Fisher) supplemented with a protease inhibitor cocktail (Roche), and were centrifuged at 15000 rpm for 20 minutes at 4°C. A small aliquot of the recovered supernatant was set aside (input). Pull-down was performed using GFP- and RFP-TRAP magnetic beads (Chromotek) according to the manufacturer's protocol. Briefly, beads were washed twice in wash buffer (10mM Tris/Cl pH 7.5, 150mM NaCl, 0.5mM EDTA). The lysate was then incubated with the equilibrated beads overnight at 4°C with end-over-end rotation. The next day, beads were magnetically separated from the supernatant (flow-through) and washed 3 times with wash buffer. Bound proteins were released from the beads in an elution buffer (200mM glycine pH 2.5). Samples were boiled in Laemmli buffer for 10 minutes at 95°C and used for immunoblotting. The following primary antibodies were used: anti-Mab414 (1: 1000, Abcam), anti-mCherry (1:1000, Novus Biologicals), anti-GFP (1:2000, Aves Labs), anti-Nup62 (1: 1000, BD labs), anti-Nup50 (1: 1000, Abcam), anti-Ran (1:2000, Sigma) and anti-importinα/KPNA2 (1: 1000, Novus Biologicals).

### RESULTS

Wildtype KPNB1 is capable of binding directly to both wildtype TDP-43 and TDP-43-CTF, a C-terminal fragment of the aggregation-prone protein (Figure 14A). KPNB1 maintains its activity against GFP-TDP-43-CTF regardless of the presence of absence of an N-terminal mCherry fluorescent protein modification (Figure 14B). The function of KPNB 1 is likely leveraged through its interaction with the C-terminal fragment of TDP-43.

### Example 4: The FG-Nup interacting sequence is required for KPNB1 to reduce TDP-43 aggregation.

### METHODS

### Cell culture and transfection

Human HEK293T cells were cultured in DMEM media (Life Technologies) containing 10% FBS and transfected with PolyMag Neo (Oz Biosciences) according to the manufacturer's protocol. SH-SY5Y neuroblastoma cells were cultured in DMEM/F12 media (Life Technologies) containing 10% FBS and transfected with NeuroMag Neo (Oz Biosciences) according to the manufacturer's protocol. HEK293T and SH-SY5Y cells were obtained from ATCC (CRL-1573 and CRL-2266, respectively).

### Immunofluorescence and image acquisition

Cells were plated on coverslips in 12-well plates and transfected for 24 hours, and then were fixed with 4% paraformaldehyde in PBS for 15 minutes at room temperature. For high-resolution imaging, z-series image stacks were acquired using an epifluorescence microscope (Nikon Eclipse Ti2) equipped with a Zyla camera (Zyla sCMOS, Andor). Within each experiment, all groups were imaged with the same acquisition settings. Image stacks were deconvolved using a 3D blind constrained iterative algorithm (Nikon NIS Elements).

### Cell lysis, subcellular fractionation, and immunoblotting

Cells were seeded in 12-well plates and transfected for 48 hours. Cells were lysed in RIPA Lysis and Extraction buffer (Fisher) supplemented with a protease inhibitor cocktail (Roche), and centrifuged at 15000 rpm for 20 minutes at 4°C. The supernatant was collected as the detergent-soluble fraction and the insoluble pellet was recovered in urea buffer (7M urea, 2M thiourea, 4% CHAPS, 50 mM Tris pH 8, Roche complete protease inhibitor cocktail). The samples were left at room temperature for 20 minutes then briefly sonicated and centrifuged at 15000 rpm for 20 minutes. The supernatant was collected as the detergent-insoluble fraction. For immunoblotting, the samples were boiled in Laemmli sample buffer for 5 minutes at 98°C and separated in BoltTM 4 to 12% Bis-Tris gels (Fisher). Proteins were blotted onto nitrocellulose membranes using an iBlotTM 2 Gel Transfer device (Fisher). Membranes were blocked with Odyssey blocking buffer (LiCor) for 1 hour followed by incubation with primary antibodies (anti-TDP-43 (1:1000, 12892-1-AP, Proteintech), anti-GFP (1:2000, Aves Labs) anti-β-tubulin (1:1000, DHSB)) overnight at 4°C and incubation with secondary antibodies (1:10000, LiCor) in PBS blocking buffer with 0.05% Tween 20 for 1 hour at room temperature. Blots were imaged using an Odyssey scanner (LiCor).

### Immunoprecipitation

HEK293T cells were seeded in 6-well plates and transfected for 48 hours. Cells were then lysed in IP Lysis buffer (Fisher) supplemented with a protease inhibitor cocktail (Roche), and were centrifuged at 15000 rpm for 20 minutes at 4°C. A small aliquot of the recovered supernatant was set aside (input). Pull-down was performed using GFP- and RFP-TRAP magnetic beads (Chromotek) according to the manufacturer's protocol. Briefly, beads were washed twice in wash buffer (10mM Tris/Cl pH 7.5, 150mM NaCl, 0.5mM EDTA). The lysate was then incubated with the equilibrated beads overnight at 4°C with end-over-end rotation. The next day, beads were magnetically separated from the supernatant (flow-through) and washed 3 times with wash buffer. Bound proteins were released from the beads in an elution buffer (200mM glycine pH 2.5). Samples were boiled in Laemmli buffer for 10 minutes at 95°C and used for immunoblotting. The following primary antibodies were used: anti-Mab414 (1: 1000, Abcam), anti-mCherry (1:1000, Novus Biologicals), anti-GFP (1:2000, Aves Labs), anti-Nup62 (1: 1000, BD labs), anti-Nup50 (1: 1000, Abcam), anti-Ran (1:2000, Sigma) and anti-importinα/KPNA2 (1:1000, Novus Biologicals).

### RESULTS

KPNB1 is capable of binding to phenylalanine and glycine rich nucleoporins (FG-Nups). C-terminal truncations up to the 9^{th} heat domain maintain this interaction (Figure 15A). Mutation of the nucleoporin interaction sequence (I178A, F217A, Y255A, and I263R; mNIS) interferes with this interaction (Figure 15B). KPNB1-mNIS also demonstrates reduced capacity to disaggregate TDP-43-CTF (Figure 15C & 15D). This suggests that the disaggregation activity of KPNB1 towards TDP-43 is dependent upon interactions with FG-Nups like NUP62.

### Example 5: TDP-CTF binds to KPNB1 via its RRM2 domain.

### METHODS

### Cell culture and transfection

Human HEK293T cells were cultured in DMEM media (Life Technologies) containing 10% FBS and transfected with PolyMag Neo (Oz Biosciences) according to the manufacturer's protocol. SH-SY5Y neuroblastoma cells were cultured in DMEM/F12 media (Life Technologies) containing 10% FBS and transfected with NeuroMag Neo (Oz Biosciences) according to the manufacturer's protocol. HEK293T and SH-SY5Y cells were obtained from ATCC (CRL-1573 and CRL-2266, respectively).

### Cell lysis, subcellular fractionation, and immunoblotting

Cells were seeded in 12-well plates and transfected for 48 hours. Cells were lysed in RIPA Lysis and Extraction buffer (Fisher) supplemented with a protease inhibitor cocktail (Roche), and centrifuged at 15000 rpm for 20 minutes at 4°C. The supernatant was collected as the detergent-soluble fraction and the insoluble pellet was recovered in urea buffer (7M urea, 2M thiourea, 4% CHAPS, 50 mM Tris pH 8, Roche complete protease inhibitor cocktail). The samples were left at room temperature for 20 minutes then briefly sonicated and centrifuged at 15000 rpm for 20 minutes. The supernatant was collected as the detergent-insoluble fraction. For immunoblotting, the samples were boiled in Laemmli sample buffer for 5 minutes at 98°C and separated in BoltTM 4 to 12% Bis-Tris gels (Fisher). Proteins were blotted onto nitrocellulose membranes using an iBlotTM 2 Gel Transfer device (Fisher). Membranes were blocked with Odyssey blocking buffer (LiCor) for 1 hour followed by incubation with primary antibodies (anti-TDP-43 (1:1000, 12892-1-AP, Proteintech), anti-GFP (1:2000, Aves Labs) anti-β-tubulin (1:1000, DHSB)) overnight at 4°C and incubation with secondary antibodies (1:10000, LiCor) in PBS blocking buffer with 0.05% Tween 20 for 1 hour at room temperature. Blots were imaged using an Odyssey scanner (LiCor).

### Immunoprecipitation

HEK293T cells were seeded in 6-well plates and transfected for 48 hours. Cells were then lysed in IP Lysis buffer (Fisher) supplemented with a protease inhibitor cocktail (Roche), and were centrifuged at 15000 rpm for 20 minutes at 4°C. A small aliquot of the recovered supernatant was set aside (input). Pull-down was performed using GFP- and RFP-TRAP magnetic beads (Chromotek) according to the manufacturer's protocol. Briefly, beads were washed twice in wash buffer (10mM Tris/Cl pH 7.5, 150mM NaCl, 0.5mM EDTA). The lysate was then incubated with the equilibrated beads overnight at 4°C with end-over-end rotation. The next day, beads were magnetically separated from the supernatant (flow-through) and washed 3 times with wash buffer. Bound proteins were released from the beads in an elution buffer (200mM glycine pH 2.5). Samples were boiled in Laemmli buffer for 10 minutes at 95°C and used for immunoblotting. The following primary antibodies were used: anti-Mab414 (1: 1000, Abcam), anti-mCherry (1:1000, Novus Biologicals), anti-GFP (1:2000, Aves Labs), anti-Nup62 (1: 1000, BD labs), anti-Nup50 (1: 1000, Abcam), anti-Ran (1:2000, Sigma) and anti-importinα/KPNA2 (1:1000, Novus Biologicals).

### RESULTS

Truncation of TDP-43-CTF at its C-terminus demonstrates that removal of the PrLD does not inhibit interaction KPNB 1. Interaction is maintained as long as residues 208-239 are present. This includes the entire RRM2 domain (Figure 16A). Within the longer form of TDP-43-CTF, containing the PrLD, deletion of residues 230-240, within the RRM2 domain, reduce binding with KPNB1 (Figure 16B). This reduction in binding does not significantly impact the capacity of KPNB1 to disaggregate TDP-43 (Figure 16C).

### Example 6: FG-Nups mediate the activity of KPNB1 towards TDP-43

### METHODS

### Cell culture and transfection

Human HEK293T cells were cultured in DMEM media (Life Technologies) containing 10% FBS and transfected with PolyMag Neo (Oz Biosciences) according to the manufacturer's protocol. SH-SY5Y neuroblastoma cells were cultured in DMEM/F12 media (Life Technologies) containing 10% FBS and transfected with NeuroMag Neo (Oz Biosciences) according to the manufacturer's protocol. HEK293T and SH-SY5Y cells were obtained from ATCC (CRL-1573 and CRL-2266, respectively).

### Immunofluorescence and image acquisition

Cells were plated on coverslips in 12-well plates and transfected for 24 hours, and then were fixed with 4% paraformaldehyde in PBS for 15 minutes at room temperature. For high-resolution imaging, z-series image stacks were acquired using an epifluorescence microscope (Nikon Eclipse Ti2) equipped with a Zyla camera (Zyla sCMOS, Andor). Within each experiment, all groups were imaged with the same acquisition settings. Image stacks were deconvolved using a 3D blind constrained iterative algorithm (Nikon NIS Elements).

### Cell lysis, subcellular fractionation, and immunoblotting

Cells were seeded in 12-well plates and transfected for 48 hours. Cells were lysed in RIPA Lysis and Extraction buffer (Fisher) supplemented with a protease inhibitor cocktail (Roche), and centrifuged at 15000 rpm for 20 minutes at 4°C. The supernatant was collected as the detergent-soluble fraction and the insoluble pellet was recovered in urea buffer (7M urea, 2M thiourea, 4% CHAPS, 50 mM Tris pH 8, Roche complete protease inhibitor cocktail). The samples were left at room temperature for 20 minutes then briefly sonicated and centrifuged at 15000 rpm for 20 minutes. The supernatant was collected as the detergent-insoluble fraction. For immunoblotting, the samples were boiled in Laemmli sample buffer for 5 minutes at 98°C and separated in BoltTM 4 to 12% Bis-Tris gels (Fisher). Proteins were blotted onto nitrocellulose membranes using an iBlotTM 2 Gel Transfer device (Fisher). Membranes were blocked with Odyssey blocking buffer (LiCor) for 1 hour followed by incubation with primary antibodies (anti-TDP-43 (1:1000, 12892-1-AP, Proteintech), anti-GFP (1:2000, Aves Labs) anti-β-tubulin (1:1000, DHSB)) overnight at 4°C and incubation with secondary antibodies (1:10000, LiCor) in PBS blocking buffer with 0.05% Tween 20 for 1 hour at room temperature. Blots were imaged using an Odyssey scanner (LiCor).

### Immunoprecipitation

HEK293T cells were seeded in 6-well plates and transfected for 48 hours. Cells were then lysed in IP Lysis buffer (Fisher) supplemented with a protease inhibitor cocktail (Roche), and were centrifuged at 15000 rpm for 20 minutes at 4°C. A small aliquot of the recovered supernatant was set aside (input). Pull-down was performed using GFP- and RFP-TRAP magnetic beads (Chromotek) according to the manufacturer's protocol. Briefly, beads were washed twice in wash buffer (10mM Tris/Cl pH 7.5, 150mM NaCl, 0.5mM EDTA). The lysate was then incubated with the equilibrated beads overnight at 4°C with end-over-end rotation. The next day, beads were magnetically separated from the supernatant (flow-through) and washed 3 times with wash buffer. Bound proteins were released from the beads in an elution buffer (200mM glycine pH 2.5). Samples were boiled in Laemmli buffer for 10 minutes at 95°C and used for immunoblotting. The following primary antibodies were used: anti-Mab414 (1: 1000, Abcam), anti-mCherry (1:1000, Novus Biologicals), anti-GFP (1:2000, Aves Labs), anti-Nup62 (1: 1000, BD labs), anti-Nup50 (1: 1000, Abcam), anti-Ran (1:2000, Sigma) and anti-importinα/KPNA2 (1:1000, Novus Biologicals).

### RESULTS

Along with interacting with KPNB1, Nup62 colocalizes with TDP-43 aggregates as long as the PrLD is maintained (Figure 17A). When V, L, I, and/or M residues within the PrLD are mutated to F (VLIM-F), KPNB1 demonstrated reduced disaggregation activity (Figure 17B). These same mutations abrogated the interaction between KPNB1 and TDP-43 (Figure 17C). Colocalization between TDP-43 and Nup62 was also reduced by VLIM-F mutations (Figure 17D). TDP-43 containing the VLIM-F mutations also demonstrate resistance to KPNB1 disaggregation (Figure 17E). This suggests that the disaggregation activity of KPNB 1 towards TDP-43 is dependent upon the ternary interaction with FG-Nups.

### Example 7: KPNB1 reduces aggregation of TDP-43 in a familial model of ALS (C9ALS)

### METHODS

### Cell culture and transfection

Human HEK293T cells were cultured in DMEM media (Life Technologies) containing 10% FBS and transfected with PolyMag Neo (Oz Biosciences) according to the manufacturer's protocol. SH-SY5Y neuroblastoma cells were cultured in DMEM/F12 media (Life Technologies) containing 10% FBS and transfected with NeuroMag Neo (Oz Biosciences) according to the manufacturer's protocol. HEK293T and SH-SY5Y cells were obtained from ATCC (CRL-1573 and CRL-2266, respectively).

### Immunofluorescence and image acquisition

Cells were plated on coverslips in 12-well plates and transfected for 24 hours, and then were fixed with 4% paraformaldehyde in PBS for 15 minutes at room temperature. For high-resolution imaging, z-series image stacks were acquired using an epifluorescence microscope (Nikon Eclipse Ti2) equipped with a Zyla camera (Zyla sCMOS, Andor). Within each experiment, all groups were imaged with the same acquisition settings. Image stacks were deconvolved using a 3D blind constrained iterative algorithm (Nikon NIS Elements).

### RESULTS

The expression of C9orf72 possessing a large GR expansion is associated with familial ALS variant C9ALS. When poly-GR C9orf72 is present, it will co-aggregate with both TDP-43 and Nup62 (Figure 18A). Introduction of KPNB1 successfully reduces this aggregation. Truncated KPNB1 (H1-H8) demonstrates increased efficacy in this familial model of aggregation, but mutation of the NIS blocks this inhibitory activity (Figure 18B). This suggests that the active truncations of KPNB1 maintain the interaction with both FG-Nups and TDP-43 to exercise its activity.

### Example 8: Nuclear import receptors are recruited by FG-Nucleoporins to reduce hallmarks of TDP-43 proteinopathy

This Example describes the discover that one or more importin polypeptides and/or fragments thereof (and/or nucleic acids designed to express an importin polypeptide and/or a fragment thereof) can rescue all hallmarks of TDP-43 pathology, by restoring TDP-43 polypeptide solubility and nuclear localization of TDP-43 polypeptides, thereby reducing neurodegeneration in cellular and animal models of ALS/FTD.

The results in this Example re-present and expand on at least some of the results provided in other Examples.

### METHODS

### DNA constructs

The expression plasmids used in this study were obtained from multiple sources or generated as summarized in Table 9. A flexible linker (GGGS)₃ (SEQ ID NO:80) was inserted between fluorescent protein fusion proteins to ensure proper protein folding.

**Table 9. Summary of DNA constructs and sources.**

| **Plasmids** | **Sources** |
|---|---|
| pEGFP-C1 / mCherry-C1 | Clontech |
| GFP-TDP-CTF/ mCherry-TDP-CTF | Chou et al., Nat. Neurosci., 21:228-239 (2018) |
| GFP-TDP-43^{WT} | Chou et al., Nat. Neurosci., 21:228-239 (2018) |
| GFP-TDP-43^{mNLS} (KRK... KVKR>AAA...AVAA) | Chou et al., Nat. Neurosci., 21:228-239 (2018) |
| GFP-sTDP | sTDP was cloned into pEGFP-C 1 |
| GFP-TDP-43^{mNLS} 1-265 | TDP-43^{mNLS} 1-265 fragment was cloned into pEGFP-C1 |
| GFP-TDP-CTF non-PrLD | TDP-CTF non-PrLD (amino-acids 208-274) was generated by PCR and cloned in pEGFP-C1 |
| GFP-TDP-43 PrLD | TDP-43 PrLD (amino-acids 275-414) was generated by PCR and cloned into pEGFP-C1 |
| GFP-TDP-CTF 229-414 → 260-414/ 208-259 → 208-219 | TDP-CTF deletion constructs were generated by PCR and cloned into pEGFP-C1 |
| GFP-TDP-CTF Q331K/ M337V/ A382T | ALS-causing mutations were amplified by PCR and cloned into GFP-TDP-CTF |
| GFP-TDP-CTF Δ274-313/ Δ314-353/ Δ354-393 | TDP-CTF PrLD deletion constructs were cloned into GFP-TDP-CTF |
| GFP-TDP-CTF Δ230-240 | TDP-CTF lacking amino-acids 230-240 was generated by PCR and cloned into pEGFP-C1 |
| GFP-TDP-CTF F-A/ F-G / F-Y | Mutations were generated by site-directed mutagenesis and cloned into GFP-TDP-CTF |
| GFP-TDP-CTF G309F/ G368W/ 2xφ/ 4xφ/ K-R/ KRED-S/ FYW-L/ VLIM-F | Mutant constructs Addgene #107822/ #107837/ #107806/ #107805/ #118796/ #118795/ # 118793/ #118794 were cloned into GFP-TDP-CTF |
| GFP-TDP-43^{mNLS} 5F-L | Mann et al., Neuron, 102:321-338 e328 (2019) |
| GFP-TDP-43^{mNLS} mNES | Mutations in the NES (I239A, L243A, L248A, I249A, I250A were inserted in GFP-TDP-43^{mNLS} |
| GFP-TDP-43^{mNLS} G368W/ 4xφ/ FYW-L/ VLIM-F | Mutant constructs (Addgene #107837/ #107805/ #118793/ #118794) were cloned into GFP-TDP-43^{mNLS} |
| GFP/ mCherry/ 3xFLAG-KPNB1 | mCherry and 3xFLAG were cloned into GFP-KPNB1 (Euroscarf # P30478) |
| Untagged KPNB1 | KPNB1 was amplified by PCR and cloned into pEGFP-C1 plasmid lacking the GFP tag |
| GFP-KPNB1 H1-9 → H1 | KPNB1 fragments were generated by PCR and cloned into pEGFP-C 1 |
| GFP-KPNB1 H1-8^{mNIS} | NIS mutations (I178A, F217A, Y255A, I263R; were generated by site-directed mutagenesis and cloned into pEGFP-C1 |
| GFP-KPNB1 H10-19 | KPNB1 H10-19 was generated by DNA synthesis and cloned into pEGFP-C1 |
| FLAG-KPNB1 H1-3 | KPNB1 H1-3 was generated by DNA synthesis and cloned into pEGFP-C1 |
| FLAG-KPNB1 H1-8^{WT}/H1-8^{mNIS} | 3xFLAG tag replaced GFP in GFP-KPNB1 H1-8^{WT}/ H1-8^{mNIS} |
| mScarlet-C1 | mScarlettag was cloned from mScarlet-I-LaminB (Addgene #98831) into pEGFP-C1 |
| GFP-TNPO1 | Guo et al., Cell, 173:677-692 e620 (2018) |
| GFP-TNPO2A/ TNPO2B | TNPO2A and 2B constructs were cloned into pEGFP-C1 |
| GFP-IPO4/ IPO7/ IPO8/ IPO9/ IPO11/ TNPO3/ IPO13 | Importin constructs were cloned into pEGFP-C1 |
| GFP-IPOS | Chao et al., Nucleic Acids Res., 40:8484-8498 (2012) |
| GFP-KPNA1 → KPNA7 | KPNA1 → KPNA7 constructs (Addgene #26677 → #26683) were cloned into pEGFP-C1 |
| GFP-XPO1/ XPO2/ XPOT/ XPO6/ XPO7/ RANBP6/ RANBP17 | Exportin constructs were cloned into pEGFP-C1 |
| GFP-XPO4 | XPO4 was cloned into pEGFP-C 1 |
| GFP-XPO5 | Addgene #58331 |
| Nup62-mCherry | mCherry replaced GFP in pNup62-EGFP3 (Euroscarf #P30484) |
| mCherry-Nup85 | mCherry replaced GFP in pEGFP3-Nup85 (Euroscarf #P30485) |
| GFP-Nup98 | Euroscarf #P30487 |
| NES-tdTomato-NLS | Hatch et al, Cell, 154:47-60 (2013) |
| GFP-(GR)₁₀₀ | A fragment with 100 GR repeats was generated by DNA synthesis and cloned into pEGFP-C1 |
| GFP-FUSΔ14 | FUS lacking the last 14 amino-acids was generated by DNA synthesis and cloned into pEGFP-C1 |
| AAV-Syn-GFP | Addgene #50465 |
| AAV-Syn-GFP-TDP-43^{mNLS} | GFP-TDP-43^{mNLS} was cloned into AAV-Syn-GFP |
| AAV-Syn-mScarlet-TDP-CTF | mScarlet-TDP-CTF was cloned into AAV-Syn-GFP |
| AAV-Syn-FLAG-mCherry | FLAG-mCherry was cloned into AAV-Syn-GFP |
| AAV-Syn-FLAG-KPNB1 H1-9^{WT}/ H1-9^{mNIS}/ H1-8^{WT}/ H1-8^{mNIS}/ H10-19 | FLAG-KPNB 1 constructs were cloned into AAV-Syn-GFP |
| pGW1-GFP | GFP was cloned into pGW1 |
| pGW1-GFP-TDP-43 WT/ mNLS | TDP-43 WT/ mNLS were cloned into pGW 1-GFP |
| pGW1-GFP-TDP-CTF | GFP-TDP-CTF was cloned into pGW1 |
| pGW1-mScarlet | mScarlet was cloned into pGW1 |
| pGW1-mScarlet-KPNB1 | KPNB1 was cloned into pGW1-mScarlet |
| pGW1-NLS-mTagBFP | NLS-mTagBFP was cloned into pGW1 |
| pGW1-HaloTag | HaloTag was cloned into pGW1 |

### Mammalian cell culture and transfection

Human HEK293T cells were cultured in DMEM media (Life Technologies) containing 10% FBS. SH-SY5Y neuroblastoma cells were cultured in DMEM/F12 media (Life Technologies) containing 10% FBS. Cells were transfected with NeuroMag Neo (OZ Biosciences) according to the manufacturer's protocol. HEK293T and SH-SY5Y cells were purchased from ATCC (CRL-1573 and CRL-2266, respectively).

### Immunostaining and image acquisition and analysis

Cells were plated on coverslips in 12-well plates. 24 hours after transfection, cells were fixed with 4% paraformaldehyde (PFA) in PBS for 15 minutes at room temperature (RT). For experiments with GFP-(GR)₁₀₀, cells were fixed 48 hours after transfection. Cells were permeabilized with 0.2% Triton X-100 in PBS for 10 minutes, blocked with 5% bovine serum albumin (BSA) for 45 minutes and incubated with the following primary antibodies: anti-FLAG (1:500), anti-TDP-43 (1:500), anti-Nup62 (1:250), anti-Nup98 (1:250), anti-Ran (1:200) and anti-KPNB1 (1:100) overnight at 4°C, followed by incubation with fluorophore-coupled secondary antibodies for 1 hour at RT.

For high-resolution fluorescence microscopy, single and multiple z-plane images were acquired using an epifluorescence microscope (Nikon Eclipse Ti2) equipped with a Zyla camera (Zyla sCMOS, Andor). Within each experiment, all groups were imaged with the same acquisition settings. Image stacks were deconvolved using a 3D blind constrained iterative algorithm (Nikon NIS Elements).

To quantitate the spatial overlap between Nup62-mCherry and GFP-tagged TDP-43 constructs, Mander's colocalization coefficient was calculated using the JACoP plugin on ImageJ (Bolte and Cordelieres, 2006). Values close to 0 and 1 indicate weak and perfect colocalization, respectively.

As a reporter for nucleocytoplasmic transport of proteins, NES-tdTomato-NLS was co-transfected with GFP or GFP-tagged KPNB1 constructs in SH-SY5Y cells. The mean pixel intensity of NES-tdTomato-NLS in the nucleus and cytoplasm was measured to calculate the nuclear-to-cytoplasmic (N-to-C) ratio.

To semi-quantitatively determine nuclear-cytoplasmic localization of GFP-tagged TDP-43^{mNLS}, HEK293T cells were co-transfected with GFP-TDP-43^{mNLS} and different mCherry-KPNB1 constructs, fixed, and z-stacks were acquired as described above. Images were blinded across conditions and TDP-43^{mNLS} fluorescent signal was characterized as nuclear/nucleocytoplasmic/cytoplasmic for at least 20 cells per condition, in triplicate.

### Cell lysis, subcellular fractionation and immunoblotting

Cells were seeded in 12-well plates and transfected for 48 hours. Cells were lysed in RIPA Lysis and Extraction buffer (Thermo Fisher Scientific) supplemented with a protease inhibitor cocktail (Roche) and centrifuged at 15000 rpm for 20 minutes at 4°C. The supernatant was collected as the detergent-soluble fraction and the insoluble pellet was recovered in urea buffer (7M urea, 2M thiourea, 4% CHAPS, 50 mM Tris pH 8, Roche complete protease inhibitor cocktail). For experiments with total protein lysate, cells were collected in urea buffer. The samples were left at RT for 20 minutes then briefly sonicated and centrifuged at 15000 rpm for 20 minutes. The supernatant was collected as the detergent-insoluble fraction. For immunoblotting, the samples were boiled in Laemmli sample buffer for 5 minutes at 98°C and separated in Bolt^{™} 4 to 12% Bis-Tris gels (Thermo Fisher Scientific). Proteins were blotted onto nitrocellulose membranes using an iBlot^{™} 2 Gel Transfer device (Thermo Fisher Scientific). Membranes were blocked with Odyssey blocking buffer (LI-COR) for 1 hour followed by incubation with the following primary antibodies: anti-mCherry (1: 1,000), anti-GFP (1:2,000), anti-β-tubulin (1: 1,000), anti-β-actin (1: 1,000) and anti-KPNB 1 (1:1,000) overnight at 4°C and incubation with secondary antibodies (1:10,000) in PBS blocking buffer with 0.05% Tween 20 for 1 hour at RT. Blots were imaged using an Odyssey scanner (LI-COR).

### Immunoprecipitation (IP)

HEK293T cells were seeded in 6-well plates. 48 hours after transfection, cells were lysed in IP Lysis buffer (Thermo Fisher Scientific) supplemented with a protease inhibitor cocktail (Roche) and centrifuged at 15000 rpm for 20 minutes at 4°C. A small aliquot of the recovered supernatant was collected as input extract, and the rest was used for IP. Pull-down was performed using GFP- and RFP-Trap magnetic beads (Chromotek) according to the manufacturer's protocol. Briefly, beads were washed twice in wash buffer (10 mM Tris/Cl pH 7.5, 150 mM NaCl, 0.5 mM EDTA). The lysate was then incubated with the equilibrated beads overnight at 4°C with end-over-end rotation. Beads were magnetically separated from the supernatant (flow-through) and washed 3 times with wash buffer. Bound proteins were released from the beads in an elution buffer (200 mM glycine pH 2.5). Samples were boiled in Laemmli buffer for 10 minutes at 95°C and used for immunoblotting. The following primary antibodies were used: anti-mCherry (1: 1,000), anti-GFP (1:2,000), anti-mAb414 (1: 1,000), anti-Nup62 (1:1,000), anti-Nup50 (1: 1,000), anti-importin-α1 (1:1,000) and anti-Ran (1:2,000).

### RNA isolation and real-time RT-PCR

RNA from cultured HEK293T cells was isolated using RNeasy Plus Mini Kit (Qiagen) according to the manufacturer's protocol. Isolated RNA was treated with RQ1 DNase (Promega) and used for the cDNA synthesis with SuperScript First-Strand Synthesis System (Invitrogen) using oligo-dT and random hexamer primers. Real-time RT-PCR was performed using TaqMan Universal PCR Master Mix (Applied Biosystems) and TaqMan gene expression assays for *EGFP* (Mr04097229_mr, Thermo Fisher Scientific) and human *GAPDH* (Hs02758991_g1, Thermo Fisher Scientific) on QuantStudio 7 Flex Real-Time PCR system (Applied Biosystems).

### Cell death assay

Cell death of SH-SY5Y cells was assessed by the uptake of the membrane-impermeant Live-or-Dye^{™} Fixable Dead Cell Dye 640/662 (Live-or-Dye^{™} Fixable Viability Staining Kit, 32007, Biotium). The dye penetrates dying cells that have compromised membrane integrity and labels amines on intracellular proteins. Cells were transfected with mCherry/mCherry-TDP-CTF and GFP/GFP-tagged KPNB 1 constructs using FuGene6 (Promega). 48 hours after transfection, cells were incubated with the dye for 30 minutes at 37°C protected from light, then fixed in 4% PFA and imaged using an epifluorescence microscope (Nikon Eclipse Ti2). Cell death was assessed by scoring the proportion of dying cells that incorporated the dye.

### Co-immunofluorescence on human post-mortem tissue

Tissue derived from spinal cord and hippocampus were obtained from patients with ALS or FTLD, respectively, in addition to tissue from neuropathologically normal control tissue blocks. The neuropathological curation of cases consists of sporadic and familial disease, where select cases were available for patients who developed ALS or FTLD due to genetic mutations in *C9orf72, SOD1,* or *TARDBP.* Post-mortem case demographics are provided in Table 7. Brains were processed in 10% neutral buffered formalin before tissue blocks were selected during gross neuroanatomically examination and embedded in paraffin for their preservation and sectioning. Double immunofluorescence staining was performed on 5 µm formalin fixed paraffin embedded (FFPE) tissue sections obtained from the spinal cord or posterior hippocampus. Briefly, FFPE tissue sections were deparaffinized by immersion in xylene and rehydrated through a series of graded ethanol solutions (100%, 90% and 70% ethanol). Tissue was rinsed in dH₂O and antigen retrieval was performed by steaming tissue sections in citrate buffer, pH 6 (Dako Target Retrieval Solution, S2369), for 30 minutes. Tissue sections were cooled to RT and rinsed with dH₂O for 10 minutes. Sections were permeabilized and blocked with serum-free protein block containing 0.3% Triton-X 100 at RT for 1 hour. Tissues were immunostained with antibodies against pTDP-43 (1:200), Nup62 (1:100) or KPNB1 (1:2000), diluted in antibody diluent (Dako, S0809) and incubated overnight at 4°C in a humidified chamber. Tissue sections were washed in tris buffered saline (TBS) containing 0.05% Tween-20 (TBS-T), three times for 10 minutes each. Alexa Fluor conjugated secondary antibodies (1:1000, F'(ab)anti-mouse 488 or anti-rat 555 or anti-rat 647, Thermo Fisher Scientific), were diluted in antibody diluent (Dako, S0809) and incubated for 2 hours at RT. Tissue was washed with TBS-T and incubated with Hoechst 33342 (1: 1000, Thermo Fisher Scientific) for 20 minutes at RT before rinsing with TBS. To quench autofluorescence, tissues were stained with 1x True Black lipofuscin autofluorescence quencher (Biotium, 23007) for 30 seconds and rinsed with dH₂O for 5 minutes. Tissue sections were mounted with glass slides using ProLong Glass Antifade Mountant (Invitrogen). High-resolution imaging was conducted, and optical sections were acquired, according to the depth of the tissue to capture the full contour of the pTDP-43 aggregate. Z-series were obtained using an ECLIPSE Ti2 fluorescence microscope (Nikon) equipped with a Spectra X multi-LED light engine (Lumencor), single bandpass filter cubes for DAPI, EGFP/FITC and TRITC (Chroma), and a ZYLA 4.2 PLUS sCMOS camera (Andor), using NIS Elements HC V5.30 software (Nikon). Immunofluorescent staining of all cases and CNS regions was conducted at the same time and all pathological subgroups were imaged with the same acquisition settings. Out of focus light from each z-series was eliminated using the Clarify.ai module of the NIS-Elements Advanced Research (V5.30) deconvolution package. Clarified z-series are shown as optical projections and 3D volume rendered views to demonstrate colocalization of KPNB 1 and Nup62 with pTDP-43 inclusions. Fluorescent tile images of the same tissue sections were captured using a 40x objective on an inverted fluorescent microscope (Keyence BZ-X800) and high-resolution image stitching was completed using the Keyence analyzer package (BZ-X series).

**Table 7. Demographic information of human post-mortem cases used for Nup62 and KPNB1 co-immunostaining with pTDP-43^{S409/410}.**

| **Case ID** | **Clinical Dx** | **Prim.path Dx** | **Age** | **Sex** | **Genetics** | **TDP-43 subtype** | **Braak stage** | **Thai phase** | **Brain weight (g)** | **CNS region** |
|---|---|---|---|---|---|---|---|---|---|---|
| **C9/ALS #1** | ALS | ALS-C9 | 66 | M | C9orf72 | B | II | 0 | 1320 | Spinal cord |
| **C9/ALS #2** | ALS | ALS-C9 | 67 | F | C9orf72 | B | II | 0 | 1120 | Spinal cord |
| **sALS #1** | ALS | ALS | 76 | M | - | B | III | 0 | 1340 | Spinal cord |
| **sALS #2** | ALS | ALS | 58 | F | - | B | 0 | 0 | 1260 | Spinal cord |
| **SOD1/ALS #1** | ALS | ALS-SOD1 | 66 | F | SOD1^{#} | - | 0 | 0 | 1360 | Spinal cord |
| **SOD1/ALS #2** | ALS | ALS-SOD1 | 48 | M | SOD* | - | 0 | 0 | 1260 | Spinal cord |
| **FUS/ALS** | ALS | ALS-FUS | 68 | F | undetermined | - | II | 0 | 1410 | Spinal cord |
| **TARDBP/ALS** | ALS | ALS | 67 | M | TARDBP‡ | - | I | 0 | 1080 | Spinal cord |
| **Control #1** | MSA | Normal | 77 | M | - | - | II | 0 | 1000 | Spinal cord |
| **Control #2** | Normal | Normal | 64 | M | - | - | II | 0 | 1420 | Spinal cord |
| **C9/FTLD-A #1** | FTD | FTLD-C9 | 69 | F | C9orf72 | A | III | 0 | 880 | Hippocampus |
| **C9/FTLD-A #2** | FTD | FTLD-C9 | 69 | M | C9orf72 | A | IV | 0 | 1320 | Hippocampus |
| **C9/FTLD-B #1** | FTD | FTLD-C9 | 77 | M | C9orf72 | B | II | 0 | 1300 | Hippocampus |
| **C9/FTLD-B#2** | FTD | FTLD-C9 | 62 | M | C9orf72 | B | II | 0 | 1060 | Hippocampus |
| **sFTLD-A #1** | FTD | FTLD | 70 | F | - | A | II | 3 | 1040 | Hippocampus |
| **sFTLD-A #2** | FTD | FTLD | 76 | F | - | A | 0 | 0 | 1040 | Hippocampus |
| **sFTLD-B #1** | DLB | FTLD | 70 | M | - | B | III | 0 | 1200 | Hippocampus |
| **sFTLD B#2** | PSP | FTLD | 62 | F | - | B | III | 0 | 1260 | Hippocampus |
| **Control #3** | Normal | Normal | 58 | M | - | - | II | 0 | 1280 | Hippocampus |
| **Control #4** | Depression | Normal | 59 | M | - | - | 0 | 0 | 1540 | Hippocampus |

### Organotypic brain slice cultures, transduction and immunohistochemistry

Brain slice cultures (BSCs) were prepared from postnatal day 8-9 (P8-9) C57BL/6 mice. Pups were placed in a chamber with isofluorane and decapitated after confirmation with toe-pinch response. The hemi-brains were dissected to collect the cortex and hippocampus in sterile-filtered ice-cold dissection buffer containing Hank's balanced salt solution (HBSS), calcium, magnesium, no phenol red (Thermo Fisher Scientific), 2 mM ascorbic acid (Sigma Aldrich), 39.4 µM ATP (Sigma Aldrich) and 1% (v/v) penicillin/streptomycin (Thermo Fisher Scientific). Each hemi-brain was placed on filter paper and cut into 350 µm slices using a McIllwain^{™} tissue chopper (Stoelting Co.). Slices were placed in 6-well tissue culture plates to contain three slices per semi-porous membrane insert per well (0.4 µm pore diameter, Millipore Sigma). BSCs were maintained at 37°C and 5% CO₂ in sterile-filtered culture medium containing Basal Medium Eagle (Thermo Fisher Scientific), 26.6 mM HEPES (pH 7.1, Thermo Fisher Scientific), 511 µM ascorbic acid, 1% (v/v) GlutaMAX (Thermo Fisher Scientific), 0.033% (v/v) insulin (Sigma Aldrich), 1% (v/v) penicillin/streptomycin (Thermo Fisher Scientific) and 25% (v/v) heat-inactivated horse serum (Sigma Aldrich). Culture medium was changed every 3-4 days.

For AAV transduction of BSCs, AAV plasmids were first transfected in HEK293T using Polyethylenimine Hydrochloride (Polysciences). 3-4 days after transfection, cell media was collected and centrifuged at 800 rcf for 5 minutes to recover the supernatant containing secreted AAVs. AAVs were applied directly into the culture medium on the first day of the BSC (DIV0).

For immunostaining of BSCs, slices were fixed 12-15 days after transduction (DIV12-15) with 4% PFA for 1 hour at RT, permeabilized with 0.5% Triton X-100 overnight at 4°C and blocked with 20% BSA for 4 hours. BSCs were incubated with the following primary antibodies: anti-FLAG (1:500), anti-GFP (1:500) and anti-TDP-43 (1:500) in 5% BSA for 48 hours at 4°C, followed by incubation with fluorophore-coupled secondary antibodies for 4 hours at RT. To stain the nuclei, slices were incubated with the Hoechst dye (1:5000) for 30 minutes at RT, then coverslipped with ProLong Glass Antifade Mountant (Thermo Fisher Scientific). Nuclear-cytoplasmic localization of TDP-43 was semi-quantitatively determined as described above for HEK293T cells.

### Statistical analysis

Data from different experiments were analyzed by mean value analysis (Student's t-test) or analysis of variance (one- or two-way ANOVA) using GraphPad Prism software. Bonferroni's post hoc test was used. Differences were considered statistically significant when p < 0.05. All values are mean and SEM.

**Table 8. Summary of statistical analyses.**

| Fig. 31D | | | |
|---|---|---|---|
| Test used | Two-way ANOVA; Bonferroni post hoc test | | |
| n | mCherry | n=79 | Three |
| | mCherry-KPNB1 FL | n=66 | independent |
| | mCherry-KPNB1 H1-9 | n=81 | experiments |
| | mCherry-KPNB1 H1-8^{WT} | n=76 | |
| | mCherry-KPNB1 H1-8^{mNIS} | n=80 | |
| | mCherry-KPNB1 H10-19 | n=74 | |
| F (DFn, DFd) | F (10, 36) = 7.067 | | |
| | F (5, 36) = 9.552e-016 | | |
| | F (2, 36) = 54.45 | | |
| P Value | p < 0.001 | | |
| | p > 0.999 | | |
| | p < 0.001 | | |
| Post hoc test: cytoplasmic GFP-TDP-43^{mNLS} | mCherry vs mCherry-KPNB1 H1-9^{WT} | p = 0.002 | |
| | mCherry vs mCherry-KPNB1 H1-8^{WT} | p < 0.001 | |
| | mCherry-KPNB1 H1-8^{WT} vs mCherry-KPNB1 H1-8^{mNIS} | p = 0.002 | |
| Post hoc test: nucleocytoplasmic GFP-TDP-43^{mNLS} | mCherry vs mCherry-KPNB1 H1-9^{WT} | p = 0.009 | |
| | mCherry vs mCherry-KPNB1 H1-8^{WT} | p = 0.001 | |
| | mCherry-KPNB1 H1-8^{WT} vs mCherry-KPNB1 H1-8^{mNIS} | p = 0.016 | |

| Fig. 31F | | | |
|---|---|---|---|
| Test used | Two-way ANOVA; Bonferroni post hoc test | | |
| n | FLAG-mCherry | n=150 | Three independent experiments |
| | FLAG-KPNB1 H1-9^{WT} | n=152 | |
| | FLAG-KPNB1 H1-9^{mNIS} | n=151 | |
| | FLAG-KPNB1 H1-8^{WT} | n=156 | |
| | FLAG-KPNB1 H1-8^{mNIS} | n=151 | |
| | FLAG-KPNB1 H10-19 | n=154 | |
| F (DFn, DFd) | F (10, 36) = 152.0 | | |
| | F (5, 36) = 1.183e-007 | | |
| | F (2, 36) = 148.8 | | |
| P Value | p < 0.001 | | |
| | p > 0.999 | | |
| | p < 0.001 | | |
| Post hoc test: cytoplasmic GFP-TDP-43^{mNLS} | FLAG-mCherry vs FLAG-KPNB1 H1-9^{WT} | p < 0.001 | |
| | FLAG-mCherry vs FLAG-KPNB1 H1-9^{mNIS} | p < 0.001 | |
| | FLAG-mCherry vs FLAG-KPNB1 H1-8^{WT} | p < 0.001 | |
| | FLAG-KPNB1 H1-9^{WT} vs FLAG-KPNB1 H10-19 | p < 0.001 | |
| | | p < 0.001 | |
| | FLAG-KPNB1 H1-8^{WT} vs FLAG-KPNB1 H1-8^{mNIS} | | |
| Post hoc test: nucleocytoplasmic GFP-TDP-43^{mNLS} | FLAG-mCherry vs FLAG-KPNB1 H1-9^{WT} | p < 0.001 | |
| | FLAG-mCherry vs FLAG-KPNB1 H1-9^{mNIS} | p < 0.001 | |
| | FLAG-mCherry vs FLAG-KPNB1 H1-8^{WT} | p < 0.001 | |
| | FLAG-KPNB1 H1-9^{WT} vs FLAG-KPNB1 H1-9^{mNIS} | p < 0.001 | |
| | | p < 0.001 | |
| | FLAG-KPNB1 H1-9^{WT} vs FLAG-KPNB1 H10-19 | p < 0.001 | |
| | FLAG-KPNB1 H1-8^{WT} vs FLAG-KPNB1 H1-8^{mNIS} | | |
| Post hoc test: nuclear GFP-TDP-43^{mNLS} | FLAG-mCherry vs FLAG-KPNB1 H1-9^{WT} | p < 0.001 | |
| | FLAG-mCherry vs FLAG-KPNB1 H1-8^{WT} | p < 0.001 | |
| | FLAG-KPNB1 H1-9^{WT} vs FLAG-KPNB1 H1-9^{mNIS} | p < 0.001 | |
| | | p < 0.001 | |
| | FLAG-KPNB1 H1-9^{WT} vs FLAG-KPNB1 H10-19 | p < 0.001 | |
| | FLAG-KPNB1 H1-8^{WT} vs FLAG-KPNB1 H1-8^{mNIS} | | |

| Fig. 38B | | | |
|---|---|---|---|
| Test used | Two-way ANOVA; Bonferroni post hoc test | | |
| n | GFP | n=168 | Three independent experiments |
| | FLAG-KPNB1 H1-9^{WT} | n=178 | |
| | FLAG-KPNB1 H1-9^{mNIS} | n=158 | |
| | FLAG-KPNB1 H1-8^{WT} | n=162 | |
| | FLAG-KPNB1 H1-8^{mNIS} | n=160 | |
| | FLAG-KPNB1 H10-19 | n=161 | |
| F (DFn, DFd) | F (10, 36) = 41.48 | | |
| | F (5, 36) = 1.775e-008 | | |
| | F (2, 36) = 22.51 | | |
| P Value | p < 0.001 | | |
| | p > 0.999 | | |
| | p < 0.001 | | |
| Post hoc test: cytoplasmic mScarlet-TDP-CTF | GFP vs FLAG-KPNB1 H1-9^{WT} | p < 0.001 | |
| | GFP vs FLAG-KPNB1 H1-9^{mNIS} | p < 0.001 | |
| | GFP vs FLAG-KPNB1 H1-8^{WT} | p < 0.001 | |
| | FLAG-KPNB1 H1-9^{WT} vs FLAG-KPNB1 H10-19 | p < 0.001 | |
| | | p < 0.001 | |
| | FLAG-KPNB1 H1-9^{mNIS} vs FLAG-KPNB1 H1-8^{mNIS} | p < 0.001 | |
| | FLAG-KPNB1 H1-8^{WT} vs FLAG-KPNB1 H1-8^{mNIS} | | |
| Post hoc test: nucleocytoplasmic mScarlet-TDP-CTF | GFP vs FLAG-KPNB1 H1-9^{mNIS} | p < 0.001 | |
| | GFP vs FLAG-KPNB1 H1-8^{WT} | p = 0.001 | |
| | FLAG-KPNB1 H1-9^{WT} vs FLAG-KPNB1 H1-9^{mNIS} | p = 0.002 | |
| | | p = 0.03 | |
| | FLAG-KPNB1 H1-9^{WT} vs FLAG-KPNB1 H1-8^{WT} | p < 0.001 | |
| | | p = 0.006 | |
| | FLAG-KPNB1 H1-9^{mNIS} vs FLAG-KPNB1 H1-8^{mNIS} | | |
| | FLAG-KPNB1 H1-8^{WT} vs FLAG-KPNB1 H1-8^{mNIS} | | |
| Post hoc test: nuclear mScarlet-TDP-CTF | GFP vs FLAG-KPNB1 H1-9^{WT} | p < 0.001 | |
| | GFP vs FLAG-KPNB1 H1-8^{WT} | p < 0.001 | |
| | FLAG-KPNB1 H1-9^{WT} vs FLAG-KPNB1 H1-9^{mNIS} | p < 0.001 | |
| | | p < 0.001 | |
| | FLAG-KPNB1 H1-9^{WT} vs FLAG-KPNB1 H1-8^{WT} | p < 0.001 | |
| | | p = 0.001 | |
| | FLAG-KPNB1 H1-9^{WT} vs FLAG-KPNB1 H10-19 | | |
| | FLAG-KPNB1 H1-8^{WT} vs FLAG-KPNB1 H1-8^{mNIS} | | |

### RESULTS

### A subset of β-importin family nucleocytoplasmic import receptors (NIRs) reduce cytoplasmic aggregation of TDP-CTF

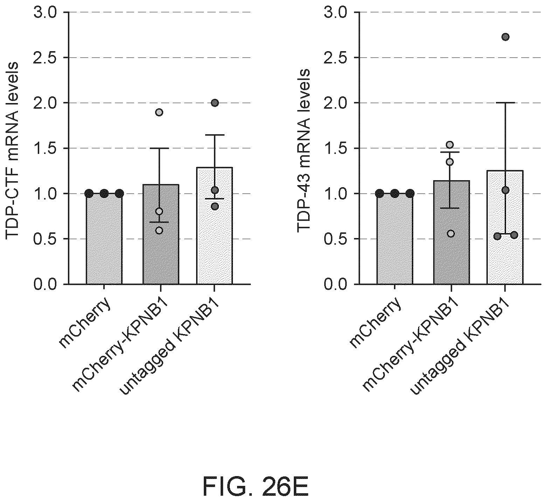

TDP-CTF is a 25-kDa C-terminal fragment of TDP-43 that lacks the NLS but contains parts of RRM2 and the complete PrLD and is a major component of insoluble cytoplasmic aggregates in cortical and hippocampal regions of ALS and FTLD-TDP patient brain tissue. Its expression recapitulates histopathological features of TDP-43 pathology by forming detergent-insoluble inclusions that are positive for phospho-TDP-43^{S409/410}, p62/SQSTM1, and ubiquitin, and induces high levels of toxicity in neuronal cell lines and primary neurons. KPNB1 reduces TDP-CTF cytoplasmic aggregation in human neuroblastoma SH-SY5Y cells, rendering its localization more nucleocytoplasmic (Figure 26A). In complementary biochemical fractionation experiments, KPNB1 expression significantly reduced the detergent-insoluble protein levels of TDP-CTF and variants carrying ALS-causing point mutations without affecting endogenous nuclear TDP-43 levels (Figures 26A and 26C). Expression of mCherry-KPNB1 or untagged-KPNB 1 significantly reduced insoluble TDP-CTF protein levels, with very little effect on soluble TDP-CTF (Figure 26B). In addition, both constructs significantly reduced total protein levels of TDP-CTF, but not TDP-43^{WT}, indicating that KPNB 1 specifically targets insoluble TDP-CTF and reduces its aggregation in a tag-independent manner (Figure 26D). KPNB1 did not affect transcript levels of either TDP-CTF or TDP-43 (Figure 26E).

Mammalian cells express several members of the α- and β-karyopherin families of nuclear transport receptors that share a similar curved α-solenoid structure composed of stacks of related armadillo or HEAT repeats, respectively. To determine whether KPNB 1 was the sole transport factor able to reduce TDP-43 pathology, the effect of all 7 α-importin, 20 β-importin, and exportin protein family members on TDP-CTF aggregates in SH-SY5Y cells was tested using fluorescence microscopy (Figure 19A) and quantitative western blot analysis of insoluble proteins (Figure 19B). Based on microscopy results, transport receptors were subdivided into four categories: "no effect", "co-aggregation", "reduced aggregation" (including proteins that only reduced the size of individual TDP-CTF aggregates), and "no aggregation". Several β-importins, including IPO3/TNPO2, IPO4, IPO9, and IPO13, were able to reduce insoluble TDP-CTF levels similarly to KPNB1. TNPO1/KPNB2 did not reduce insoluble TDP-CTF protein levels but reduced the size of cytoplasmic TDP-CTF inclusions (Figures 19 A and 19B). No such effect was found for α-importins and exportins, with some exportins showing co-aggregation (Figures 19 A and 19B). These results suggest that while KPNB1 and other β-importins differ in their NLS recognition and substrate specificity for nuclear import, they share a common mechanism in reducing TDP-CTF aggregation.

### The N-terminal half of KPNB1 is required and sufficient to reduce TDP-CTF aggregation and toxicity

KPNB1, the best characterized member of the NIR family, was focused on to gain a better mechanistic understanding of how NIRs reduce TDP-CTF aggregation, and to determine the region that is required and sufficient for this activity. Since KPNB1 is comprised of 19 highly flexible arrays of short amphiphilic tandem α-helical HEAT repeats, a series of constructs based on these repeats was generated. First, the N-terminal half (HEAT repeats 1-9, H1-9) and the C-terminal half of KPNB 1 (H10-19) were tested. Much like the full-length protein, KPNB1 H1-9 showed diffuse localization with enrichment in the nuclear envelope, whereas KPNB1 H10-19 was mainly present in the nucleoplasm (Figure 20A). Expression of full-length KPNB 1 and H1-9 but not H10-19 reduced TDP-CTF aggregate formation (Figure 20A) and decreased detergent-insoluble levels of TDP-CTF (Figure 20B) without affecting endogenous nuclear TDP-43 protein levels (Figure 27). Co-expression of full-length KPNB1 or H1-9 also significantly reduced TDP-CTF-induced cell death by ~58%, whereas KPNB1 H10-19 had no effect (Figure 20C), demonstrating that the N-terminal part of KPNB 1 is required and sufficient to reduce cytoplasmic TDP-43 aggregation and toxicity.

To further delineate the active region in the KPNB1 H1-9 fragment, a series of constructs with C-terminal HEAT repeat deletions, ranging from H1-8 down to H1, was generated. Only KPNB1 H1-8 was strongly enriched on the nuclear envelope, and was identified as the smallest fragment with full activity towards reducing TDP-CTF aggregation, rendering TDP-CTF diffuse and significantly decreasing insoluble TDP-CTF protein levels by ~77% (Figures 20D and 20E). KPNB1 H1-7 and KPNB1 H1-6 were less active, only slightly reducing insoluble TDP-CTF protein levels and aggregate size, while even smaller KPNB1 fragments colocalized with TDP-CTF aggregates without exerting any significant chaperone activity to reduce aggregation and solubility (Figures 20E and 20E).

*Reduction of TDP-CTF aggregation depends on the FG-Nup interaction domain of KPNB1* binding sites for KPNB1 interactors that regulate its nuclear import activity, including Ran^{GTP}, importin-α, and FG-Nups which can bind KPNB1 at two distinct Nup-interacting sites (NIS1 and 2) were mapped (Figure 21A). Co-immunoprecipitation (co-IP) experiments with KPNB1 constructs were used to map high-affinity interactions with FG-Nups including Nup62 and Nup50, but also with importin-α1 and Ran. KPNB1 H1-8 and H1-9 interacted with high-molecular weight FG-Nups with even greater affinity than full-length KPNB 1 (Figure 21B). While structural studies have shown that importin-α contacts KPNB1 H7-19 (Cingolani et al., Nature, 399:221-229 (1999)), these results indicate that H1-8, perhaps via the acidic loop in H8, may be required and sufficient for high affinity interaction.

KPNB1 constructs with the greatest effect on TDP-CTF aggregation are also the constructs that bind to FG-Nups; this raised the question whether the proposed chaperone activity of KPNB 1 depends on its interaction with FG-Nups. Therefore missense mutations (I178A, F217A, Y255A, I263R) into NIS1 of KPNB1 H1-8 (H1-8^{mNIS}) were introduced to reduce Nup-binding (Figure 21C). While KPNB1 H1-8^{WT} expression caused diffuse nucleocytoplasmic distribution of TDP-CTF, reduced the formation of TDP-CTF cytoplasmic inclusions, and decreased insoluble TDP-CTF, KPNB1 H1-8^{mNIs} had lost most of its activity (Figures 21D and 21E).

To determine whether KPNB 1 can also reduce the aggregation of cytoplasmically mislocalized endogenous TDP-43, a C9ALS/FTD disease model based on expression of a *C9orf72* repeat expansion-derived poly(GR) di-peptide repeat protein, which has been shown to induce endogenous TDP-43 mislocalization in mice, was used. It was found that poly(GR)₁₀₀ forms both nuclear and cytoplasmic aggregates in cell culture, but only cytoplasmic aggregates were positive for endogenous TDP-43 and Nup62. Cells with nuclear GR aggregates exhibited an irregular nuclear envelope, nucleocytoplasmic distribution of TDP-43, and abnormal aggregation of Nup62 in the cytoplasm (Figures 28A and 28B). While KPNB1 H1-8^{WT} did not affect GR inclusion formation, it abolished the accumulation of TDP-43 and Nup62 in these aggregates in ~60% of cells (Figures 21F-21I). This effect was dependent on the FG-Nup interaction domain as most GR aggregates remained positive for TDP-43 and Nup62 in the presence of KPNB 1 H1-8^{mNIS} (Figures 21F-21I). Taken together, these results suggest a role for FG-Nups in facilitating KPNB 1-mediated reduction of TDP-43 aggregation.

### KPNB1 associates with TDP-CTF via both the RRM2 and PrLD

To identify the TDP-43 regions required for recruiting KPNB1, we conducted a series of interaction domain mapping experiments. Co-IP experiments in HEK293T cell lysates showed that GFP-TDP-CTF strongly interacts with untagged KPNB1 both via the remaining RRM2 domain fragment present in TDP-CTF (aa208-274), and to a lesser degree its PrLD (aa275-414) (Figure 29).

To delineate the interaction domain within the RRM2 region of TDP-CTF (non-PrLD), a series of TDP-CTF deletion constructs (Figure 30A) was tested in co-IP experiments with KPNB1 and mapped the main interaction domain to TDP-CTF amino-acids 230-240 (Figures 30V and 30C). This was confirmed by a TDP-CTF deletion construct lacking these ten amino-acids (TDP-CTF^{Δ230-240}) that interacts significantly weaker with KPNB1 in comparison to TDP-CTF^{WT} (Figure 30D). KPNB1 was still able to reduce TDP-CTF^{Δ230-240} aggregates and insoluble protein levels, similarly to TDP-CTF^{WT} (Figures 30E and 30F). This led to a conclusion that although region 230-240 in TDP-CTF can mediate interaction with KPNB1, it is not required for its TDP-CTF aggregation-reducing activity.

These findings led to an examination of whether the PrLD is required for reducing TDP-43 aggregation, by co-expressing KPNB1 with TDP-CTF, TDP-43^{mNLS} (full-length TDP-43 with mutant NLS), or sTDP (short-TDP), an alternative splicing isoform of TDP-43 where the PrLD is replaced by a nuclear export signal (NES). KPNB1 expression not only reduced TDP-CTF and TDP-43^{mNLS} aggregates, but also increased the nuclear localization of TDP-43^{mNLS} (Figure 22A). In contrast, sTDP aggregates were reduced in size but still present upon KPNB1 expression. Western blot analysis of insoluble TDP-43 protein showed that KPNB1 reduced the levels of all three TDP-43 constructs, but sTDP to a much lesser extent (Figure 22B), indicating an NLS-independent mode of interaction and an important role for the PrLD in enabling KPNB1 to reduce protein aggregation.

### Nup62 associates with the TDP-43 PrLD and recruits KPNB1 into aggregates

The TDP-PrLD fragment (aa275-414) is mostly soluble in cells but sometimes forms small cytoplasmic granules that are positive for Nup62 and KPNB 1 (Figures 31A and 31B). Since it was found that the aggregation-reducing activity of KPNB1 depends on its FG-Nup interaction domain (Figure 21), it was examined whether either FG-Nups or Ran can recruit KPNB1 to TDP-43 aggregates. Pull-down experiments in HEK293T cell lysates confirmed that TDP-PrLD interacts with FG-Nups Nup62 and Nup98, and KPNB1, but not Ran (Figure 31C), suggesting that Ran is not required for the association between TDP-CTF and KPNB 1. To investigate the role of FG-Nups in recruiting KPNB1 to TDP-43 aggregates, a battery of GFP-tagged TDP-43 constructs were tested for colocalization with mCherry-tagged Nup62, which by itself forms big cytoplasmic aggregates in cell culture and has been previously shown to colocalize with TDP-43 aggregates. It was found that TDP-CTF, TDP-PrLD, and TDP-43^{mNLS} strongly colocalize with Nup62-mCherry in HEK293T cells (Figure 22C and Figure 32). While the N-terminal half of TDP-43^{mNLS} (TDP-43^{mNLS} 1-265) and TDP-PrLD are soluble when co-expressed with mCherry, only TDP-PrLD co-aggregates with Nup62-mCherry. Strikingly, sTDP aggregates that lack the PrLD do not colocalize with Nup62 but accumulate around Nup62 inclusions (Figure 22C and Figure 32). Similar to Nup62, Nup98 also strongly colocalized with TDP-CTF, TDP-43^{mNLS} and TDP-PrLD, but not sTDP (Figure 35). sTDP also showed reduced interaction with endogenous Nup62, Nup98, and KPNB1, as compared to TDP-CTF (Figure 22D). These findings may explain why KPNB1 cannot reduce sTDP aggregation as efficiently as TDP-CTF (Figure 22A and 22B). In comparison with TDP-43^{mNLS}, sTDP lacks the PrLD but also has an intact NLS and an additional C-terminal NES that could interfere with the TDP-43-Nup62 interaction. A sTDP construct that lacks both sequences (sTDP^{mNLS} ΔNES) was co-expressed with Nup62-mCherry and it was found to still form aggregates surrounding Nup62 foci (Figure 33), confirming that it is the TDP-43 PrLD that is required for Nup62 association.

It was next tested whether expression of Nup62 can increase the association between TDP-CTF aggregates and KPNB1 in HEK293T cells. KPNB1 strongly reduces the prevalence of TDP-CTF aggregates; the rare remaining TDP-CTF aggregates weakly overlap with KPNB 1, and co-expression of Nup62 dramatically increased the recruitment of KPNB 1 into TDP-CTF aggregates (Figure 22E). Nup62 and KPNB1 did not colocalize with sTDP aggregates. While the non-FG nucleoporin Nup85 showed strong colocalization with TDP-CTF, it did not recruit KPNB 1 into aggregates (Figure 22E). These findings raised the question whether this FG-Nup-dependent recruitment of NIRs into TDP-43 aggregates may contribute to their aggregation-reducing activity.

### The association of TDP-43 PrLD with Nup62 is required for KPNB1-mediated reduction of aggregation

To test whether specific mutations in the PrLD of TDP-43 that hinder its association with Nup62 also inhibit the KPNB1-dependent reduced aggregation, an extensive series of PrLD mutant variants were generated (Figure 34). FUSΔ14 was used as a negative control, as FUS aggregates do not colocalize with Nup62 (Figure 34D). Deleting any of the three segments that make up the PrLD (Δ274-313, Δ314-353 or Δ354-393) did not prevent co-aggregation with Nup62, suggesting a multivalent interaction across the PrLD instead of one specific interaction domain (Figure 34A). Since the TDP-43 PrLD harbors six FG motifs, including four GXFG repeats (Figure 34E), it was sought to determine whether TDP-CTF and Nup62 can associate via their respective FG repeats. However, TDP-CTF mutants where the phenylalanine residues in the PrLD were replaced by alanine, glycine or tyrosine residues (F-A, F-G or F-Y) still strongly co-aggregated with Nup62 (Figure 34A). The role of charged (KRED), aliphatic (VLIM), and aromatic (FYW) amino-acid residues was also examined for PrLD association with Nup62. Only one of these mutations had a strong effect on TDP-CTF colocalization with Nup62: substituting nine aliphatic residues with phenylalanine (VLIM-F) not only rendered TDP-CTF^{VLIM-F} more aggregation-prone, but also abrogated its colocalization with Nup62 (Figures 34A and 34B) and Nup98 (Figure 35). Similarly, TDP-43^{mNLS} aggregates with VLIM-F mutations in the PrLD were proximal to Nup62 granules but did not overlap (Figure 34E). This allowed investigation of whether reduced Nup62 association would impact KPNB1-dependent reduction of aggregation. When compared with TDP-CTF^{WT}, TDP-CTF^{VLIM-F} interaction with KPNB1 was reduced in co-IP experiments (Figure 34F) and its insoluble protein levels were only slightly affected by KPNB 1 (Figures 34G and 34H). While it is possible that the recruitment of FG-Nups and KPNB1 into TDP-CTF^{VLIM-F} inclusions is prevented by their dense nature, other TDP-CTF mutations promoting its aggregation did not affect association with Nup62. Taken together, these results strongly suggest that FG-Nup interaction plays a crucial role in recruiting KPNB 1 to reduce protein aggregation.

### KPNB1 reduces TDP-CTF and Nup62 aggregates in a similar manner

One compelling model for the canonical activity of NIRs in nuclear import is based on their structural flexibility that allows them to form multivalent interactions with FG motifs. It was examined whether KPNB1 may dissolve or prevent Nup62-TDP-43 co-aggregate formation via a similar mechanism. Co-expression of Nup62 with KPNB1^{WT} leads to the formation of smaller Nup62 aggregates that strongly colocalize with KPNB 1, whereas KPNB1^{mNIS} weakly associated with Nup62 aggregates and had no effect on their size (Figure 22F). Similarly, KPNB1 H1-8^{WT} abolished Nup62 aggregates, while KPNB1 H1-8^{mNIS} did not (Figure 22F and Figure 36).

It was observed that only a subset of β-importins can reduce TDP-CTF aggregates, although importins and exportins interact with Nup62 and other FG-Nups during nuclear transport. IPO13 reduces TDP-CTF aggregation even more strongly that KPNB 1, whereas TNPO1 partially disassembles TDP-CTF into smaller aggregates, and exportins XPO1, XPO7, and RANBP17 co-aggregate with TDP-CTF (Figure 19A). These transport receptors were co-expressed with Nup62-mCherry and a very similar effect was found on Nup62 (Figure 22G). All three exportins strongly associated with Nup62 aggregates, but without affecting their morphology, whereas IPO13 caused strong dissociation and TNPO1 partially disassembled TDP-CTF into smaller aggregates (Figure 22G). These results indicate that specific transport receptors have a similar effect on the formation of both TDP-43 and Nup62 aggregates, suggesting a common mechanism.

### KPNB1 expression restores the nuclear localization of mislocalized TDP-43 independent of its NLS in primary neurons and mouse brain tissue

Following up on the unexpected finding that KPNB 1 expression increases the nuclear localization of TDP-43^{mNLS} (Figure 22A), the nucleocytoplasmic (N-to-C) ratio of TDP-43 constructs over time upon KPNB1 expression was measured in primary neuron cultures. KPNB1 further increased the N-to-C ratio of TDP-43^{WT} as soon as 24 hours after transfection, despite it being mostly nuclear (Figures 23A and 23B and Figure 37). However, KPNB1 also significantly increased the nuclear localization of cytoplasmic TDP-43^{mNLS} and TDP-CTF 48 and 72 hours post-transfection, demonstrating that KPNB1 can increase the nuclear import of TDP-43 independently of its NLS (Figures 23A and 23B and Figure 37).

To determine whether both aggregation-reducing and nuclear translocation activities reside in the same domains of KPNB1, the effect of truncated KPNB1 constructs on the localization of TDP-43^{mNLS} was tested in HEK293T cells. Full-length KPNB 1, as well as KPNB1 H1-9 and H1-8 strongly increased the levels of nuclear TDP-43^{mNLS}, whereas KPNB1 H1-7 had a weak nuclear import activity (Figures 23C and 23D). In addition, KPNB1 H1-8^{mNIS} did not change the localization of TDP-43^{mNLS} in comparison with KPNB 1 H1-8^{WT}, indicating that the nuclear translocation activity of KPNB1 also depends on the FG-Nup interaction domain.

To determine the effect of KPNB 1 on TDP-43 localization in the context of intact CNS tissue, AAV-transduction of organotypic mouse brain slice cultures (BSCs) was employed (Figure 23E). BSCs were co-transduced with AAV-GFP-TDP-43^{mNLS} and various AAV-FLAG-KPNB1 constructs, or AAV-FLAG-mCherry as a control. 12 days after transduction, TDP-43^{mNLS} was cytoplasmic without any obvious signs of aggregation (Figure 23E). Expression of KPNB1 H1-9^{WT} and H1-8^{WT} strongly increased the nuclear localization of TDP-43^{mNLS} (arrowheads), whereas KPNB1 constructs carrying NIS mutations to prevent FG-Nup-binding had no effect (Figures 23E and 23F). The effect of these constructs on AAV-mScarlet-TDP-CTF, which forms small cytoplasmic aggregates in this model, was tested (Figure 38A, arrows). TDP-CTF became very nuclear in presence of KPNB1 H1-9^{WT} and KPNB1 H1-8^{WT}. This effect was less pronounced with KPNB1 H1-9^{mNIS} and completely absent with KPNB1 H1-8^{mNIS} (Figure 38). KPNB1 H10-19 had no effect on the localization of TDP-43^{mNLS} and TDP-CTF (Figures 23E and 23F and Figure 38). Taken together, these data demonstrate that KPNB 1 reduces TDP-43 cytoplasmic aggregation and mislocalization in an FG-Nup-dependent but NLS-independent manner, both in primary neuron and BSC models of TDP-43 proteinopathy.

### Nup62 and KPNB1 are sequestered into cytoplasmic pTDP-43 aggregates in ALS/FTD postmortem tissue

To determine Nup62 and pTDP-43 are mislocalized in pTDP-43 inclusions in ALS/FTLD, Nup62 and KPNB1 co-immunostainings were performed in patient tissue with phospho-TDP-43 (pTDP-43) pathology (case demographics summarized in Table 7). pTDP-43 inclusions were positive for both Nup62 and KPNB 1 in the spinal cord of subjects diagnosed with sporadic, C9- and mutant TARDBP-ALS (Figures 24A and 24B and Figure 39). Nup62 was almost completely absent from the nuclear rim in these cells, while KPNB 1 was still partially present in the nucleus. Similar observations were made in the hippocampus of sporadic and C9-FTLD-TDP patients, although Nup62 was still weakly detected on the nuclear envelope (Figures 24A-24D). Spinal cord tissue from subjects diagnosed with SOD1-or FUS-ALS, which do not exhibit TDP-43 pathology, were devoid of cytoplasmic Nup62 and KPNB1 aggregates (Figures 24A and 24B). Neuropathologically normal control spinal cord and hippocampus showed robust localization of Nup62 on the nuclear rim of the majority of cells and nucleoplasmic localization of KPNB 1, in the absence of pTDP-43 pathology. The finding that both Nup62 and KPNB1 are recruited to TDP-43 inclusions in ALS/FTD patients strongly support the relevance of our data from disease models for human TDP-43 proteinopathies.

Together, these results demonstrate that one or more importin polypeptides and/or fragments thereof (and/or nucleic acids designed to express an importin polypeptide and/or a fragment thereof) can be administered to a mammal (e.g., a human) having, or at risk of developing, a proteinopathy (e.g., a TDP-43 proteinopathy) to treat the mammal.

### Example 9: Treating ALS

A human identified as having, or as being at risk of developing, ALS is administered one or more IPO3 polypeptides or fragments thereof. The administered IPO3 polypeptides or fragments thereof can slow, delay, or prevent progression of neurodegeneration in the brain and/or spinal cord of the human.

### Example 10: Treating ALS

A human identified as having, or as being at risk of developing, ALS is administered nucleic acid encoding an IPO3 polypeptide or a fragment thereof under conditions where the IPO3 polypeptide or the fragment thereof is expressed within the brain and/or spinal cord of the human to slow, delay, or prevent progression of neurodegeneration in the brain and/or spinal cord of the human.

### Example 11: Treating ALS

A human identified as having, or as being at risk of developing, ALS is administered one or more IPO13 polypeptides or fragments thereof. The administered IPO13 polypeptides or fragments thereof can slow, delay, or prevent progression of neurodegeneration in the brain and/or spinal cord of the human.

### Example 12: Treating ALS

A human identified as having, or as being at risk of developing, ALS is administered nucleic acid encoding an IPO13 polypeptide or a fragment thereof under conditions where the IPO13 polypeptide or the fragment thereof is expressed within the brain and/or spinal cord of the human to slow, delay, or prevent progression of neurodegeneration in the brain and/or spinal cord of the human.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### EMBODIMENTS

Although the present invention is defined in the claims, it should be understood that the present invention can also (alternatively) be defined in accordance with the following embodiments:
1. A method for treating a mammal having a TAR DNA-binding protein 43 (TDP-43) proteinopathy, wherein said method comprises administering an importin-3 (IPO3) polypeptide or a fragment of said IPO3 polypeptide to said mammal.
2. The method of embodiment 1, wherein said IPO3 polypeptide or said fragment is effective to reduce a symptom of said TDP-43 proteinopathy.
3. The method of embodiment 2, wherein said symptom is selected from the group consisting of depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, behavior variant frontotemporal dementia (bvFTD), and primary progressive aphasia (PPA).
4. A method for reducing aggregation of TDP-43 polypeptides in a central nervous system (CNS) of a mammal having a TDP-43 proteinopathy, wherein said method comprises administering an IPO3 polypeptide or a fragment of said IPO3 polypeptide to said mammal.
5. The method of any one of embodiments 1-4, wherein said method comprises administering said IPO3 polypeptide to said mammal.
6. The method of any one of embodiments 1-4, wherein said method comprises administering said fragment of said IPO3 polypeptide to said mammal.
7. The method of embodiment 6, wherein said fragment of said IPO3 polypeptide consists of the amino acid sequence set forth in any one of SEQ ID NOs:26-55.
8. A method for treating a mammal having a TDP-43 proteinopathy, wherein said method comprises administering nucleic acid encoding an IPO3 polypeptide or a fragment of said IPO3 polypeptide to said mammal, wherein said IPO3 polypeptide or said fragment is expressed by cells in a CNS of said mammal.
9. The method of embodiment 8, wherein said IPO3 polypeptide or said fragment is effective to reduce a symptom of said TDP-43 proteinopathy.
10. The method of embodiment 9, wherein said symptom is selected from the group consisting of depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, bvFTD, and PPA.
11. A method for reducing aggregation of TDP-43 polypeptides in a CNS of a mammal, wherein said method comprises administering nucleic acid encoding an IPO3 polypeptide or a fragment of said IPO3 polypeptide to said mammal wherein said IPO3 polypeptide or said fragment is expressed by cells in the CNS of said mammal.
12. The method of any one of embodiments 8-11, wherein said nucleic acid encodes said IPO3 polypeptide.
13. The method of any one of embodiments 8-11, wherein said nucleic acid encodes said fragment of said IPO3 polypeptide.
14. The method of embodiment 13, wherein said fragment of said IPO3 polypeptide consists of the amino acid sequence set forth in any one of SEQ ID NOs:26-55.
15. The method of any one of embodiments 8-14, wherein said nucleic acid is in the form of a vector.
16. The method of embodiment 15, wherein said vector is an adeno-associated virus (AAV) vector.
17. The method of embodiment 15, wherein said vector is an expression plasmid.
18. The method of any one of embodiments 8-14, wherein said nucleic acid is contained within a nanoparticle.
19. The method of any one of embodiments 1-18, wherein said mammal is a human.
20. The method of any one of embodiments 1-19, wherein said TDP-43 proteinopathy is selected from the group consisting of Alzheimer's disease, FTD, ALS, traumatic brain injury (TBI), chronic traumatic encephalopathy (CTE), limbic-predominant age-related TDP-43 encephalopathy (LATE), dementia with Lewy bodies (DLB), Parkinson's disease, Huntington's disease, argyrophilic grain disease (AGD), hippocampal sclerosis (HS), Guam ALS, Guam parkinsonism-dementia complex (G-PDC), Perry disease, facial onset sensory and motor neuronopathy (FOSMN), inclusion body myositis (IBM), hereditary inclusion body myopathy, oculopharyngeal muscular dystrophy (OPMD), and distal myopathies with rimmed vacuoles.
21. The method of any one of embodiments 1-20, wherein said administering comprises intracerebral injection.
22. The method of any one of embodiments 1-20, wherein said administering comprises intrathecal injection.
23. A method for treating a mammal having a TDP-43 proteinopathy, wherein said method comprises administering an importin-13 (IPO13) polypeptide or a fragment of said IPO13 polypeptide to said mammal.
24. The method of embodiment 23, wherein said IP013 polypeptide or said fragment is effective to reduce a symptom of said TDP-43 proteinopathy.
25. The method of embodiment 24, wherein said symptom is selected from the group consisting of depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, bvFTD, and PPA.
26. A method for reducing aggregation of TDP-43 polypeptides in a CNS of a mammal having a TDP-43 proteinopathy, wherein said method comprises administering an IPO13 polypeptide or a fragment of said IPO13 polypeptide to said mammal.
27. The method of any one of embodiments 23-26, wherein said method comprises administering said IPO13 polypeptide to said mammal.
28. The method of any one of embodiments 23-26, wherein said method comprises administering said fragment of said IP013 polypeptide to said mammal.
29. The method of embodiment 28, wherein said fragment of said IPO13 polypeptide consists of the amino acid sequence set forth in any one of SEQ ID NOs:56-77.
30. A method for treating a mammal having a TDP-43 proteinopathy, wherein said method comprises administering nucleic acid encoding an IPO13 polypeptide or a fragment of said IPO13 polypeptide to said mammal, wherein said IPO13 polypeptide or said fragment is expressed by cells in the CNS of said mammal.
31. The method of embodiment 30, wherein said IP013 polypeptide or said fragment is effective to reduce a symptom of said TDP-43 proteinopathy.
32. The method of embodiment 31, wherein said symptom is selected from the group consisting of depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, bvFTD, and PPA.
33. A method for reducing aggregation of TDP-43 polypeptides in a CNS of a mammal, wherein said method comprises administering nucleic acid encoding an IPO13 polypeptide or a fragment of said IPO13 polypeptide to said mammal, wherein said IPO13 polypeptide or said fragment is expressed by cells in the CNS of said mammal.
34. The method of any one of embodiments 30-33, wherein said nucleic acid encodes said IPO13 polypeptide.
35. The method of any one of embodiments 30-33, wherein said nucleic acid encodes said fragment of said IPO13 polypeptide.
36. The method of embodiment 35, wherein said fragment of said IP013 polypeptide consists of the amino acid sequence set forth in any one of SEQ ID NOs:56-77.
37. The method of any one of embodiments 30-36, wherein said nucleic acid is in the form of a vector.
38. The method of embodiment 37, wherein said vector is an AAV vector.
39. The method of embodiment 37, wherein said vector is an expression plasmid.
40. The method of any one of embodiments 30-36, wherein said nucleic acid is contained within a nanoparticle.
41. The method of any one of embodiments 23-40, wherein said mammal is a human.
42. The method of any one of embodiments 23-41, wherein said TDP-43 proteinopathy is selected from the group consisting of Alzheimer's disease, FTD, ALS, TBI, CTE, LATE, DLB, Parkinson's disease, Huntington's disease, AGD, HS, Guam ALS, G-PDC, Perry disease, FOSMN, IBM, hereditary inclusion body myopathy, OPMD, and distal myopathies with rimmed vacuoles.
43. The method of any one of embodiments 23-42, wherein said administering comprises intracerebral injection.
44. The method of any one of embodiments 23-42, wherein said administering comprises intrathecal injection.

## Claims

1. An importin-13 (IPO13) polypeptide for use in a method for treating a mammal having a TAR DNA-binding protein 43 (TDP-43) proteinopathy, wherein said method comprises administering said IPO13 polypeptide or a fragment of said IPO13 polypeptide to said mammal.

2. The IPO13 polypeptide for use of claim 1, wherein said IPO13 polypeptide or said fragment is effective to reduce a symptom of said TDP-43 proteinopathy.

3. The IPO13 polypeptide for use of claim 2, wherein said symptom is selected from the group consisting of depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, behavior variant frontotemporal dementia (bvFTD), and primary progressive aphasia (PPA).

4. An IPO13 polypeptide for use in a method for reducing aggregation of TDP-43 polypeptides in a central nervous system (CNS) of a mammal having a TDP-43 proteinopathy, wherein said method comprises administering said IPO13 polypeptide or a fragment of said IPO13 polypeptide to said mammal.

5. A nucleic acid encoding an IPO13 polypeptide or a fragment of said IPO13 polypeptide for use in a method for treating a mammal having a TDP-43 proteinopathy, wherein said method comprises administering said nucleic acid to said mammal, wherein said IP013 polypeptide or said fragment is expressed by cells in the CNS of said mammal.

6. The nucleic acid for use of claim 5, wherein said IPO13 polypeptide or said fragment is effective to reduce a symptom of said TDP-43 proteinopathy.

7. The nucleic acid for use of claim 6, wherein said symptom is selected from the group consisting of depression, apathy, social withdrawal, mood swings, irritability, aggressiveness, changes in sleeping habits, wandering, loss of inhibitions, delusions, deterioration in behavior and personality affecting conduct, judgment, empathy and foresight, emotional blunting, compulsive or ritualistic behavior, changes in eating habits or diet, deficits in executive function, lack of insight, agitation, emotional instability, difficulty with producing or comprehending spoken or written language, memory loss, muscle weakness, muscle atrophy, fasciculations, spasticity, dysarthria, dysphagia, bvFTD, and PPA.

8. A nucleic acid encoding an IPO13 polypeptide or a fragment of said IPO13 polypeptide for use in a method for reducing aggregation of TDP-43 polypeptides in a CNS of a mammal, wherein said method comprises administering said nucleic acid to said mammal, wherein said IP013 polypeptide or said fragment is expressed by cells in the CNS of said mammal.

9. The IPO13 polypeptide for use of any one of claims 1-4 or the nucleic acid for use of any one of claims 5-8, wherein said fragment of said IP013 polypeptide consists of the amino acid sequence set forth in any one of SEQ ID NOs:56-77.

10. The nucleic acid for use of any one of claims 5-9, wherein said nucleic acid is in the form of a vector, optionally wherein said vector is an adeno-associated virus (AAV) vector, or wherein said vector is an expression plasmid.

11. The nucleic acid for use of any one of claims 5-9, wherein said nucleic acid is contained within a nanoparticle.

12. The IPO13 polypeptide for use of any one of claims 1-4 and 9 or the nucleic acid for use of any one of claims 5-11, wherein said mammal is a human.

13. The IPO13 polypeptide for use of any one of claims 1-4, 9 and 12 or the nucleic acid for use of any one of claims 5-12, wherein said TDP-43 proteinopathy is selected from the group consisting of Alzheimer's disease, FTD, ALS, traumatic brain injury (TBI), chronic traumatic encephalopathy (CTE), limbic-predominant age-related TDP-43 encephalopathy (LATE), dementia with Lewy bodies (DLB), Parkinson's disease, Huntington's disease, argyrophilic grain disease (AGD), hippocampal sclerosis (HS), Guam ALS, Guam parkinsonism-dementia complex (G-PDC), Perry disease, facial onset sensory and motor neuronopathy (FOSMN), inclusion body myositis (IBM), hereditary inclusion body myopathy, oculopharyngeal muscular dystrophy (OPMD), and distal myopathies with rimmed vacuoles.

14. The IPO13 polypeptide for use of any one of claims 1-4, 9 and 12-13 or the nucleic acid for use of any one of claims 5-13, wherein said administering comprises intracerebral injection.

15. The IPO13 polypeptide for use of any one of claims 1-4, 9 and 12-13 or the nucleic acid for use of any one of claims 5-13, wherein said administering comprises intrathecal injection.
